# EUROPEAN PATENT SPECIFICATION

(11) **EP 1 307 455 B1**
(45) Date of publication and mention of the grant of the patent: **06.04.2005**
(21) Application number: 00976625.4
(22) Date of filing: 19.10.2000
(51) Int. Cl.: C07D 487/04

(54) **INHIBITORS OF ALPHA-L BETA-2 MEDIATED CELL ADHESION**
INHIBITOREN DER ALPHA-L BETA-2 VERMITTELTEN ZELLADHÄSION
INHIBITEURS D'ADHESION CELLULAIRE INDUITE PAR ALPHA-L BETA-2

(30) Priority: 20.10.1999 US 160629 P; 07.06.2000 US 209847 P
(43) Date of publication of application: 07.05.2003
(73) Proprietor: TANABE SEIYAKU CO., LTD., Chuo-ku, Osaka-shi, Osaka 541-8505 (JP)
(72) Inventor: SIRCAR, Ila, San Diego, CA 92130 (US); MORNINGSTAR, Marshall, 3, San Diego, CA 92129 (US); FURTH, Paul, Bonita, CA 92192 (US); SMITH, Nicholas, 1, San Diego, CA 92109 (US); TEEGARDEN, Bradley, R., San Diego, CA 92123 (US); GRIFFITH, Ronald, C., Escondido, CA 92029 (US)
(74) Representative: HOFFMANN - EITLE
(86) International application number: PCT/US2000/029273
(87) International publication number: WO 2001/030781

(56) References cited:
- WO-A-99/10312
- DYATKIN, ALEXY B.: "Ring-closing olefin metathesis for the synthesis of 1,8-diazabicyclo[4.3.0]non-3-ene-7,9-dione s" TETRAHEDRON LETT. (1997), 38(12), 2065-2066 , XP004055934
- BIANCO, ALBERTO ET AL: "Solid-Phase Synthesis and Structural Characterization of Highly Substituted Hydroxyproline-Based 2,5-Diketopiperazines" J. ORG. CHEM. (2000), 65(7), 2179-2187 , XP002171605
- KELLY, TERENCE A. ET AL: "Cutting edge: a small molecule antagonist of LFA-1-mediated cell adhesion" J. IMMUNOL. (1999), 163(10), 5173-5177 , XP002171606

## Description

### BACKGROUND OF THE INVENTION

### Field of the Invention

The present invention relates to small molecules that are potent inhibitors of α_{L}β₂ mediated cell adhesion which could be useful for the treatment of inflammatory diseases.

### Description of Related Art

The integrin family of proteins are heterodimeric receptors which are expressed on all cell types to mediate cell to cell binding and adhesion to extracellular matrix. The β₂ (CD18) integrin subfamily is comprised of 3 members, α_{L}β₂ integrin (LFA-1, CD11a/CD18), α_{M}β₂ integrin (Mac-1, CD11b/CD18), and gp 150 β₂ integrin (α_{X}β₂ integrin, CD11c/CD18) that are primarily expressed on leukocytes (Sanchez-Madrid et al., J. Exp. Med., 158, 1785-1803 (1983)). α_{L}β₂ integrin is found mostly on T and B lymphocytes, while α_{M}β₂ integrin is present on activated neutrophils, NK cells and some myeloid cells. The α_{L}β₂ integrin binds intracellular adhesion molecules ICAM-1, 2 and 3 found on multiple cell types such as vascular endothelial cells, dendritic cells, epithelial cells, macrophage and T lymphoblasts (Dustin et al., J. Immunology, 137, 245-254 (1986)). Recently there has been evidence presented that α_{L}β₂ integrin binds to ICAM-4 and a novel ligand expressed in brain telencephalin. It has been shown that the I domain of the alpha chain is the major recognition site for its ligands.

α_{L}β₂ integrin adhesion to ICAM-1 is necessary for immune responsiveness of T-lymphocytes to antigens, lymphocyte homing and circulation, and cell emigration to sites of inflammation (Springer, Ann. Rev. Physiol., 57, 827 (1995)). A dominant role of α_{L}β₂ integrin in mediating inflammatory events is shown in several different animal models of inflammatory diseases in which antibodies to α_{L}β₂ integrin or ICAM-1 significantly inhibit development of therapeutic end points (Rothlein et al., Kidney International, 41, 617 (1992); Iigo et al., J. Immunology, 147, 4167 (1991); Bennet et al., J. Pharmacol. and Exp. Therapeutics, 280, 988 (1997)).

Also, β₂ integrin subfamily are thought to play a critical role in several types of inflammatory disease processes by interacting with ICAMs. Support for the importance of β₂ integrin in mediating inflammatory responses has been demonstrated by the evidence that transendothelial migration *in vitro* is markedly inhibited by monoclonal antibodies against β₂ integrin or ICAM-1 (Smith, Can. J. Physiol. Pharmacol., 71, 76 (1993). Furthermore, blockade of α_{L}β₂ integrin has been shown to inhibit neutrophil influx in almost every system, including skin, peritoneum, synovium, lung, kidney, and heart. As one of the primary ligands for the β₂ integrin, it would also be expected that blockade of ICAM-1 would inhibit the inflammatory response (Albelda et al., The FASEB Journal, 8, 504 (1994)).

Moreover, it has been shown that antibodies against α_{L}β₂ integrin suppress rejection after transplantation. WO 94/04188 discloses the use of monoclonal antibodies directed against α_{L}β₂ integrin for all transplantation's, including graft vs. host or host vs. graft diseases.

### SUMMARY OF THE INVENTION

The present invention relates to a compound of the formula (I): 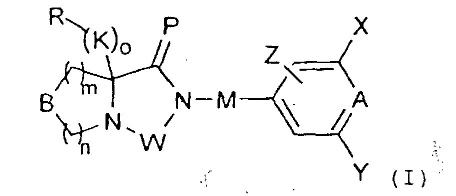 wherein
A is =C(Z¹)-, or =N-;
B is -C(R¹) (R²)-, -CH=CH-, -S-, -SO-, -SO₂-, -O-, -N(R³)-, -N(COR⁴¹)-, -N(CSR⁴¹)-, -N(SO₂R⁵)-, -N(R³)CO-, -N(COR⁴¹)CO-, -N(CSR⁴¹)CO-, or -N(SO₂R⁵)CO-;
K is -CH₂-, -CH(OH)-, -C(=O)-, or -CF₂-;
M is a single bond, -(CH₂)ₚ-, -C(=O)- or -NH-;
W is one of the following groups: 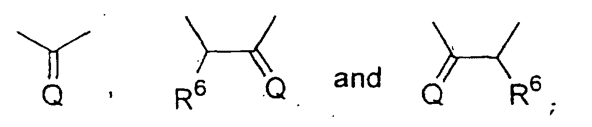 X and Y are independently
1) H,
2) halogen
3) NO₂
4) CN,
5) C₁₋₆ alkylthio,
6) NR³R⁶
7) C₁₋₆ alkyl optionally substituted with halogen,
8) C₁₋₆ alkoxy,
9) COR⁴²,
10) phenyl optionally substituted with:
   a) C₁₋₆ alkyl optionally substituted with halogen,
   b) C₁₋₆ alkoxy optionally substituted with halogen, or
   c) CN,
11) isoxazolyl optionally substituted with C₁₋₆ alkyl,
12) pyrrolyl optionally substituted with C₁₋₆ alkoxycarbonyl or formyl, or
13) pyridyl,
Z and Z¹ are independently
1) H,
2) OH,
3) halogen,
4) NO₂,
5) CF₃,
6) NR³R⁶,
7) NHCOR⁴¹,
8) C₁₋₆ alkoxy optionally substituted with:
   a) carboxyl,
   b) C₁₋₆ alkoxycarbonyl, or
   c) phenyl,
   or
9) COR⁴²;
P and Q are independently O or S;
R is phenyl, naphthyl, pyridyl, benzofuryl or thiazolyl, and said phenyl, naphthyl, pyridyl, benzofuryl and thiazolyl may be substituted with a group selected from:
1) halogen,
2) OH,
3) CN,
4) C₁₋₆ alkyl optionally substituted with a group selected from:
   a) halogen,
   b) OR⁶, or
   c) COR⁴¹,
5) C₁₋₆ alkoxy optionally substituted with a group selected from:
   a) halogen,
   b)NR³R⁶,
   c) pyridyl, and
   d) piperidinyl,
6) NO₂,
7) NR³R⁶,
8) NHCOR⁴¹,
9) NHSO₂R⁵,
10) COR⁴²,
11) C(=NH)NH₂,
12) CONHOH,
13) C₁₋₆ alkylthio which may be substituted with halogen,
14) C₁₋₆ alkylsulfinyl which may be substituted with halogen,
15) C₁₋₆ alkylsulfonyl which may be substituted with halogen,
16) C₁₋₃ alkylenedioxy optionally substituted with:
   a) C₁₋₆ alkyl, or
   b) halogen,
17) -C(=O)-(natural α-amino acid residue)
   wherein said natural α-amino acid residue may be esterified with C₁₋₆ alkyl group,
18) phenyl optionally substituted with:
   a) C₁₋₆ alkoxy,
   b) C₁₋₆ alkyl optionally substituted with OR⁶, N(C₁₋₆ alkyl)₂ or COR⁴²,
   c) CN,
   d) COR⁴²,
   e) C₂₋₇ alkenyl optionally substituted with COOR⁵,
   f) NR⁶R⁶,
   g) NO₂,
   h) NHCOR⁴¹,
   i) NHSO₂R⁵,
   j) N(SO₂R⁵)₂,
   k) NHCONHR⁵,
   l) N(CONHR⁵)₂,
   m) NHCSNHR⁵, or
   n) pyrrolidinyl which may be substituted with C₁₋₆ alkyl,
19) furyl optionally substituted with CHO,
20) thienyl optionally substituted with CHO,
21) pyrrolyl optonally substituted with CHO and C₁₋₆ alkoxycarbonyl,
22) dihydroxazolyl optionally substituted with C₁₋₆ alkyl,
23) isoxazolyl optionally substituted with C₁₋₆ alkyl,
24) benzothienyl,
25) pyridyl,
26) tetrazolyl, and
27) thiazolyl which may be substituted with C₁₋₆ alkyl;
R¹ and R² are independently
1) H,
2) halogen,
3) OR³,
4) OCOR⁵,
5) SO₂R⁵,
6) NR³R⁶,
7) NR⁶COR⁴¹,
8) NR⁶CSR⁴¹,
9) NR⁶SO₂R⁵,
10) OCONR³R³,
11) N₃,
12) CN,
13) COR⁴², or
14) phenyl
or R¹ and R² combine with each other at the terminal thereof to form:
1) oxo,
2) methylene substituted with carboxyl, C₂₋₇ alkoxycarbonyl, or CONR^{a}R^{b}, or
3) C₂₋₃ alkylenedioxy;
R^{a} and R^{b} combine with each other at the terminals thereof to form a 3-7 membered ring together with the nitrogen atom to which they are attached, where said 3-7 membered ring may include additional heteroatoms such as oxygen, nitrogen and sulfur and may be substituted with C₁₋₆ alkyl, oxo, hydroxy, C₁₋₆ alkoxy or NR⁶R⁶;
R³ is
1) H,
2) C₁₋₆ alkyl which may be substituted with:
   a) OH,
   b) phenyl optionally substituted with halogen or C₁₋₆ alkoxy,
   c) carboxyl,
   d) carbamoyl,
   e) NR⁶R⁶,,
   f) C₁₋₆ alkoxycarbonyl,
   g) morpholinyl,
   h) pyridyl,
   i) thienyl, or
   j) pyrrolidinyl optionally substituted with oxo,
3) C₃₋₆ cycloalkyl,
4) phenyl which may be substituted with halogen,
5) pyridyl, which may be substituted with C₁₋₆ alkyl, or
6) morpholinyl;
R⁴¹ is
l) C₁₋₆ alkyl which may be substituted with a group selected from:
   a) NR⁶R⁶,
   b) carboxyl,
   c) -CONR^{c}R^{d} where R^{c} and R^{d} are independently selected from hydrogen and C₁₋₆ alkyl, or R^{c} and R^{d} combine with each other at the terminals thereof to form a 3-7 membered ring together with the nitrogen atom to which they are attached, and said 1-3 heteroatoms such as oxygen, nitrogen and sulfur and may be substituted with C₁₋₆ alkyl, oxo, hydroxy, C₁₋₆ alkoxy or NR⁶R⁶,
   d) C₁₋₆ alkoxycarbonylamino,
   e) C₁₋₆ alkylsulfonylamino,
   f) C₂₋₇ alkanoylamino, and
   g) pyridyl,
2) C₁₋₆ alkoxy which may be substituted with NR⁶R⁶ or phenyl,
3) phenyl which may be substituted with:
   a) carboxyl,
   b) C₁₋₆ alkoxycarbonyl, or
   c) NR⁶R⁶,
4) isoxazolyl which may be substituted with C₁₋₆ alkyl,
5) pyridyl,
6) thienyl,
7) furyl,
8) NR^{a}R^{b},
9) C₃₋₆ cycloalkyl, or
10) NR³R⁶;
R⁴² is
1) H,
2) OH,
3) C₁₋₆ alkyl which may be substituted with NR⁶R⁶ or phenyl,
4) C₁₋₆ alkoxy which may be substituted with NR⁶R⁶,
5) phenyl,
6) NR³R⁶, or
7) NR^{a}R^{b};
R⁵ is
1) C₁₋₆ alkyl which may be substituted with COR⁴², or
2) phenyl or naphthyl;
R⁶ is
1) H, or
2) C₁₋₆ alkyl which may be substituted with -N(C₁₋₆ alkyl)₂;
m is 0, 1, 2 or 3; n is 0, 1 or 2; o is 1 or 2; p is 1 or 2;
or a pharmaceutically acceptable salt thereof.

### Detailed Description of the Invention

The desired compound of the present invention may exist in the form of optical isomers based on asymmetric atoms thereof, and the present invention also includes these optical isomers and mixtures thereof.

In an embodiment of the present invention, the steric configuration of a bond need not be fixed. The compound of the present invention may be a compound with a sole configuration or a mixture with several different configurations.

Examples of said -NR^{a}R^{b} group may be morpholinyl, pyrrolidinyl, piperazinyl, and piperidyl.

In a more preferred embodiment of the present invention, X and Y are independently selected from:
1) halogen,
2) NO₂,
3) C₁₋₆ alkyl optionally substituted with halogen,
4) C₁₋₆ alkoxy group,
5) C₁₋₇ alkanoyl group,
6) CN,
7) carboxyl,
8) C₁₋₆ alkylthio,
9) NR³R⁶,
10) phenyl optionally substituted with a) C₁₋₆ alkyl optionally substituted with halogen, b) C₁₋₆ alkoxy optionally substituted with halogen, or c) CN,
11) isoxazolyl optionally substituted with C₁₋₆ alkyl,
12) pyrrolyl optionally substituted with C₁₋₆ alkoxycarbonyl or formyl,
13) pyridyl;
R is phenyl, naphthyl, pyridyl, benzofuryl or thiazolyl, and said phenyl, naphthyl, pyridyl, benzofuryl and thiazolyl may be substituted with a group selected from:
1) halogen,
2) OH,
3) CN,
4) C₁₋₆ alkyl optionally substituted with a group selected from a) halogen, b) OR⁶, or c) COR⁴¹,
5) C₁₋₆ alkoxy optionally substituted with a group selected from a) halogen, b) NR³R⁶,c) pyridyl, or d) piperidinyl,
6) NO₂,
7) NR³R⁶,
8) NHCOR⁴¹,
9) NHSO₂R⁵,
10) COR⁴²,
11) C(=NH)NH₂,
12) CONHOH,
13) C₁₋₆ alkylthio which may be substituted with halogen,
14) C₁₋₆ alkylsulfinyl which may be substituted with halogen,
15) C₁₋₆ alkylsulfonyl which may be substituted with halogen,
16) C₁₋₃ alkylenedioxy optionally substituted with a) C₁₋₆ alkyl, or b) halogen,
17) -C(=O)-(natural α-amino acid residue), wherein said natural α-amino acid is selected from aspartic acid, alanine, phenylalanine, and asparagine, and said natural α-amino acid residue may be esterified with C₁₋₆ alkyl group,
18) phenyl optionally substituted with a) C₁₋₆ alkoxy, b) C₁₋₆ alkyl optionally substituted with OR⁶, N(C₁₋₆ alkyl)₂, or COR⁴², c) CN, d) COR⁴², e) C₂₋₇ alkenyl optionally substituted with COOR⁵, f) NR⁶R⁶, g) NO₂, h) NHCOR⁴¹, i) NHSO₂R⁵, j) N(SO₂R⁵)₂, k) NHCONHR⁵, l) N(CONHR⁵)₂, m) NHCSNHR⁵, or n) pyrrolidinyl which may be substituted with C₁₋₆ alkyl,
19) furyl optionally substituted with CHO,
20) thienyl optionally substituted with CHO,
21) pyrrolyl optionally substituted with CHO and C₁₋₆ alkoxycarbonyl,
22) dihydroxazolyl optionally substituted with C₁₋₆ alkyl,
23) isoxazolyl optionally substituted with C₁₋₆ alkyl,
24) benzothienyl,
25) pyridyl,
26) tetrazolyl, and
27) thiazolyl which may be substituted with C₁₋₆ alkyl; R¹ and R² are independently selected from H, halogen, OR³, OCOR⁵, NR³R⁶, NR⁶COR⁴¹, NHCSR⁴¹, NHSO₂R⁵, N₃, COR⁴², or phenyl; or R¹ and R² combine with each other at the terminal thereof to form oxo;
R³ is
1) hydrogen,
2) C₁₋₆ alkyl optionally substituted with a) OH, b) phenyl optionally substituted with halogen or C₁₋₆ alkoxy, c) carboxyl, d) carbamoyl, e) NR⁶R⁶, f) C₁₋₆ alkoxycarbonyl, g) morpholinyl, h) pyridyl, i) thienyl, or j) pyrrolidinyl optionally substituted with oxo;
3) C₃₋₆ cycloalkyl,
4) phenyl optionally substituted with halogen,
5) pyridyl optionally substituted with C₁₋₆ alkyl, or
6) morpholinyl;
R⁴¹ is
1) C₁₋₆ alkyl optionally substituted with a group selected from a) NR⁶R⁶, b) carboxyl, c) -CONR^{c}R^{d} where R^{c} and R^{d} are independently selected from hydrogen and C₁₋₆ alkyl, or R^{c} and R^{d} combine with each other at the terminals thereof to form a 3-7 membered ring together with the nitrogen to which they are attached, and said 3-7 membered ring may include additional 1-3 heteroatoms, such as oxygen, nitrogen and sulfur and may be substituted with C₁₋₆ alkyl, oxo, hydroxy, C₁₋₆ alkoxy or NR⁶R⁶, d) C₁₋₆ alkoxycarbonylamino, e) C₁₋₆ alkylsulfonylamino, f) C₂₋₇ alkanoylamino, and g) pyridyl;
2) C₁₋₆ alkoxy optionally substituted with NR⁶R⁶ or phenyl;
3) NR³R⁶;
4) phenyl optionally substituted with a) carboxyl, b) C₁₋₆ alkoxycarbonyl, or c) NR⁶R⁶,
5) C₃₋₆ cycloalkyl,
6) isoxazolyl optionally substituted with C₁₋₆ alkyl,
7) pyridyl,
8) thienyl,
9) furyl, or
10) NR^{a}R^{b} (for example, pyrrolidinyl which may be substituted with a hydroxyl, and morpholinyl);
R⁴² is
1) H,
2) OH;
3) C₁₋₆ alkyl optionally substituted with NR⁶R⁶ or phenyl,
4) C₁₋₆ alkoxy optionally substituted with NR⁶R⁶,
5) NR³R⁶,
6) NR^{a}R^{b} (for example, pyrrolidinyl which may be substituted with a hydroxyl, and morpholinyl), or
7) pyridyl which may be substituted with C₁₋₆ alkyl; R⁵ is C₁₋₆ alkyl optionally substituted with COR⁴², or phenyl or naphthyl; R⁶ is hydrogen or C₁₋₆ alkyl optionally substituted with -N(C₁₋₆ alkyl)₂.

Among the desired compounds of the present invention, a more preferred compound is represented by the following formula (I-A):

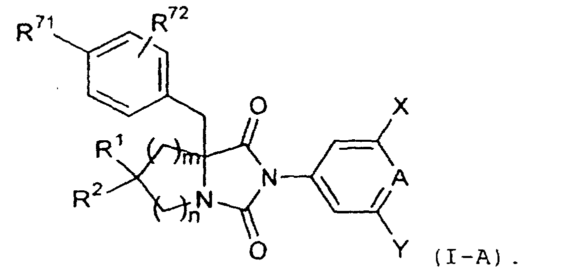

In the above formula (I-A), A is =C(Z¹)-, or =N-, and X and Y are independently H, halogen, NO₂, CN, C₁₋₆ alkylthio, NR³R⁶, or C₁₋₆ alkyl optionally substituted with halogen. Z¹ is selected from 1) H, 2) OH, 3) halogen, 4) NR³R⁶, 5) NHCOR⁴¹, 6) C₁₋₆ alkoxy optionally substituted with a) carboxyl, b) C₁₋₆ alkoxycarbonyl, or c) phenyl, and 7) COR⁴². R¹ and R² are independently H, halogen, OR³, NR³R⁶, NHCOR⁴¹, NHCSR⁴¹, NHSO₂R⁵, N₃, COR⁴², or phenyl, or R¹ and R² combine with each other at the terminal thereof to form oxo. R⁷¹ is a group selected from: 1) H, 2) OH, 3) halogen, 4) CN, 5) C₁₋₆ alkyl optionally substituted with halogen or OR⁶, 6) C₁₋₆ alkoxy optionally substituted with halogen, NR³R⁶, pyridyl, or piperidinyl, 7) NO₂, 8) NR³R⁶, 9) NHCOR⁴¹, 10) NHSO₂R⁵, 11) COR⁴², 12) C(=NH)NH₂, 13) CONHOH, 14) C₁₋₆ alkylthio, 15) C₁₋₆ alkylsulfinyl, 16) C₁₋₆ alkylsulfonyl, 17) phenyl which may be substituted with a group selected from a) C₂₋₇ alkenyl substituted with COOR⁵, b) COR⁴², c) C₁₋₆ alkyl optionally substituted with OR⁶, d) C₁₋₆ alkoxy and e) CN, 18) thienyl which may be substituted with CHO, 19) furyl which may be substituted with CHO, 20) tetrazolyl, 21) dihydroxazolyl, 22) pyrrolyl which may be substituted with CHO, 23) isoxazolyl substituted C₁₋₆ alkyl, or 24) benzothienyl. R⁷² is a group selected from 1) hydrogen, 2) halogen, 3) CN, 4) C₁₋₆ alkyl optionally substituted with OR⁶, or 5) NO₂, or, R⁷¹ and R⁷² combine with each other at the terminal thereof to form C₁₋₃ alkylenedioxy optionally substituted with halogen. m is 0, 1, or 2, and n is 0 or 1.

In a preferred compound among the compound (I-A), A is =CH- or =N-, X and Y are independently halogen, NO₂, NR³R⁶, or C₁₋₆ alkyl optionally substituted with halogen. One of R¹ and R² is H, and the other is H, OH, halogen, NR³R⁶, NHCOR⁴¹, NHCSR⁴¹, NHSO₂R⁵, N₃, COR⁴², or phenyl, or R¹ and R² combine with each other at the terminal thereof to form oxo. R⁷¹ is a group selected from H, halogen, CN, C₁₋₆ alkyl optionally substituted with halogen, C₁₋₆ alkoxy optionally substituted with halogen, COR⁴², C₁₋₆ alkylthio, phenyl which may be substituted with a group selected from a) C₂₋₇ alkenyl substituted with COOR⁵, b) COR⁴², c) C₁₋₆ alkyl optionally substituted with OR⁶, d) C₁₋₆ alkoxy, and e) CN, thienyl which may be substituted with CHO, furyl which may be substituted with CHO, pyrrolyl which may be substituted with CHO, isoxazolyl substituted with C₁₋₆ alkyl. R⁷² is hydrogen, or, R⁷¹ and R⁷² combine with each other at the terminal thereof to form C₁₋₃ alkylenedioxy substituted with halogen. m is 1, and n is 1.

In a more preferred compound among the compound (I-A), X and Y are independently halogen, and one of R¹ and R² is H, and the other is H, OH, NR³R⁶, NHCOR⁴¹, NHCSR⁴¹, or COR⁴². R⁴¹ is 1) C₁₋₆ alkyl optionally substituted with a group selected from: a) NR⁶R⁶, b) carboxyl, c) carbamoyl, d) piperazinylcarbonyl optionally substituted with C₁₋₆ alkyl, e) C₂₋₇ alkanoylamino, and f) pyridyl; 2) C₁₋₆ alkoxy; 3) NR³R⁶; 4) C₃₋₆ cycloalkyl; 5) pyridyl; 6) thienyl; 7) furyl or 8) pyrrolidinyl. R⁴² is NR³R⁶ or morpholinyl. R⁷¹ is 1) halogen, 2) CN, or 3) C₁₋₆ alkoxy optionally substituted with halogen, and R⁷² is hydrogen.

In a further more preferred compound among the compound (I-A), X and Y are independently halogen, particularly chroline atom, and one of R¹ and R² is H, and the other is OH, NHCOR⁴¹ or COR⁴². R⁴¹ is C₁₋₆ alkyl optionally substituted with a group selected from carboxyl, carbamoyl, and piperazinylcarbonyl substituted with C₁₋₆ alkyl; NH₂; NH(C₁₋₆ alkyl); pyridyl; or pyrrolidinyl. R⁴² is NH₂, NH(C₁₋₆ alkyl) or morpholinyl. R⁷¹ is C₁₋₆ alkoxy substituted with halogen, and R⁷² is hydrogen.

Among the desired compounds of the present invention, another more preferred compound is represented by the following formula (I-B):

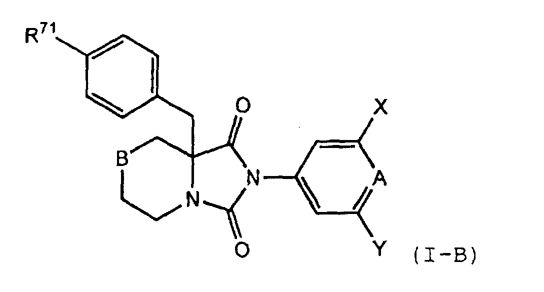

In the above formula (I-B), A is =CH- or =N-, B is -S-, -SO-, -SO₂-, -N(R³)-, -N(COR⁴¹)-, -N(CSR⁴¹)-, or -N(SO₂R⁵)-. X and Y are independently H, halogen, NO₂, or C₁₋₆ alkyl. R⁷¹ is a group selected from 1) H, 2) halogen, 3) CN, 4) C₁₋₆ alkyl optionally substituted with halogen, 5) C₁₋₆ alkoxy optionally substituted with halogen, 6) COR⁴², 7) C₁₋₆ alkylthio, 8) phenyl, 9) thienyl, 10) furyl, 11) pyrrolyl, 12) isoxazolyl substituted with C₁₋₆ alkyl, wherein said phenyl may be substituted with a group selected from a) C₂₋₇ alkenyl substituted with COOR⁵, b) COR⁴², c) C₁₋₆ alkyl optionally substituted with OR⁶, d) C₁₋₆ alkoxy, and e) CN, and said thienyl, furyl and pyrrolyl may be substituted with CHO.

In a more preferred compound among the compound (I-B), B is -N(COR⁴¹)-, and X and Y are independently halogen. R⁴¹ is 1) C₁₋₆ alkyl optionally substituted with a group selected from: a) NR⁶R⁶,b) carbamoyl, and d) piperazinylcarbonyl optionally substituted with C₁₋₆ alkyl; 2) C₁₋₆ alkoxy; or 3) NR³R⁶; and R⁷¹ is 1) halogen, or 2) C₁₋₆ alkoxy optionally substituted with halogen.

Among the desired compounds of the present invention, another more preferred compound is represented by the following formula (I-C):

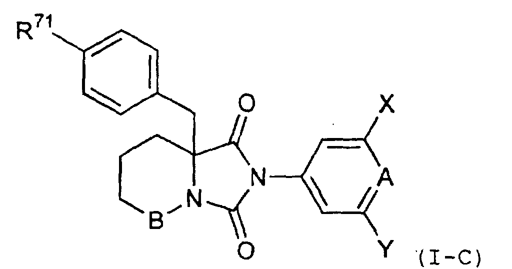

In the above formula (I-C), A is =CH- or =N-, B is -N(R³)-, -N(COR⁴¹)-, -N(CSR⁴¹)-, or -N(SO₂R⁵)-. X and Y are independently H, halogen, NO₂, or C₁₋₆ alkyl. R⁷¹ is a group selected from 1) H, 2) halogen, 3) CN, 4) C₁₋₆ alkyl optionally substituted with halogen, 5) C₁₋₆ alkoxy optionally substituted with halogen, 6) COR⁴², 7) C₁₋₆ alkylthio, 8) phenyl, 9) thienyl, 10) furyl, 11) pyrrolyl, 12) isoxazolyl substituted with C₁₋₆ alkyl, wherein said phenyl may be substituted with a group selected from a) C₂₋₇ alkenyl substituted with COOR⁵, b) COR⁴², c) C₁₋₆ alkyl optionally substituted with OR⁶, d) C₁₋₆ alkoxy, and e) CN, and said thienyl, furyl and pyrrolyl may be substituted with CHO.

Among the desired compounds of the present invention, another more preferred compound is represented by the following formula (I-D):

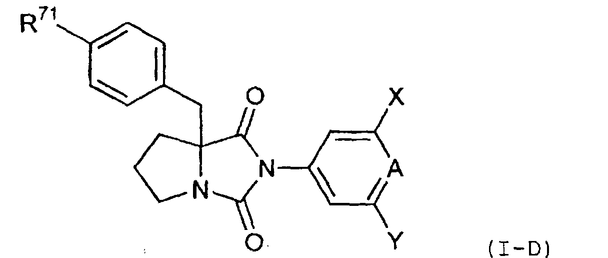

In the above formula (I-D), A is =CH-, or =N-. X is H or halogen, Y is 1) pyrrolyl optionally substituted with formyl, 2) phenyl optionally substituted with a) CN, b) C₁₋₆ alkyl optionally substituted with halogen, c) C₁₋₆ alkoxy optionally substituted with halogen, or 3) isoxazolyl optionally substituted with C₁₋₆ alkyl. R⁷¹ is a group selected from 1) H, 2) halogen, 3) CN, 4) C₁₋₆ alkyl optionally substituted with halogen, 5) C₁₋₆ alkoxy optionally substituted with halogen, 6) COR⁴², 7) C₁₋₆ alkylthio, 8) phenyl, 9) thienyl, 10) furyl, 11) pyrrolyl, 12) isoxazolyl substituted with C₁₋₆ alkyl, wherein said phenyl may be substituted with a group selected from a) C₂₋₇ alkenyl substituted with COOR⁵, b) COR⁴², c) C₁₋₆ alkyl optionally substituted with OR⁶, d) C₁₋₆ alkoxy, and e) CN, and said thienyl, furyl and pyrrolyl may be substituted with CHO.

Preferred compounds of the present invetnion may be selected from the group consisting of:
(5R, 7R)-5-[4-(trifluoromethoxy)benzyl]-3-(3,5-dichlorophenyl)-7-hydroxy-1,3-diazabicyclo[3.3.0]octane-2,4-dione;
(5R, 7S)-5-[4-(trifluoromethoxy)benzyl]-3-(3,5-dichlorophenyl)-7-acetylamino-1,3-diazabicyclo[3.3.0]octane-2,4-dione;
(5R, 7S)-5-[4-(trifluoromethoxy)benzyl]-3-(3,5-dichlorophenyl)-7-(3-carbamoylpropionylamino)-1,3-diazabicyclo[3.3.0]octane-2,4-dione;
(5R, 7S)-5-[4-(trifluoromethoxy)benzyl]-3-(3,5-dichlorophenyl)-7-[3-(4-methyl-1-piperazinylcarbonyl)propionylamino]-1,3-diazabicyclo[3.3.0]octane-2,4-dione;
(5R, 7S)-5-[4-(trifluoromethoxy)benzyl]-3-(3,5-dichlorophenyl)-7-nicotinoylamino-1,3-diazabicyclo[3.3.0]octane-2,4-dione;
(5R, 7S)-5-[4-(trifluoromethoxy)benzyl]-3-(3,5-dichlorophenyl)-7-(1-pyrrolidinylcarbonylamino)-1,3-diazabicyclo[3.3.0]octane-2,4-dione;
(5R, 7R)-5-[4-(trifluoromethoxy)benzyl]-3-(3,5-dichlorophenyl)-7-carbamoyl-1,3-diazabicyclo[3.3.0]octane-2,4-dione;
(5R, 7R)-5-[4-(trifluoromethoxy)benzyl]-3-(3,5-dichlorophenyl)-7-morphlinocarbonyl-1,3-diazabicyclo[3.3.0]octane-2,4-dione;
(5R, 7R)-5-[4-(trifluoromethoxy)benzyl]-3-(3,5-dichlorophenyl)-7-dimethylcarbamoyl-1,3-diazabicyclo[3.3.0]octane-2,4-dione; (5R, 7R)-5-[4-(trifluoromethoxy)benzyl]-3-(3,5-dichlorophenyl)-7-methylcarbamoyl-1,3-diazabicyclo[3.3.0]octane-2,4-dione; and (5R, 7R)-5-[4-(trifluoromethoxy)benzyl]-3-(3,5-dichlorophenyl)-7-morpholinocarbamoyl-1,3-diazabicyclo[3.3.0]octane-2,4-dione.

The desired compound of the present invention may be clinically used either in a free form or in the form of pharmaceutically acceptable salts thereof. Pharmaceutically acceptable salts include an acid-addition salt with an inorganic acid or an organic acid (e.g., hydrochloride, sulfate, nitrate, hydrobromide, methanesulfonate, p-toluenesulfonate, acetate), and a salt with an inorganic base, an organic base or an amino acid (e.g., triethylamine salt, a salt with lysine, an alkali metal salt, an alkali earth metal salt and the like). Pharmaceutically acceptable salts also include an intramolecular salt thereof, or a solvate or hydrate thereof.

The compound of the present invention may be formulated into a pharmaceutical composition comprising a therapeutically effective amount of the compound as defined above and a pharmaceutically acceptable carrier or diluent. The pharmaceutically acceptable carrier or diluent may be, for example, binders (e.g., syrup, gum arabic, gelatin, sorbitol, tragacanth, polyvinylpyrrolidone), excipients (e.g., lactose, sucrose, corn starch, potassium phosphate, sorbitol, glycine), lubricants (e.g., magnesium stearate, talc, polyethylene glycol, silica) disintegrators (e.g., potato starch), wetting agents (e.g., sodium laurylsulfate), and the like.

The desired compound of the present invention or pharmaceutically acceptable salts thereof may be administered either orally or parenterally, and it may be used as a suitable pharmaceutical preparation. These pharmaceutical preparations may be in the form of a solid preparation such as a tablet, a granule, a capsule, and a powder, or in the form of a liquid preparation such as solution, suspension, and emulsion, when administered orally. When administered parenterally, the pharmaceutical preparation may be in the form of suppository, an injection preparation or an intravenous drip preparation using distilled water for injection, a physiological salt solution, an aqueous glucose solution, and so on, and an inhalation by a conventional process.

The dose of the desired compound of the present invention or a pharmaceutically acceptable salt thereof varies depending on an administration method, age, sex, body weight, and condition of a patient, but, in general, the daily dose is preferably about 0.1 to 100 mg/kg/day, particularly preferably 1 to 100 mg/kg/day.

The compound of the present invention has an excellent activity in inhibiting α_{L}β₂ mediated cell adhesion, and can be used for treating or preventing α_{L}β₂ adhesion mediated conditions in a mammal such as a human.

The compound of the present invention may be used for treatment or prevention of numerous inflammatory diseases such as rheumatoid arthritis, asthma, allergy conditions, adult respiratory distress syndrome, AIDS, cardiovascular diseases, thrombosis or harmful platelet aggregation, reocclusion following thrombolysis, allograft rejection, reperfusion injury, stroke, psoriasis, eczema, skin inflammatory diseases such as contact dermatitis and atopic dermatitis, osteoporosis, osteoarthritis, atherosclerosis (including graft arteriosclerosis after transplantation), neoplastic diseases including metastasis of neoplastic or cancerous growth, wound healing enhancement, eye diseases such as detaching retina, Type I diabetes, multiple sclerosis, systemic lupus erythematosus (SLE), inflammatory and immunoinflammatory conditions including ophthalmic inflammatory conditions and inflammatory bowel diseases (Crohn's disease, ulcerative colitis), regional enteritis, Sjogren's Syndrome, and other autoimmune diseases, pancreatitis, delayed graft function, intimal hyperplasia; myocardial reinfarction or restenosis after surgery such as percutaneous transluminal coronary angioplasty (PTCA).

The compound of the present invention may also be used for cancer, such as radiation induced pneumonitis.

The compound of the present invention may also be used for transplantation, including the rejection (i.e., chronic rejection and acute rejection) after transplantation, and host vs. graft or graft vs. host diseases.

The compound of the present invention may be preferably used for treatment or prevention of psoriasis, rheumatoid arthritis, inflammatory bowel diseases (Crohn's disease, ulcerative colitis), systemic lupus erythematosus, atopic dermatitis, Sjogren's Syndrome, rejection after transplantation, and graft vs. host disease.

According to the present invention, the desired compound (I) can be prepared by the following methods:

### Method A:

The desired compound (I) or a pharmaceutically acceptable salt thereof may be prepared by:
(1) cyclizing the compound of the formula (II): 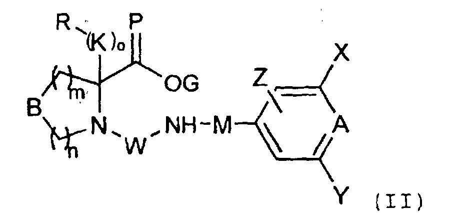 wherein OG is a hydroxyl group, a protected hydroxyl group, or a resin-bound hydroxyl group, and the other symbols are the same as defined above, and
(2) converting the resulting cyclized compound into a pharmaceutically acceptable salt thereof by a conventional method, if desired.

When OG is a protected hydroxyl group, the protecting group can be selected from the conventional protecting groups for a carboxyl group (i.e., C₁₋₆ alkyl group, benzyl group).

When OG is a resin-combined hydroxyl group, the resin may be selected from resin polymers which are conventionally used for a solid phase peptide synthesis. Merrifield resin may be given as an example of such resin polymers.

The cyclization can be carried out in the presence of an acid or a base in a suitable solvent.

The acid can be selected from organic acids (i.e., p-toluenesulfonic acid, and trifluoroacetic acid) and inorganic acids (i.e., hydrochloric acid, sulfuric acid, and nitric acid).

The base can be selected from conventional bases such as alkali metal alkoxide (e.g., NaOEt, NaOMe).

The solvent can be selected from any one which does not disturb the cyclization reaction, for example, CH₂Cl₂, THF, CH₃CN, DMF, alcohols (methanol, ethanol, etc.) or a mixture thereof. The reaction is carried out at a temperature of 0 °C to boiling point of the solvent, preferably at 50 °C to 100 °C.

The cyclization of the compound (II) can be also carried out in the presence of a condensing reagent with or without a base in a suitable solvent or without a solvent. The condensing reagent can be selected from SOCl₂ and conventional condensing reagents which can be used for a peptide synthesis, for example, BOP-Cl, BOP reagent, DCC, EDC or CDI.

The base can be selected from an organic base (e.g., DIEA, DMAP, DBU, Et₃N), an alkali metal hydride (e.g., NaH, LiH), an alkali metal carbonate (e.g., Na₂CO₃, K₂CO₃), an alkali metal hydrogen carbonate (e.g., NaHCO₃, KHCO₃), an alkali metal amide (e.g., NaNH₂), an alkali metal alkoxide (e.g., NaOMe, KOMe), a C₁₋₆ alkyl alkali metal salt(e.g., n-BuLi, t-BuLi), an alkali metal hydroxide (e.g., NaOH, KOH), an alkaline earth metal hydroxide (e.g., Ba(OH)₂), and the like.

The solvent can be selected from any one which does not disturb the cyclization reaction, for example, CH₂Cl₂, THF, DMF or a mixture thereof. The reaction is carried out at a temperature of 0 °C to the boiling point of the solvent, preferably at room temperature.

In this method, any functional group in the compound (II) can be protected with a conventional protecting group before the cyclization reaction. The protecting group can be removed after the cyclization by a conventional method according to the protecting group to be removed, for example, hydrolysis with a base or an acid, acid treatment, and catalytic reduction.

### Method B:

The desired compound (I) or a pharmaceutically acceptable salt thereof, may be prepared by:
(1) reacting the compound of the formula (III): 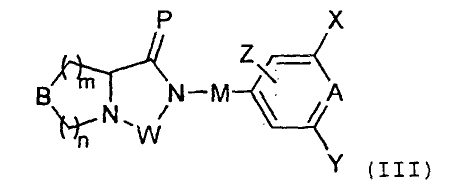 wherein the symbols are the same as defined above, with the compound of the formula (IV):

   R-(K)ₒ-L (IV)

   wherein L is a leaving group and the other symbols are the same as defined above, and
(2) converting the resulting compound into a pharmaceutically acceptable salt thereof by a conventional method, if desired.

The leaving group L can be selected from conventional leaving groups, such as a halogen atom (e.g., chlorine, bromine, iodine), an alkylsulfonyloxy group (e.g., methylsulfonyloxy group) and an arylsulfonyloxy group (e.g., p-tolylsulfonyloxy group).

The condensation reaction can be carried out in the presence of a base in a suitable solvent.

The base can be selected from conventional bases such as alkali metal hydride (i.e., NaH, KH), alkali metal alkoxide (i.e., NaOMe, NaOEt) and alkali metal amide (i.e., NaNH₂, LDA, KHMDS) .

The solvent can be selected from any one which does not disturb the condensation reaction, for example, DME, THF, DMF, HMPA or a mixture thereof. The reaction can be carried out at a temperature of -78 °C to room temperature.

In this method, any functional group in the compound (III) and comound (IV) can be protected with a conventional protecting group before the condensation reaction, if necessary. The protecting group can be removed after the condensation by a conventional method according to the protecting group to be removed, for example, hydrolysis with a base or an acid, acid treatment, and catalytic reduction.

### Method C:

The desired compound (I) or a pharmaceutically acceptable salt thereof, may be prepared by:
(1) reacting the compound of the formula (V): 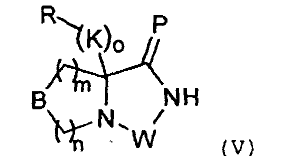 wherein the symbols are the same as defined above, with the compound of the formula (VI): 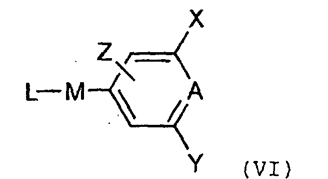 wherein the symbols are the same as defined above, and
(2) converting the resulting compound into a pharmaceutically acceptable salt thereof by a conventional method, if desired.

The leaving group L can be selected from conventional leaving groups such as those mentioned in Method B.

The condensation reaction can be carried out in the presence of a base in a suitable solvent.

The base can be selected from conventional bases such as alkali metal hydride (i.e., NaH, KH), alkali metal alkoxide (i.e., NaOMe, NaOEt) and alkali metal amide (i.e., NaNH₂, LDA, KHMDS).

The solvent can be selected from any one which does not disturb the condensation reaction, for example, DME, THF, DMF, HMPA or a mixture thereof. The reaction is carried out at a temperature of -78 °C to room temperature.

In this method, any functional group in the compound (V) and comound (VI) can be protected with a conventional protecting group before the condensation reaction, if necessary. The protecting group can be removed after the condensation by a conventional method according to the protecting group to be removed, for example, hydrolysis with a base or an acid, acid treatment, and catalytic reduction.

### Method D:

Among the desired compound (I), the compound (I) wherein K is -CH(OH)-, -C(=O)- or -CF₂- may be prepared according to the following method. 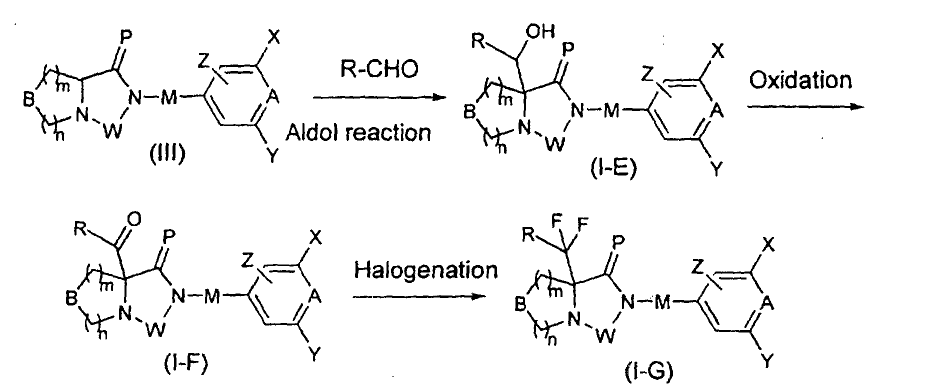 (The symbols are the same as defined above.)
Step 1: Among the desired compound (I), the compound (I-E) can be prepared by reacting the compound (III) with the compound of the formula (VII):

   R-CHO (VII)

   wherein R is the same as defined above.
   The reaction can be carried out by a conventional aldol reaction. For example, the reaction can be carried out in the presence of a base (e.g., LDA, KHMDS, n-BuLi, NaH, KH, MeONa) in a suitable solvent (THF, DMF, MeOH) at a temperature of -78 °C to room temperature.
Step 2: Among the desired compound (I), the compound (I-F) can be prepared by oxidating the compound (I-E).
   The oxidation reaction can be carried out by a conventional method. For example, the oxidation reaction can be carried out by reacting the compound (I-E) with an oxidating reagent (e.g., PCC, PDC, "Dess-Martin Periodinane" reagent) in a suitable solvent (e.g., CH₂Cl₂, toluene) at a temperature of 0 °C to 50 °C.
Step 3: Among the desired compound (I), the compound (I-G) can be prepared by halogenating the compound (I-F).

The halogenation reaction can be carried out by reacting the compound from step 2 with a halogenating reagent (e.g., (diethylamino)sulfur trifluoride) in a suitable solvent (e.g., CCl₄) at a temperature of 0 °C to 50 °C.

The compound (I) of the present invention can be converted into each other in accordance with the following Schemes 1 to 6: 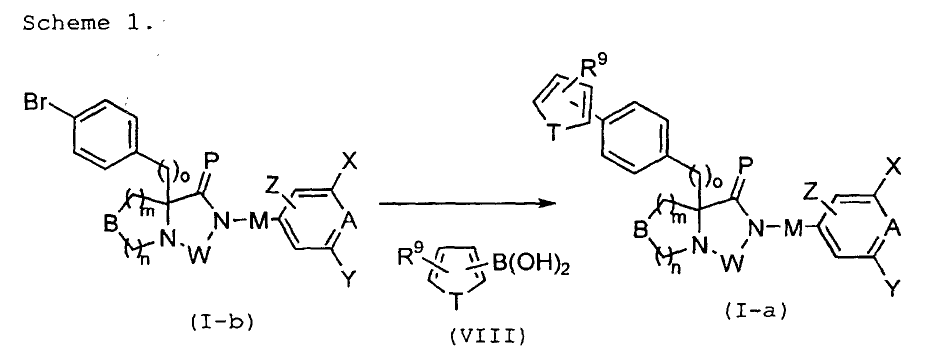 (In Scheme 1, T is -O-, -S-, -NBoc-, -CH=CH- or -CH=N-, R⁹ is H, CN, COCH₃ or CHO, and the other symbols are the same as defined above.)

The compound (I-a) can be prepared by coupling the compound (I-b) with the compound (VIII).

The coupling reaction can be carried out by a conventional aryl coupling method such as Suzuki coupling method. References of Suzuki coupling method are: (a) Suzuki et al., *Synth. Commun.* **1981**, *11,* 513, (b) Suzuki *Pure and Appl*. *Chem.* **1985**, *57*, 1749-1758, (c) Suzuki et al., *Chem. Rev.* **1995**, *95*, 2457-2483, (d) Shieh et al., *J. Org. Chem.* **1992**, *57*, 379-381, (e) Martin et al., *Acta Chemica Scandinavica*, **1993**, *47*, 221-230.

For example, the coupling reaction can be carried out in a suitable solvent in the presence of a Pd catalyst and a base.

As a Pd catalyst, Pd(PPh₃)₄ may be preferably used. The base can be selected from conventional inorganic bases such as K₂CO₃ and Na₂CO₃. The solvent can be selected from any one which does not disturb the coupling reaction, for example, toluene, DME, DMF, H₂O or a mixture thereof. The coupling reaction can be carried out, for example, at a temperature of room temperature to 100 °C, preferably at a temperature of 80 °C to 100 °C.

Compounds of (I-a) where T is NBOC can be converted to compounds where T = NH, which can be further transformed to NR'' (wherein R" is C₁₋₆ alkyl, C₁₋₆ alkylsulfonyl, arylsulfonyl, heteroarylsulfonyl, C₂₋₇ alkanoyl (wherein said C₁₋₆ alkyl, C₁₋₆ alkylsulfonyl, arylsulfonyl, heteroarylsulfonyl, C₂₋₇ alkanoyl is optionally substituted with, for example, amino or carboxy), or CONHR''' (wherein R''' may be C₁₋₆ alkyl, aryl and said C₁₋₆ alkyl and ary may be substituted). Removal of BOC group can be cariied out by a conventional method (e.g., acid (TFA) treatment) in a suitable solvent. Transformation from NH to NR'' may be carried out by the condensation reaction of compound (I-a) and appropreate halides such as C₁₋₆ alkyl halide, C₁₋₆ alkylsulfonyl halide, arylsulfonyl halide, heteroarylsulfonyl halide, C₂₋₇ alkanoyl halide in a suitable solvent (e.g., CH₂Cl₂, THF, DMF, toluene) in the presence of a base (e.g., NaOH, KOH, K₂CO₃, Na₂CO₃, NaH). The reaction can be carried out at a temperature of 0 °C to 100 °C. 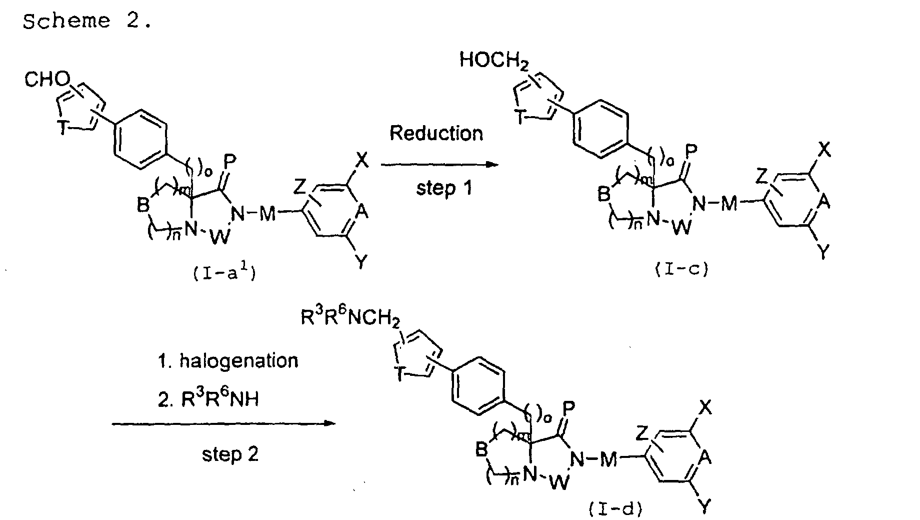 (In the Scheme 2, the symbols are the same as defined above.)
Step 1: The compound (I-c) can be prepared by reducing the compound (I-a¹). The reduction can be carried out by a conventional method, for example, by reacting the compound (I-a¹) with NaBH₄ in a suitable solvent. The solvent can be selected from any one which does not disturb the reduction, for example, toluene, DME, THF, Et₂O or a mixture thereof. The reaction can be carried out at a temperature of -78 °C to 50 °C, preferably at a temperature of 0 °C to room temperature.
Step 2: The compound (I-d) can be prepared by the amination of the compound (I-c). The amination can be carried out by a usual method, for example, by 1) halogenating the compound (I-c) with a halogenating reagent in a suitable solvent, and 2) reacting the resulting halogenated compound with a requisite amine with or without a base in a suitable solvent. In the halogenation of the compound (I-c) with a halogenating reagent, the halogenating reagent can be selected from conventional halogenating reagents such as thionyl chloride. The solvent can be selected from any one which does not disturb the halogenation, for example, toluene, CH₂Cl₂, THF, Et₂O or a mixture thereof. The reaction can be carried out at a temperature of -78 °C to 50 °C, preferably at a temperature of 0 °C to room temperature. In the reaction of the halogenated compound thus obtained and the amine, the base can be selected from conventional inorganic bases (i.e., NaHCO₃, K₂CO₃) and organic bases (i.e., pyridine, Et₃N, N,N-dimethylaniline, i-Pr₂NEt). The solvent can be selected from any one which does not disturb the reaction, for example, toluene, DMF, CH₂Cl₂, DME, THF, Et₂O or a mixture thereof. The reaction can be carried out at a temperature of -78 °C to 50 °C, preferably at a temperature of 0 °C to room temperature. 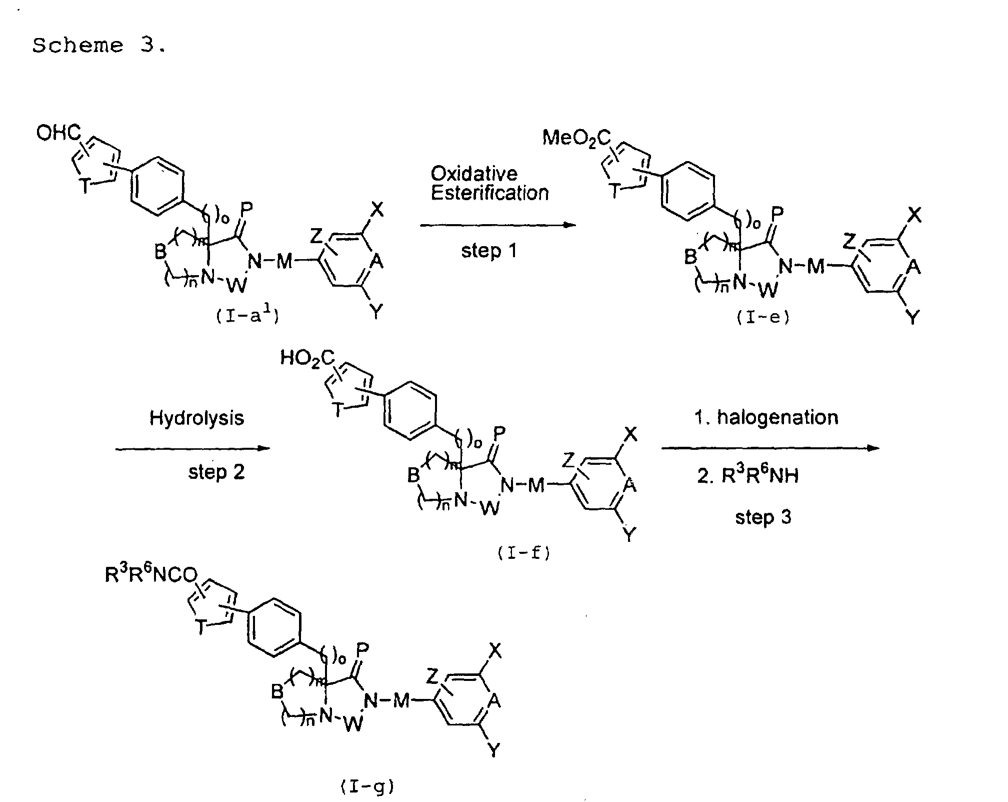 (In the Scheme 3, the symbols are the same as defined above.)

Step 1: The compound (I-e) can be prepared by the oxidative esterification of the compound (I-a¹). The reaction can be carried out by a usual method, for example, by reacting the compound (I-a¹) with an oxidizing reagent such as MnO₂ in the presence of an alkali metal cyanide such as NaCN, MeOH and an acid in a suitable solvent. As the acid, acetic acid can be preferably used. The solvent can be selected from any one which does not disturb the reaction, for example, MeOH, AcOH, H₂O or a mixture thereof. The reaction can be carried out at a temperature of 0 °C to 50 °C, preferably at a temperature of 0 °C to room temperature.
Step 2: The compound (I-f) can be prepared by hydrolyzing the compound (I-e). The hydrolysis can be carried out by a usual procedure, for example, by treating the compound (I-e) with a base in a suitable solvent. The base can be selected from conventional inorganic bases such as LiOH, NaOH and KOH. The solvent can be selected from any one which does not disturb the hydrolyzing reaction, for example, THF, MeOH, EtOH, H₂O or a mixture thereof. The reaction can be carried out at a temperature of -78 °C to 50 °C, preferably at a temperature of 0 °C to room temperature.
Step 3: The compound (I-g) can be prepared by the amidation of the compound (I-f). The amidation can be carried out by a usual method. For example, the compound (I-g) can be prepared by 1) reacting the compound (I-f) with a halogenating reagent, and 2) reacting the acid chloride compound thus obtained with a requisite amine in the presence or absence of a base in a suitable solvent. The reaction of the compound (If) with a halogenating reagent can be carried out at a reflux temperature. The halogenating reagent can be selected from conventional halogenating reagents such as thionyl chloride. In the reaction of the acid chloride compound and the amine, the base can be selected from conventional inorganic bases such as K₂CO₃, Na₂CO₃ and NaHCO₃, and conventional organic bases such as pyridine, Et₃N, i-Pr₂EtN, aniline, N,N-dimethylaniline. The solvent can be selected from any one which does not disturb the coupling reaction, for example, toluene, CH₂Cl₂, THF, Et₂O, H₂O or a mixture thereof. The reaction can be carried out at a temperature of -78 °C to 50 °C, preferably at a temperature of 0 °C to room temperature. 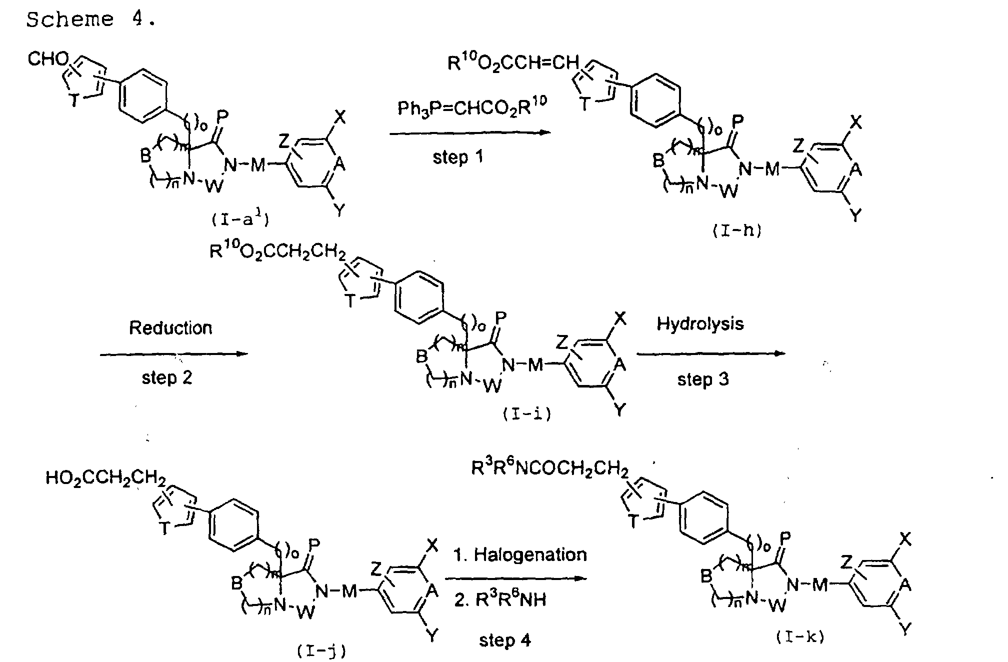
(In the Scheme 4, R¹⁰ is C₁₋₆ alkyl, and the other symbols are the same as defined above.)
Step 1: The compound (I-h) can be prepared by reacting the compound (I-a¹) with a conventional Wittig reagent, Ph₃P=CHCO₂R¹⁰. The solvent can be selected from any one which does not disturb the coupling reaction, for example, toluene, DMSO, DMF, THF, Et₂O or a mixture thereof. The Wittig reaction can be carried out at a temperature of 0 °C to 150 °C, preferably at a temperature of room temperature to 120 °C.
Step 2: The compound (I-i) can be prepared by reducing the compound (I-h). The reduction can be carried out by a usual method, for example, by treating the compound (I-h) with a reducing reagent such as NaBH₄ in the presence of NiCl₂ in a suitable solvent. The solvent can be selected from any one which does not disturb the reduction reaction, for example, MeOH, EtOH, AcOH, THF, Et₂O, H₂O or a mixture thereof. The reaction can be carried out at a temperature of -78 °C to 50 °C, preferably at a temperature of 0 °C to room temperature.
Step 3: The compound (I-j) can be prepared by hydrolyzing the compound (I-i). The hydrolysis can be carried out in a similar method as described in the Scheme 3, step 2.
Step 4: The compound (I-k) can be prepared by the amidation of the compound (I-j). The amidation can be carried out in a similar method as described in the Scheme 3, step 3. 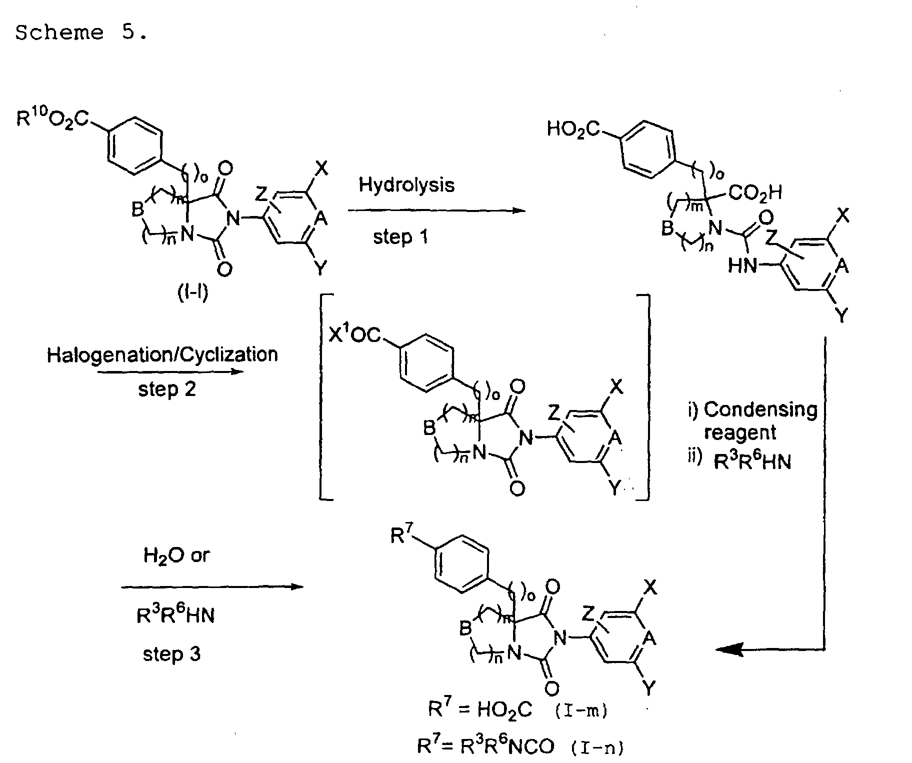
(In the Scheme 5, X¹ is a halogen, and the other symbols are the same as defined above.)

Among the compound (I), the compound (I-m) and the compound (I-n) can be prepared by the steps comprising 1) hydrolyzing the compound (I-l), 2) reacting the resulting compound with a conventional halogenating reagent such as thionyl chloride, and 3) reacting the resulting cyclized acid chloride compound with H₂O or a requisite amine. These Steps 1 to 3 can be carried out by a similar method as described in the Scheme 3, steps 2 and 3.

An alternative procedure for the preparation of the compound (I-n) from the compound (I-l) comprises the steps of 1) hydrolysis of the compound (I-l), and 2) cyclization of the resulting diacid with condensing reagent followed by treatment with the requisite amine.

The hydrolysis can be carried out as described in the Scheme 3, step 2. The cyclization reaction can be carried out in the presence of a condensing reagent with or without a base in a suitable solvent or without a solvent. The condensing reagent can be selected from any one which can be used for a conventional amide bond synthesis, for example, BOP-Cl, BOP reagent, DCC, EDC or CDI, preferably EDC.

The base can be selected from an organic base (e.g., DIEA, DMAP, DBU, Et₃N), an alkali metal hydride (e.g., NaH, LiH), an alkali metal carbonate (e.g., Na₂CO₃, K₂CO₃), an alkali metal hydrogen carbonate (e.g., NaHCO₃, KHCO₃), an alkali metal amide (e.g., NaNH₂), an alkali metal alkoxide (e.g., NaOMe, KOMe), a lower alkyl alkali metal salt(e.g., n-BuLi, t-BuLi), an alkali metal hydroxide (e.g., NaOH, KOH), an alkaline earth metal hydroxide (e.g., Ba(OH)₂), and the like.

The solvent can be selected from any one which does not disturb the condensation reaction, for example, CH₂Cl₂, THF, DMF or a mixture thereof. The reaction is carried out at a temperature of 0 °C to room temperature, preferably at room temperature. 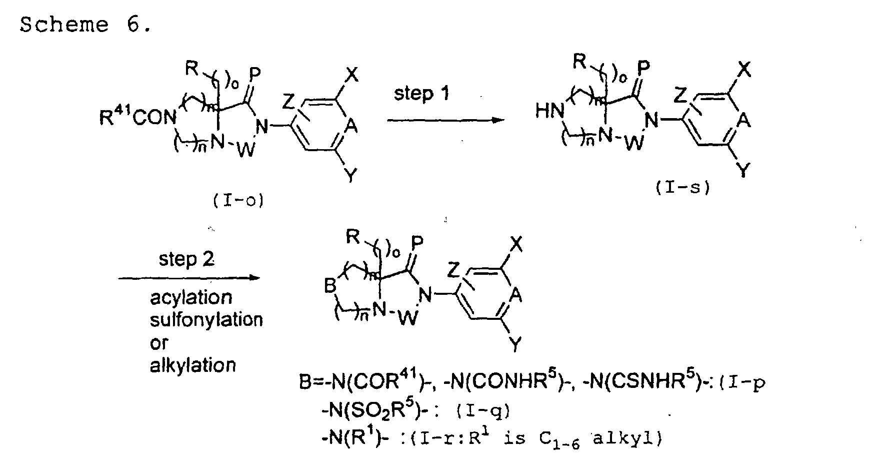 (In the Scheme 6, the symbols are the same as defined above.)

Among the desired compound (I), the compound (I-p), (I-q), or (I-r) can be prepared from the compound (I-o) by 1) removing the COR⁴¹ group, and 2) acylating, sulfonylating or alkylating the resulting compound (I-s).
Step 1: Removal reaction can be carried out by a conventional method, which is selected according to the type of the group to be removed, e.g., 1) catalytic reduction using a catalyst such as palladium on activated carbon under a hydrogen atmosphere, 2) a treatment with an acid such as hydrogen chloride or TFA, at room temperature or with heating in an organic solvent, e.g., CH₂Cl₂, THF, MeOH, EtOH and MeCN, or without an organic solvent.
Step 2: Acylation: The compound (I-p) (B is -N(COR⁴¹)-, -N(CONHR⁵)- or -N(CSNHR⁵)-) can be prepared by the N-acylation of the compound (I-s). The N-acylation reaction can be carried out by a conventional method using 1) an acylating reagent, e.g., a C₂₋₇ alkanoyl halide, a C₁₋₇ alkanoic acid anhydride, a C₁₋₆ alkyl halogenoformate such as a C₁₋₆ alkyl chloroformate, an aryl carbonyl halide, a chlorosulfonyl isocyanate, a C₁₋₆ alkyl isocyanate, a C₁₋₆ alkyl isothiocyanate, an aryl isocyanate or an isocyanate, or 2) a condensing reagent (e.g., CDI, thioCDI) and a requisite amine or alcohol. The N-acylation can be carried out at a temperature of 0 °C to 100 °C (preferably at a temperature of room temperature to 90 °C) in the presence or absence of a base (e.g., DIEA, DMAP, pyridine, NaHCO₃, Na₂CO₃, KHCO₃, K₂CO₃) in an organic solvent (e.g., THF, CH₃CN, CH₂Cl₂, DMF, toluene, acetone and the mixture thereof).

Sulfonylation: The compound (I-q) (B is -N(SO₂R⁵)-) can be prepared by the N-sulfonylation of the compound (I-s). The N-sulfonylation reaction can be carried out by a conventional method using a requisite C₁₋₆ alkylsulfonyl halide or an arylsulfonyl halide in the presence of a base (e.g., Et₃N, DIEA, NaHCO₃, KHCO₃, Na₂CO₃, K₂CO₃) at a temperature of 0 °C to room temperature (preferably at room temperature) in an organic solvent (e.g., CH₂Cl₂, THF, DMF, CH₃CN, toluene, acetone and the mixture thereof).

Alkylation: The compound (I-r) (B is -N(R¹)-, R¹ is C₁₋₆ alkyl) can be prepared by the reductive alkylation of the compound (I-s). The reductive alkylation can be carried out by a conventional method, for example, using aldehydes (e.g., formaldehyde, acetaldehyde), a reducing agent (e.g., sodium cyanoborohydride, NaBH₄) and an acid (e.g., HCl) at room temperature in an organic solvent (e.g., MeOH, EtOH, THF, dioxane) or H₂O, or the mixture thereof.

The desired compound (I) of the present invention can be also converted into each other in accordance with one of the following procedures according to the type of the substituent thereof.

### Procedure A: Acylation of Hydroxyl Group

(A-1) The compound (I) wherein Z, Z¹, R¹, R², R³, R⁴¹, R⁴², R⁵, R⁶ or the substituent of R is a substituted or unsubstituted C₂₋₇ alkanoyloxy group can be prepared by the acylation of the compound (I) wherein the corresponding Z, Z¹, R¹, R², R³, R⁴¹, R⁴², R⁵, R⁶ or the substituent of R is hydroxy. The acylation can be carried out using a C₂₋₇ alkanoyl halide which may be substituted in the presence of a base (e.g., Et₃N, DIEA, NaHCO₃, KHCO₃, Na₂CO₃, K₂CO₃, KHCO₃, Cs2CO3) at a temperature of room temperature to 50 °C in a suitable solvent (e.g., CH₂Cl₂, THF, DMF, CH₃CN, toluene).
(A-2) Among the compound (I) wherein B is -CH(OCOR⁵)- can be prepared by the acylation of the compound (I) wherein B is -CH(OH)-. The acylation reaction can be carried out using R⁵COCl in the presence of a base (e.g., Et₃N, DIEA, NaHCO₃, KHCO₃, Na₂CO₃, K₂CO₃, KHCO₃, Cs₂CO₃) at a temperature of 0 °C to 50 °C in an organic solvent (e.g., CH₂Cl₂, THF, DMF, CH₃CN, toluene).

### Procedure B: Wittig reaction

Among the compound (I) wherein B is -C(R¹)(R²)- and R¹ and R² combine with each other at the terminal thereof to form methylene substituted with C₁₋₆ alkoxycarbonyl can be prepared by the Wittig reaction of the compound (I) wherein B is -C(=O)-.

The Wittig reaction can be carried out by a conventional method using a Wittig reagent, for example, (triphenyl-phosphoranylidene)acetic acid C₁₋₆ alkyl ester at a room temperature of 50 °C to 100 °C in an organic solvent (e.g., toluene, THF).

### Procedure C: Azidation

The compound (I) wherein B is -CH(N₃)- can be prepared from the compound (I) wherein B is -CH(OH)-. This procedure comprises steps of 1) converting hydroxyl group to an eliminating group such as methanesulfonyloxy group and toluenesulfonyl group, and 2) reacting the compound from step-1 with an alkali metal azide. Step-1 can be carried out by reacting the compound (I) wherein B is -CH(OH)- with methanesulfonyl chloride or toluenesulfonyl chloride in the presence of a base (e.g., Et₃N, DIEA, NaHCO₃, KHCO₃, Na₂CO₃, K₂CO₃, KHCO₃, Cs₂CO₃) at a temperature of 0 °C to 50 °C in an organic solvent (e.g., CH₂Cl₂, THF, DMF, CH₃CN, toluene). Step-2 can be carried out at a temperature of 0 °C to 100 °C in an organic solvent (e.g., CH₂Cl₂, THF, DMF, CH₃CN, toluene).

### Procedure D: Reduction of Azido Group

The compound (I) wherein B is -CH(NH₂)- can be prepared by reducing the compound (I) wherein B is -CH(N₃)-. The reduction can be carried out by chemical reduction using metal and inorganic acid (e.g., Fe/HCl, Sn/HCl, SnCl₂/HCl, Zn/AcOH and the like) at a temperature of 0 °C to 50 °C in an organic solvent (e.g., MeOH, EtOH, AcOH), water or a mixture thereof. The reduction reaction can be carried out at a temperature of 0 °C to 100 °C in an organic solvent (e.g., CH₂Cl₂, THF, DMF, CH₃CN, toluene). In addition, the reduction can be carried out under catalytic hydrogenation condition (e.g., H₂/Pd-C). The reduction reaction can be carried out at room temperature in an organic solvent (e.g.,EtOAc, THF, EtOH).

### Procedure E: Acylation of Amino Group

(E-1) The compound (I) wherein Z, Z¹, R¹, R², R³, R⁴¹, R⁴², R⁵, R⁶ or the substituent of R is a substituted or unsubstituted C₂₋₇ alkanoylamino group can be prepared by the acylation of the compound (I) wherein the corresponding Z, Z¹, R¹, R², R³, R⁴¹, R⁴², R⁵, R⁶ or the substituent of R is amino. The acylation reaction can be carried out by a usual method as described in the Scheme 6, step 2.
(E-2) The compound (I) wherein B is -CH(NHCOR⁴¹)-, -CH(NHCONR³R⁶)- or -CH(NHCSNR³R⁶)-, and/or R has NHCOR⁴¹ as a substituent thereof can be prepared by the N-acylation of the compound (I) wherein B is -CH(NH₂)- and/or R has NH₂ as a substitutent.

The N-acylation can be performed with using 1) an acylating reagent (e.g., R⁴¹COCl, R³R⁶NCO, R³R⁶NCS) or 2) a condensing reagent (e.g., CDI, EDC) and a requisite acid (e.g., R⁴¹COOH). The reaction can be carried out by a usual method as described in the Scheme 6, step 2.

### Procedure F: Sulfonylation of Amino Group

(F-1) The compound (I) wherein Z, Z¹, R¹, R², R³, R⁴¹, R⁴², R⁵, R⁶ or the substituent of R is a substituted or unsubstituted C₁₋₆ alkanesulfonylamino group can be prepared by the sulfonylation of the compound (I) wherein the corresponding Z, Z¹, R¹, R², R³, R⁴¹, R⁴², R⁵, R⁶ or the substituent of R is amino. The sulfonylation reaction can be carried out by a usual method as described in the Scheme 6, step 2.
(F-2) The compound (I) wherein B is -CH(NHSO₂R⁵)- and/or R has NHSO₂R⁵ as a substituent can be prepared by the N-sulfonylation of the compound (I) wherein B is -CH(NH₂)- and/or R has NH₂ as a substituent. The N-sulfonylation reaction can be carried out by a usual method as described in the Scheme 6, step 2.

### Procedure G: Preparation of Oxo Group

The compound (I) wherein B is -C(=O)- can be prepared by oxidating the compound (I) wherein B is -CH(OH)-. The oxidation can be carried out in the presence of an oxidating reagent (e.g., PDC, PCC, Swern reagent, and MnO₂) at a temperature of -78 °C to 50 °C in an organic solvent (e.g., CH₂Cl₂, THF, DMF, CH₃CN, toluene) .

### Procedure H: Elimination of Azido Group

The compound (I) wherein B is -CH=CH- can be prepared from the compound (I) wherein B is -CH(N₃)- by β-elimination of N₃ and hydrogen attached to the carbon atom which is adjacent to B. The elimination reaction can be carried by a usual manner, preferably in the presence of a base at a temperature of 0 °C to 50 °C in an organic solvent (e.g., CH₂Cl₂, THF, DMF, CH₃CN, toluene).

### Procedure I: Reduction of Nitro Group

The compound (I) wherein R has NH₂ as a substitutent thereof can be prepared by the reduction of the compound (I) wherein R has NO₂ as a substitutent. The reduction reaction can be carried out by a conventional method. Such method can be selected from, for example, 1) a catalytic reduction using a catalyst (e.g., Raney-nickel, and a palladium on activated carbon) under a hydrogen atmosphere at room temperature in an organic solvent (e.g., methanol, H₂O or the mixture thereof), 2) chemical reduction using metal and inorganic acid (e.g., Fe/HCl, Sn/HCl, SnCl₂/HCl) or 3) reduction with a reducing agent (e.g., Na₂S₂O₄) in a suitable solvent (e.g., methanol, ethanol, H₂O or the mixture thereof) or without a solvent at a temperature of 0 °C to 80 °C.

### Procedure J: Demethylation of Methoxy Group

The compound (I) wherein Z or Z¹ is hydroxy and/or R has hydroxyl as a substituent thereof can be prepared by the demethylation of the compound (I) wherein Z or Z¹ is methoxy and/or R has methoxy as a substituent. The demethylation reaction can be carried out by a conventional method, for example, a treatment with BBr₃ or HBr for the demethylation of a methoxy group, at a temperature of -78 °C to 50 °C in a suitable solvent (e.g., AcOH, water).

### Procedure K: Deprotection of Protected Carboxyl Group

The compound (I) wherein Z, Z¹, R¹, R², R³, R⁴¹, R⁴², R⁵, R⁶ or the substituent of R is carboxyl or has carboxyl can be prepared by the hydrolysis of the compound (I) wherein the corresponding Z, Z¹, R¹, R², R³, R⁴¹, R⁴², R⁵, R⁶ or the substituent of R is C₁₋₆ alkoxycarboxyl group or has C₁₋₆ alkoxycarboxyl. The hydrolysis can be carried out by a conventional method, for example, hydrolysis using a base (e.g., NaOH, LiOH, KOH) or an acid (e.g., hydrochloric acid), and treatment with an acid (e.g., TFA), at room temperature in an organic solvent (e.g., MeOH, EtOH or THF) or without an organic solvent.

### Procedure L: Alkylation of Hydroxyl Group

(L-1) The compound (I) wherein Z, Z¹, R¹, R², R³, R⁴¹, R⁴², R⁵, R⁶ or the substituent of R is a substituted or unsubstituted C₁₋₆ alkoxy group can be prepared by the alkylation of the compound (I) wherein the corresponding Z, Z¹, R¹, R², R³, R⁴¹, R⁴², R⁵, R⁶ or the substituent of R is hydroxy. The alkylation reaction can be carried out using a halogenated C₁₋₆ alkane which may be substituted (e.g., methyl iodide, benzyl bromide) in the presence of a base (e.g., Et₃N, DIEA, NaHCO₃, KHCO₃, Na₂CO₃, K₂CO₃, KHCO₃, Cs₂CO₃) at a temperature of room temperature to 50 °C in an organic solvent (e.g., CH₂Cl₂, THF, DMF, CH₃CN, toluene).
   The alkylation reaction can be also carried out by using a conventional alkylation method such as Mitsunobu Reaction (for reference of Mitsunobu reaction: (a) Mitsunobu, *Synthesis,* 1-28, (1981), (b) Hughes, *Organic Reactions,* 42, 335 (1992), (c) Mitsuhashi et al., *J. Am. Chem. Soc.,* 94, 26 (1972)).
(L-2) Among the compound (I) wherein B is -CH(OR³)- and R³ is C₁₋₆ alkyl which may be substituted can be prepared by the alkylation of the compound (I) wherein B is -CH(OH)-. The alkylation reaction can be carried out using a C₁₋₆ alkyl halide which may be substituted in the presence of a base (e.g., Et₃N, DIEA, NaHCO₃, KHCO₃, Na₂CO₃, K₂CO₃, KHCO₃, Cs₂CO₃) at a temperature of room temperature to 50 °C in an organic solvent (e.g., CH₂Cl₂, THF, DMF, CH₃CN, toluene). The alkylation reaction can be also carried out by a conventional alkylation method such as Mitsunobu Reaction.

### Procedure M: Conversion of Carboxyl Group into Carbamoyl Group

(M-1) The compound (I) wherein Z, Z¹, R¹, R², R³, R⁴¹, R⁴², R⁵, R⁶ or the substituent of R is a substituted or unsubstituted carbamoyl group can be prepared by condensing the compound (I) wherein Z, Z¹, R¹, R², R³, R⁴¹, R⁴², R⁵, R⁶ or the substituent of R is carboxyl with a substituted or unsubstituted amine.
   The condensation reaction can be carried out by the conventional method for a usual peptide synthesis. The condensation reaction of the compound (I) with a substituted or unsubstituted amine is carried out in the presence of a condensing reagent with or without a base in a suitable solvent or without a solvent. The condensing reagent can be selected from any one which can be used for a conventional amide bond synthesis, for example, BOP-Cl, BOP reagent, DCC, EDC or CDI.
   The base can be selected from an organic base (e.g., DIEA, DMAP, DBU, Et₃N), an alkali metal hydride (e.g., NaH, LiH), an alkali metal carbonate (e.g., Na₂CO₃, K₂CO₃), an alkali metal hydrogen carbonate (e.g., NaHCO₃, KHCO₃), an alkali metal amide (e.g., NaNH₂), an alkali metal alkoxide (e.g., NaOMe, KOMe), a lower alkyl alkali metal salt (e.g., n-BuLi, t-BuLi), an alkali metal hydroxide (e.g., NaOH, KOH), an alkaline earth metal hydroxide (e.g., Ba(OH)₂), and the like.
   The solvent can be selected from any one which does not disturb the condensation reaction, for example, CH₂Cl₂, THF, DMF or a mixture thereof. The reaction is carried out at a temperature of 0 °C to room temperature, preferably at room temperature.
   The condensation reaction of a substituted or unsubstituted amine with the reactive derivative of the compound (I), for example, with an acid halide (e.g., an acid chloride), a reactive ester (e.g., an ester with p-nitrophenol), an anhydride thereof, a mixed anhydride with other carboxylic acid (e.g., a mixed anhydride with acetic acid), and the like, is carried out in the presence of a base or without a base in a solvent or without a solvent.
   The base can be selected from an organic base (e.g., DIEA, DMAP, DBU, Et₃N), an alkali metal hydride (e.g., NaH, LiH), an alkali metal carbonate (e.g., Na₂CO₃, K₂CO₃), an alkali metal hydrogen carbonate (e.g., NaHCO₃, KHCO₃), an alkali metal amide (e.g., NaNH₂), an alkali metal alkoxide (e.g., NaOMe, KOMe), a lower alkylalkali metal salt(e.g., n-BuLi, t-BuLi), an alkali metal hydroxide (e.g., NaOH, KOH), an alkaline earth metal hydroxide (e.g., Ba(OH)₂), and the like.
   The solvent can be selected from any one which does not disturb the condensation reaction, for example, CH₂Cl₂, C₂H₄Cl₂, Et₂O, THF, DMF, CH₃CN, DMSO, benzene, toluene or a mixture thereof. The reaction is carried out at a temperature of -30 °C to 100 °C.
(M-2) The compound (I) wherein Z or Z¹ is COR⁴² and R⁴² is NR³R⁶ can be prepared by condensing the compound (I) wherein Z or Z¹ is carboxyl with HNR³R⁶.
   The condensation reaction can be carried out by a conventional method as described in (M-1).
(M-3) The compound (I) wherein R has -C(=O)-(α-amino acid residue) as a substitutent thereof can be prepared by condensing the compound (I) wherein R has carboxyl group as a substitutent thereof with an α-amino acid.

The condensation reaction can be carried out by a conventional method as described in (M-1).

### Procedure N: Conversion of Halogen Atom to Aryl Group or Heteroaryl Group

The compound (I) wherein X and Y are aryl which may be substitituted or heteroaryl which may be substituted can be prepared by reacting the compound (I) wherein X and Y is halogen with a (substituted or unsubstituted aryl)boronic acid or a (substituted or unsubstituted heteroaryl)boronic acid using a conventional aryl coupling method such as Suzuki Coupling method. The coupling reaction can be carried out in a similar procedure as described in Scheme 1.

### Procedure O: Oxidation of Sulfur Atom

(O-1) The compound (I) wherein B is -SO- or -SO₂- can be prepared by oxidizing the compound (I) wherein B is -S- with an oxidant such as a peracid (e.g., mCPBA, H₂O₂, AcOOH, PhCOOOH) in a suitable solvent (e.g., CH₂Cl₂) at room temperature or under cooling.
(O-2) The compound (I) wherein R has C₁₋₆ alkylsulfinyl or C₁₋₆ alkylsulfonyl as a substitutent thereof can be prepared by oxidizing the compound (I) wherein R has C₁₋₆ alkylthio as a substitutent. The oxidation can be carried out in a similar manner as described in the above procedure (O-1) .

### Procedure P: Acylation of pyrrolyl group

(P-1) The compound (I) wherein X and/or Y is formyl-substituted pyrrolyl can be prepared by formylation of the compound (I) wherein X and/or Y is unsubstituted pyrrolyl. The formylation can be carried out by a conventinal method, for example, with using a Vilsmeier reagent (DMF-POCl₃) in a suitable solvent (e.g., DMF) at a temperature of 0 °C to 50 °C, preferably at room temperature.
(P-2) The compound (I) wherein R¹ is lower alkanoyl-substituted pyrrolyl (said lower alkanoyl may be substituted with halogen) can be prepared by the acylation of the compound (I) wherein R¹ is unsubstituted pyrrolyl. The acylation can be carried out by reacting the compound (I) with a requisite lower alkanoyl halide (e.g., acetyl chloride) or a requisite lower alkanoic acid anhydride (e.g., acetic anhydride, trifluoroacetic anhydride) in the presence or absence of a Lewis acid (e.g., SnCl₂, TiCl₄, AlCl₃) in a suitable solvent (e.g., CH₂Cl₂, CCl₄) at a temperature of 0 °C to 50 °C, preferably at room temperature.

### Procedure Q: Amination of Halogen

The compound (I) wherein X and/or Y is NR³R⁶ can be prepared by the amination of the compound (I) wherein X and/or Y is a halogen with an amine compound, HNR³R⁶. The amination can be carried out in a suitable solvent (e.g., THF, toluene, DMF) or without solvent in a sealed tube at a temperature of 50 °C to 150 °C.

### Procedure R: Halogenation of Oxo Group

The compound (I) wherein B is -CF₂- can be prepared by halogenating the compound (I) wherein B is -C(=O)-. The halogenation can be carried out with a halogenating reagent (e.g., (diethylamino)sulfur trifluoride) in a suitable solvent such as halogenomethane (e.g., CH₂Cl₂) at a temperature of 0 °C to 50 °C, preferably at a room temperature.

### Procedure S: Conversion of CN group into Tetrazolyl group

The compound (I) wherein R is tetrazolyl-substituted aryl or tetrazolyl-substituted heteroaryl can be prepared by reacting the compound (I) wherein R is cyano-substituted aryl or cyano-substituted heteroaryl with an alkali metal azide (e.g., NaN₃). The reaction can be carried out in a suitable solvent (e.g., DMF, DMSO, toluene) at a temperature of 50 °C to 150 °C.

### Procedure T: Conversion of CN group into Amidino group

The compound (I) wherein R is amidino-substituted aryl or amidino-substituted heteroaryl can be prepared by 1) alcoholyzing the compound (I) wherein R is a cyano-substituted aryl or a cyano-substituted heteroaryl, and 2) reacting the resulting ester with ammonia. The alcoholysis (step 1) can be carried out in the presence of an acid (e.g., HCl) in a suitable alcohol (e.g., MeOH, EtOH) at a temperature of 50 °C to 150 °C, preferably at room temperature. The reaction of the resulting ester with ammonia (step 2) can be carried out in a suitable solvent (e.g., MeOH, EtOH) at a temperature of 50 °C to 150 °C, preferably at room temperature.

### Procedure U: Halogenation of Aryl or Heteroaryl

The compound (I) wherein R is halogenoaryl or halogenoheteroaryl (e.g., fluorophenyl) can be prepared by reacting the compound (I) wherein R is aryl or heteroaryl with a halogenating reagent (e.g., fluoropyridinium triflate) in a suitable solvent (e.g., CH₃CN) at a temperature of 0 °C to 100 °C.

### Procedure V: Conversion of Amino group into pyrrolyl group or dimethylpyrrolyl group

(V-1) The compound wherein B is -CH(pyrrolyl)- can be prepared by reacting the compound (I) wherein B is -CH(NH₂)-with 2,5-dimethoxyfuran in the presence of a base (e.g., NaOAc) in a suitable solvent (e.g., acetic acid). The reaction can be carried out at a temperature of 0 °C to 100 °C.
(V-2) The compound wherein B is -CH(dimethylpyrrolyl)-can be prepared by reacting the compound (I) wherein B is -CH(NH₂)- with 2,5-hexanedione in the presence of acid (e.g., AcOH) in a suitable solvent (e.g., EtOH, AcOH)

The starting compound of the formula (II) can be prepared by the following scheme: 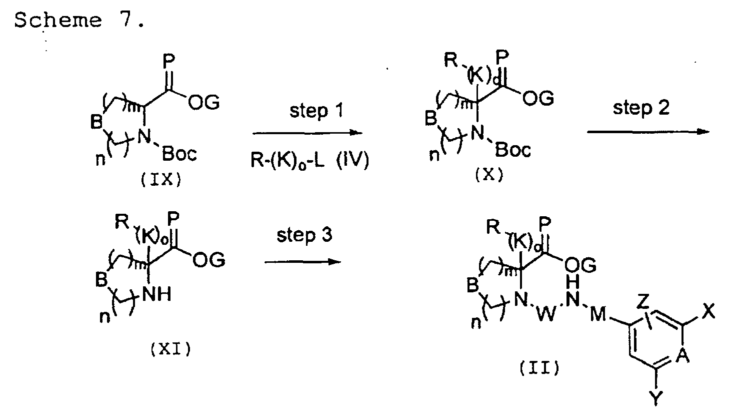 (In the Scheme 7, the symbols are the same as defined above.)
Step 1: The compound (X) can be prepared by reacting the compound (IX) with the compound (IV). The reaction can be carried out in a similar manner as described in Method B.
Step 2: The compound (XI) can be prepared by deprotecting the compound (X). The deprotection can be carried out by a usual manner used for the deprotection of a Boc protected amino group.
Step 3: The compound (II) can be prepared by reacting the compound (XI) with the compound of the formula (XII-a): 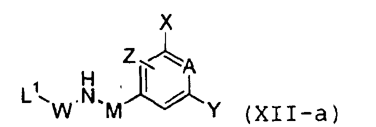
wherein L¹ is a leaving group and the other symbols are the same as defined above, or with the compound of the formula (XII-b) : 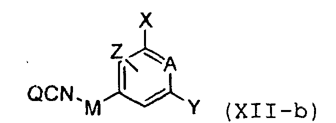 wherein the symbols are the same as defined above.

The leaving group may be selected from conventional leaving groups such as halogen atoms (i.e., chlorine and bromine).

The reaction can be carried out in the presence or absence of a base in a suitable solvent or without a solvent. The base can be selected from conventional inorganic bases such as K₂CO₃, Na₂CO₃ and NaHCO₃, and conventional organic bases such as pyridine, Et₃N, iPr₂EtN, aniline, and N,N-dimethylaniline. The solvent can be selected from any one which does not disturb the coupling reaction, for example, toluene, DME, DMF, THF, CH₂Cl₂ or a mixture thereof. The coupling reaction can be carried out, for example, at a temperature of -78 °C to 50 °C, preferably at a temperature of 0 °C to room temperature.

The compound (II) wherein OG is a resin-combined hydroxyl group may be also prepared in accordance with the following scheme: 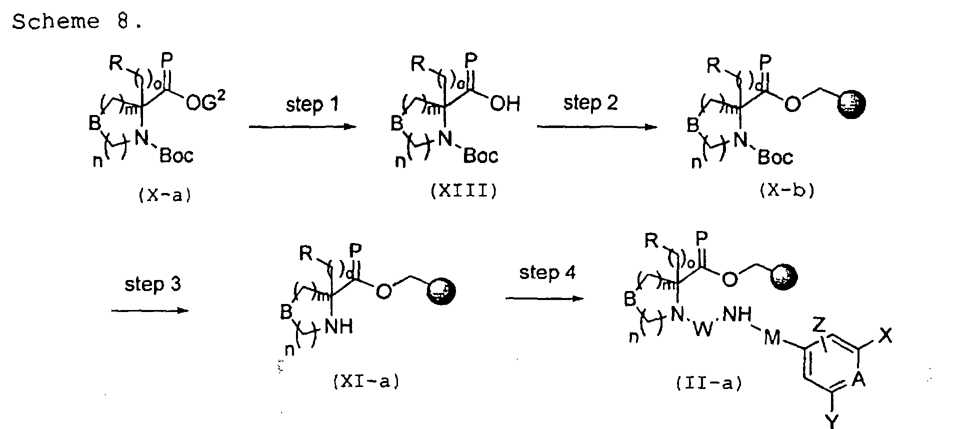 (In the Scheme 8, OG² is a protected hydroxyl group, 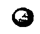 is a resin polymer and other symbols are the same as defined above.)
Step 1: The compound (XIII) can be prepared by deprotecting the compound (XI-a). The deprotection can be carried out by a usual manner used for the deprotection of a protected carboxyl group, for example, by hydrolysis.
Step 2: The reaction can be carried out by a conventional method for solid phase synthesis (e.g., Horiki's method, Horiki et al., Chem. Lett. 1978 (2) 165-168). For example, the reaction can be carried out by heating the compound thus obtained with Merrifield resin in the presence of KF in a suitable solvent such as DMF.
Step 3: The removal of Boc group can be carried out by a similar method as described in the Scheme 7, step 2.
Step 4: The reaction can be carried out by a similar method as described in the Scheme 7, step 3.

The starting compound of the formula (III) can be prepared in accordance with the following scheme: 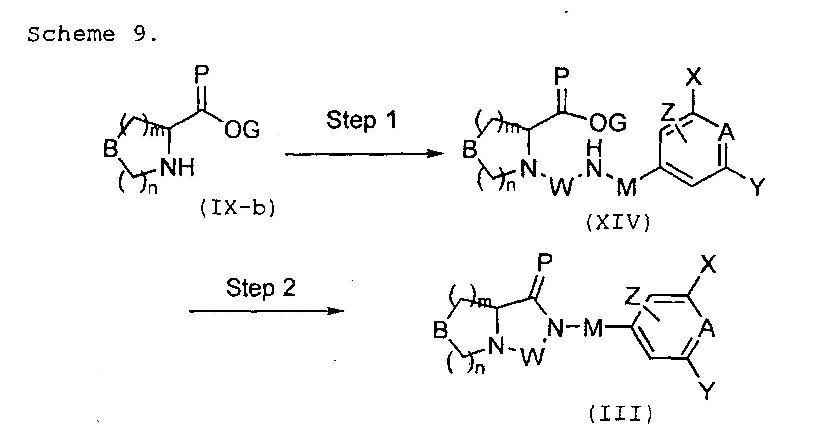 (In the Scheme 9, the symbols are the same as defined above.)
Step 1: The compound (XIV) can be prepared by reacting the compound (IX-b) with the compound (XII-a) or with the compound (XII-b). The reaction can be carried out in a similar manner as described in the scheme 6, step 3.
Step 2: The compound (III) can be prepared by cyclizing the compound (XIV). The cyclization can be carried out in a similar manner as described in Method A.

The starting compound (III) can be also prepared in accordance with the following scheme: 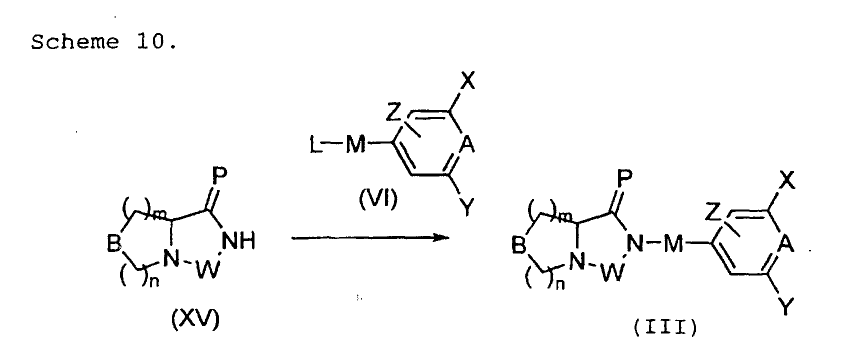 (In the Scheme 10, the symbols are the same as defined above.)

The compound (III) can be prepared by reacting the compound (XV) with the compound (VI). The reaction can be carried out in a similar manner as described in the Method C. The Starting compound (XV) can be prepared by a conventional method (for example, *J*. *Med*. *Chem*., 1995, *38*, 3566).

The starting compound (V) can be prepared in accordance with the following scheme: 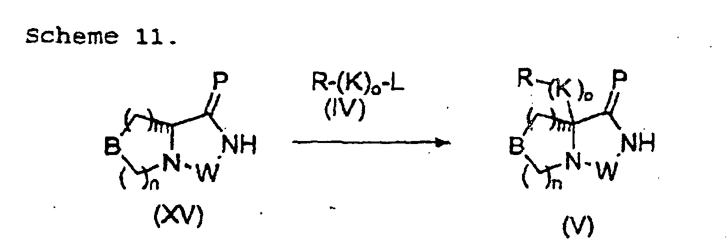 (In the Scheme 11, the symbols are the same as defined above.)

The compound (V) can be prepared by reacting the compound (XV) with the compound (IV). The reaction can be carried out in a similar manner as described in the Method B.

In the present description and the claims, the C₁₋₆ alkyl means a straight chain or branched chain alkyl group having 1 to 6 carbon atoms, for example, methyl, ethyl, propyl, butyl, etc., preferably one having 1 to 4 carbon atoms. The C₃₋₆ cycloalkyl means a cycloalkyl group having 3 to 6 carbon atoms, for example, cyclopropyl, cyclobutyl, cyclopentyl, etc. The C₁₋₆ alkoxy means a straight chain or branched chain alkoxy group having 1 to 6 carbon atoms, for example, methoxy, ethoxy, propoxy, butoxy, etc., preferably one having 1 to 4 carbon atoms. The C₁₋₆ alkoxy also includes a cycloalkyloxy group having 3 to 6 carbon atoms, for example, cyclopropyl, cyclobutyl, cyclopentyl, etc. The C₁₋₆ alkoxycarbonyl means a straight chain or branched chain alkoxycarbonyl group comprising an alkoxy group having 1 to 6 carbon atoms and carbonyl group, for example methoxycarbonyl, ethoxycarbonyl, propoxycarbonyl, and butoxycarbonyl, etc., preferably one having 1 to 4 carbon atoms.

The C₁₋₇ alkanoyl means a straight chain or branched chain alkanoyl group having 1 to 7 carbon atoms, for example, formyl, acetyl, propionyl, butyryl, etc., preferably one having 1 to 5 carbon atoms. The C₂₋₇ alkenyl means a straight chain or branched chain alkenyl group having 2 to 7 carbon atoms, for example vinyl, allyl, crotyl, etc., preferably one having 2 to 5 carbon atoms. The "α-amino acid residue" means an amino acid residue which is obtained by removing a hydrogen atom from the amino group of the corresponding α-amino acid (e.g., aspartic acid, glutamic acid, glutamine, serine, sarcosine, proline, phenylalanine, leucine, glycine, tryptophan, cysteine, histidine, tyrosine, and valine).

### Abbreviations

- Ac:: Acetyl
- Ac₂O:: Acetic anhydride
- AcOH:: Acetic acid
- ACOOH:: Peracetic acid
- AcOEt:: Ethyl acetate (= EtOAc)
- BOC:: t-Butoxycarbonyl (= t-Boc)
- BOP-Cl:: Bis (2-oxo-3-oxazolidinyl) phosphinic chloride
- BOP Reagent:: Benzotriazol-1-yloxy-tris (dimethylamino)-phosphonium hexafluorophosphate
- BSA:: Bovine serum albumin
- CDI:: 1,1'-Carbonyldiimidazole
- DAST:: Diethylaminosulfur trifluoride
- DCC:: 1,3-Dicyclohexylcarbodiimide
- DCM:: Dichloromethane
- DEAD:: Diethyl azodicarboxylate
- DME:: Dimethoxyethane
- DMAP:: 4-Dimethylaminopyridine
- DMF:: Dimethyl formamide
- DIEA:: Diisopropylethylamine
- DMSO:: Dimethyl sulfoxide
- EDC:: 1-(3-Dimethylaminopropyl)-3-ethylcarbodiimide hydrochloride
- Et:: Ethyl
- EtOH:: Ethanol
- HBSS:: Hank's balanced salt solution
- HBTU:: O-Benzotriazol-1-yl-N,N,N',N'-tetramethyluronium hexafluorophosphate
- HMPA:: Hexamethylphosphoramide
- HOBT:: 1-Hydroxybenzotriazole
- HSA:: Human serum albumin
- KHMDS:: Potassium hexamethyldisilazide (=Potassium bis(trimethylsilyl)amide)
- LDA:: Lithium diisopropylamide
- mCPBA:: m-Chloroperbenzoic acid (= MCPBA)
- Me:: Methyl
- MeOH:: Methanol
- n-Bu:: n-Butyl
- PBS:: Phosphate buffered saline
- PCC:: Pyridinium dichromate
- PDC:: Pyridinium chlorochromate
- Ph:: Phenyl
- i-Pr:: i-Propyl
- t-Bu:: tert-Butyl
- THF:: Tetrahydrofuran
- Tf:: Trifluoromethanesulfonyl
- TFA:: Trifluoroacetic acid
- TBDMS:: tert-Butyl-dimethylsilyl
- Tris:: Tris(hydroxymethyl)aminomethane

The compound of the present invention is exemplified by the following examples but not limited thereby.

### Examples

### Example 1. 5-(4-Bromobenzyl)-3-(3,5-dichlorophenyl)-1,3-diazabicyclo[3.3.0]octane-2,4-dione

(1) To a solution of N-(tert-butoxycarbonyl)proline methyl ester (1.30 g) in THF (20 mL) at -78 °C was added a solution of KHMDS (1.25 g) in THF (20 mL). After 45 minutes, a solution of 4-bromobenzyl bromide (1.59 g) in THF (20 mL) was added at -78 °C. The reaction mixture was warmed to room temperature over 3 hours and stirred for an additional 3 hours. The mixture was diluted with EtOAc, washed with water and brine, dried (Na₂SO₄), filtered and concentrated. Purification by flash column chromatography (silica gel; EtOAc/hexane 1:2) afforded N-(tert-butoxycarbonyl)-2-(4-bromobenzyl)proline methyl ester (0.474g). MS (m/z) 398 (MH⁺).
(2) To a solution of the compound obtained above (0.474 g) in CH₂Cl₂ (5 mL) was added TFA (1.5 mL). After 1 hour, the reaction mixture was diluted with CH₂Cl₂, washed with aqueous NaHCO₃ and brine, dried (Na₂SO₄) and concentrated to give 2-(4-bromobenzyl)proline methyl ester. MS (m/z) 298 (MH⁺).
(3) 3,5-Dichlorophenylisocyanate (0.50 g) was added to a solution of the compound obtained above in THF (10 mL) and DIEA (2.0 mL). After 4 hours stirring at room temperature, the reaction mixture was concentrated. The residue was redissolved in EtOAc, washed with aqueous NaHCO₃ and brine, dried (Na₂SO₄), filtered and concentrated. Purification by chromatography (silica gel: EtOAc/hexane: 1/4, Chromatotron (Harrison Research)) afforded 0.33 g of N-[(3,5-dichlorophenyl)carbamoyl]-2-(4-bromobenzyl)proline methyl ester. MS (m/z) 485 (MH⁺). mp 170.9 °C.
(4) The compound obtained above (0.153 g) was dissolved in hot EtOH (10 mL) and cooled to 0 °C. To the solution at 0 °C was added NaOEt (0.010 g). After 45 minutes of stirring, the mixture was concentrated. Purification by chromatography (silica gel: 15% EtOAc/hexane, Chromatotron) afforded the titled compound (0.129 g). MS (m/z) 453 (MH⁺) . mp 69.4 °C.

### Example 2. (5S,7R)-5-(4-Bromobenzyl)-3-(3,5-dichlorophenyl)-7-hydroxy-1,3-diazabicyclo[3.3.0]octane-2,9-dione

(1) To a solution of (4R)-N-(*tert*-Butoxycarbonyl)-4-hydroxy proline benzyl ester (Williams *et al., J. Org. Chem.* **1994,** *59,* 3612-3625) (20.75 g) in acetonitrile (200 mL) was added imidazole (4.43 g) and *tert*-butyldimethylsilylchloride (14.47 g). After stirring overnight, the reaction was concentrated. The residue was redissolved in EtOAc, washed with satd. NaHCO₃ and brine, dried (Na₂SO₄), filtered and concentrated. The compound thus obtained, (4R)-N-(tert-Butoxycarbonyl)-4-(tert-butyldimethylsilyl-oxy)proline benzyl ester (5.60 g), was treated in a similar manner as described in Example 1 (1) to give (2S,4R)-N-(tert-butoxycarbonyl)-2-(4-bromobenzyl)-4-(tert-butyldimethylsilyl-oxy)proline benzyl ester (4.26 g).
(2) To a solution of the compound obtained above (1.00 g) in CH₂Cl₂ (20 mL) was added TBDMSOTf (0.58 mL). After 15 minutes, the mixture was diluted with CH₂Cl₂, washed with water and brine, dried (Na₂SO₄), filtered and concentrated to yield 0.85 g of 2-(4-bromobenzyl)-4-(tert-butyldimethylsilyloxy)proline benzyl ester as a mixture of diastereoisomers. MS (m/z) 504 (MH⁺).
(3) The compound obtained above (0.38 g) was treated in a similar procedure as described in Example 1 (3) and (4) to give (5S, 7R)-5-(4-bromobenzyl)-3-(3,5-dichlorophenyl)-7-(tert-butyldimethylsilyloxy)-1,3-diazabicyclo-[3.3.0]octane-2,4-dione. Separation of the diastereoisomers (5S,7R; 0.23 g) and (5R,7R; 0.045 g) was achieved via flash chromatography eluting with CH₂Cl₂.
(4) To a solution of the compound obtained above (5S,7R; 1.75g) in THF (20 mL) was added HF-pyridine (65-70%, 2.00 mL). After stirring overnight, additional HF-pyridine (65-70%, 1.00 mL) was added. After 20 hours, the mixture was concentrated. The residue was dissolved in EtOAc, washed with 0.5 N HCl, satd. NaHCO₃ and brine, dried (Na₂SO₄), filtered and concentrated to give 1.43 g of the titled compound. MS (m/z) 469 (MH⁺). mp 136.1 °C.

### Example 3. (5S, 7S)-5-(4-Bromobenzyl)-3-(3,5-dichlorophenyl)-7-azido-1,3-diazabicyclo[3.3.0]octane-2,4-dione

(1) To a solution of (5S, 7R)-5-(4-bromobenzyl)-3-(3,5-dichloro-phenyl)-7-hydroxy-1,3-diazabicyclo[3.3.0]octane-2,4-dione (0.81 g) in CH₂Cl₂ (5 mL) and DIEA (0.50 mL) at 0 °C was added methanesulfonyl chloride (0.19 mL). After 1 hour at 0 °C, the mixture was diluted with CH₂Cl₂, washed with satd. NaHCO₃ and brine, dried (Na₂SO₄) and concentrated to give (5S, 7R)-5-(4-bromobenzyl)-3-(3,5-dichlorophenyl)-7-methylsulfonyloxy-1,3-diazabicyclo[3.3.0]octane-2,4-dione. MS (m/z) 494 (MH⁺).
(2) To a solution of the compound obtained above in DMF (4 mL) at 75 °C was added sodium azide (0.54 g). After heating for 2 hours the mixture was diluted with Et₂O and water. The organic solution was collected, washed with water and brine, dried (Na₂SO₄), filtered and concentrated. Purification by chromatography (silica gel: 2% methanol/CH₂Cl₂, Chromatotron) afforded the titled compound (0.82 g). MS (m/z) 494 (MH⁺), mp 58.9 °C.

### Example 4. (5S,7S)-5-(4-Bromobenzyl)-3-(3,5-dichlorophenyl)-7-acetoxy-1,3-diazabicyclo[3.3.0]octane-2,4-dione

To a solution of (5S,7R)-5-(4-bromobenzyl)-3-(3,5-dichlorophenyl)-7-hydroxy-1,3-diazabicyclo[3.3.0]octane-2,4-dione (0.21 g) in THF (5mL) was added DEAD (0.09 mL), triphenylphosphine (0.15 g), and acetic acid (0.035 mL) . After stirring overnight at room temperature, the mixture was concentrated. Purification by chromatography (Silica gel: 25% EtOAc/hexane, Chromatotron) afforded the titled compound (0.151 g). MS (m/z) 511 (MH⁺), mp 149.8 °C.

### Example 5. (5S,7S)-5-(4-Bromobenzyl)-3-(3,5-dichlorophenyl)-7-hydroxy-1,3-diazabicyclo[3.3.0]octane-2,4-dione

To a solution of (5S,7S)-5-(4-bromobenzyl)-3-(3,5-dichloro-phenyl)-7-acetoxy-1,3-diazabicyclo[3.3.0]octane-2,4-dione (0.10 g) in THF (5 mL), MeOH (0.10 mL), and water (0.10 mL) was added 2N LiOH (0.11 mL). After 4 hours, the mixture was quenched with acetic acid (0.10 mL) and concentrated. Purification by chromatography (Silica gel: 4% methanol/CH₂Cl₂, Chromatotron) afforded the titled compound (0.0435 g). MS (m/z) 469 (MH⁺), mp 88.3 °C.

### Example 6. 6-(4-Bromobenzyl)-8-(3,5-dichlorophenyl)-1,8-diazabicyclo[4.3.0]nonane-7,9-dione

The titled compound was prepared by a similar procedure as described in Example 1 starting from pipecolinic acid except that cyclization occurred during treatment with 3,5-dichlorophenyl isocyanate and DIEA. MS (m/z) 467 (MH⁺). mp 128.8 °C.

### Example 7. 6-[4-(3-Pyridyl)benzyl]-8-(3,5-dichlorophenyl)-1,8-diazabicyclo[4.3.0]nonane-7,9-dione

To a solution of 6-(4-bromobenzyl)-8-(3,5-dichlorophenyl)-1,8-diazabicyclo[4.3.0]nonane-7,9-dione (0.360 g) and 3-pyridineboronic acid (0.225 g) in DME (20 mL) was added CsF (0.615 g) and the mixture was degassed with nitrogen. After 10 minutes, Pd(PPh₃)₄ was added and the mixture was heated under reflux for 6 hours. The reaction mixture was concentrated. The residue was redissolved in EtOAc, washed with water and brine, dried (Na₂SO₄), filtered, and concentrated. Purification by chromatography (Silica gel: hexane/acetone: 3/2 , Chromatotron) afforded the titled compound (0.172 g). MS (m/z) 466 (MH⁺). mp 276.5 °C.

The following compounds (Examples 8-11) were prepared in a fashion similar to Example 7 by replacing 3-pyridineboronic acid with requisite boronic acids.

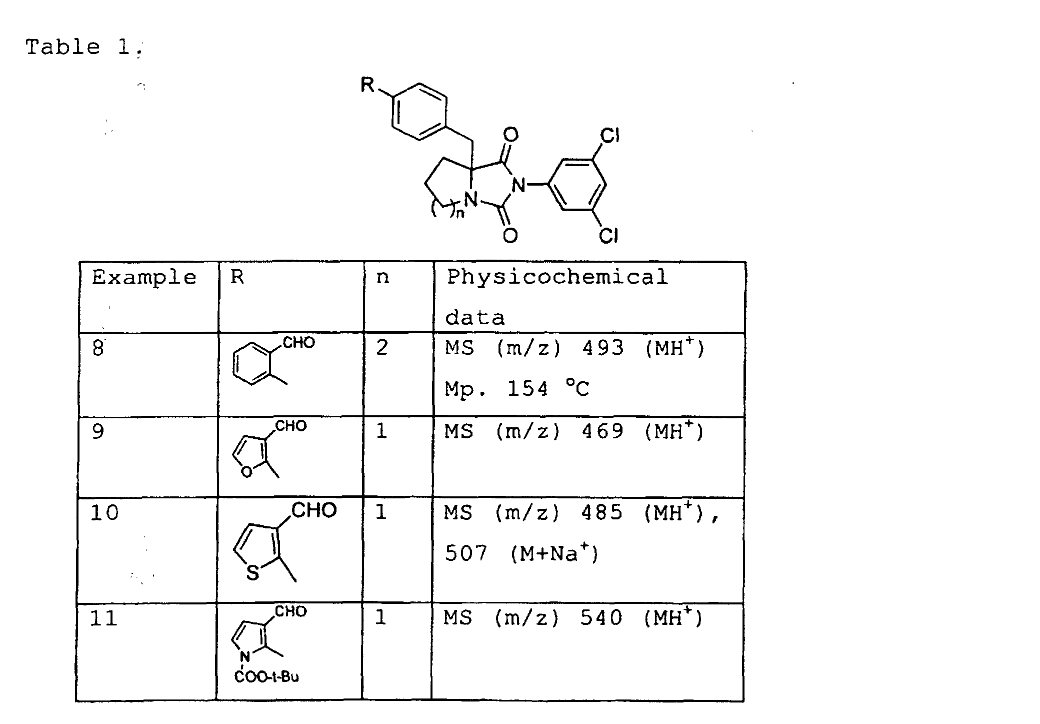

### Example 12. 6-[4-[2-(Hydroxymethyl)phenyl]benzyl]-8-(3,5-dichlorophenyl)-1,8-diazabicyclo[4.3.0]nonane-7,9-dione.

NaBH₄ (13.7 mg) was added to a solution of 6-[4-(2-formyl-phenyl)benzyl]-8-(3,5-dichlorophenyl)-1,8-diazabicyclo[4.3.0]-nonane-7,9-dione (0.178 mg) in THF (5 mL) at 0 °C. After stirring for 30 minutes, the reaction mixture was diluted with water and extracted with EtOAc. The extract was washed with brine, dried (Na₂SO₄), filtered, and concentrated. Purification by chromatography (Silica gel: EtOAc/hexane: 1/2, Chromatotron) afforded the titled compound (167 mg). MS (m/z) 477 (M⁺-OH).

### Example 13. 6-[4-[2-(N,N-Dimethylaminomethyl)phenyl]benzyl]-8-(3,5-dichlorophenyl)-1,8-diazabicyclo[4.3.0]nonane-7,9-dione.

SOCl₂ (0.15 mL) was added to a solution of 6-[4-[2-(hydroxymethyl)phenyl]benzyl]-8-(3,5-dichlorophenyl)-1,8-diazabicyclo[4.3.0]nonane-7,9-dione (0.121 g) in CH₂Cl₂ (5 mL). The mixture was stirred for 1 hour and then evaporated. The residue was dissolved in toluene, evaporated and dried under vacuum. The residue was dissolved in DMF (4 mL) and the solution was added to an ice cold solution of dimethylamine (1 mL, 2M in THF) in DMF. The reaction mixture was stirred for 18 hours at room temperature and diluted with EtOAc. Aqueous NaHCO₃ was added and the organic layer was separated. The extract was washed with water, brine, dried (MgSO₄), filtered, and concentrated. Purification by chromatography (Silica gel: CHCl₃/MeOH: 50/1 to 20/1, Chromatotron) afforded the titled compound (70 mg). It was converted to the HCl salt. MS(m/z) 522(MH⁺).

### Example 14. 6-[4-(2-Methoxycarbonylphenyl)benzyl]-8-(3,5-dichlorophenyl)-1,8-diazabicyclo[4.3.0]nonane-7,9-dione

To a solution of 6-[4-(2-formylphenyl)benzyl]-8-(3,5-dichlorophenyl)-1,8-diazabicyclo[4.3.0]nonane-7,9-dione (0.206 g) in MeOH (20 mL) was added NaCN (0.204 g), AcOH (0.086 mL) and MnO₂ (activated, 1.84 g) and the mixture was stirred overnight at room temperature. The inorganics were removed by filtration through Celite and the filtrate was evaporated. The residue was dissolved in EtOAc and washed with water, brine, dried (MgSO₄), filtered, and concentrated. Purification by chromatography (Silica gel: EtOAc/hexane: 1/2) afforded the titled compound (0.161 g). It was converted to the HCl salt. MS (m/z) 545(M⁺+Na), 523 (MH⁺).

### Example 15. 6-[4-(2-Carboxyphenyl)benzyl]-8-(3,5-dichlorophenyl)-1,8-diazabicyclo[4.3.0]nonane-7,9-dione.

A solution of 6-[4-(2-methoxycarbonylphenyl)benzyl]-8-(3,5-dichlorophenyl)-1,8-diazabicyclo[4.3.0]nonane-7,9-dione (0.141 g) in MeOH/H₂O (5/1 mL) containing NaOH (0.087 g) was heated at 50 °C for 2 h. The reaction mixture was cooled in ice and acidified with 0.5 N HCl. It was extracted with EtOAc and the extract was washed with water, brine, dried (MgSO₄), filtered, and concentrated. Purification by chromatography (Silica gel: CHCl₃/MeOH: 100/1 to 50/1, Chromatotron) afforded the titled compound (114 mg). MS (m/z) 531(M⁺+Na).

### Example 16. 6-[4-[2-(N,N-Dimethylcarbamoyl)phenyl]benzyl]-8-(3,5-dichlorophenyl)-1,8-diazabicyclo[4.3.0]nonane-7,9-dione.

A mixture of the 6-[4-(2-carboxyphenyl)benzyl]-8-(3,5-dichlorophenyl)-1,8-diazabicyclo[4.3.0]nonane-7,9-dione (0.089 g) and SOCl₂ (1.0 mL) was heated at 100 °C for 1 hour. The solution was evaporated. The residue was dissolved in toluene, evaporated and dried under vacuum. The residue was dissolved in THF (4 mL) and the solution was added to an ice cold solution of dimethylamine (1 mL, 2M in THF) in THF (1 mL). The reaction mixture was stirred for 3 hours at room temperature and diluted with EtOAc and water. The organic layer was separated and washed with 0.5 N HCl (10 mL), water and brine. It was dried (MgSO₄), filtered, and concentrated. Purification by chromatography (Silica gel: EtOAc/hexane: 1/2 to 1/1, Chromatotron) afforded the titled compound (85 mg). MS (m/z) 536 (MH⁺).

### Example 17. 6-[4-[2-(2-Methoxycarbonylvinyl)phenyl]benzyl]-8-(3,5-dichlorophenyl)-1,8-diazabicyclo[4.3.0]nonane-7,9-dione

A mixture of 6-[4-(2-formylphenyl)benzyl]-8-(3,5-dichloro-phenyl)-1,8-diazabicyclo[4.3.0]nonane-7,9-dione (0.205 g) and methyl (triphenylphosphoranylidene)acetate (0.223g) in toluene (5 mL) was refluxed for 8 hours. The mixture was evaporated and the residue was purified by chromatography (Silica gel: EtOAc/hexane: 1/4 to 1/3, Chromatotron) to afford the titled compound (0.229 g). MS (m/z) 571 (M⁺ +Na) .

### Example 18. 6-[4-[2-(2-Methoxycarbonylethyl)phenyl]benzyl]-8-(3,5-dichlorophenyl)-1,8-diazabicyclo[4.3.0]nonane-7,9-dione

NiCl₂.6H₂O (0.17 g) was added to a solution of 6-[4-[2-(2-methoxycarbonylvinyl)phenyl]benzyl]-8-(3,5-dichlorophenyl)-1,8-diazabicyclo[4.3.0]nonane-7,9-dione (0.196 g). NaBH₄ (0.14 g) was added portionwise to the mixture and the mixture was stirred for 1 hour. The mixture was diluted with EtOAc and water and filtered through Celite. The organic layer was separated and washed with water, brine, dried (MgSO₄), filtered, and concentrated. Purification by chromatography (Silica gel: EtOAc/hexane: 1/4, Chromatotron) afforded the titled compound (0.125 g). MS (m/z) 573 (M⁺+Na).

### Example 19. 6-[4-[2-(2-Carboxyethyl)phenyl]benzyl]-8-(3,5-dichlorophenyl)-1,8-diazabicyclo[4.3.0]nonane-7,9-dione.

An aqueous solution of NaOH (2 mL; 0.5 N) was added to a solution of 6-[4-[2-(2-methoxycarbonylethyl)phenyl]benzyl]-8-(3,5-dichlorophenyl)-1,8-diazabicyclo[4.3.0]nonane-7,9-dione (0.11 g) in MeOH (7 mL) and the mixture was stirred for 18 hours. The reaction mixture was acidified, diluted with water and extracted with EtOAc. The extract was washed with water, brine, dried (MgSO₄), filtered, and concentrated to give the titled compound (0.109 g). MS (m/z) 559 (M⁺+Na).

### Example 20. 6-[4-[2-[2-(N,N-Dimethylcarbamoyl)ethyl]phenyl]-benzyl]-8-(3,5-dichlorophenyl)-1,8-diazabicyclo[4.3.0]nonane-7,9-dione.

The titled compound was prepared in a manner similar to Example 16. MS (m/z) 564 (MH⁺).

### Example 21. 5-(4-Cyanobenzyl)-3-(3,5-dichlorophenyl)-1,3-diazabicyclo[3.3.0]octane-2,4-dione

(1) Et₃N (1.7 mL) was added to an ice-cold solution of (S)-proline methyl ester hydrochloride (1.31 g) and 3,5-dichlorophenylisocyanate (1,97 g) in CH₂Cl₂ (40 mL). The mixture was stirred for 3 hours. It was washed with water, brine, dried (MgSO₄), filtered, and concentrated. Purification by chromatography (Silica gel: EtOAc/hexane: 1/3) afforded (S)-N-[N-(3,5-dichlorophenyl)carbamoyl]proline methyl ester (2.27 g). mp. 110-112 °C; MS (m/z) 339 (M⁺+Na).
(2) A solution of the compound obtained above (14.3 g) in toluene (100 mL) containing p-toluenesulfonic acid (0.5 g) was refluxed for 2hours. The solution was diluted with EtOAc and was washed with satd. NaHCO₃, water, brine, dried (MgSO₄), filtered, and concentrated. Purification by chromatography (Silica gel: CHCL₃/MeOH: 10/1) and recrystallization from EtOH afforded 8.87 g of (S)-3-(3,5-dichlorophenyl)-1,3-diazabicyclo[3.3.0]octane-2,4-dione. mp. 169 °C; MS (m/z) 307 (M⁺+Na) .
(3) A solution of n-BuLi (2.2 mL) in hexane (1.6 N) was added to a solution of diisopropylamine (0.381 g) in THF (5 mL) at -78 °C. After 15 minutes, a solution of the compound obtained above (0.894 g) in THF (8 mL) was added to the mixture. HMPA (8 mL) and additional THF (8 mL) were added to the mixture. After 35 minutes, a solution of 4-(bromomethyl)benzonitrile (0.697 g) in THF (8 mL) was added and the mixture stirred for 30 minutes at that temperature. The mixture was slowly warmed to room temperature, stirred for 30 minutes and finally quenched with 0.5 N HCl (10 mL). The mixture was diluted with water and extracted with EtOAc. The extract was washed with water, brine, dried (Na₂SO₄), filtered, and concentrated. Purification by chromatography (Silica gel: EtOAc/hexane: 1/2, Chromatotron) afforded the titled compound (0.833 g). MS (m/z) 422 (M⁺+Na).

### Example 22. 5-(4-Methoxycarbonylbenzyl)-3-(3,5-dichlorophenyl)-1,3-diazabicyclo[3.3.0]octane-2,4-dione

The titled compound was prepared in a fashion similar to Example 21. MS (m/z) 455 (M⁺+Na).

### Example 23. 5-[4-(N,N-Dimethylcarbamoyl)benzyl]-3-(3,5-dichlorophenyl)-1,3-diazabicyclo[3.3.0]octane-2,4-dione

(1) NaOH (0.406 g) was added to a solution of 5-(4-methoxycarbonylbenzyl)-3-(3,5-dichlorophenyl)-1,3-diazabicyclo[3.3.0]octane-2,4-dione (0.601 g) in MeOH/H₂O (20mL/4mL) and the mixture was heated at 45 °C for 18 hours. The reaction mixture was cooled, acidified , diluted with water and extracted with EtOAc. The extract was washed with water, brine, dried (MgSO₄), filtered, and concentrated. The residue was recrystallized from EtOAc/hexane (1:1) to give N-[N-(3,5-dichlorophenyl)carbamoyl]-2-(4-carboxybenzyl)proline (0.529 g). mp. 144.6 °C; MS (m/z) 459 (M⁺ +Na) .
(2) A mixture of the compound obtained above (0.074 g) and SOCl₂ (1.0 mL) was heated at 100 °C for 1 hour. The solution was evaporated. The residue was dissolved in toluene, evaporated and dried under vacuum. The residue was dissolved in THF (4 mL) and the solution was added to an ice cold solution of dimethylamine (1 mL, 2M in THF) in THF (1 mL). The reaction mixture was stirred for 3 hour at room temperature and diluted with EtOAc and water. The organic layer was separated and washed with 0.5 N HCl (10 mL), water and brine. It was dried (MgSO₄), filtered, and concentrated. Purification by chromatography (Silica gel: EtOAc, Chromatotron) afforded the titled compound (47 mg). MS (m/z) 468(M⁺+Na).

### Example 24. 5-(4-Carboxybenzyl)-3-(3,5-dichlorophenyl)-1,3-diazabicyclo[3.3.0]octane-2,4-dione.

A mixture of N-[N-(3,5-dichlorophenyl)carbamoyl]-2-(4-carboxybenzyl)proline (0.080 g) and SOCl₂ (2.0 mL) was heated at 100 °C for 1 hour. The solution was evaporated. The residue was dissolved in toluene, evaporated and dried under vacuum. The residue was dissolved in a mixture of THF/H₂O (4/0.4 mL) and the solution was stirred for 18 hours. It was diluted with EtOAc and water. The organic layer was separated, washed with water, brine, dried (MgSO₄), filtered, and concentrated. Purification by chromatography (Silica gel: EtOAc/hexane: 1/1) afforded the titled compound (48 mg). MS (m/z) 441 (M⁺ +Na).

### Example 25. 5-[4-[N-(2-Hydroxyethyl)carbamoyl]benzyl]-3-(3,5-dichlorophenyl)-1,3-diazabicyclo[3.3.0]octane-2,4-dione.

The titled compound was prepared in a fashion similar to Example 23 but replacing dimethyl amine with 2-aminoethanol. MS (m/z) 484 (M⁺ +Na).

### Example 26. 4-(tert-Butoxycarbonyl)-6-(4-bromobenzyl)-8-(3,5-dichlorophenyl)-1,4,8-triazabicyclo[4.3.0]nonane-7,9-dione

(1) To a solution of ethyl 1-benzyloxycarbonyl-4-(tert-butoxycarbonyl)-2-piperazinecarboxylate (2.49 g) (Tet. Lett. 30(39), 5193-5196) in EtOH was added Pd/C (0.25 g). The solution was degassed with nitrogen and hydrogen gas was bubbled into the mixture. The reaction mixture was stirred for 3 hours under hydrogen atmosphere. The mixture was filtered through Celite and concentrated to give 1.15 g of ethyl 4-(tert-butoxycarbonyl)-2-piperazinecarboxylate.
(2) To a solution of the compound obtained above in THF (10 mL) was added 3,5-dichlorophenyl isocyanate (1.00g). After stirring overnight, the solution was concentrated. Purification by chromatography (Silica gel: EtOAc/hexane: 1/4, Chromatotron) afforded ethyl 1-[N-(3,5-dichlorophenyl)carbamoyl]-4-tert-butoxycarbonyl-2-piperazinecarboxylate (1.36 g). MS (m/z) 446 (MH⁺).
(3) The compound obtained above was treated in a manner similar to Example 1 (4) to yield 4-(tert-butoxycarbonyl)-8-(3,5-dichlorophenyl)-1,4,8-triazabicyclo[4.3.0]nonane-7,9-dione (0.72 g). MS (m/z) 422 (M⁺+Na) mp 60.2 °C.
(4) To a solution of the compound obtained above (0.67g) in THF (5 mL) at -78 °C was added LDA (1.3 eq.). After 30 minutes at -78 °C, a solution of 4-bromobenzyl bromide (0.57 g) in THF (2 mL) was added to the mixture. The reaction mixture was allowed to warm to room temperature over 1 hour. After stirring for 4 hours the mixture was concentrated. The residue was redissolved in EtOAc, washed with 1N HCl, aqueous NaHCO₃, and brine, dried (Na₂SO₄), filtered, and concentrated.
Purification by chromatography (Silica gel: EtOAc/hexane: 1/4, Chromatotron) afforded the title compound (0.89 g). MS (m/z) 590 (M⁺+Na). mp 79.5 °C.

### Example 27. 6-(4-Bromobenzyl)-8-(3,5-dichlorophenyl)-1,4,8-triazabicyclo[4.3.0]nonane-7,9-dione

To a solution of 4-(tert-butoxycarbonyl)-6-(4-bromobenzyl)-8-(3,5-dichlorophenyl)-1,4,8-triazabicyclo[4.3.0]nonane-7,9-dione (0.82g) in CH₂Cl₂ (10 mL) was added TFA (0.5 mL). Additional TFA (0.5 mL) was added after 1 hour and 2.5 hours. After 5 hours, the reaction mixture was diluted with CH₂Cl₂, washed with aqueous NaHCO₃, and brine, dried (Na₂SO₄), filtered, and concentrated to yield the titled compound (0.63 g). MS (m/z) 468 (MH⁺). mp 92.5 °C.

### Example 28. 4-Acetyl-6-(4-bromobenzyl)-8-(3,5-dichlorophenyl)-1,4,8-triazabicyclo[4.3.0]nonane-7,9-dione

To a solution of 6-(4-bromobenzyl)-8-(3,5-dichlorophenyl)-1,4,8-triazabicyclo[4.3.0]nonane-7,9-dione (0.062 g) in THF (2.5 mL) and DIEA (0.030 mL) was added acetyl chloride (0.015 mL). After 30 minutes, the reaction mixture was diluted with EtOAc and the solution washed with NaHCO₃, and brine, dried (Na₂SO₄), filtered, and concentrated. Purification by chromatography (Silica gel: CH₂Cl₂/MeOH: 99/1, Chromatotron) afforded the titled compound (0.046 g,). MS (m/z) 510 (MH⁺). mp 89.6 °C.

### Example 29. 4-Methyl-6-(4-bromobenzyl)-8-(3,5-dichlorophenyl)-1,4,8-triazabicyclo[4.3.0]nonane-7,9-dione

To a solution of 6-(4-bromobenzyl)-8-(3,5-dichlorophenyl)-1,4,8-triazabicyclo[4.3.0]nonane-7,9-dione (0.072g) in formic acid (0.40 mL) and water (0.10 mL) was added 37% formaldehyde (0.050 mL). After 1 hour at reflux, the reaction mixture was diluted with EtOAc and the solution washed with aqueous NaHCO₃, and brine, dried (Na₂SO₄), filtered, and concentrated to yield the titled compound (74.3 g). MS (m/z) 482 (MH⁺). mp 221 °C.

### Example 30. 4-(tert-Butoxycarbonylmethyl)-6-(4-bromobenzyl)-8-(3,5-dichlorophenyl)-1,4,8-triazabicyclo[4.3.0]nonane-7,9-dione

To a solution of 6-(4-bromobenzyl)-8-(3,5-dichlorophenyl)-1,4,8-triazabicyclo[4.3.0]nonane-7,9-dione (0.11g) in THF (5 mL) was added DIEA (0.1 mL) and tert-butyl bromoacetate (0.06 mL). Additional tert-butyl bromoacetate (0.040 mL) was added after 16 hours. After 40 hours, the reaction mixture was concentrated and the residue was diluted with EtOAc and washed with water, NaHCO₃, and brine, dried (Na₂SO₄), filtered, and concentrated. Purification by chromatography (Silica gel: EtOAc/hexane: 1/5, Chromatotron) afforded the titled compound (0.124 g). MS (m/z) 582 (MH⁺). mp 74.7 °C.

### Example 31. 4-[(Dimethylamino)acetyl]-6-(4-bromobenzyl)-8-(3,5-dichlorophenyl)-1,4,8-triazabicyclo[4.3.0]nonane-7,9-dione

To a solution of 6-(4-bromobenzyl)-8-(3,5-dichlorophenyl)-1,4,8-triazabicyclo[4.3.0]nonane-7,9-dione (0.085g) in THF (5 mL) was added DIEA (0.1 mL), HOBt (0.57 g), EDC (0.054 g) and N,N-dimethylglycine (0.037 g). After stirring overnight, the reaction was concentrated and the residue was diluted with EtOAc and washed with water, NaHCO₃, and brine, dried (Na₂SO₄), filtered, and concentrated. Purification by chromatography (Silica gel: 2% MeOH/CH₂Cl₂, Chromatotron) afforded the titled compound. (0.093 g). MS (m/z) 553 (MH⁺). mp 73.5 °C.

### Example 32. 4-[2-(Diethylamino)ethyl]-6-(4-bromobenzyl)-8-(3,5-dichlorophenyl)-1,4,8-triazabicyclo[4.3.0]nonane-7,9-dione

The titled compound was prepared in a fashion similar to Example 30.
HCl salt: MS (m/z) 567 (MH⁺), mp 223 °C.

### Example 33. 4-Methanesulfonyl-6-(4-bromobenzyl)-8-(3,5-dichlorophenyl)-1,4,8-triazabicyclo[4.3.0]nonane-7,9-dione

To a solution of 6-(4-bromobenzyl)-8-(3,5-dichlorophenyl)-1,4,8-triazabicyclo[4.3.0]nonane-7,9-dione (0.063g) in CH₂Cl₂ (6 mL) was added DIEA (0.05 mL) and methanesulfonyl chloride (0.015 mL). After 1 hour, the mixture was concentrated and purified by chromatography (Silica gel: 4% MeOH/CH₂Cl₂, Chromatotron) afforded the titled compound. (0.069 g). MS (m/z) 546 (MH⁺). mp 229 °C.

### Example 34. 4-Carboxymethyl-6-(4-bromobenzyl)-8-(3,5-dichlorophenyl)-1,4,8-triazabicyclo[4.3.0]nonane-7,9-dione

To a solution of 4-(tert-butoxycarbonylmethyl)-6-(4-bromobenzyl)-8-(3,5-dichlorophenyl)-1,4,8-triazabicyclo[4.3.0]nonane-7,9-dione (0.080 g) in CH₂Cl₂ (10 mL) was added TFA (0.10 mL). Additional TFA (0.5 mL) was added after 3 and 22 hours. After stirring for 48 hours, the mixture was concentrated and purified by chromatography (Silica gel: 8% MeOH/CH₂Cl₂, Chromatotron) afforded the titled compound. (0.083 g). MS (m/z) 526 (MH⁺). mp 236.5 °C.

### Example 35. 5-(4-Bromobenzyl)-3-(3,5-dichlorophenyl)-1,3-diazabicyclo[3.3.0]octane-2,4,7-trione

(1) To a solution of 1-benzyloxycarbonyl-4-oxoproline (10.61 g, J. Am. Chem. Soc., 79, 185 (1957)) in methanol (150 mL) was bubbled HCl gas. After 30 minutes, the reaction mixture was concentrated and the residue was diluted with EtOAc and washed with aqueous NaHCO₃ and brine, dried (Na₂SO₄), filtered, and concentrated. Purification by chromatography (Silica gel: EtOAc/hexane: 1/3) afforded 10.26 g of 1-benzyloxycarbonyl-4,4-dimethoxyproline methyl ester.
(2) The compound obtained above was treated in a similar manner as described in Example 1 (1) to give 1-benzyloxycarbonyl-2-(4-bromobenzyl)-4,4-dimethoxyproline methyl ester.
(3) To a solution of the compound obtained above (4.76 g) in acetic acid (100 mL) was bubbled HBr gas for 15 minutes. After stirring overnight, Et₂O was added to the mixture. The resulting solid was collected and added to a mixture of ethyl acetate and aqueous NaHCO₃. The organic solution was washed with aqueous NaHCO₃ and brine, dried (Na₂SO₄), filtered, and concentrated to give 2-(4-bromobenzyl)-4-oxoproline methyl ester.
(4) The compound obtained above was reacted with 3,5-dichlorophenyl isocyanate in a fashion similar to Example 1 (3) to afford N-[N-(3,5-dichlorophenyl)carbamoyl]-2-(4-bromobenzyl)-4-oxoproline methyl ester. MS (m/z) 499 (MH⁺). mp 194.2 °C.
(5) A solution of the compound obtained above (0.115g) in toluene (4 mL) containing p-toluenesulfonic acid (10 mg) was refluxed for 5 h and then concentrated. Purification by chromatography (Silica gel: EtOAc/hexane: 1/4) afforded 97.6 mg of the titled compound. mp. 85.4 °C ; MS (m/z) 467 (MH⁺).

### Example 36. 5-(4-Bromobenzyl)-3-(3,5-dichlorophenyl)-7-methoxycarbonylmethylene-1,3-diazabicyclo[3.3.0]octane-2,4-dione.

The titled compound (mixture of E/Z isomers (1.2/1.0)) was prepared in a fashion similar to Example 17 from 5-(4-bromobenzyl)-3-(3,5-dichlorophenyl)-1,3-diazabicyclo[3.3.0]-octane-2,4,7-trione. MS (m/z) 523 (MH⁺), mp 74.9 °C.

### Example 37. 5-(4-Bromobenzyl)-3-(3,5-dichlorophenyl)-7-carboxymethyl-1,3-diazabicyclo[3.3.0]oct-6-ene-2,4-dione

To a solution of 5-(4-Bromobenzyl)-3-(3,5-dichlorophenyl)-7-methoxycarbonylmethylene-1,3-diazabicyclo[3.3.0]octane-2,4-dione (0.564 g) in THF (4 mL) and methanol (1 mL) at 0 °C was added 2N LiOH (1.20 mL) . After 2 hours, the reaction mixture was concentrated and the residue was suspended in 1 N HCl and filtered. A solution of the filtrate (0.10 g) in thionyl chloride (5 mL) was refluxed for 30 minutes. The reaction mixture was concentrated and the residue was diluted with EtOAc and water. The organic solution was collected and dried (Na₂SO₄), filtered, and concentrated. Purification by chromatography (Silica gel: 4% MeOH/CH₂Cl₂, Chromatotron) afforded 0.063 g of the titled compound. MS (m/z) 509 (MH⁺), mp 101.5 °C.

### Example 38. (8R)-5-(4-Bromobenzyl)-3-(3,5-dichlorophenyl)-8-phenyl-1,3-diazabicyclo[3.3.0]octane-2,4-dione

The titled compound was prepared in a fashion similar to Example 1. MS (m/z) 530 (MH⁺), mp. 72.4 °C.

The following compounds (Examples 39-44) were prepared in a fashion similar to Example 1 using the requisite isocyanate, provided that KHMDS was replaced with LDA.

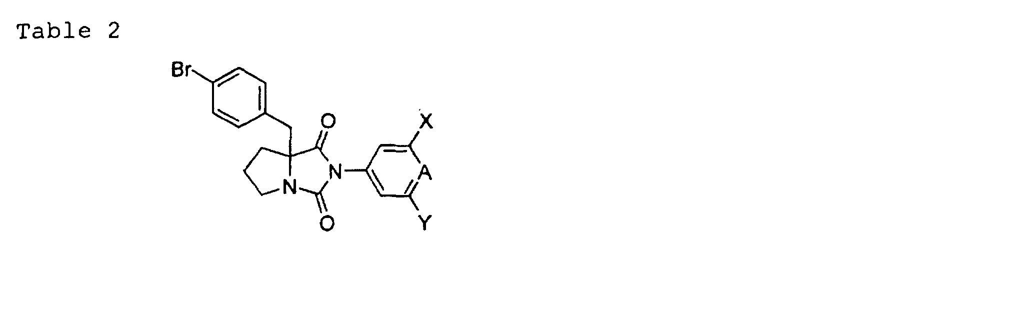

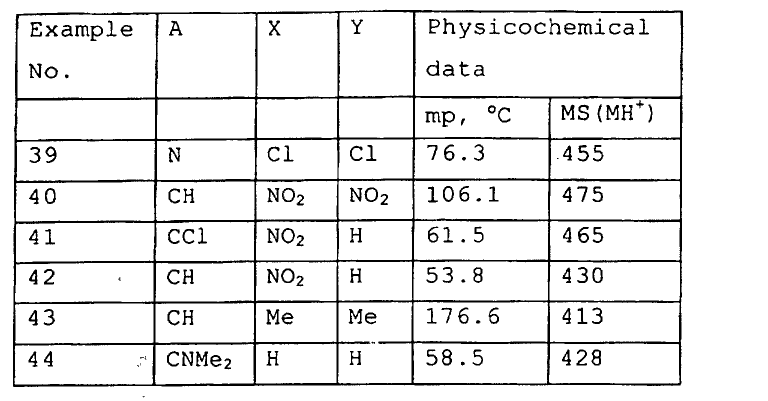

### Example 45. 6-[4-[2-[[2-(dimethylamino)ethoxy]methyl]phenyl]-benzyl]-8-(3,5-dichlorophenyl)-1,8-diazabicyclo[4.3.0]nonane-7,9-dione

(1) To a solution of 6-[4-[2-(hydroxymethyl)phenyl]benzyl]-8-(3,5-dichlorophenyl)-1,8-diazabicyclo[4.3.0]nonane-7,9-dione (0.318 g) in CH₂Cl₂ (5 mL) was added thionyl chloride (0.2 mL) and the mixture was stirred for 1 hour at room temperature. The mixture was concentrated and the residue was redissolved in CH₂Cl₂ (5 mL) and evaporated again. The residue was dried under vacuum to give 6-[4-[2-(chloromethyl)phenyl]benzyl]-8-(3,5-dichlorophenyl)-1,8-diazabicyclo[4.3.0]nonane-7,9-dione.
(2) NaH (60% in oil, 0.090 mg) was added to a solution of N,N-dimethylethanolamine (0.58 mg) in DMF (5 mL) and the mixture was stirred for 30 minutes. To the reaction mixture was added a solution of the compound obtained above in DMF (2 mL) and the mixture was stirred overnight at room temperature. The mixture was cooled in ice-water and quenched with EtOAc/water. The organic layer was separated and washed with water and brine. It was dried, filtered, stripped and the residue was purified by chromatography (silica gel: CHCl₃/MeOH: 50/1 to 30/1) to yield 84 mg of the titled compound. It was dissolved in MeOH and treated with 1N HCl/Et₂O (2 mL). The mixture was evaporated and dried under vacuum to yield 88 mg of the hydrochloride of the titled compound. MS (m/z) 566 (MH⁺).

### Example 46. (5R, 7S)-5-(4-Bromobenzyl)-3-(3,5-dichlorophenyl)-7-azido-1,3-diazabicyclo[3.3.0]octane-2,4-dione

(1) To a solution of (4R)-N-(*tert*-Butoxycarbonyl)-4-hydroxy proline benzyl ester (8.96 g) in CH₂Cl₂ (100 mL) and DIEA (8 mL) at 0 °C was added methanesulfonyl chloride (3.00 mL). After 1 hour, the reaction mixture was allowed to stir at room temperature. After 2.5 hours, the mixture was diluted with CH₂Cl₂, washed with satd. NaHCO₃ and brine, dried (Na₂SO₄), filtered and concentrated. To a solution of the residue in DMF (100 mL) was added sodium azide (7.80 g) and placed in a preheated oil bath (75 °C). After stirring overnight, the reaction was diluted with water and extracted with Et₂O. The organic layer was washed with brine, dried (Na₂SO₄), filtered and concentrated. Purification by chromatography (silica gel: EtOAc/hexane: 1/10) afforded 7.70 g of (4S)-N-(*tert*-butoxycarbonyl)-4-azidoproline benzyl ester.
(2) The compound obtained above (5.69 g) was treated in a similar manner as described in Example 26 (4) to give (a mixture of diastereoisomers) (2R,4S)-N-(*tert*-butoxycarbonyl)-2-(4-bromobenzyl)-4-azidoproline benzyl ester (4.51 g).
(3) The compound obtained above (1.66 g)was treated in a similar procedure as described in Example 2 (2) to give (2R,4S)-2-(4-bromobenzyl)-4-azidoproline benzyl ester (1.0 g) and (2S,4S)-2-(4-bromobenzyl)-4-azidoproline benzyl ester (0.125 g) after purification by flash chromatography.
(4) The compound obtained above (2R,4S; 0.36 g) was treated in a similar procedure as described in Example 1 (3) and (4) to give (5R, 7S)-5-(4-bromobenzyl)-3-(3,5-dichlorophenyl)-7-azido-1,3-diazabicyclo[3.3.0]octane-2,4-dione (0.28 g). MS (m/z) 495 (MH⁺) .

### Example 47. (5R, 7S)-5-(4-Bromobenzyl)-3-(3,5-dichlorophenyl)-7-amino-1,3-diazabicyclo[3.3.0]octane-2,4-dione

To a solution of (5R, 7S)-5-(4-bromobenzyl)-3-(3,5-dichlorophenyl)-7-azido-1,3-diazabicyclo[3.3.0]octane-2,4-dione (0.83 g) in acetic acid (20 mL) was added zinc powder (0.90 g). After 1 hour at room temperature, the mixture was concentrated. The residue was suspended in satd. NaHCO₃ and EtOAc. The mixture was filtered and the organic layer was collected, washed with brine, dried (Na₂SO₄), filtered and concentrated. Purification of the residue by chromatography (silica gel: CH₂Cl₂: Chromatotron) afforded the titled compound (0.42 g). MS (m/z) 468 (MH⁺). mp 64.4 °C.

### Example 48. (5R, 7S)-5-(4-Bromobenzyl)-3-(3,5-dichlorophenyl)-7-acetamido-1,3-diazabicyclo[3.3.0]octane-2,4-dione

To a solution of (5R, 7S)-5-(4-Bromobenzyl)-3-(3,5-dichlorophenyl)-7-amino-1,3-diazabicyclo[3.3.0]octane-2,4-dione (0.103 g) in THF (4 mL) was added acetic anhydride (0.031 mL). After 5 hours, the reaction mixture was concentrated and purified by chromatography (silica gel: CH₂Cl₂, Chromatotron) to afford the titled compound (0.125 g) . MS (m/z) 510 (MH⁺). mp 140.7 °C.

### Example 49. (5R, 7S)-5-(4-Bromobenzyl)-3-(3,5-dichlorophenyl)-7-methanesulfamide-1,3-diazabicyclo[3.3.0]octane-2,4-dione

The titled compound was prepared in a fashion similar to Example 33: MS (m/z) 546 (MH⁺). mp 125.8 °C.

### Example 50. (5R, 7S)-5-(4-Bromobenzyl)-3-(3,5-dichlorophenyl)-7-dimethylamino-1,3-diazabicyclo[3.3.0]octane-2,4-dione

The titled compound was prepared in a fashion similar to Example 29: MS (m/z) 496 (MH⁺). mp 54 °C.

### Example 51. 5-(4-Cyanobenzyl)-3-[3-chloro-5-(1-pyrrolyl)-phenyl]-1,3-diazabicyclo[3.3.0]octane-2,4-dione

(1) To a solution of 5-chloro-1,3-phenylenediamine (2.00 g) in acetic acid (20 mL) was added 2,5-dimethoxytetrahydrofuran (1.8 mL). After 4 hours at reflux, the reaction mixture was concentrated. The residue was suspended in satd. NaHCO₃ and EtOAc. The reaction mixture was filtered and the organic layer was collected, washed with satd. NaHCO₃ and brine, dried (Na₂SO₄), filtered and concentrated. Purification of the residue by chromatography (silica gel: EtOAc/hexane: 1/4) afforded 0.54 g of 3-chloro-5-(1-pyrrolyl)aniline. MS (m/z) 193 (MH⁺) .
(2) To a solution of the compound obtained above (0.35 g) in CH₂Cl₂ (15 mL) was added a solution of trichloroacetyl chloride (0.17 g) dissolved in CH₂Cl₂ (2 mL). After 45 minutes at room temperature, 2-(4-cyanobenzyl)proline methyl ester (0.38 g) (prepared in a similar fashion to Example 1 (1) and (2)) dissolved in CH₂Cl₂ (10 mL) and triethylamine (2 mL) were added. After stirring overnight, the mixture was concentrated. The residue was dissolved in EtOAc, washed with satd. NaHCO₃ and brine, dried (Na₂SO₄), filtered and concentrated. Purification by chromatography (silica gel: EtOAc/hexane: 1/4, Chromatotron) afforded 0.51 g of N-[[3-chloro-5-(1-pyrrolyl)phenyl]carbamoyl]-2-(4-bromobenzyl)proline methyl. MS (m/z) 463 (MH⁺).
(3) The titled compound was prepared from the above compound in a fashion similar to Example 1 (4): MS (m/z) 431 (MH⁺). mp 266.5 °C.

### Example 52. 5-(4-Cyanobenzyl)-3-[3-chloro-5-(2-formyl-1-pyrrolyl)-phenyl]-1,3-diazabicyclo[3.3.0]octane-2,4-dione

To a solution of POCl₃ (0.1 mL) and DMF (0.3 mL) was added after 45 minutes a solution of 5-(4-cyanobenzyl)-3-[3-chloro-5-(1-pyrrolyl)-phenyl]-1,3-diazabicyclo[3.3.0]octane-2,4-dione (0.22 g) in DMF (0.5 mL). After 1 hour at room temperature, the reaction mixture was diluted with water (2 mL) and stirred. After 30 minutes, the mixture was diluted with CH₂Cl₂ and satd. NaHCO₃. The organic layer was collected, washed with brine, dried (Na₂SO₄), filtered and concentrated. Purification by chromatography (silica gel: EtOAc/hexane: 1/3, Chromatotron) afforded 0.091 g of the titled compound. MS (m/z) 459 (MH⁺). mp 109.7 °C.

### Example 53. 2-(tert-Butoxycarbonyl)-6-(4-bromobenzyl)-8-(3,5-dichlorophenyl)-1,2,8-triazabicyclo[4.3.0]nonane-7,9-dione

(1) In direct analogy to the method of Chen *et al*. (Bioorganic and Med. Chem. Lett. 9 (1999), 1587), 1,2-di(tert-butoxycarbonyl)-hexahydropyridazine-3-carboxylic acid ethyl ester was synthesized from ethyl-5-bromo-pentanoate. MS (m/z) 381 (M+Na)⁺.
(2) To a solution of freshly prepared LDA (13.2 mmol) in dry THF (30 mL) was added a solution of the compound obtained above (4.0 g) in dry THF (20 mL). The yellow solution was stirred at -78 °C for 30 minutes whereupon a solution of *p*-bromobenzylbromide (3.9 g) in dry THF (20 mL) was added *via* cannula. The reaction mixture was stirred at -78 °C for 30 minutes then warmed to -30 °C over 2 hours. After warming to room temperature, the reaction mixture was shaken with EtOAc/H₂O. The organic layer was separated and the aqueous layer was extracted with EtOAc. The combined organic layer was dried over MgSO₄, filtered and concentrated. Purification by liquid chromatography (silica gel: EtOAc/hexane: 12/88) gave 1,2-di(tert-butoxycarbonyl)-3-(4-bromobenzyl)hexahydropyridazine-3-carboxylic acid ethyl ester (2.3 g). MS (m/z) 327 (M-2Boc)⁺.
(3) The compound obtained above was dissolved in TFA (50% in CH₂Cl₂, 30 mL) and stirred at room temperature for 3 hours. The mixture was washed with NaHCO₃ until effervescence ceased. The organic layer was dried over MgSO₄ and concentrated to give 3-(4-bromobenzyl)hexahydropyridazine-3-carboxylic acid ethyl ester (650 mg). MS (m/z) 327 (M⁺).
(4) Di-tert-butyl dicarbonate (454 mg) was added to a solution of the compound obtained above (650 mg) and triethylamine (0.32 mL) in CH₂Cl₂ (20 mL) at 0 °C. After 5 hours at 0 °C, the reaction mixture was warmed to room temperature and stirred for additional 20 hours. The mixture was shaken with EtOAc/H₂O, and the organic layer was separated and the aqueous layer was extracted with EtOAc. The combined organic layer was dried over MgSO₄, filtered and concentrated. Purification by liquid chromatography (silica gel: EtOAc/hexane: 35/65) gave 1-(tert-butoxycarbonyl)-3-(4-bromobenzyl)hexahydropyridazine-3-carboxylic acid ethyl ester (580 mg).
(5) 3,5-Dichlorophenylisocyanate (306 mg) was added to a solution of the compound obtained above (580 mg) in dry toluene and the reaction mixture was heated to 95 °C. The progress of the reaction was monitored by MS (following appearance of a peak at 470). Two further portion of 3,5-dichlorophenylisocyanate (306 mg) were added at 24 hours and 48 hours. After a total of 50 hours, the reaction mixture was shaken with EtOAc/H₂O. The organic layer was separated and the aqueous layer was extracted with EtOAc. The combined organic layer was dried over MgSO₄, filtered and concentrated. Purification by liquid chromatography (silica gel: EtOAc/Hexane: 30/70) and recrystallization from EtOAc/hexane to give the titled compound (303 mg). MS (m/z) 592 (M+Na)⁺.

### Example 54. 6-(4-Bromobenzyl)-8-(3,5-dichlorophenyl)-1,2,8-triazabicyclo[4.3.0]nonane-7,9-dione

2-(tert-Butoxycarbonyl)-6-(4-bromobenzyl)-8-(3,5-dichlorophenyl)-1,2,8-triazabicyclo[4.3.0]nonane-7,9-dione (260 mg) was dissolved in TFA/CH₂Cl₂ (50%, 5 mL) and stirred at room temperature for 3 hours. The reaction mixture was then concentrated *in vacuo*, redissolved in CH₂Cl₂ (5 mL) and DIEA (0.22 mL) was added. The mixture was stirred at room temperature for 30 minutes and was shaken with CH₂Cl₂/H₂O. The organic layer was separated and the aqueous layer was extracted with CH₂Cl₂. The combined organic layer was dried over MgSO₄, filtered and concentrated. The residue was recrystallized from EtOAc/hexane to give the titled compound (180 mg). MS (m/z) 470 (M⁺).

### Example 55. 3-(2,6-dichloro-4-pyridyl)-5-(4-bromobenzyl)-1,3-diazabicyclo[3.3.0]octane-2,4-dione (Solid phase synthesis)

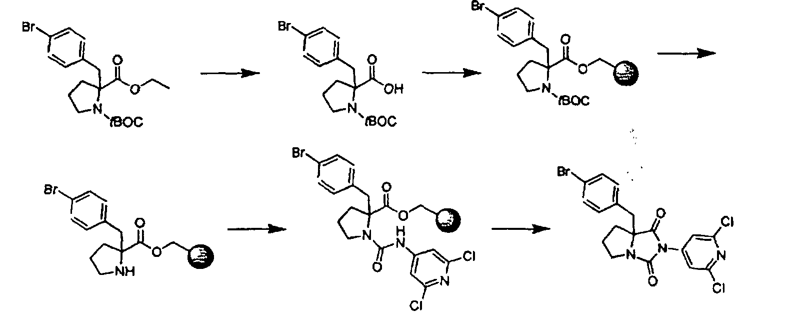

(In the above scheme, 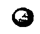 means resin polymer.)
(1) A mixture of N-(tert-butoxycarbonyl)-2-(4-bromobenzyl)proline ethyl ester (3.00 g), LiOH (10 mmol), THF (25 mL), MeOH (10 mL) and H₂O (5 mL) was stirred at room temperature overnight. The mixture was then heated at 74 °C overnight. The mixture was concentrated in vacuo, and water was added. The mixture was washed with Et₂O and the aqueous layer was made acidic with the addition of 1N H₂SO₄. The aqueous layer was extracted with EtOAc. The EtOAc layer was washed with brine, dried over Na₂SO₄, and evaporated to give N-(tert-butoxycarbonyl)-2-(4-bromobenzyl)proline (3.02 g). MS (m/z) 284 (MH⁺-*t*-Boc); mp: 143.9 °C.
(2) To a solution of the compound obtained above (2.45 g) in DMF (100 mL) was added Merrifield Resin (4.70 g) and KF (1.1 g). The resulting mixture was stirred under N₂ at 80 °C overnight. The resin was filtered, and washed thoroughly with MeOH, H₂O and CH₂Cl₂, then dried in vacuo overnight to yield the resin-bound N-(tert-butoxycarbonyl)-2-(4-bromobenzyl)proline (6.66 g); FTIR (cm⁻¹) 1740, 1697, 1397, 1131, 1011.
(3) The compound obtained above was then deprotected using a 50% solution of TFA in CH₂Cl₂, followed by thorough washing, to give the resin-bound 2-(4-bromobenzyl)proline (6.09 g); FTIR (cm⁻¹) 1722.
(4) To the compound obtained above (200 mg) was added a DMF solution of 2,6-dichloro-4-pyridyl isocyanate (5 equiv.). This mixture was shaken at room temperature in a sealed receptacle overnight. The mixture was filtered, and the resin was washed with DMF, water and MeOH, and air-dried to give the resin-bound N-[(2,6-dichloro-4-pyridyl)carbamoyl]-2-(4-bromobenzyl)proline.
(5) A solution of DIEA (350 µL) in DMF (5 mL) was added to the compound obtained above, and the resulting mixture was then shaken at 50 °C for 2 hours, then at room temperature overnight. The resin was filtered and washed with MeOH, and the filtrate was evaporated. The residue was purified by reverse phase HPLC (gradient of 0%-70% CH₃CN in water) to yield 3-(2,6-dichloro-4-pyridyl)-5-(4-bromobenzyl)-1,3-diazabicyclo[3.3.0]octane-2,4-dione (58.0 mg). MS (m/z): 456 (MH⁺); mp: 76.3 °C;

### Example 56: 8-(t-Butoxycarbonyl)-5-(4-cyanobenzyl)-3-(2,6-dichloro-4-pyridyl)-1,3,8-triazabicyclo[3.3.0]octane-2,4-dione

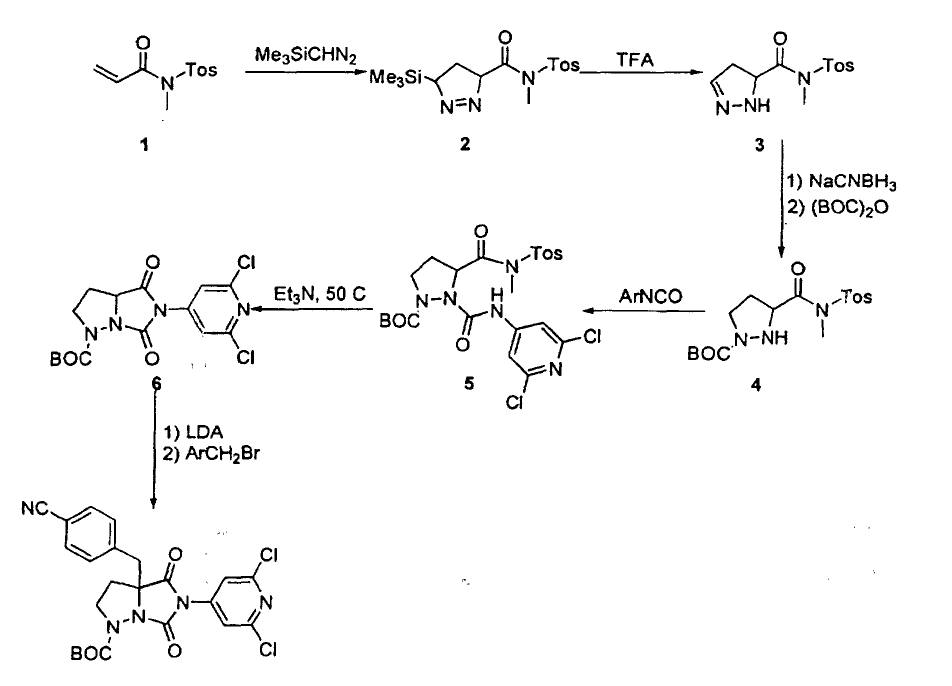

(1) Aza-proline derivative **4** was synthesized from N-methyl-N-tosylacrylamide **1** according to the method of Carreira *et al (J. Am. Chem. Soc*., 1997, 119, 8379).
(2) To a solution of aza-proline **4** (1.7 g) in dry CH₂Cl₂ (30 mL) was added 3,5-dichloropyrid-4-yl isocyanate (1 g) under N₂. The solution was stirred at room temperature for 18 hours. The reaction mixture was concentrated *in vacuo* and purified by chromatography (SiO₂, EtOAc/hexanes 25/75) to give the urea **5** (2.2 g). MS (m/z) 573 (MH⁺).
(3) To a suspension of the urea **5** (1.95 g) in dry toluene/DME (50 mL/12 mL) was added triethylamine (0.18 mL). The reaction mixture was heated at 50 °C for 18 hours, whereupon it was concentrated *in vacuo* and purified by chromatography (SiO₂, EtOAc/DCM 1/9) to give the bicyclic derivative **6** (0.95 g). MS (m/z) 387 (M).
(4) Freshly prepared LDA (1.55 mmol) was added to a solution of the bicyclic compound **6** (0.5 g) in dry THF/DMPU (8 mL/1 mL) at -78 °C under N₂ to give an orange solution. After stirring at -78 °C for 20 minutes, 4-cyano-α-bromotoluene (382 mg) in THF (5 mL) was added via cannula and the reaction mixture stirred at -78 °C for 2.5 hours, then at room temperature for 0.5 hour. Water (30 mL) and EtOAc (50 mL) were added and the mixture was shaken. The aqueous phase was separated and extracted with EtOAc. The combined organics were dried over Na₂SO₄, filtered and concentrated *in vacuo.* Purification of the residue by HPLC (SiO₂, MeCN/H₂O 50/50) gave the titled compound (156 mg). MS (m/z) 502 (M⁺), mp 179 °C.

### Example 57: 5-(4-Cyanobenzyl)-3-(2,6-dichloro-4-pyridyl)-1,3,8-triazabicyclo [3.3.0] octane-2,4-dione.

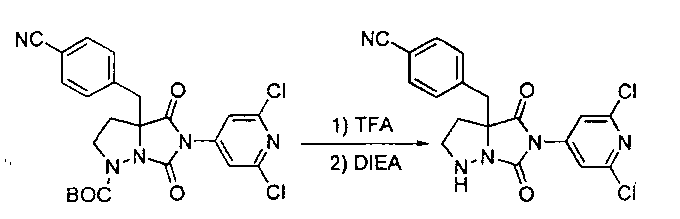

To a solution of the compound from Example 56 (102 mg) in CH₂Cl₂ (5 mL) at -10 °C was added TFA (1 mL). After warming to room temperature, the reaction mixture was stirred for a total of 1 hour while monitoring the progress by TLC. Upon consumption of the starting material, the reaction mixture was cooled to 0 °C and DIEA (4.5 mL) was added carefully. After 2 hours, water (20 mL) and EtOAc (30 mL) were added and the mixture shaken. The aqueous phase was then separated and extracted with EtOAc. The combined organic layers were dried over Na₂SO₄, filtered and concentrated *in vacuo.* The residue was purified by liquid chromatography (SiO₂, EtOAc) and recrystallization from EtOAc/hexanes to give the titled compound (62 mg). MS (m/z) 402 (M); mp 187 °C.

### Example 58: (5R, 7R)-5-[4-(Trifluoromethoxy)benzyl]-3-(2,6-dichloro-4-pyridyl)-7-hydroxy-1,3-diazabicyclo[3.3.0]octane-2,4-dione; and

### Example 59: (5S,7R)-5-[4-(Trifluoromethoxy)benzyl]-3-(2,6-dichloro-4-pyridyl)-7-hydroxy-1,3-diazabicyclo[3.3.0]octane-2,4-dione

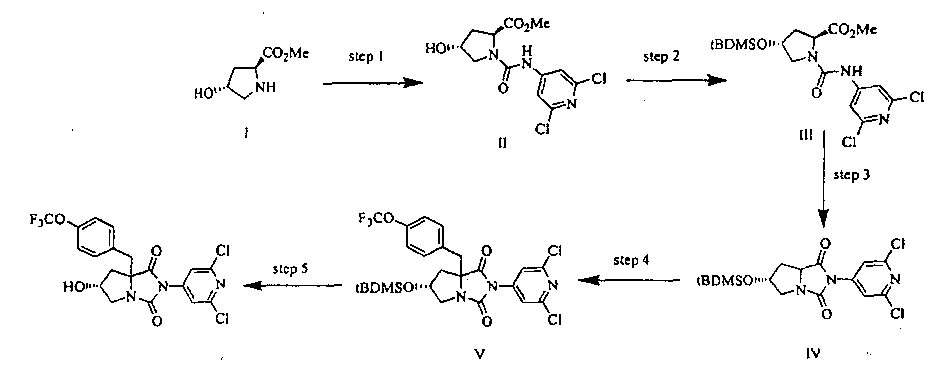

Step-1. To a suspension of L-4-trans-hydroxyproline methyl ester hydrochloride (5.25 g) in THF (50 mL) and DIEA (10 mL) was added 2,6-dichloropyrid-4-yl isocynate (7.00 g). After stirring overnight, the reaction mixture was concentrated. The residue was dissolved in EtOAc, washed with 0.5 N HCl, NaHCO₃, brine, dried (Na₂SO₄), filtered and concentrated. The residue was suspended in CH₂Cl₂ and the white solid filtered to give the urea II (7.12 g).
Step-2. To a suspension of the above urea derivative II (7.12 g) in CH₃CN (150 mL) was added imidazole (2.92 g) and tBDMSCl (6.52 g). After 7 hours, the reaction mixture was concentrated. The residue was dissolved in EtOAc, washed with 0.5 N HCl, NaHCO₃, brine, dried (Na₂SO₄), filtered and concentrated to give the desired product III (10.23 g).
Step-3. To a solution of the above crude urea derivative (10.23 g) in CH₃CN (100 mL) was added DIEA (5.0 mL) and heated to reflux. After refluxing for 2 days, the reaction mixture was concentrated and purified by flash chromatography (2:98 MeOH/CH₂Cl₂) to yield the desired compound IV (7.16 g).
Step-4. The compound from step 3 (3.55 g) was benzylated in a similar procedure as described for Example 56, step 4 yielding 5-[4-(Trifluoromethoxy)benzyl]-3-(2,6-dichloro-4-pyridyl)-7-(tert-butyldimethylsilyloxy)-1,3-diazabicyclo[3.3.0]octane-2,4-dione. Separation of the diastereomers 5R,7R (2.76 g) and 5S,7R (0.3 g) was achieved by flash chromatography eluting with CH₂Cl₂.
Step-5. Both diastereomers from step 4 were treated separately in a similar procedure as described in Example 2 (step 4) to give the following compounds:
   (5R,7R)-5-[4-(Trifluoromethoxy)benzyl]-3-(2,6-dichloro-4-pyridyl)-7-hydroxy-1,3-diazabicyclo[3.3.0]octane-2,4-dione; 1.86 g, MS (m/z) 476 [MH⁺], mp 52.4 °C
   (5S,7R)-5-[4-(Trifluoromethoxy)benzyl]-3-(2,6-dichloro-4-pyridyl)-7-hydroxy-1,3-diazabicyclo[3.3.0]octane-2,4-dione: 0.18 g, MS (m/z) 476 [MH⁺]. mp 162.3 °C.

### Example 60: (5R,7R)-5-(4-Cyanobenzyl)-3-(2,6-dichloro-4-pyridyl)-7-hydroxy-1,3-diazabicyclo[3.3.0]octane-2,4-dione

The titled compound was prepared in a manner similar to Example 58. MS (m/z) 417 [MH⁺]. mp 97.6 °C

### Example 61: (5R, 7S)-5-(4-Bromobenzyl)-3-(3,5-dichlorophenyl)-7-hydroxy-1,3-diazabicyclo[3.3.0]octane-2,4-dione

The titled compound was prepared in a manner similar to Example 58 using 4-bromobenzyl bromide in step 4. MS (m/z) 469 [MH⁺] . mp 60.4 °C.

### Example 62: (5R, 7S)-5-[4-(Trifluoromethoxy)benzyl]-3-(2,6-dichloro-4-pyridyl)-7-azido-1,3-diazabicyclo[3.3.0]octane-2,4-dione

The titled compound was prepared from (5R, 7R)-5-[4-(Trifluoromethoxy)benzyl]-3-(2,6-dichloro-4-pyridyl)-7-hydroxy-1,3-diazabicyclo[3.3.0]octane-2,4-dione in a manner similar to Example 3 except that no heat was used in step 2. MS (m/z) 501 [MH⁺]. mp 139.9 °C.

### Example 63. (5R, 7S)-5-[4-(Trifluoromethoxy)benzyl]-3-(2,6-dichloro-4-pyridyl)-7-amino-1,3-diazabicyclo[3.3.0]octane-2,4-dione

The titled compound was prepared from (5R, 7S)-5-[4-(Trifluoromethoxy)benzyl]-3-(2,6-dichloro-4-pyridyl)-7-azido-1,3-diazabicyclo[3.3.0]octane-2,4-dione in a manner similar to Example 47. MS (m/z) 475 [MH⁺]. mp 150.6 °C.

### Example 64. (5R, 7S)-5-[4-(Trifluoromethoxy)benzyl]-3-(2,6-dichloro-4-pyridyl)-7-acetamido-1,3-diazabicyclo[3.3.0]octane-2,4-dione

The titled compound was prepared from (5R, 7S)-5-[4-(Trifluoromethoxy)benzyl]-3-(2,6-dichloro-4-pyridyl)-7-amino-1,3-diazabicyclo[3.3.0]octane-2,4-dione in a manner similar to Example 48. MS (m/z) 517 [MH⁺]. mp 126.9 °C.

### Example 65. (5R, 7S)-5-[4-(Trifluoromethoxy)benzyl]-3-(2,6-dichloro-4-pyridyl)-7-(ethylurea)-1,3-diazabicyclo[3.3.0]octane-2,4-dione

To a solution of (5R, 7S)-5-[4-(Trifluoromethoxy)benzyl]-3-(2,6-dichloro-4-pyridyl)-7-amino-1,3-diazabicyclo[3.3.0]octane-2,4-dione (0.085 g) in THF (5 mL) was added ethyl isocynate (25 mL). After stirring overnight at room temperature the reaction mixture was concentrated and purified by chromatography (silica gel: 98:2 CH₂Cl₂:MeOH, Chromatotron) to afford the titled compound (0.095 g). MS (m/z) 546 [MH⁺]. mp 227.5 °C.

### Example 66. (5R, 7S)-5-[4-(Trifluoromethoxy)benzyl]-3-(2,6-dichloro-4-pyridyl)-7-(3-carboxypropanoylamino)-1,3-diazabicyclo[3.3.0]octane-2,4-dione

To a solution of (5R, 7S)-5-[4-(Trifluoromethoxy)benzyl]-3-(2,6-dichloro-4-pyridyl)-7-amino-1,3-diazobicyclo[3.3.0]octane-2,4-dione (0.155 g) in CH₂Cl₂ (4 mL) was added succinic anhydride (0.072 g) and DMAP (catalytic amount). After 4 hours, the reaction mixture was concentrated and purified by chromatography (silica gel: 95:5 CH₂Cl₂:MeOH, Chromatotron) to afford the titled compound (0.182 g). MS (m/z) 575 [MH⁺]. mp 101.9 °C.

### Example 67. (5R, 7S)-5-[4-(Trifluoromethoxy)benzyl]-3-(2,6-dichloro-4-pyridyl)-7-(isonicotinoylamino)-1,3-diazabicyclo[3.3.0]octane-2,4-dione

To a solution of (5R, 7S)-5-[4-(Trifluoromethoxy)benzyl]-3-(2,6-dichloro-4-pyridyl)-7-amino-1,3-diazobicyclo[3.3.0]octane-2,4-dione (0.075 g) in THF (5 mL) was added isonicotinic acid (0.041 g), EDC (0.050 g), HOBt (0.057 g), and DIEA (0.10 mL). After stirring overnight at room temperature, the reaction mixture was concentrated. The residue was dissolved in EtOAc, washed with NaHCO₃ and brine, dried (Na₂SO₄), filtered, concentrated and purified by chromatography (silica gel: 95:5 CH₂Cl₂:MeOH Chromatotron) to afford the titled compound (0.103 g). MS (m/z) 580 [MH⁺]. mp 148 °C.

### Example 68. (5R, 7S)-5-[4-(Trifluoromethoxy)benzyl]-3-(2,6-dichloro-4-pyridyl)-7-(dimethylaminoacetylamino)-1,3-diazabicyclo[3.3.0]octane-2,4-dione

The titled compound was prepared in an analogous manner to Example 67. MS (m/z) 580 [MH⁺]. mp 252 °C.

### Example 69. (5R, 7S)-5-[4-(Trifluoromethoxy)benzyl]-3-(2,6-dichloro-4-pyridyl)-7-[3-(4-methyl-1-piperazinylcarbonyl)propanoylamino]-1,3-diazabicyclo[3.3.0]octane-2,4-dione

To a solution of the compound from Example 66 (0.12 g) in THF (5mL) was added N-methyl piperazine (50 µL), EDC (0.90 g), HOBt (0.090 g), and DIEA (0.10 mL). After stirring overnight the reaction mixture was concentrated. The residue was dissolved in EtOAc, washed with water and brine, dried (Na₂SO₄), filtered, concentrated and purified by chromatography (silica gel: 95:5 CH₂Cl₂: MeOH Chromatotron) to afford the titled compound (0.10 g). MS (m/z) 657 [MH⁺]. mp 70 °C.

### Example 70. (5R)-5-[4-(Trifluoromethoxy)benzyl]-3-(2, 6-dichloro-4-pyridyl)-7-oxo-1,3-diazabicyclo[3.3.0]octane-2,4-dione

PDC (0.40 g) was added to a solution of (5R, 7R)-5-[4-(Trifluoromethoxy)benzyl]-3-(2,6-dichloro-4-pyridyl)-7-hydroxy-1,3-diazobicyclo[3.3.0]octane-2,4-dione (0.21 g) in CH₂Cl₂ (10 mL) . Additional PDC was added after 24 hours (0.43 g) and 96 hours (1.19 g). The reaction mixture was diluted with CH₂Cl₂, washed with water, 0.5 N HCl, and brine, dried (Na₂SO₄), filtered, concentrated and purified by chromatography (silica gel: CH₂Cl₂, Chromatotron) to afford the titled compound (0.134 g). MS (m/z) 474 [MH⁺]. mp 166 °C.

### Example 71: (5R, 7S)-5-(4-Bromobenzyl)-3-(3,5-dichlorophenyl)-7-hydroxy-1,3-diaza bicyclo[3.3.0]octane-2,4-dione

The titled compound was prepared in a manner similar to Example 2 using (4S)-N-(*tert*-butoxycarbonyl) 4-hydroxy L-proline benzyl ester as starting material. MS (m/z) 470 [MH⁺]. mp 113.6 °C.

### Example 72: (5R, 7R)-5-(4-Bromobenzyl)-3-(3,5-dichlorophenyl)-7-azido-1,3-diazabicyclo[3.3.0]octane-2,4-dione

The titled compound was prepared from (5R, 7S)-5-(4-bromobenzyl)-3-(3,5-dichlorophenyl)-7-hydroxy-1,3-diazabicyclo[3.3.0]octane-2,4-dione in a manner similar to Example 3. MS (m/z) 495 [MH⁺]. mp 83.4 °C.

### Example 73 (5R, 7R)-5-(4-Bromobenzyl)-3-(3,5-dichlorophenyl)-7-amino-1,3-diazabicyclo[3.3.0]octane-2,4-dione

The titled compound was prepared from (5R, 7R)-5-(4-bromobenzyl)-3-(3,5-dichlorophenyl)-7-azido-1,3-diazabicyclo[3.3.0]octane-2,4-dione in a manner similar to Example 47. MS (m/z) 469 [MH⁺]. mp 87.4 °C.

During the reduction process, two elimination products were isolated in 11 % combined yield as a 10:1 mixture of Example 74 and Example 75.

### Example 74. (5S)-5-(4-Bromobenzyl)-3-(3,5-dichlorophenyl)-1,3-diazabicyclo[3.3.0]oct-6-en-2,4-dione

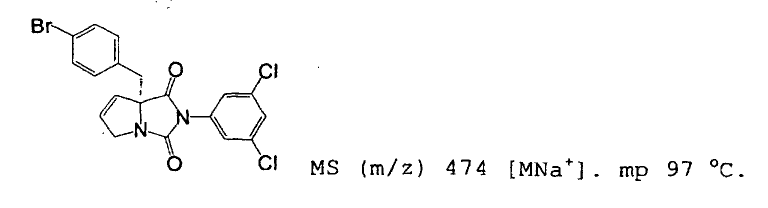

### Example 75. (5R)5-(4-Bromobenzyl)-3-(3,5-dichlorophenyl)-1,3-diazabicyclo[3.3.0]oct-7-en-2,4-dione

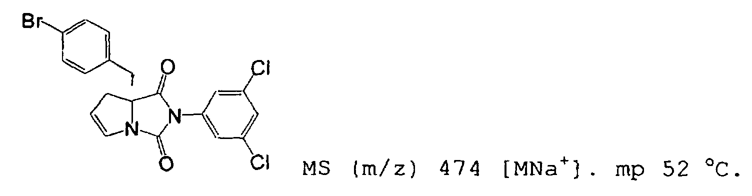

### Example 76. (5R, 7R)-5-(4-Bromobenzyl)-3-(3,5-dichlorophenyl)-7-acetamido-1,3-diazabicyclo[3.3.0]octane-2,4-dione

The titled compound was prepared from (5R, 7R)-5-(4-bromobenzyl)-3-(3,5-dichlorophenyl)-7-amino-1,3-diazabicyclo[3.3.0]octane-2,4-dione in a manner similar to Example 48. MS (m/z) 511 [MH⁺]. mp 156.9 °C.

### Example 77. (5R, 7R)-5-(4-Bromobenzyl)-3-(3,5-dichlorophenyl)-7-benzamide-1,3-diazabicyclo[3.3.0]octane-2,4-dione

To a solution of (5R, 7R)-5-(4-bromobenzyl)-3-(3,5-dichlorophenyl)-7-amino-1,3-diazabicyclo[3.3.0]octane-2,4-dione (0.065 g) in THF (5 mL) was added DIEA (0.50 mL) and benzoyl chloride (0.20 mL). After 3.5 hours, the reaction mixture was concentrated and purified by chromatography (silica gel: 98:2 CH₂Cl₂: methanol, Chromatotron) to afford the titled compound (0.073 g). MS (m/z) 594 [MNa⁺]. mp 149.6 °C.

The following compounds were prepared in an analogous manner to Example 77.

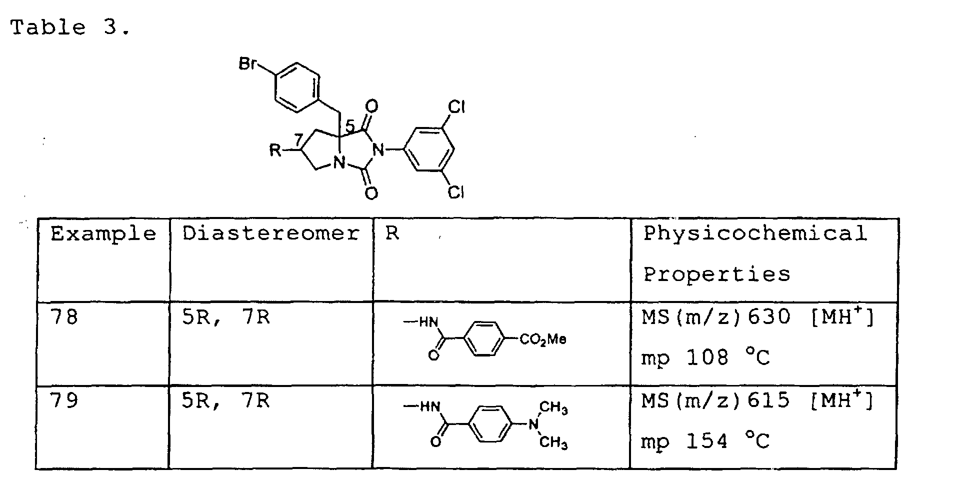

### Example 80. (5R, 7R)-5-(4-Bromobenzyl)-3-(3,5-dichlorophenyl)-7-(4-t-butoxycarbonylbenzoylamino)-1,3-diazabicyclo[3.3.0]octane-2,4-dione

The titled compound was prepared from (5R, 7R)-5-(4-bromobenzyl)-3-(3,5-dichlorophenyl)-7-amino-1,3-diazabicyclo[3.3.0]octane-2,4-dione in a manner similar to Example 67. MS (m/z) 670 [M-H]⁻. mp 112 °C.

### Example 81. (5R, 7R)-5-(4-Bromobenzyl)-3-(3,5-dichlorophenyl)-7-(4-carboxybenzoylamino)-1,3-diazabicyclo[3.3.0]octane-2,4-dione.

To a solution of Example 80 (0.161 g) in CH₂Cl₂ (4 mL) was added TFA (1 mL). After 3 hours at room temperature, the reaction mixture was diluted with CH₂Cl₂, washed with water and brine, dried (Na₂SO₄), filtered, concentrated and purified by chromatography (silica gel: 80:20 CH₂Cl₂:MeOH, Chromatotron) to afford the titled compound (0.133 g). MS (m/z) 614 [M-H]⁻.

### Example 82. 5-(4-Bromobenzyl)-3-(3,5-dichlorophenyl)-8-oxo-1,3-diazabicyclo[3.3.0]octane-2,4-dione

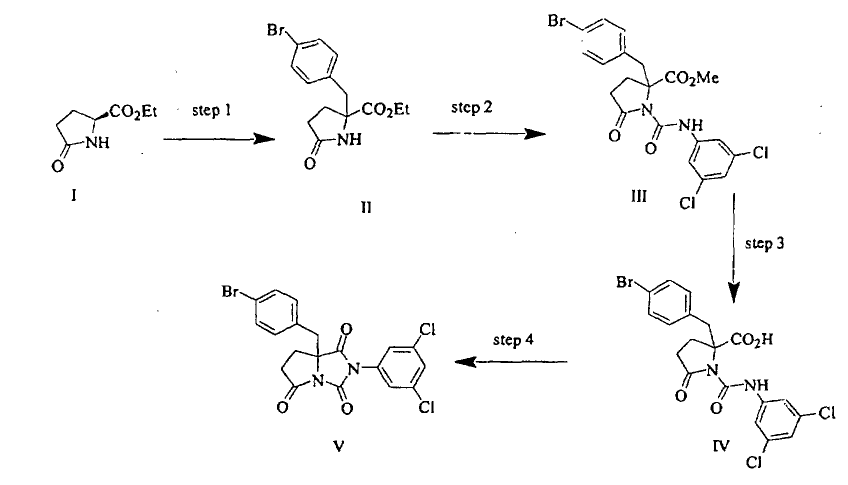

Step-1. To a solution of ethyl pyroglutamic ester (4.09 g) in THF at -78 °C was added KHMDS (12.75 g). After 30 minutes, 4-bromobenzyl bromide (6.79 g) in THF (25 mL) was added. The reaction mixture was allowed to warm to room temperature over 1 hour. After 1 hour at room temperature, the reaction mixture was concentrated. The residue was dissolved in EtOAc, washed with 0.1 N HCl and brine, dried (Na₂SO₄), filtered, concentrated and purified by chromatography (silica gel; 95:5 CH₂Cl₂: MeOH) to afford the benzylated derivative (5.00 g).
Step-2. The urea compound was prepared in a similar procedure as described in Example 1 (step 3).
Step-3. The compound obtained above (0.10 g) in THF (10 mL) was treated with 2 N LiOH (0.1 mL). After 2 hours, the reaction mixture was concentrated. The residue was dissolved in EtOAc, washed with 0.5 N HCl and brine, dried (Na₂SO₄), filtered, concentrated to give the acid (0.1 g).
Step-4. To a solution of the crude acid obtained above in THF (10 mL) was added EDC (0.17 g), HOBt (0.11 g) and DIEA (0.1 mL). After stirring overnight the reaction mixture was concentrated. The residue was dissolved in EtOAc, washed with 0.5 N HCl and brine, dried (Na₂SO₄), filtered, concentrated and purified by chromatography (silica gel: 95:5 CH₂Cl₂:MeOH, chromatotron) to afford the titled compound (0.048 g). MS (m/z) 491 [MNa⁺]. mp 229.6 °C.

### Example 83. (7S)-5-(4-Bromobenzyl)-3-(3,5-dichlorophenyl)-7-phenyl-1,3-diazabicyclo[3.3.0]octane-2,4-dione

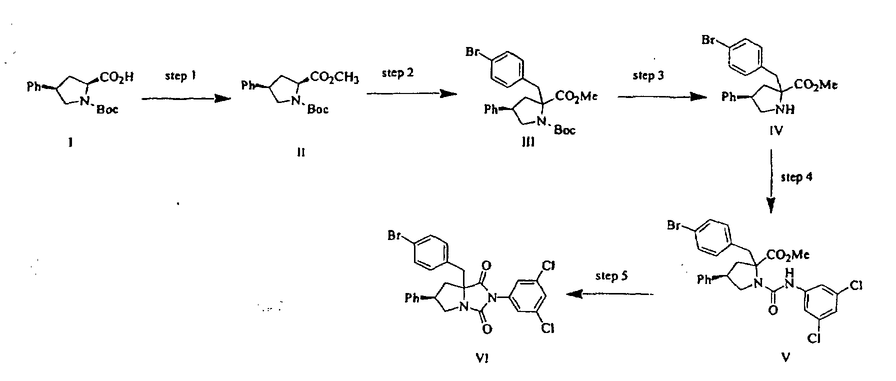

Step-1. To a solution of acid I (1.0 g) in CH₂Cl₂ (10 mL) was added DIEA (0.90 mL), HOBt (0.53 g), EDC (0.83 g) and MeOH (0.10 mL). After stirring overnight the reaction mixture was diluted with CH₂Cl₂, washed with 1 N HCl, NaHCO₃ and brine, dried (Na₂SO₄), filtered, concentrated to yield the desired ester II (0.97 g).
Step-2. The compound above was treated in a similar procedure as described in Example 26 (step 4) yielding a mixture of diastereomers of the comound III.
Step-3. The compound obtained above was treated in a manner similar to Example 1 (step 2).
Step-4. The compound obtained above was treated in a manner similar to Example 1 (step 3).
Step-5. The titled compound was prepared in a manner similar to Example 21 (step 2). MS (m/z) 551 (MNa⁺). mp. 123.5 °C .

### Example 84. 5-(4-Bromobenzyl)-3-(2-chloro-6-dimethylamino-4-pyridyl)-1,3-diazabicyclo[3.3.0]octane-2,4-dione.

A mixture of Example 39 (0.26 g) and (Me)₂NH (2 M in THF, 5 mL) was heated in a sealed tube at 80 °C for 2 hours. The solution was concentrated and the residue was crystallized from EtOH to yield the titled compound (0.16 g). MS (m/z) 464 (MH⁺); mp. 194.3 °C.

### Example 85. 5-(4-Bromobenzyl)-3-(2-chloro-6-methylamino-4-pyridyl)-1,3-diazabicyclo[3.3.0]octane-2,4-dione

By following the above procedure (Example 84) but replacing dimethylamine with methylamine the titled compound was obtained. MS (m/z) 450 (MH⁺).

The following compounds were prepared using the requisite boronic acid in a manner similar to Example 7. Purification by chromatography (SiO₂, EtOAc/hexanes 1/1) gave a mixture of the mono-subatituted bicycle **1** and the di-substituted bicycle **2** which was further purified by HPLC (MeCN/H₂O 50/50) to give the compounds:

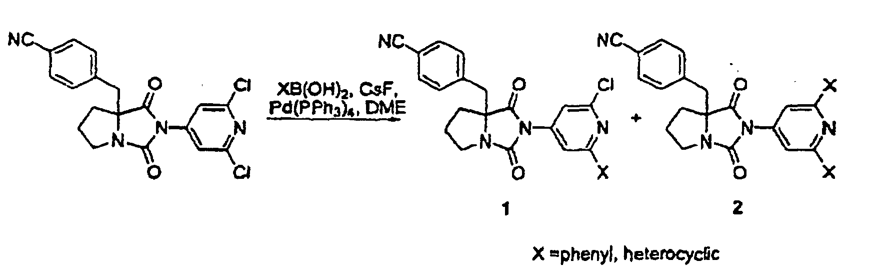

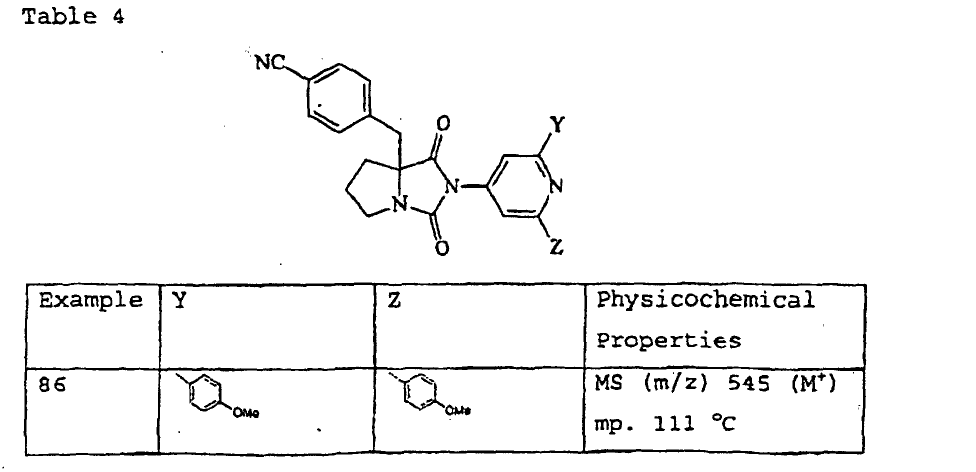

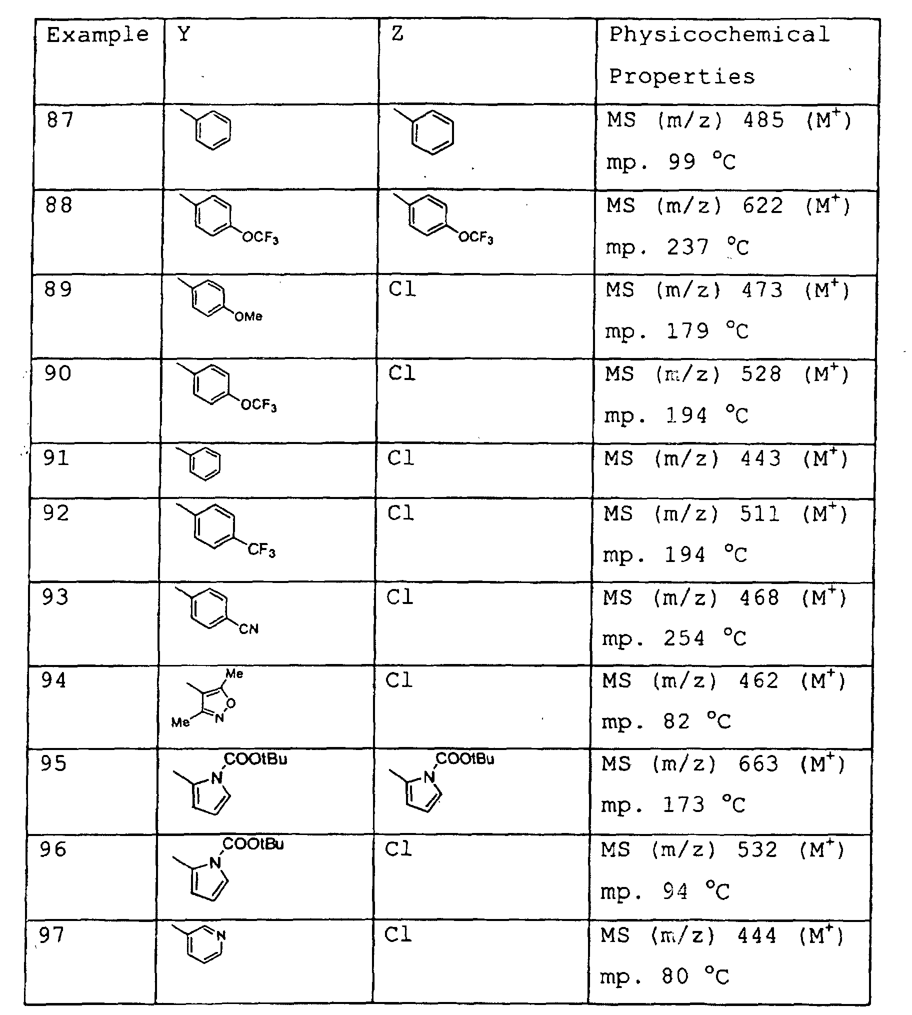

### Example 98: 5-(4-Cyanobenzyl)-3-[2,6-bis(2-pyrrolyl)-4-pyridyl)-1,3-diazabicyclo[3.3.0]octane-2,4-dione

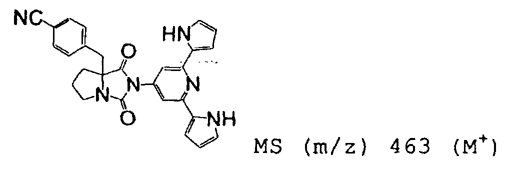

### Example 99: 5-(4-Cyanobenzyl)-3-(2-chloro-6-(2-pyrrolyl)-4-pyridyl)-2,3-diazabicyclo[3.3.0]octane-2,4-dione

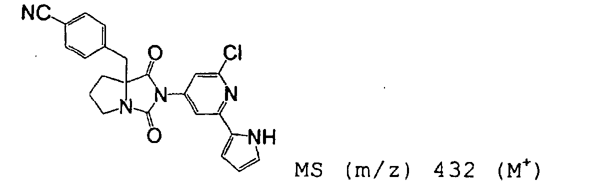

The title compounds were prepared from Example 95 and Example 96 respectively by removal of the BOC group with TFA.

### Example 100. 6-(4-Bromobenzyl)-8-(2,6-dichloro-4-pyridyl)-1,2,8-triazabicyclo[4.3.0] nonan- 7,9-dione

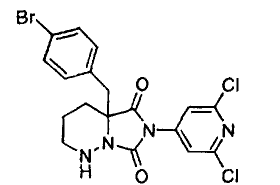

The titled compound was prepared in a manner analogous to Examples 53 and 54. MS m/z 470 (MH⁺); mp. 190 °C (dec).

Examples 101-105 were prepared in accordance with the following scheme.

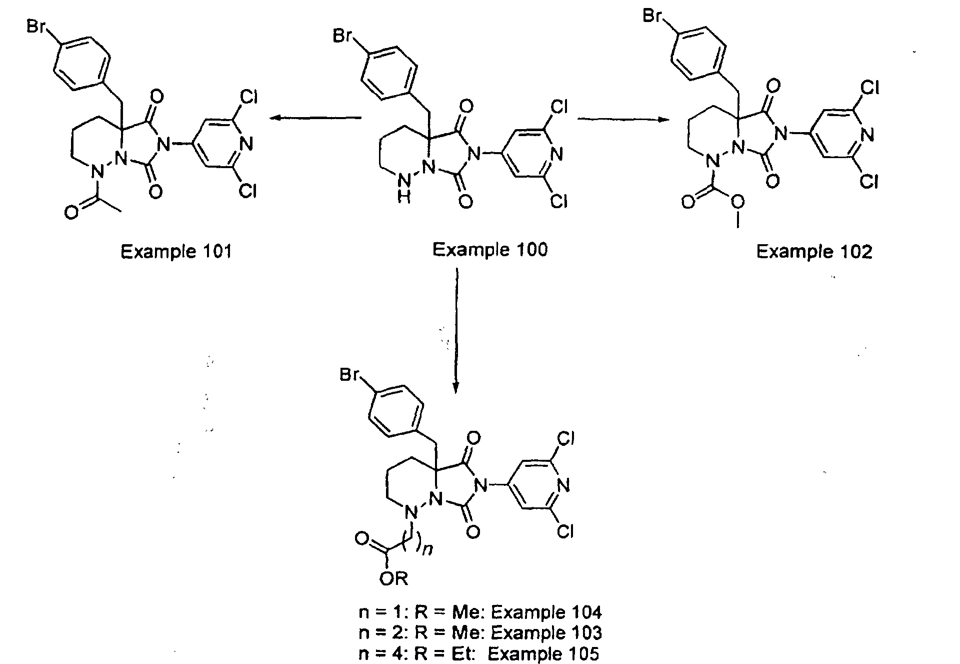

### Example 101. 6-(4-Bromobenzyl)-8-(2,6-dichloro-4-pyridyl)-2-acetyl-1,2,8-triazabicyclo[4.3.0]nonane-7,9-dione.

To a solution of the compound from Example 100 (80 mg) in dry THF (2 mL) under N₂ was added DIEA (0.073 mL) followed by AcCl (0.024 mL) and the reaction mixture stirred at room temperature for 18 hours. Water (10 mL) and EtOAc (10 mL) were added and the mixture was shaken. The aqueous phase was then separated and extracted with EtOAc. The combined organics were dried over MgSO₄, filtered and concentrated *in vacuo.* The residue was purified by chromatography (silica gel; EtOAc/hexanes; chromatotron) to give the titled compound (71 mg). MS (m/z) 513 (MH⁺); mp. 91 °C.

### Example 102. 6-(4-Bromobenzyl)-8-(2,6-dichloro-4-pyridyl)-2-methoxycarbonyl-1,2,8-triazabicyclo[4.3.0]nonane-7,9-dione.

To a solution of the compound from Example 100 (60 mg) in dry THF (2 mL) was added DIEA (0.044 mL), methylchloroformate (0.02 mL) and DMAP (catalytic amount). The reaction mixture was heated at 80 °C for 3 days during which more methylchloroformate (0.1 mL) was added after 24 hours and 48 hours. Water (10 mL) and EtOAc (10 mL) were added and the mixture shaken. The aqueous phase was separated and extracted with EtOAc. The combined organics were dried over MgSO₄, filtered and concentrated *in vacuo.* The residue was purified by chromatography (silica gel; EtOAc/hexanes; chromatotron) to give the titled compound (37 mg). MS (m/z) 529 (MH⁺); Mp. 81 °C.

### Example 103. 6-(4-Bromobenzyl)-8-(2,6-dichloro-4-pyridyl)-2-[2-(methoxycarbonyl)ethyl]-1,2,8-triazabicyclo[4.3.0]nonane-7,9-dione.

The compound from Example 100 (80 mg) was dissolved in methyl 3-bromopropanoate (0.5mL) and DIEA (0.089 mL) and heated at 73 °C for 3 days. The reaction mixture was concentrated and purified by chromatography (silica gel; EtOAc/hexanes; chromatotron) to give the titled compound (38 mg). MS (m/z) 557 (MH⁺); mp. 157 °C.

The following compounds were prepared in a similar manner.

### Example 104. 6-(4-Bromobenzyl)-8-(2,6-dichloro-4-pyridyl)-2-methoxycarbonylmethyl-1,2,8-triazabicyclo[4.3.0]nonane-7,9-dione: MS (m/z) 543 (MH⁺).

### Example 105. 6-(4-Bromobenzyl)-8-(2,6-dichloro-4-pyridyl)-2-[4-(ethoxycarbonyl)butyl]-1,2,8-triazabicyclo[4.3.0]nonane-7,9-dione: MS (m/z) 599 (MH⁺).

### Example 106. 5-(4-Cyanobenzyl)-3-(3,5-dichlorobenzyl)-1,3-diazabicyclo[3.3.0]octane-2,4-dione

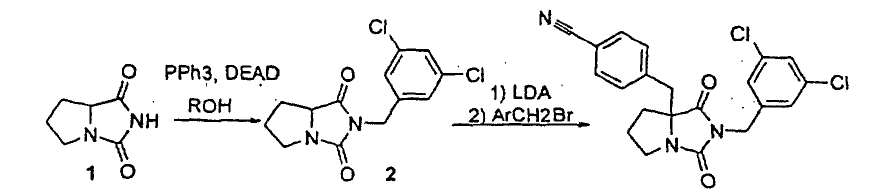

Step 1: To a solution of 1,3-diazabicyclo[3.3.0]octane-2,4-dione 1 (500 mg, *J, Med. Chem*., 1995, *38*, 3566), PPh₃ (1.2 g) and 3,5-dichlorobenzyl alcohol (690 mg) in THF (10 mL) at 0°C was added DEAD (0.7 mL) drop-wise over 45 minutes. The mixture was allowed to warm to room temperature and H₂O /EtOAc (50 mL each) were added and the mixture was shaken. The aqueous phase was then separated and extracted with EtOAc. The combined organics were dried over MgSO₄, filtered and concentrated *in vacuo*. The residue was purified by chromatography (SiO₂, EtOAc/Hexanes) and recrystallization from EtOH/H₂O to give the desired N-benzyl derivative **2** (550 mg). MS (m/z) 299 (M⁺).
Step 2: The alkylation was done in a manner similar to Example 56 to give the titled compound.
   MS (m/z) 414 (M⁺); mp. 124 °C.

The following compounds were prepared in a manner analogous to Example 106.

### Example 107: 5-(4-Cyanobenzyl)-3-[2-(3-chlorophenyl)ethyl]-1,3-diazabicyclo[3.3.0]octane-2,4-dione

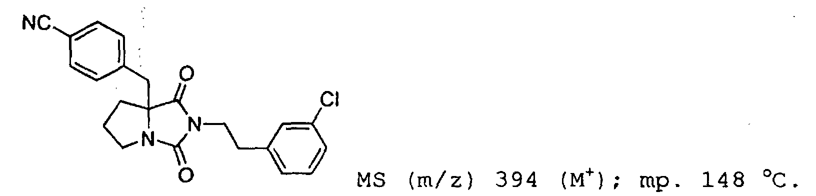

### Example 108: 5-(4-Cyanobenzyl)-3-(4-pyridylmethyl)-1,3-diazabicyclo[3.3.0]octane-2,4-dione

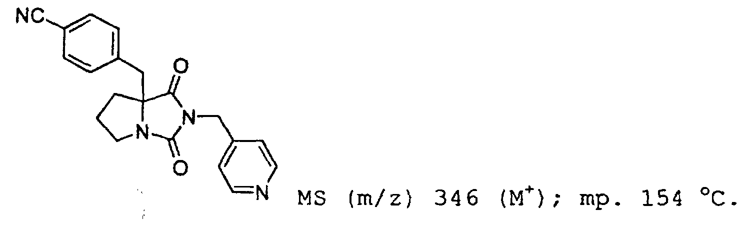

### Example 109. 5-(4-Bromobenzyl)-3-(3,4-dichlorobenzoyl)-1,3-diazabicyclo[3.3.0]octane-2,4-dione

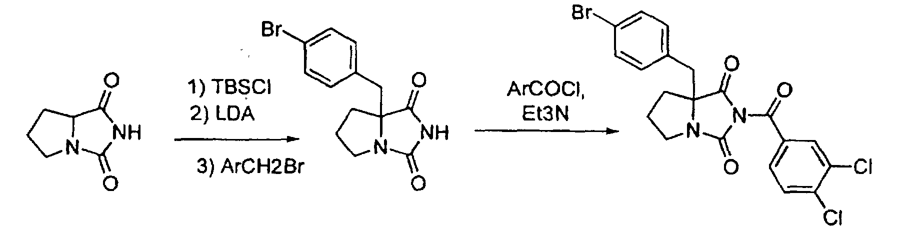

Step 1: To a solution of 1,3-diazabicyclo[3.3.0]octane-2,4-dione (5.24 g) in DMF (50 mL) was added Et₃N (6.2 mL) and tBDMSCl (6.2 g). The reaction mixture was stirred at room temperature for 2 hours whereupon water (30 mL) and DCM/hexanes (1/9, 100 mL) were added and the mixture was shaken. The aqueous phase was separated and extracted with DCM/hexanes (1/9). The combined organic layers were washed with water, dried over MgSO₄, filtered and concentrated *in vacuo* to give the protected imide (9.9 g) which was used without further purification. The alkylation was done in a manner similar to Example 106 to yield the desired benzylated material.
Step 2: To a solution of the compound from step-1 (100 mg), and Et₃N (0.054 mL) in THF (2 mL) was added 3,4-dichlorobenzoylchloride (74 mg). The reaction mixture was stirred at room temperature for 4 hours, concentrated *in vacuo* and purified by chromatography (silica gel; EtOAc/hexanes; Chromatotron) to give the titled compound (54 mg). MS (m/z) 483 (MH⁺); mp. 158 °C.

### Example 110. 5-(4-Bromobenzyl)-3-(3,5-dichlorophenyl)-7,7-difluoro-1,3-diazabicyclo[3.3.0]octane-2,4-dione:

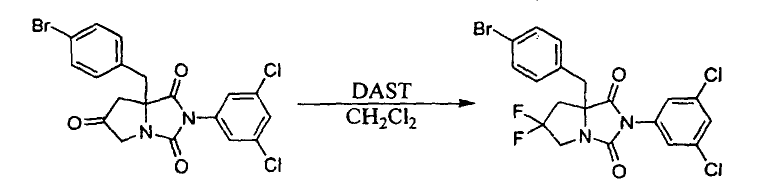

In a plastic vial, the compound from Example 35 (167 mg) was dissolved in anhydrous CH₂Cl₂ (5 mL) and DAST (47 µL) was added. The reaction mixture was stirred at room temperature for 6 hours, followed by refluxing for 2 hours. The reaction mixture was then cooled to room temperature and 10 % NaHCO₃ (2 mL) was added. The mixture was extracted with EtOAc and the combined organic layers were dried over Na₂SO₄, filtered and evaporated. The residue was purified by chromatography (SiO₂, hexanes to 2:1 hexanes/EtOAc gradient elution) and HPLC (¹⁸C-Waters 40 x 210 mm, 1% HOAc/CH₃CN gradient) provided the titled compound (38 mg). MS (m/z) 488 (MH⁺) mp. 160.3 °C.

### Example 111. 5-(4-Cyano-α-hydroxybenzyl)-3-(3,5-dichlorophenyl)1,3-diazabicyclo[3.3.0]octane-2,4-dione

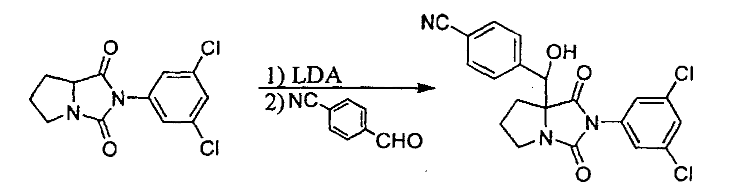

To a solution of 3-(3,5-dichlorophenyl)-1,3-diazabicyclo[3.3.0]octane-2,4-dione, (5.63 g) in anhydrous THF (20 mL) at -78 °C was added LDA prepared from n-BuLi (13.6 mL) and diisopropylamine (3.33 mL). The reaction mixture was stirred at -78 °C for 30 minutes then at 0 °C for 1 hour. To this mixture was added in one portion 4-cyanobenzaldehyde (3.64 g). The reaction mixture was allowed to warm to room temperature for 3 hours then poured onto 1 N HCl (50 mL). The mixture was extracted with EtOAc and the combined organic layers were dried over Na₂SO₄, filtered and the solvent evaporated. The residue was purified by chromatography (SiO₂, hexanes to 1:1 hexanes/EtOAc gradient elution) and HPLC (¹⁸C-Waters 40 x 210 mm, 1% HOAc/CH₃CN gradient) to provide the titled product (5.5 g). MS (m/z) 416 (MH⁺); mp. 184.4 °C.

### Example 112. 5-(4-Cyanobenzoyl)-3-(3,5-dichlorophenyl)-1,3-diazabicyclo[3.3.0]octane-2,4-dione

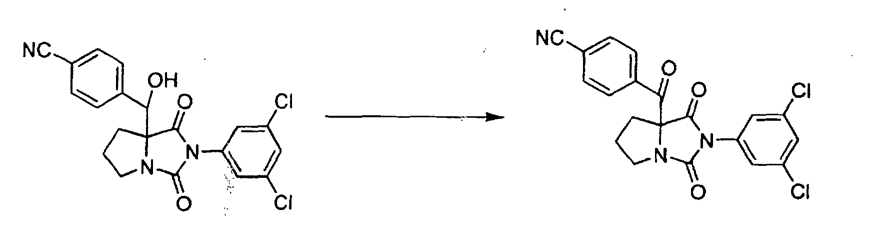

PCC (2.43 g) was added to a solution of the compound from Example 111 (3.90 g) in CH₂Cl₂ (20 mL) and the reaction mixture stirred at room temperature for 48 hours. The mixture was filtered though a plug of SiO₂ (washing with 1:1 CH₂Cl₂:EtOAc). The solvent was evaporated and the residue was purified by chromatography (SiO₂, hexanes to 1:1 hexanes/EtOAc gradient elution) providing the titled compound (1.97 g). MS (m/z) 414 (MH⁺). mp. 72.1 °C.

### Example 113. 5-(4-Cyano-α,α-difluorobenzyl)-3-(3,5-dichlorophenyl)-1,3-diazabicyclo[3.3.0]octane-2,4-dione

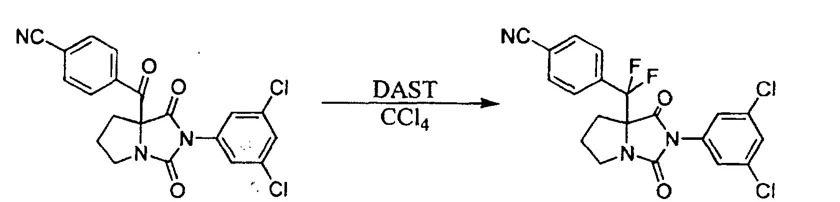

In a plastic tube, the compound from Example 112 (154 mg) was dissolved in CCl₄ (4 mL) and treated with DAST (98.3 µL). This reaction mixture was stirred for 22 hours at room temperature. The reaction mixture was quenched with 10 % NaHCO₃ (5 mL) and then extracted with EtOAc. The combined organics were dried over Na₂SO₄, filtered and the solvent evaporated. Chromatography of the residue (SiO₂, hexanes to 2:1 hexanes/EtOAc gradient elution) provided the titled compound (141 mg). MS (m/z) 434 (MH⁺). mp. 66 °C.

### Examples 114-116 were prepared in accordance with the following scheme (Scheme 12):

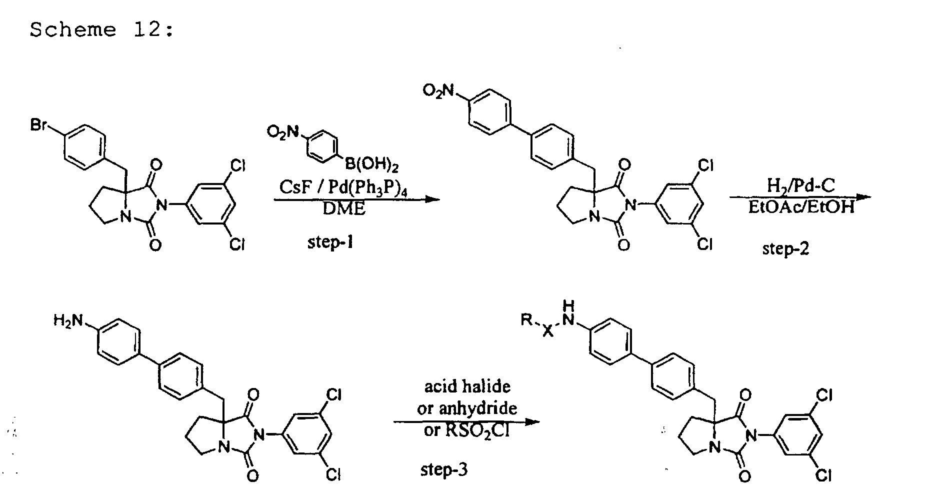

### Example 114. 5-[4-(4-Nitrophenyl)benzyl]-3-(3,5-dichlorophenyl)-1,3-diazabicyclo[3.3.0]octane-2,4-dione (Scheme 12, step 1) :

The titled compound was prepared in a manner similar to the compound in Example 7. MS (m/z) 496 (MH⁺). mp. 72-73 °C.

### Example 115. 5-[4-(4-Aminophenyl)benzyl]-3-(3,5-dichlorophenyl)-1,3-diazabicyclo[3.3.0]octane-2,4-dione (Scheme 12, step 2)

The compound from Example 114 (140 mg) was dissolved in EtOAc (15 mL) and EtOH (5 mL). The solution was degassed (pump/N₂ purge) to remove O₂. Pd-C (5 mg, 5% degussa type) was added and H₂ was bubbled through the solution for 5 minutes. The reaction mixture was stirred under H₂ for 4.5 hours. The mixture was filtered through celite (washing with EtOAc). The solvent was evaporated and the residue purified by HPLC (¹⁸C-Waters 40 x 210 mm, 1% HOAc/CH₃CN gradient) to provide the titled compound. MS (m/z) 466 (MH⁺). mp. 278 °C (dec.).

### Example 116. 5-[4-(4-Acetylaminophenyl)benzyl]-3-(3,5-dichlorophenyl)-1,3-diazabicyclo[3.3.0]octane-2,4-dione (Scheme 12, step 3)

The compound from Example 115 (38 mg) was dissolved in CH₂Cl₂ (1 mL) and pyridine (1 mL). To this solution was added Ac₂O (200 µL) and the reaction mixture was refluxed for 15 minutes. The solvent was evaporated and the residue was purified by chromatography (SiO₂, hexanes to 1:1 hexanes/EtOAc gradient elution) to provide the titled compound (41 mg). MS (m/z) 508 (MH⁺). mp. 123.9 °C.

The following compounds were prepared in a manner similar to Example 116:

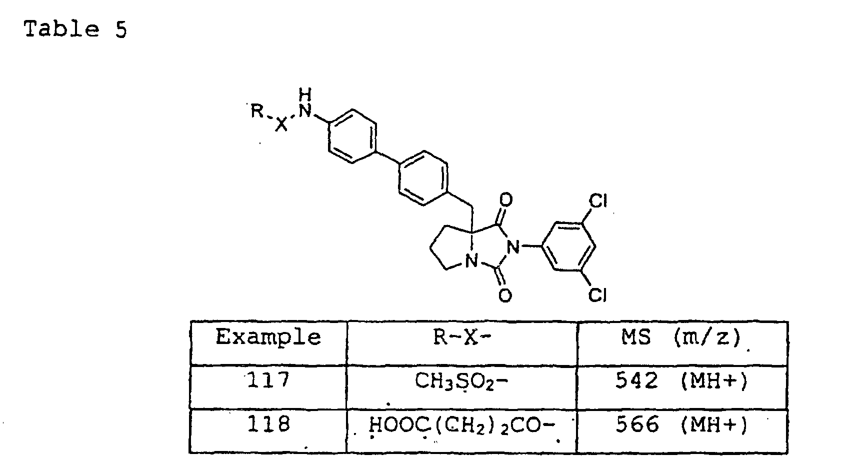

The corresponding 3-aminophenyl derivatives were prepared in an analogous manner.

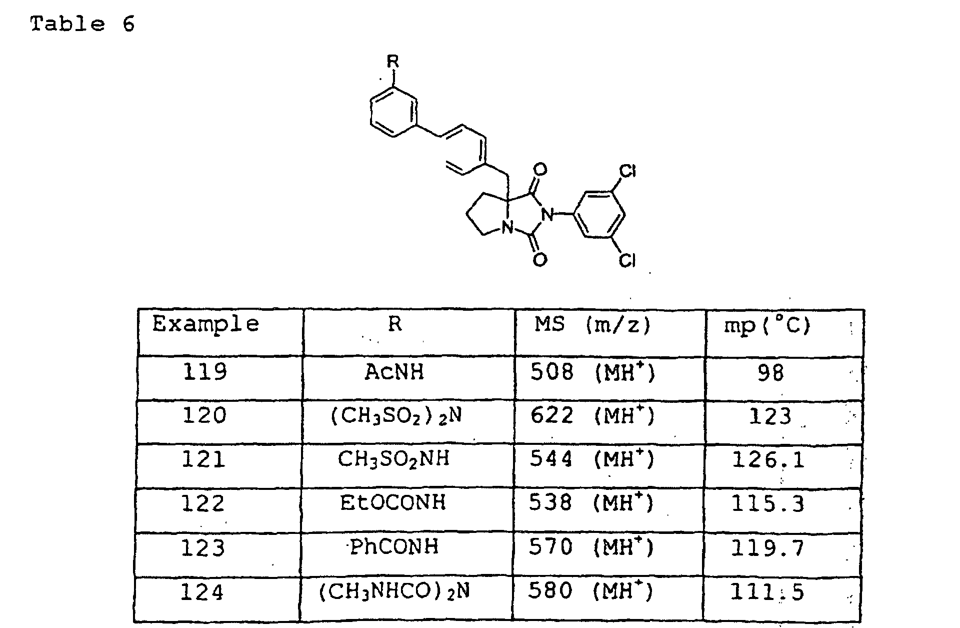

### Example 125: 5-[4-[3-(Phenylthioureido)phenyl]benzyl]-3-(3,5-dichlorophenyl)-1,3-diazabicyclo[3.3.0]octane-2,4-dione

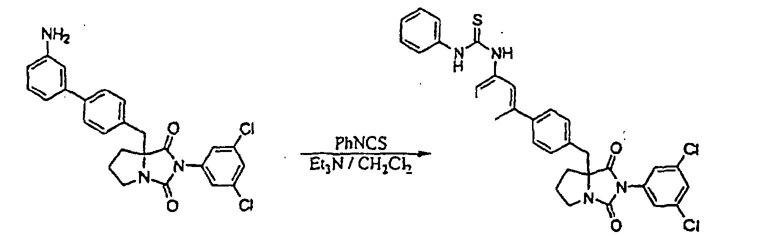

To a solution of the compound from Example 220 (100 mg) in CH₂Cl₂ (3 mL) and Et₃N (60 µL) was added PhNCS (31 µL) and the reaction mixture was stirred overnight at room temperature. The mixture was diluted with CH₂Cl₂ and then extracted in succession with 1N HCl, brine and NaHCO₃. The organic phase was dried over Na₂SO₄, filtered and the solvent was evaporated. The residue was purified, by chromatography (SiO₂, hexanes to 4:1 hexanes/EtOAc gradient elution) and reverse phase HPLC (¹⁸C, Waters 210 x 40 mm, 0.1 N HOAc to CH₃CN gradient) to provide the titled compound (35 mg). MS (m/z) 601 (MH⁺). mp. 128.6 °C.

### Example 126: 5-[4-[3-(Phenylureido)phenyl]benzyl]-3-(3,5-dichlorophenyl)-1,3-diazabicyclo[3.3.0]octane-2,4-dione

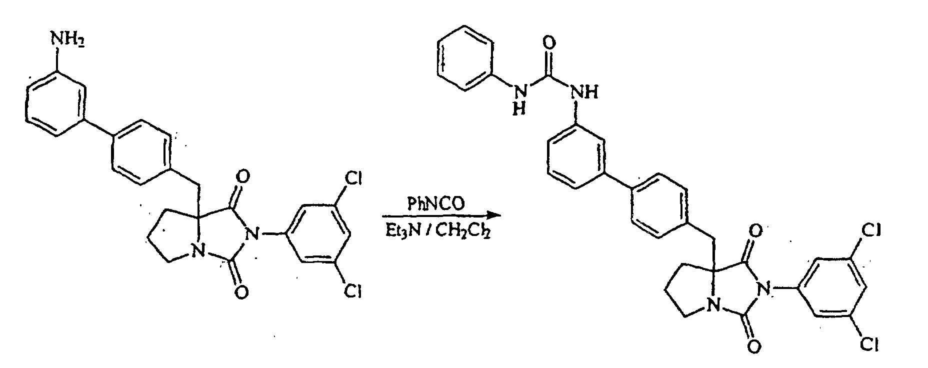

To a solution of the compound from Example 220 (100 mg) in CH₂Cl₂ (3 mL) and Et₃N (60 µL) was added PhNCO, (29 µL) and the reaction mixture was stirred overnight at room temperature. The mixture was diluted with CH₂Cl₂ and then extracted in succession with 1N HCl, brine and NaHCO₃. The organic phase was dried over Na₂SO₄, filtered and the solvent was evaporated. The residue was purified by chromatography (SiO₂, hexanes to 4:1 hexanes/EtOAc gradient elution) to provide the titled compound (95 mg). MS (m/z) 585 (MH⁺). mp. 150.2 °C.

### Example 127: 5-[4-[3-(2-oxo-1-pyrrolidinyl)phenyl]benzyl]-3-(3,5-dichlorophenyl)-1,3-diazabicyclo[3.3.0]octane-2,4-dione

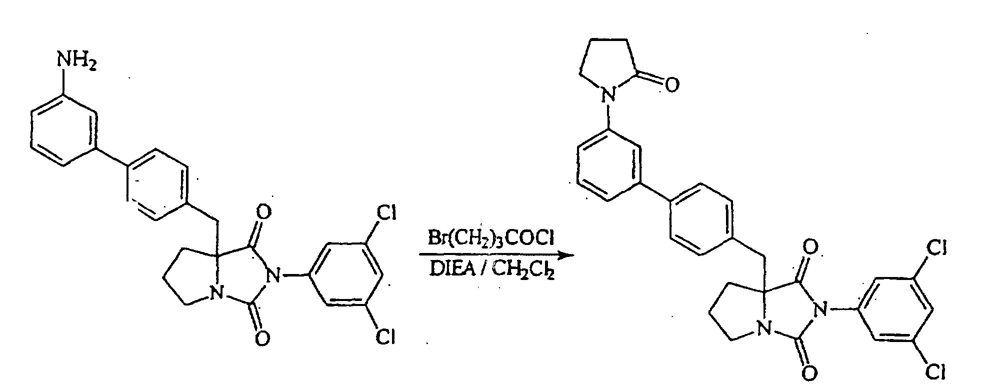

To a solution of the compound from Example 220 (100 mg) in CH₂Cl₂ (3 mL) and DIEA (75 µL) was added Br(CH₂)₃COCl (30 µL) and the reaction mixture was stirred for 48 hours at room temperature. The mixture was diluted with CH₂Cl₂ and then extracted in succession with 1N HCl, brine and NaHCO₃. The organic phase was dried over Na₂SO₄, filtered and the solvent was evaporated. The residue was purified by chromatography was evaporated. The residue was purified by chromatography (SiO₂, hexanes to 4:1 hexanes/EtOAc gradient elution) to provide the titled compound (40 mg). MS (m/z) 534 (MH⁺). mp. 86.5 °C.

### Example 128. 5-[4-(5-tetrazolyl)benzyl)-3-(3,5-dichlorophenyl)-1,3-diazabicyclo[3.3.0]octane-2,4-dione:

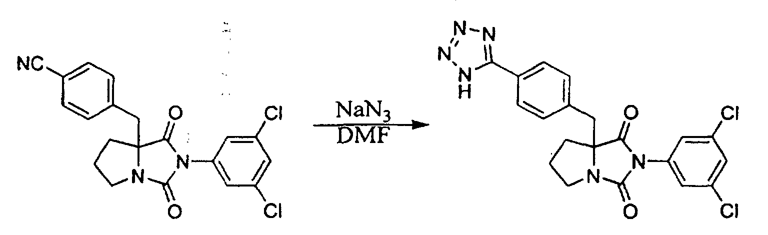

NaN₃ (64.5 mg) was added to a solution of Example 21 (137 mg) in DMF (3 mL). The reaction mixture was sealed and heated at 140 °C for 72 hours. The reaction mixture was cooled to room temperature and the solvent removed under reduced pressure. The residue was purified by HPLC (¹⁸C-Waters 40 x 210 mm, 1% HOAc/CH₃CN gradient) to provide the titled compound (27 mg). MS (m/z) 457 (MH⁺). mp. 194.8 °C.

### Example 129. 5-(4-Amidinobenzyl)-3-(3,5-dichlorophenyl)-1,3-diazabicyclo[3.3.0]octane-2,4-dione:

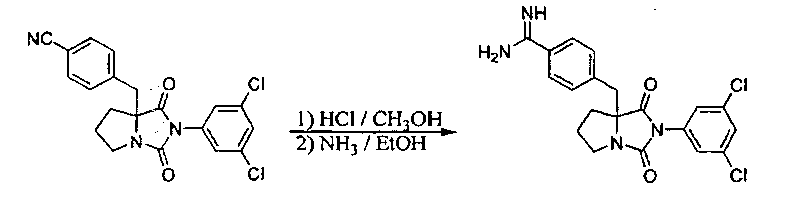

Anhydrous HCl was bubbled through a solution of Example 21 (219 mg) in CH₃OH (10 mL) for 15 minutes at 0 °C. The reaction mixture was sealed in a pressure tube and allowed to stir for 48 hours at room temperature. Evaporation of the solvent followed by repeated dissolution and re-evaporation with CH₃OH provided the intermediate imino ether. The imino ether was then treated with NH₃/EtOH (2 M, 20 mL), resealed and stirred for 48 hours. Evaporation of the solvent followed by purification by HPLC (¹⁸C-Waters 40 x 210 mm, 1% HOAc/CH₃CN gradient) provided the titled compound (177 mg). MS (m/z) 431 (MH⁺); mp. 187.6 °C.

### Example 130: 5-(4-Cyanobenzyl)-3-(2,6-dichloro-4-pyridyl)-1,3-diazabicyclo[3.3.0]octane-2,4-dione

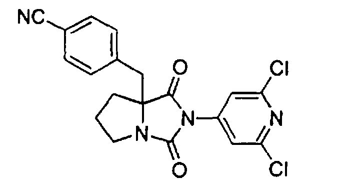

This compound was prepared in a manner similar to Example 21. MS (m/z) 401(M⁺). mp 96 C .

Examples 131-133 were prepared in accordance with the following scheme (Scheme 13):

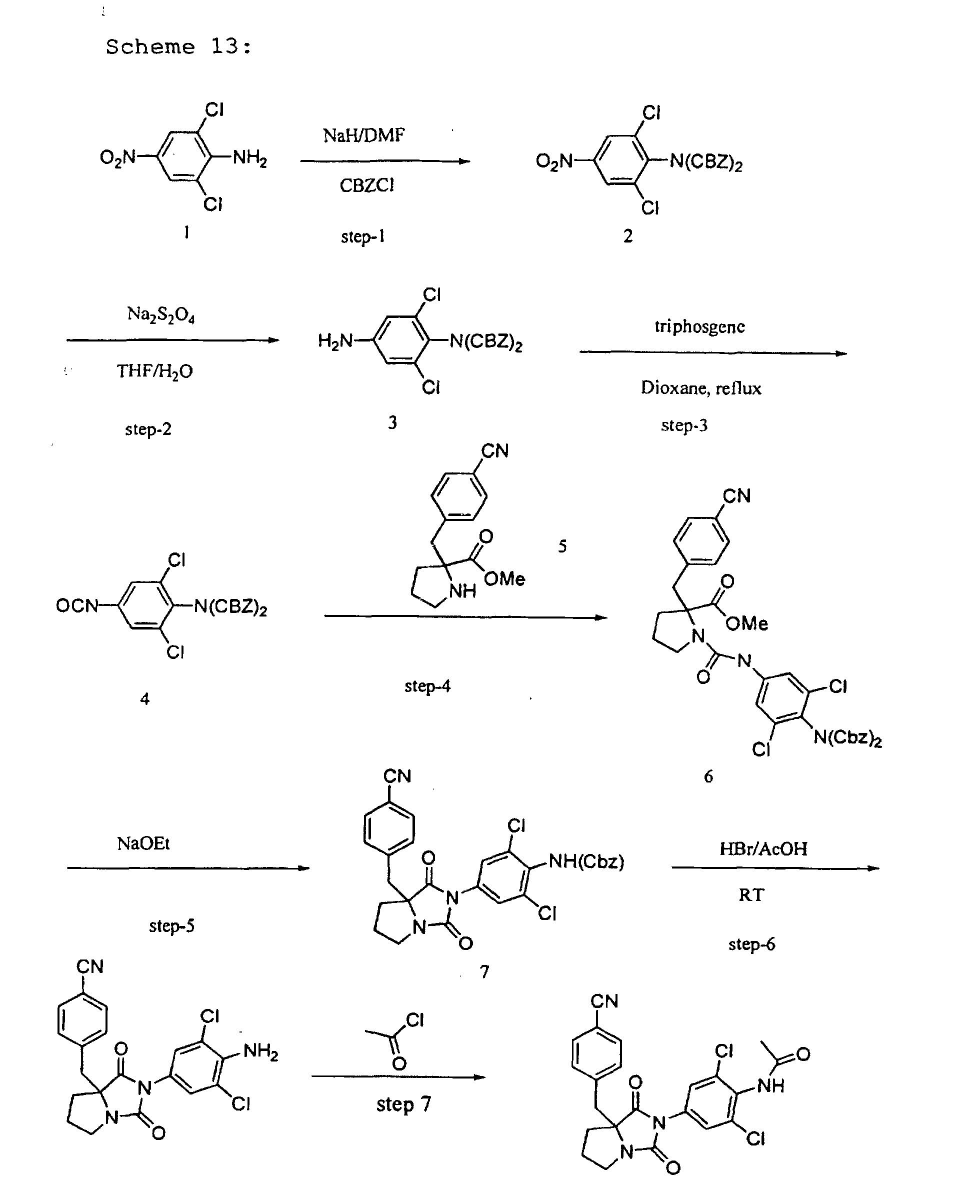

### Example 131. 5-(4-Cyanobenzyl)-3-(3,5-dichloro-4-benzyloxycarbonylaminophenyl)-1,3-diazabicyclo[3.3.0]octane-2,4-dione

Step-1. NaH (0.64 g, 60% in oil) was added to an ice-cold solution of the 2,6-dichloro-4-nitroaniline 1 (3 g) in DMF (20 mL) and the reaction mixture was stirred for 20 minutes. CbzCl (2.72 g) was added slowly and the mixture was allowed to warm up to room temperature and stirred for overnight. The solution was partitioned between EtOAc/H₂O. The combined organic layers were dried over Na₂SO₄, filtered and concentrated. The product was purified by flash chromatography on silica gel to yield the desired compound (2.7 g). MS (m/z) 475 (MH⁺).
Step-2. The CBZ protected aniline from above (2.7 g) was dissolved in THF/H₂O (15/10 mL). The mixture was cooled to 0 °C, followed by the addition of Na₂S₂O₄ (2.0 g). The reaction solution was stirred at 0 °C for 3 hours. The solution was extracted with EtOAc. The combined organic layers were dried over Na₂SO₄, filtered and concentrated. The product was purified by flash chromatography on silica gel to yield the desired compound (2.1g). MS (m/z) 445 (MH⁺) .
Step-3. Triphosgene (1.34g) was added to a solution of the compound from step-2 (2 g) in dioxane (20 mL). The reaction solution was heated under reflux for overnight. Solvent was evaporated under vacuum. The product was used for the next step without further purification.
Step-4. The compound from step-3 (1.23 g) was added to an ice-cold solution of the proline derivative (compound **5**, 0.58 g) in THF (10 mL) and the reaction mixture was stirred at 0 °C for 30 minutes. It was allowed to warm up to room temperature and stirred overnight. The reaction mixture was partitioned between EtOAc/H₂O. The combined organic layers were dried over Na₂SO₄, filtered and evaporated. The product was purified by flash chromatography on silica gel (1:1 EtOAc/Hexanes) to yield the titled compound (1.34 g). MS (m/z) 715 (MH⁺).
Step-5. The urea compound from step-4 (260 mg) was cyclized in a manner similar to Example 1, step-4, to yield the titled compound (140 mg). ESMS (m/z) 549 (MH⁺).

### Example 132. 5-(4-Cyanobenzyl)-3-(3,5-dichloro-4-aminophenyl)-1,3-diazabicyclo[3.3.0]octane-2,4-dione (Scheme 13, step 6)

The compound from Example 131 (100 mg) was dissolved in HBr (2 mL, 30% in AcOH). The resulting solution was stirred at room temperature for 30 minutes. The solution was extracted with EtOAc/H₂O. The combined organic layers were dried over Na₂SO₄, filtered and evaporated. The product was purified by preparative TLC to yield the titled compound (52 mg). MS (m/z) 415 (MH⁺). mp. 103 °C.

### Example 133. 5-(4-Cyanobenzyl)-3-(3,5-dichloro-4-acetylaminophenyl)-1,3-diazabicyclo[3.3.0]octane-2,4-dione (Scheme 13, step 7)

Acetyl chloride (10 µL ) was added to a solution of the compound of Example 132 (20 mg) and DIEA (2 drops) in THF (1 mL). The resulting solution was stirred at room temperature for 2 hours. The solution was partitioned with EtOAc/H₂O. The combined organic layers were dried over Na₂SO₄, filtered and concentrated. The product was purified by preparative TLC to yield the titled compound (15 mg). MS (m/z) 457 (MH⁺).

Examples 134-137 were prepared according to the following scheme (Scheme 14):

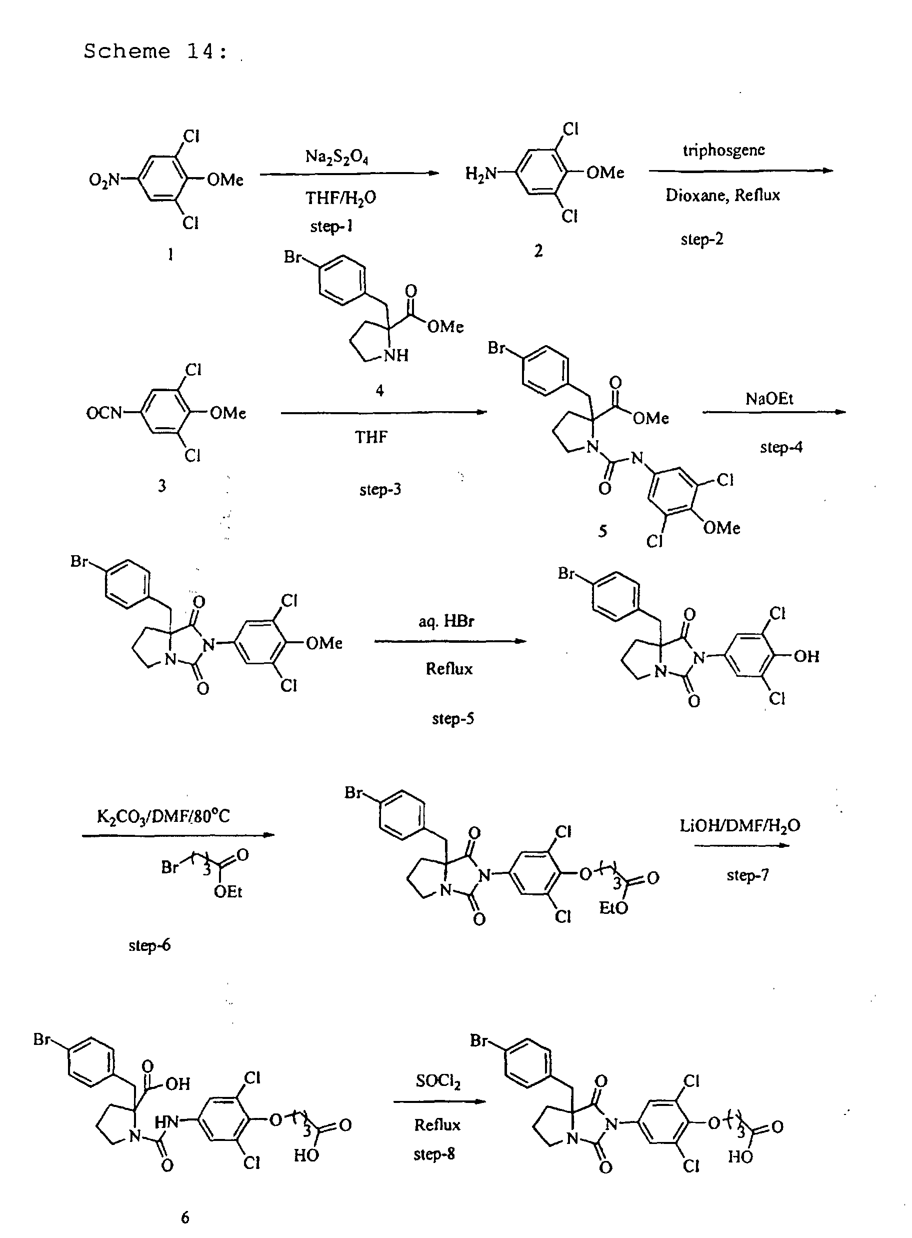

### Example 134. 5-(4-Bromobenzyl)-3-(3,5-dichloro-4-methoxyphenyl)-1,3-diazabicyclo[3.3.0]octane-2,4-dione.

Step-1. 2,6-Dichloro-4-nitroanisole (2 g) was reduced to the corresponding aniline (1.5 g) as described in the Example 131. MS (m/z) 192 (MH⁺).
Step-2. A solution of the above aniline (2.12 g) and triphosgene (3.28 g) in dioxane (20 mL) was refluxed for 24 hours. The solvent was evaporated and the product was used for next step without further purification.
Step-3. The above isocyanate (2.46 g) was added to an ice-cold solution of the proline derivative (3 g) in THF (20 mL). The resulting solution was stirred at 0 °C for 30 minutes and then allowed to warm up to room temperature and stirred overnight. The reaction mixture was partitioned between EtOAc/H₂O. The combined organic layers were dried over Na₂SO₄, filtered and evaporated. The product was purified by flash chromatography on silica gel (1:1 EtOAc/hexanes) to yield the desired compound (3.2 g). MS (m/z) 515 (MH⁺).
Step-4. The above urea (1.48 g) was cyclized with NaOEt (0.215 g) to yield the titled compound (1.1g). MS (m/z) 483 (MH⁺) .

### Example 135. 5-(4-Bromobenzyl)-3-(3,5-dichloro-4-hydroxyphenyl)-1,3-diazabicyclo[3.3.0]octane-2,4-dione (Schem 14, step 5)

The compound from Example 134 (0.8 g) was taken in aqueous HBr (10 mL) and the solution was refluxed overnight. The aqueous solution was extracted with EtOAc. The combined organic layers were dried over Na₂SO₄, filtered and evaporated. The residue was purified by flash chromatography on silica gel (1:1 EtOAc/Hexanes) to yield the titled compound (0.65 g). MS (m/z) 469 (MH⁺).

### Example 136. 5-(4-Bromobenzyl)-3-[3,5-dichloro-4-(3-ethoxycarbonylpropyloxy)phenyl]-1,3-diazabicyclo[3.3.0]octane-2,4-dione (Scheme 14, step 6)

Ethyl 4-bromobutyrate (28 mg) was added to a mixture of the compound from Example 135 (55 mg) and K₂CO₃ (18 mg) in 2 mL anhydrous DMF and the resulting mixture was heated at 80 °C for 5 hours. The solution was partitioned between EtOAc/H₂O. The combined organic layers were dried over Na₂SO₄, filtered and concentrated. The residue was purified by preparative TLC to yield the titled compound (61 mg). MS (m/z) 583 (MH⁺) .

### Example 137. 5-(4-Bromobenzyl)-3-[3,5-dichloro-4-(3-carboxypropyloxy)phenyl]-1,3-diazabicyclo[3.3.0]octane-2,4-dione (Scheme 14, step 7, 8)

LiOH (15 mg) was added to a solution of the compound from Example 136 (30 mg) in anhydrous DMF (1 mL) and the reaction mixture was stirred at room temperature for overnight. The solution was partitioned with EtOAc/H₂O. The combined organic layers were dried over Na₂SO₄, filtered and concentrated to dryness to give the diacid. MS (m/Z) 573 (MH⁺).

The dried diacid was taken in SOCl₂ (1 mL) and the solution was heated to reflux for 1 hour. SOCl₂ was evaporated. The residue was partitioned between EtOAc/1N HCl. The combined organic layers were dried over Na₂SO₄, filtered and evaporated. The residue was purified by preparative TLC (1:1 EtOAc/Hexanes) to give the titled compound. MS (m/z) 555 (MH⁺).

### Example 138: 5-(4-Bromobenzyl)-3-[3,5-dichloro-4-(benzyloxy)phenyl]-1,3-diazabicyclo[3.3.0]octane-2,4-dione

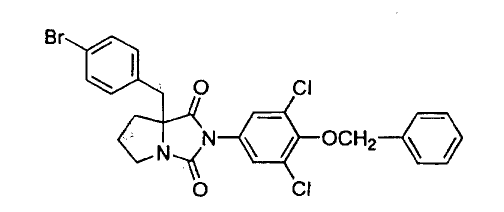

This compound was synthesized from Example 135 in a manner analogous to Example 136. MS (m/z) 559 (MH⁺); mp.62 °C.

### Example 139: 5-(4-Cyanobenzyl)-3-(3,5-dichloro-4-methoxyphenyl)-1,3-diazabicyclo[3.3.0]octane-2,4-dione

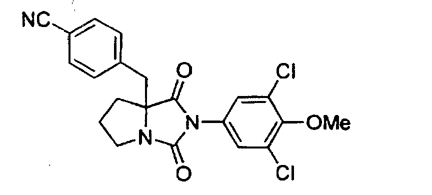

The titled compound was prepared in a manner similar to Example 134. MS m/z 430 (MH⁺). mp. 95.1 °C.

### Example 140: 5-(4-Bromobenzyl)-3-[3,5-dichloro-4-(5-ethoxycarbonylpentyloxy)phenyl]-1,3-diazabicyclo[3.3.0]octane-2,4-dione

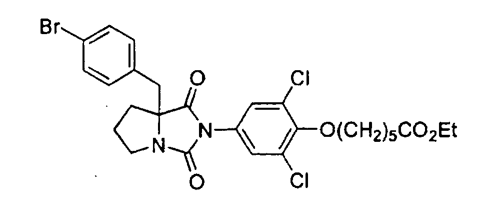

This compound was prepared in a manner similar to Example 136. MS m/z 612 (MH⁺).

### Example 141: 5-(4-Bromobenzyl)-3-[3,5-chloro-4-(5-carbxypentyloxy)phenyl]-1,3-diazabicyclo[3.3.0]octane-2,4-dione

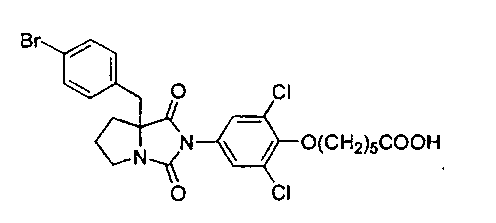

This compound was prepared in a manner similar to Example 137. MS m/z 584 (MH⁺).

### Example 142: 5-(4-Methoxycarbonylbenzyl)-3-(3,5-dichloro-4-hydroxyphenyl)-1,3-diazabicyclo[3.3.0]octane-2,4-dione

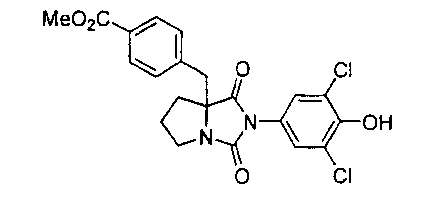

A mixture of the compound from Example 139 (20 mg) and aqueous HBr (5 mL) was heated under reflux for 40 minutes. EtOAc was added and the organic layer was separated. The aqueous layer was extracted with EtOAC. The combined organic layers were dried over Na₂SO₄, filtered and evaporated. The residue was purified by preparative TLC (EtOAc). The obtained material was dissolved in MeOH (2 mL) and few drops of SOCl₂ were added. The reaction mixture was heated under reflux for 1 hour. The residue was partitioned between EtOAc and water. The organic layer was dried over Na₂SO₄, filtered and concentrated. The product was purified via preparative TLC. MS m/z 449 (MH⁺) . mp. 105.3 °C .

### Example 143: 5-[4-((L)-N²-asparaginocarbonyl)benzyl]-3-(3,5-dichlorophenyl)-1,3-diazabicyclo[3.3.0]octane-2,4-dione t-butyl ester

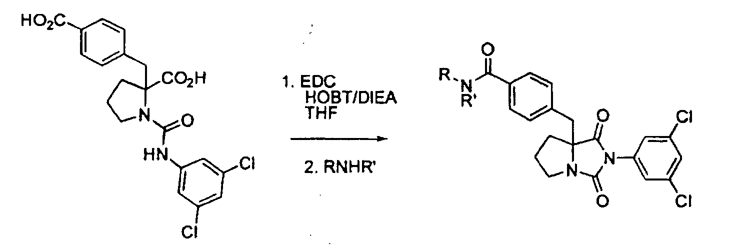

To a solution of the diacid-urea (compound from Example 23, step-1, 145.1 mg) in THF (10 mL) and DMF (1 mL) was added EDC (193 mg), HOBT (130 mg) and *N,N*-diisopropylethylamine (300 µL). Upon stirring under Ar at room temperature for 8 hours, L-asparagine *tert*-butyl ester (116 mg) was added. Stirring continued overnight. The solution was partitioned between EtOAc/HCl (0.5 N). The EtOAc layer was separated and washed successively with water, NaHCO₃ (saturated), and brine. It was dried over Na₂SO₄, filtered and concentrated. The residue was purified by chromatography (SiO₂, hexanes/EtOAc gradient elution) to provide the titled compound (177.8 mg). MS m/z 589 (MH⁺). mp. 144.2 °C .

The following compounds were prepared in an analogous manner. The free acids were obtained via TFA deprotection of the t-butyl esters.

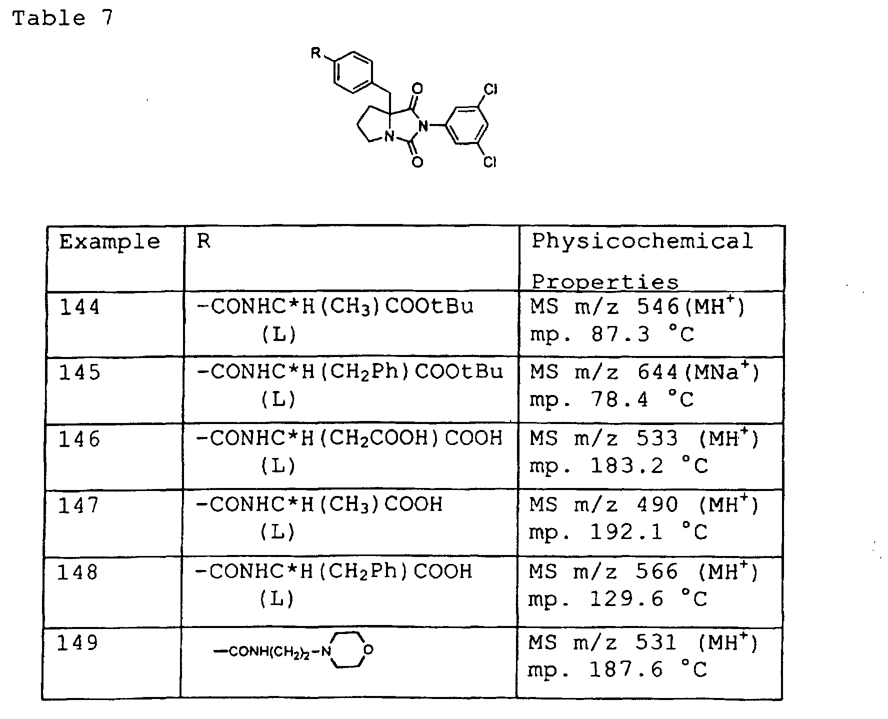

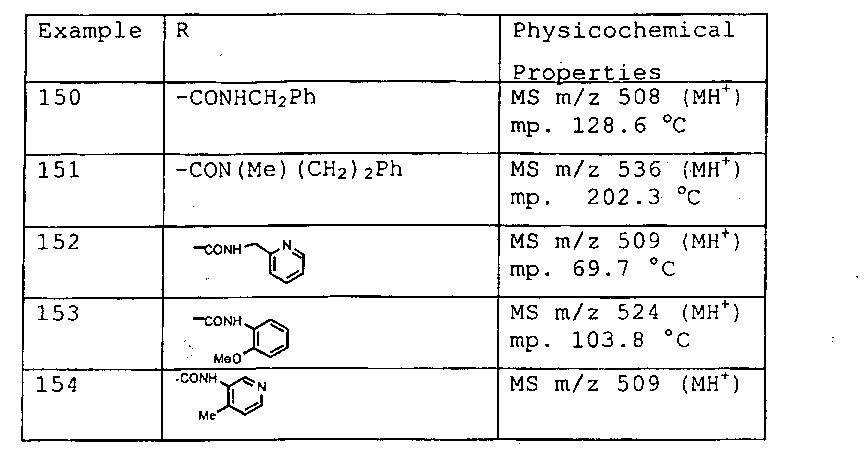

### Example 155: 6-(4-Methoxycarbonylbenzyl)-8-(3,5-dichlorophenyl)-1,8-diazabicyclo[4.3.0]nonane-7,9-dione

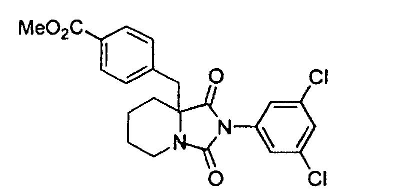

This compound was prepared by a method similar to Example 21 starting from pipecolic acid. MS m/z 447 (MH⁺). mp. 51 °C.

The following compounds were prepared starting from Example 155 by following methodology similar to Example 23.

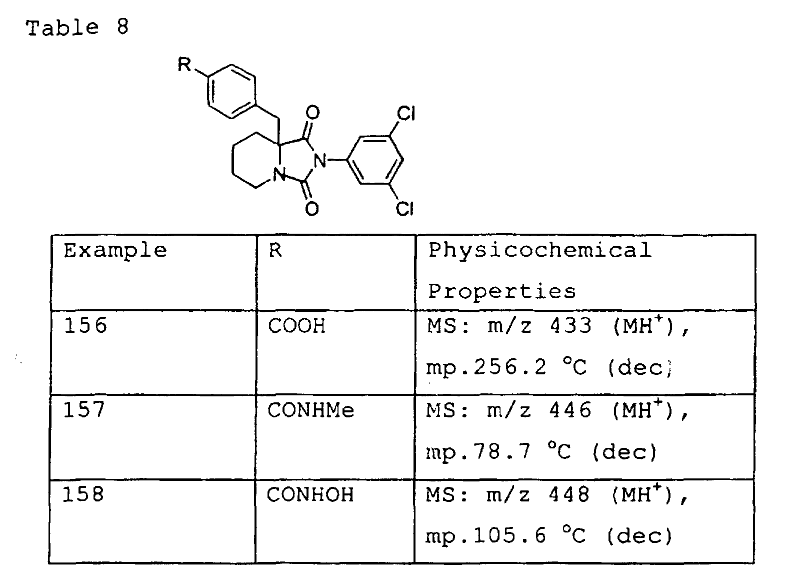

### Example 159: 6-[4-(4,4-Dimethyl-4,5-dihydro-2-oxazolyl)benzyl]-8-(3,5-dichlorophenyl)-1,8-diazabicyclo[4.3.0]nonane-7,9-dione

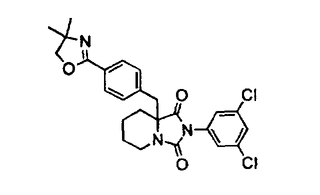

Step-1. Thionyl chloride (2 mL) was added to Example 156 (0.644 g) and the mixture was heated at 100 °C for 2 minutes and stirred overnight at room temperature. The mixture was evaporated and the residue was dried under high vacuum to give the acid chloride. This was used as is for the next step (0.655 g). MS: m/z 451 (MH⁺).
Step-2. A mixture of the above acid chloride (0.425 g) and 2-amino-2-methyl-1-propanol (0.27 mL) in CH₂Cl₂ (5 mL) was stirred at room temperature for 24 hours. The solvent was evaporated and the residue was dried under high vacuum. Thionyl chloride (5 mL) was added and the solution was warmed to 50 °C for 30 minutes. The mixture was evaporated and the residue was purified via HPLC (CH₃CN/0.1 N HOAc) to give the desired compound (30 mg). MS: m/z 486 (MH⁺), mp. 87.2 °C.
The following compounds (Examples 160 -162) were synthesized by following methods similar to Examples 26 and 27.

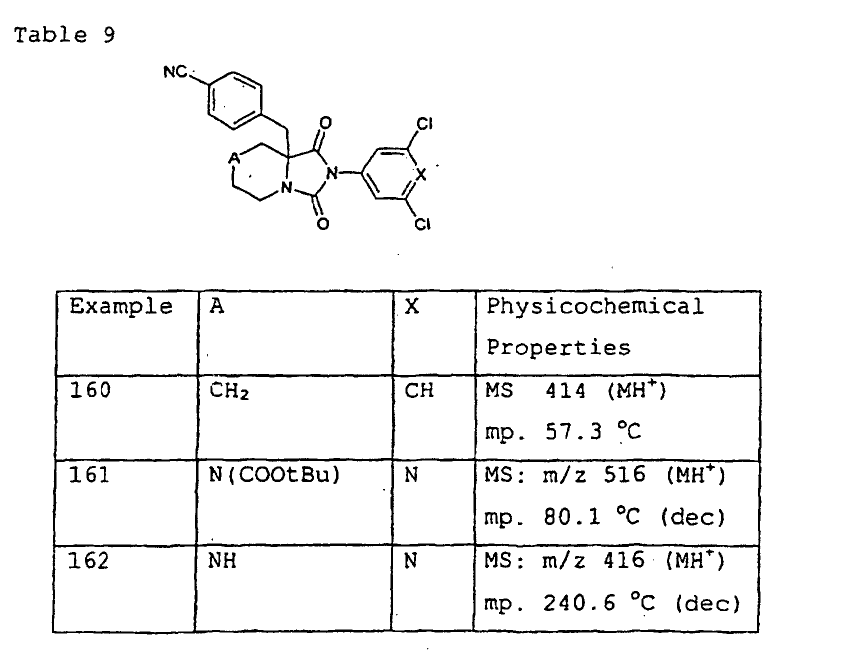

### Example 163: 6-(4-Cyanobenzyl)-4-(3-carboxypropionyl)-8-(2,6-dichloro-4-pyridyl)-1,4,8-triazabicyclo[4.3.0]nonane-7,9-dione

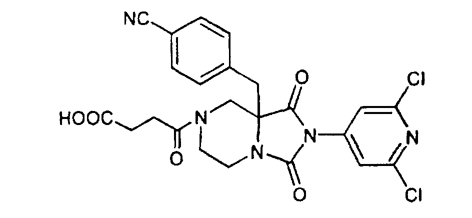

This compound was prepared from Example 162 in a manner similar to Example 64. MS m/z 516 (MH⁺); mp. 100.9 °C (dec)

The compound from Example 163 was converted to the following compounds in a manner similar to Example 69.

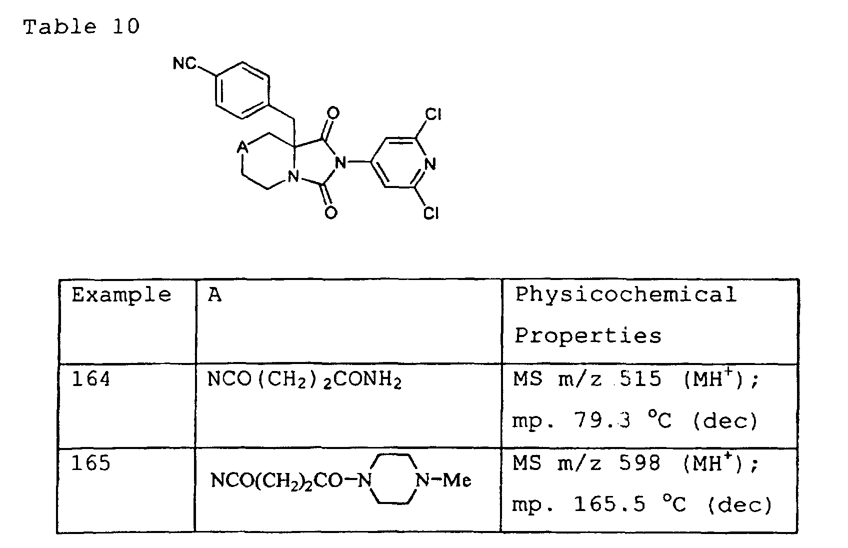

### Example 166: 6-(4-Cyanobenzyl)-4-dimethylaminoacetyl-8-(2,6-dichloro-4-pyridyl)-1,4,8-triazabicyclo[4.3.0]nonane-7,9-dione

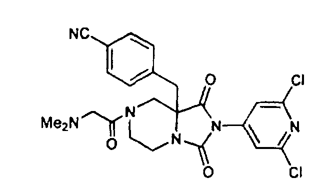

This compound was prepared in a manner similar to Example 31. MS m/z 501 (MH⁺). HCl salt, mp. 260.8 °C (dec).

### Example 167: 6-(4-Bromobenzyl)-8-(3,5-dichlorophenyl)-1,8-diaza-4-thiabicyclo[4.3.0]nonane-7,9-dione

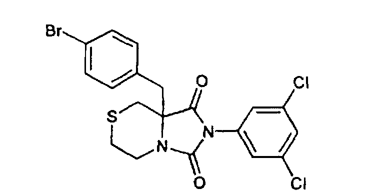

This compound was prepared starting from thiazine 2-carboxylic acid in a manner similar to Example 26.

The following compounds were obtained from Example 167 via oxidation with mCPBA.

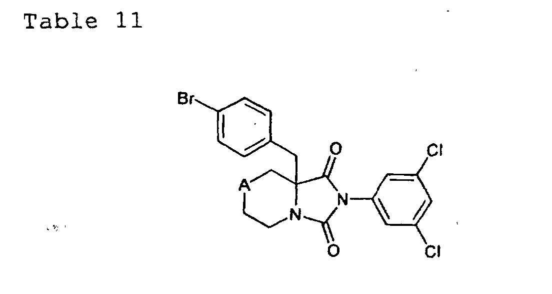

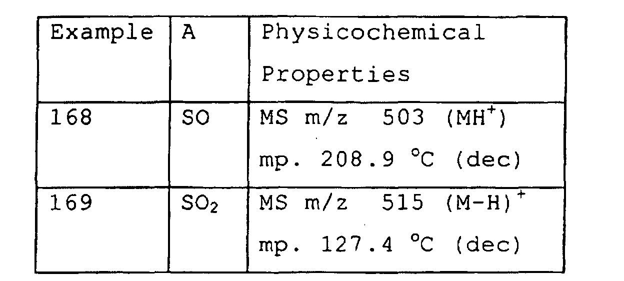

The compounds in the following tables (Tables 12, 13 and 14) were prepared in a manner similar to Example 21 by following Method B.

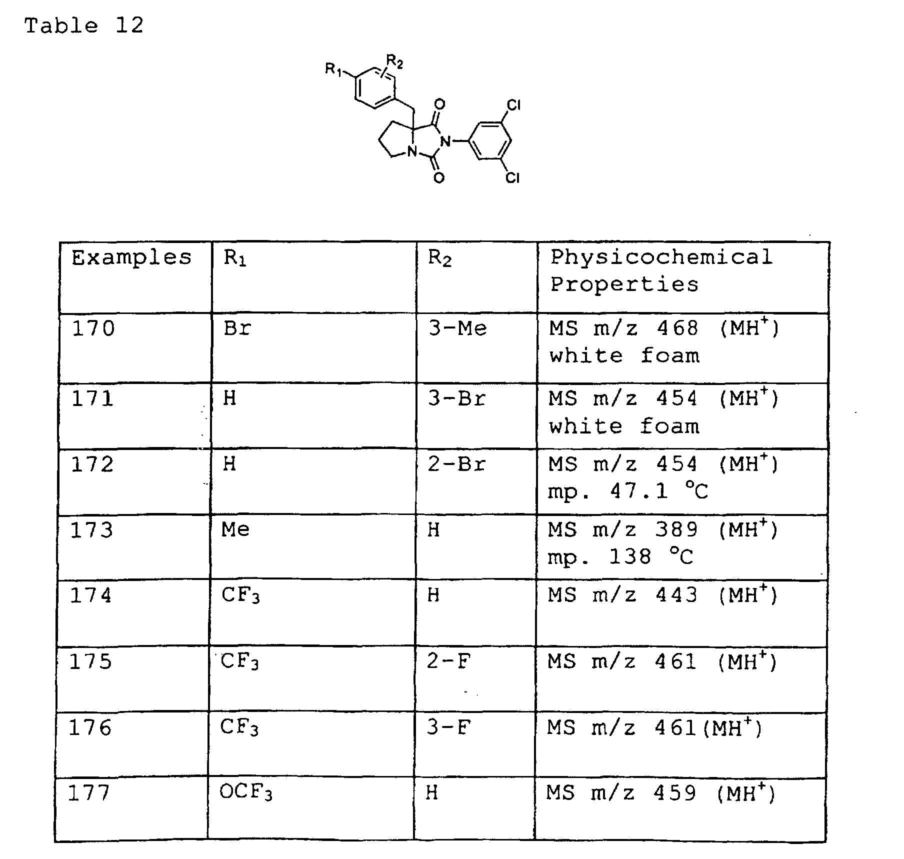

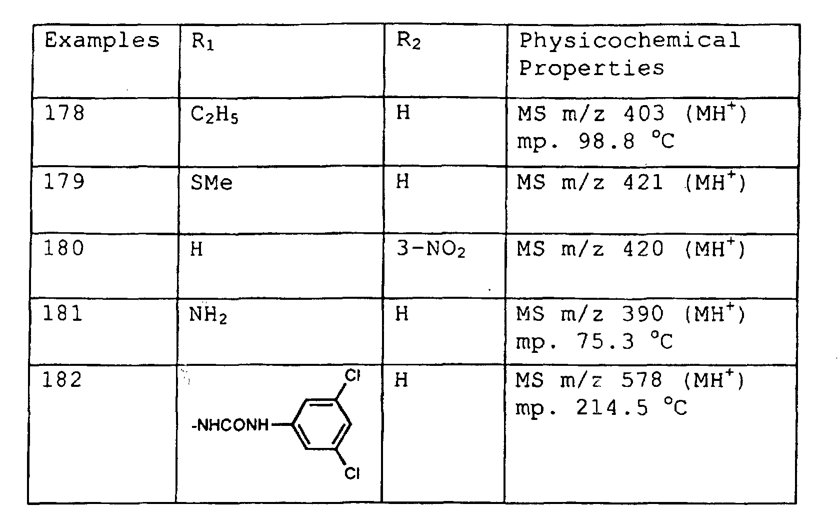

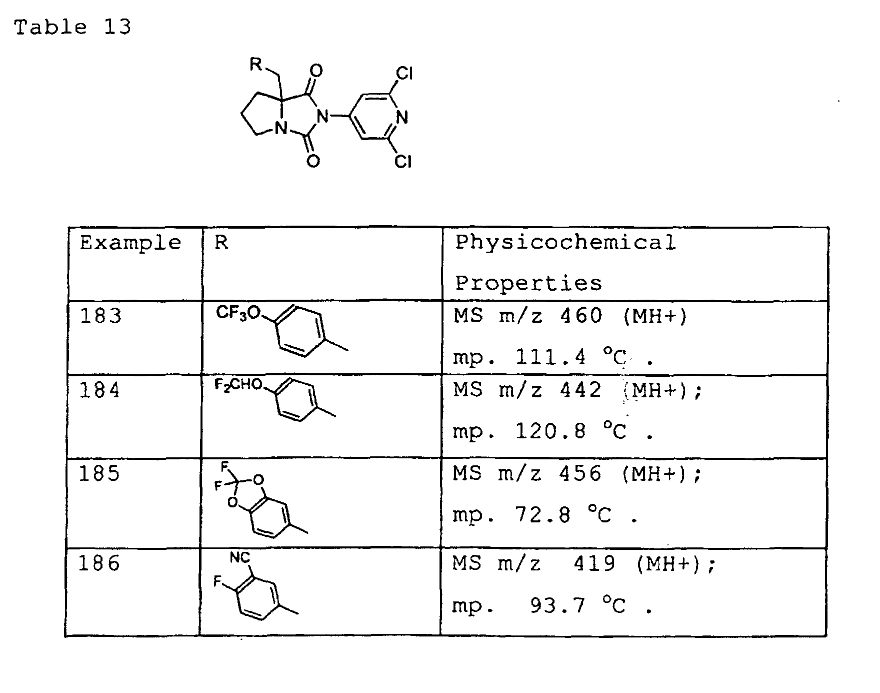

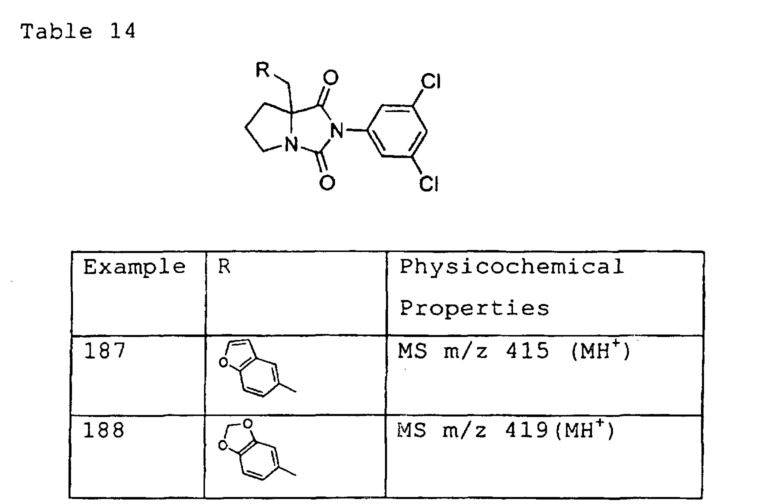

### Example 189: 5-(4-Methylsulfinylbenzyl)-3-(3,5-dichlorophenyl)-1,3-diazabicyclo[3.3.0]octane-2,4-dione; and

### Example 190: 5-(4-Methylsulfonylbenzyl)-3-(3,5-dichlorophenyl)-1,3-diazabicyclo[3.3.0]octane-2,4-dione.

MCPBA (95 mg) was added to a solution of Example 179 (0.15 g) in CH₂Cl₂ (10 mL) and the solution was stirred for 15 hours at room temperature. EtOAc was added and the combined solution was washed successively with saturated NaHCO₃, water and brine. The solution was dried and evaporated. The residue was purified via HPLC to yield the titled compounds.

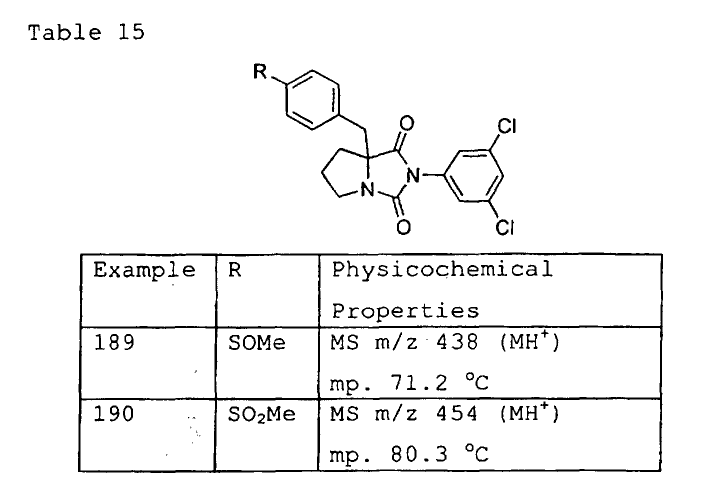

The following compounds were prepared in a manner similar to Example 56, step 4.

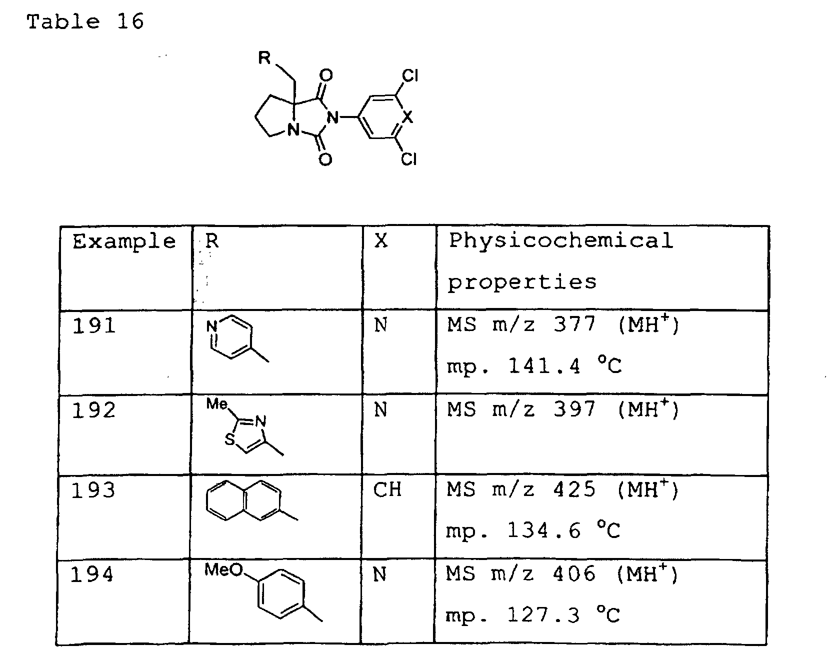

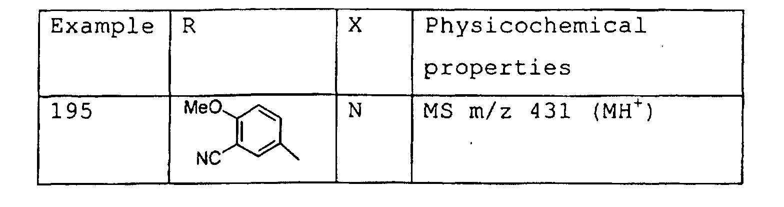

### Example 196: 5-(4-Hydroxybenzyl)-3-(2,6-dichloro-4-pyridyl)-1,3-diazabicyclo[3.3.0]octane-2,4-dione

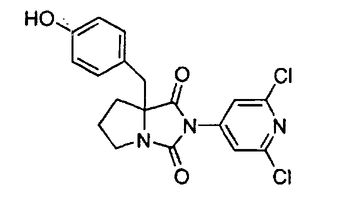

A solution of BBr₃ in CH₂Cl₂ (1 M, 8 mL) was added dropwise to an ice-cold solution of the compound from Example 194 (1.10 g) in CH₂Cl₂ (30 mL) with stirring. The slurry was stirred at 0 °C for 30 minutes and at room temperature for an additional 30 minutes. The reaction mixture was quenched with water and diluted with EtOAc. The solution was washed successively with water, saturated NH₄Cl, brine, dried and evaporated to give the desired product (0.96 g) . mp. 164.9 °C ; MS m/z 393 (MH⁺).

### Example 197: 5-(3-Cyano-4-hydroxybenzyl)-3-(2,6-dichloro-4-pyridyl)-1,3-diazabicyclo[3.3.0]octane-2,4-dione

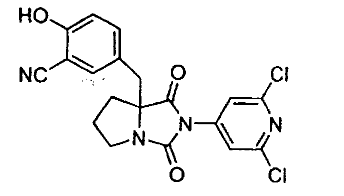

This compound was prepared in a manner similar to Example 196 from the compound of Example 195. MS. 417 (MH⁺). mp. 113.5 °C.

### Example 198: 5-[4-[2-(4-Pyridyl)ethoxy]benzyl)-3-(2,6-dichloro-4-pyridyl)-1,3-diazabicyclo[3.3.0]octane-2,4-dione

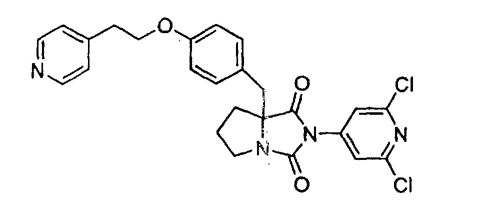

To a mixture of the compound from Example 196 (0.15 g), Ph₃P (0.22 g) and 4-hydroxyethylpyridine ((0.070 mL) in anhydrous CH₂Cl₂ (3 mL) was added DEAD (0.15 mL) under N₂. After 30 minutes the reaction mixture was concentrated and the residue was purified via HPLC to give the desired compound (97 mg). MS m/z 498(MH⁺).

The following compounds were prepared in a manner similar to Example 198 by using requisite hydroxy compounds.

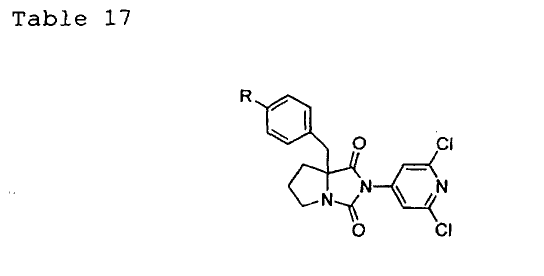

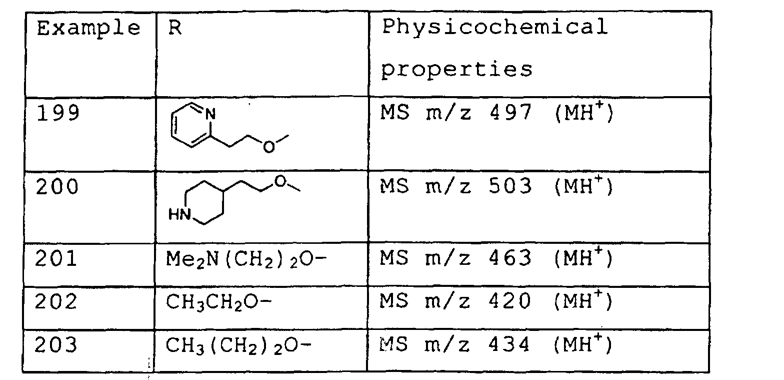

### Example 204: 5-(4-i-Propoxybenzyl)-3-(2,6-dichloro-4-pyridyl)-1,3-diazabicyclo[3.3.0]octane-2,4-dione

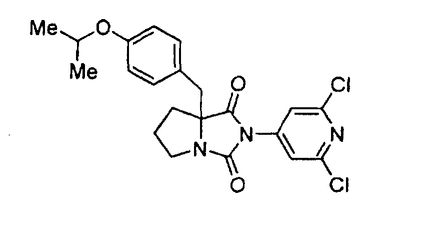

2-Iodopropane (0.5 ml) was added to a suspension of Example 196 (0.17 g) and CS₂CO₃ (0.28 g) in DMF (3 mL) and the mixture was stirred for 4 hours. The mixture was diluted with citric acid and the solution was extracted with EtOAc. EtOAc layer was washed with water and brine, dried and evaporated. The residue was purified via HPLC to yield the titled compound (0.13 g). MS m/z 435(MH⁺).

### Example 205: 5-(4-i-Butoxybenzyl)-3-(2,6-dichloro-4-pyridyl)-1,3-diazabicyclo[3.3.0]octane-2,4-dione

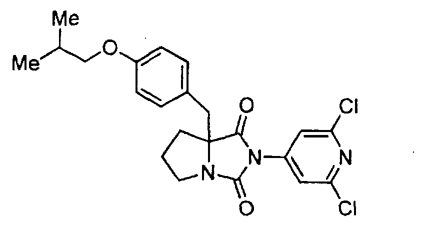

This compound was prepared in an analogous manner to Example 204. MS m/z 447(MH⁺).

### Example 206: 5-(4-Ethoxy-3-fluorobenzyl)-3-(2,6-dichloro-4-pyridyl)-1,3-diazabicyclo[3.3.0]octane-2,4-dione

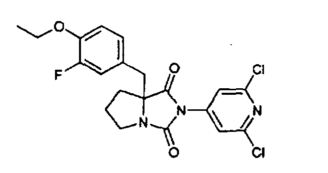

To a solution of the compound from Example 202 (0.24 g) in CH₃CN (15 mL) was added 3,5-dichloro-1-fluoropyridinium triflate (0.38 g) and the mixture was refluxed for 30 hours. The mixture was concentrated and purified by HPLC to give the desired compound (0.094 g). MS m/z 438 (MH⁺).

The following compounds were prepared in a manner (Suzuki coupling method) similar to Example 7.

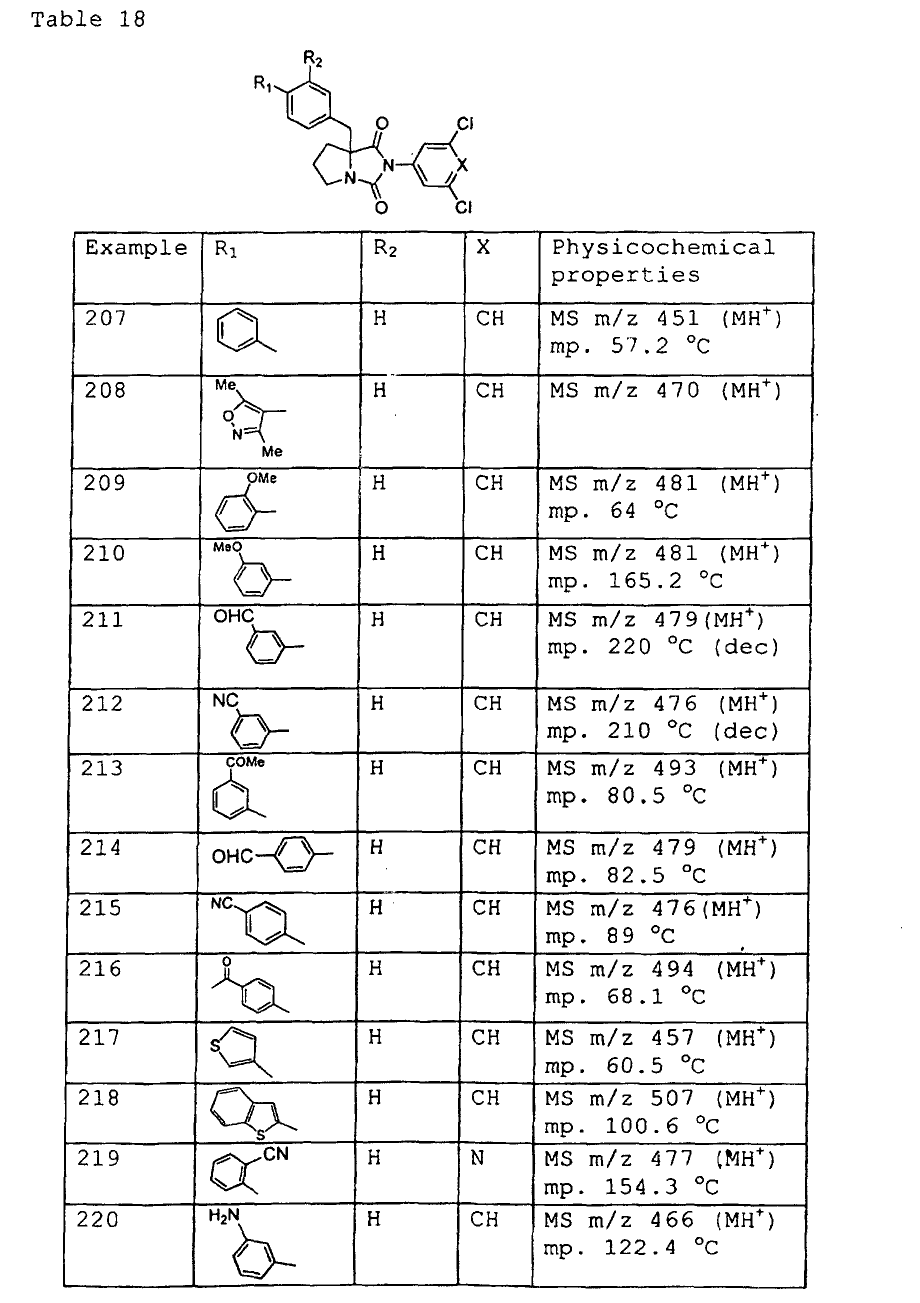

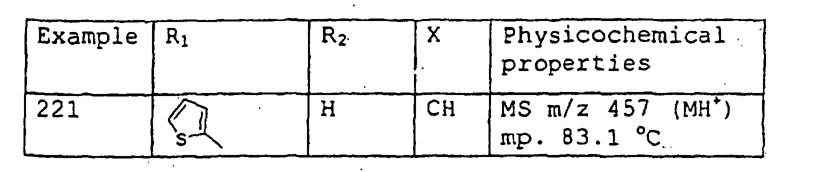

### Example 222: 5-[4-(3-Hydroxymethylphenyl)benzyl]-3-(3,5-dichlorophenyl)-1,3-diazabicyclo[3.3.0]octane-2,4-dione

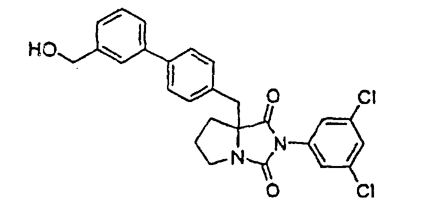

This compound was prepared in a manner similar to Example 12. MS m/z 481 (MH⁺). mp. 77.1 °C.

### Example 223: 5-[4-[3-(1-Hydroxy)ethylphenyl]benzyl]-3-(3,5-dichlorophenyl)-1,3-diazabicyclo[3.3.0]octane-2,4-dione

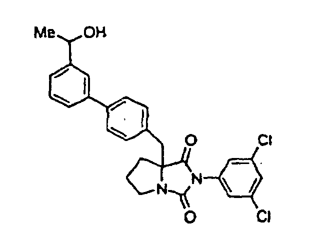

MeMgBr (1.4 M in THF, 0.7 mL) was added to a solution of the compound from Example 211 (0.4 g) in THF (10 mL) at -40 °C and the solution was stirred for 30 minutes. The reaction mixture was warmed to 0 °C and quenched with 1N HCl and extracted with EtOAc. the EtOAc layer was washed with brine, dried (MgSO₄) and evaporated. The residue was purified by chromatography (silica gel; hexanes to 50% EtOAc/hexanes; Chromatotron) and HPLC (CH₃CN/0.1 M HOAc) to give the desired compound (0.3 g). MS: m/z 517 (MNa⁺), mp. 66.5 °C.

### Example 224: (E)-5-[4-[3-(2-Methoxycarbonyl)vinylphenyl]benzyl]-3-(3,5-dichlorophenyl)-1,3-diazabicyclo[3.3.0]octane-2,4-dione

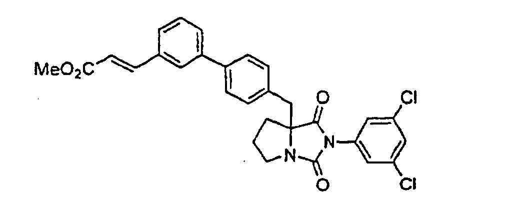

This compound was prepared in a manner similar to Example 17. MS m/z 535 (MH⁺). mp. 71.3 °C.

Examples 225 and 226 were prepared in accordance with the following scheme (Scheme 15).

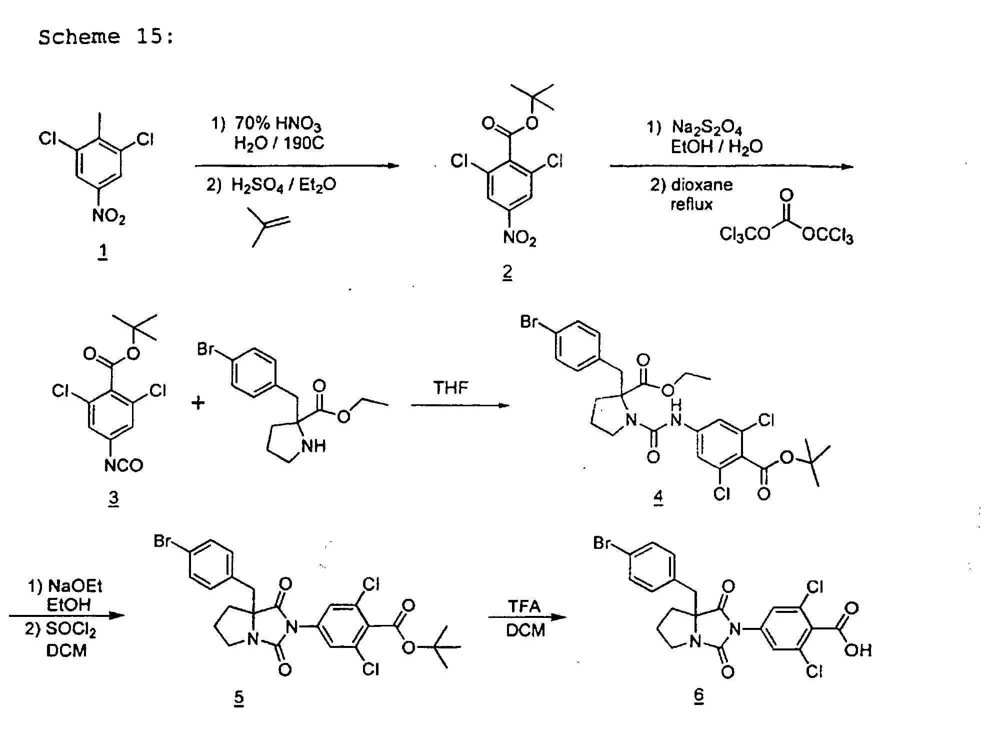

### Example 225: 5-(4-Bromobenzyl)-3-[3,5-dichloro-4-(t-butoxycarbonyl)phenyl]-1,3-diazabicyclo[3.3.0]octane-2,4-dione (Scheme 15, compound 5)

Step 1. A mixture of 2,6-dichloro-4-nitrotoluene (10 g), 70% HNO₃ (18 mL) and water (20 mL) was sealed in a steel bomb and heated with stirring in a sand bath at 195 °C for 24 hours. Additional HNO₃ (2 mL) was added and the mixture was reheated at 195 °C for 19 hours. The mixture was extracted with EtOAc and slowly treated with saturated NaHCO₃ solution. The aqueous layer was separated, acidified and re-extracted with EtOAc. It was dried (MgSO₄), filtered, concentrated and dried under high vacuum to yield the desired acid (9.9 g). MS m/z 236 (MH⁺). H₂SO₄ (2 mL) was added to a solution of the above acid (6.21 g) in Et₂O (20 mL). Isobutylene (5 mL) was condensed at -20 °C and added to the above solution. The mixture was placed in a steel bomb and stirred overnight. The bomb was opened and the mixture was taken up in Et₂O (100 mL). It was washed with 1 N NaOH (100 mL). The organic layer was dried (MgSO₄), filtered and evaporated to give the desired ester (6.21 g).
Step 2. The above nitro-ester (5.43 g) was reduced to the corresponding aniline (3.87 g) as described in the Example 131, step-2. ESMS: m/z 262 (MH⁺) A solution of the above amine (2.25 g) and triphosgene (2.6 g) in 20 mL dioxane was refluxed for 24 hours. The solvent was evaporated and the residue was used for next step without further purification.
Step 3. The above isocyanate (2.47 g) was added to an ice-cold solution of the proline derivative (2.23 g) in 50 mL THF. The resulting solution was stirred at 0 °C for 30 minutes and then allowed to warm up to room temperature and stirred overnight. The reaction mixture was partitioned between EtOAc/H₂O. The combined organic layers were dried over Na₂SO₄, filtered and evaporated. The product was purified by chromatography (silica gel, 1:1 EtOAc/hexanes) to yield the desired compound (2.46 g). ESMS: m/z 600 (MH⁺).
Step 4. The above urea (0.5 g) was hydrolyzed with NaOEt (0.06 g) in EtOH at room temperature to yield the desired acid (0.5 g). ESMS: m/z 572 (MH⁺). SOCl₂ was added to a slurry of the acid (0.092 g) in CH₂Cl₂ (10 mL) and the mixture was stirred overnight at room temperature. The mixture was evaporated and the residue was purified by chromatography (silica gel; 3/2 hexane/EtOAc; Chromatotron) to yield the titled compound (0.09 g). MS m/z 554 (MH⁺); mp. 109-110 °C.

### Example 226: 5-(4-Bromobenzyl)-3-(3,5-dichloro-4-carboxyphenyl)-1,3-diazabicyclo[3.3.0]octane-2,4-dione (Scheme 15, compound 6)

The compound from Example 225 was deprotected with TFA/CH₂Cl₂ to give the titled acid. MS m/z 492 (MH⁺); mp. 152 °C.

### Example 227: 5-(4-Bromobenzyl)-3-[3,5-dichloro-4-(carboxymethylcarbamoyl) phenyl]-1,3-diazabicyclo[3.3.0]octane-2,4-dione

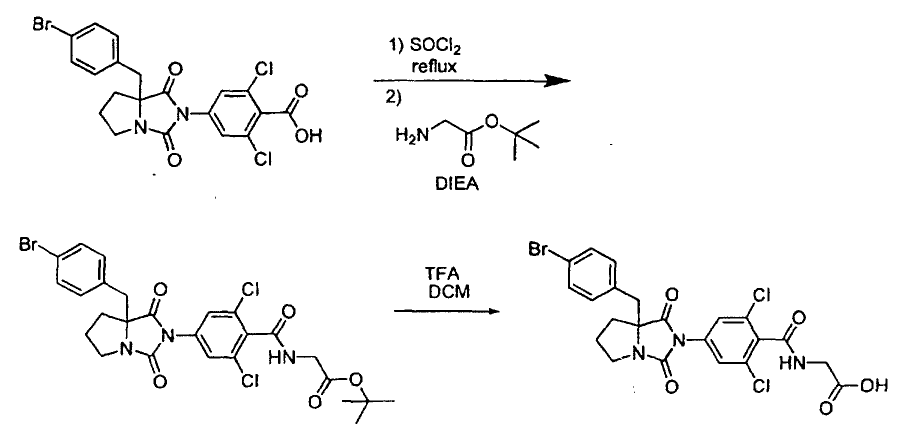

Step-1. A mixture of the compound from Example 226, SOCl₂ and a few drops of water was heated under reflux for 3days. The mixture was evaporated and dried under high vacuum to give the desired acid chloride, which was used as is for the next step.
Step-2. DIEA (0.13 mL) was added to a solution of glycine t-butyl ester hydrochloride (0.035 g) in THF (3 mL). To this solution was added a solution of the above acid chloride (0.061 g) in THF (2 mL) and the mixture was stirred overnight at room temperature. THF was evaporated and the residue was diluted with EtOAc. The solution was washed successively with 1N HCl, brine and water. It was dried (MgSO₄), filtered and concentrated to an oil which was purified via chromatography (silica gel; 3/2 hexanes/EtOAc; Chromatotron) to give the desired amide (0.061 g). MS m/z 611 (MH⁺).
Step-3. The above ester was dissolved in a mixture of TFA (1 mL) and CH₂Cl₂ (1 mL) and the solution stirred at room temperature for 4 hours. It was concentrated and the residue purified via chromatography (silica gel; CHCl₃-CHCl₃/MeOH 10%) to give the titled compound (0.045 g). MS m/z 555 (MH⁺), mp. 157-158 °C.

The following examples were prepared in a similar manner using requisite amino acid derivatives.

### Example 228: 5-(4-Bromobenzyl)-3-(3,5-dichloro-4-L-alaninocarbonyl)-1,3-diazabicyclo[3.3.0]octane-2,4-dione

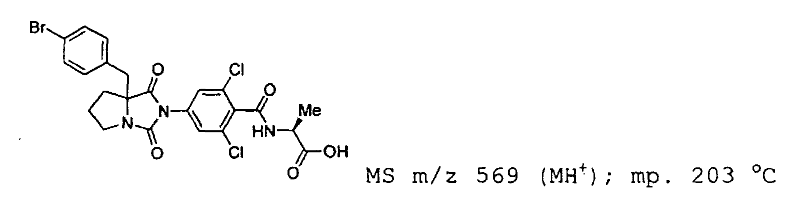

### Example 229: 5-(4-Bromobenzyl)-3-(3,5-dichloro-4-L-asparaginocarbonylphenyl)-1,3-diazabicyclo[3.3.0]octane-2,4-dione

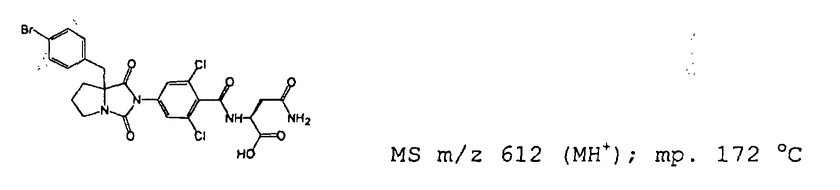

The following compounds were prepared in a manner similar to Example 1 with the exception of the use of LDA in place of KHMDS in step-1.

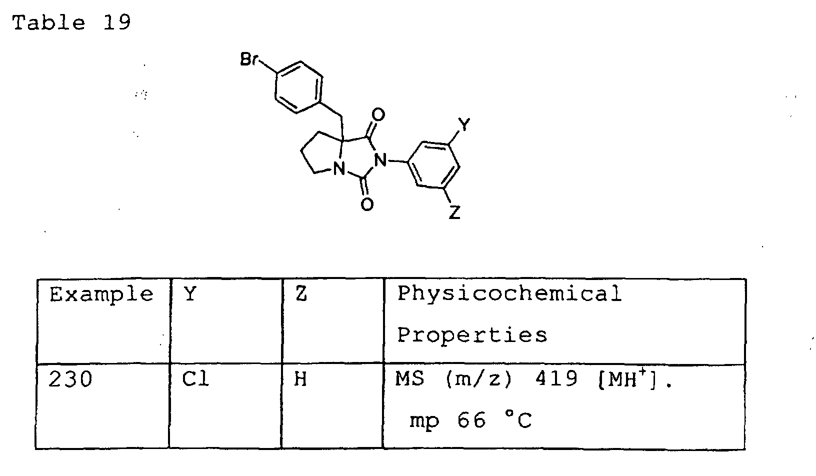

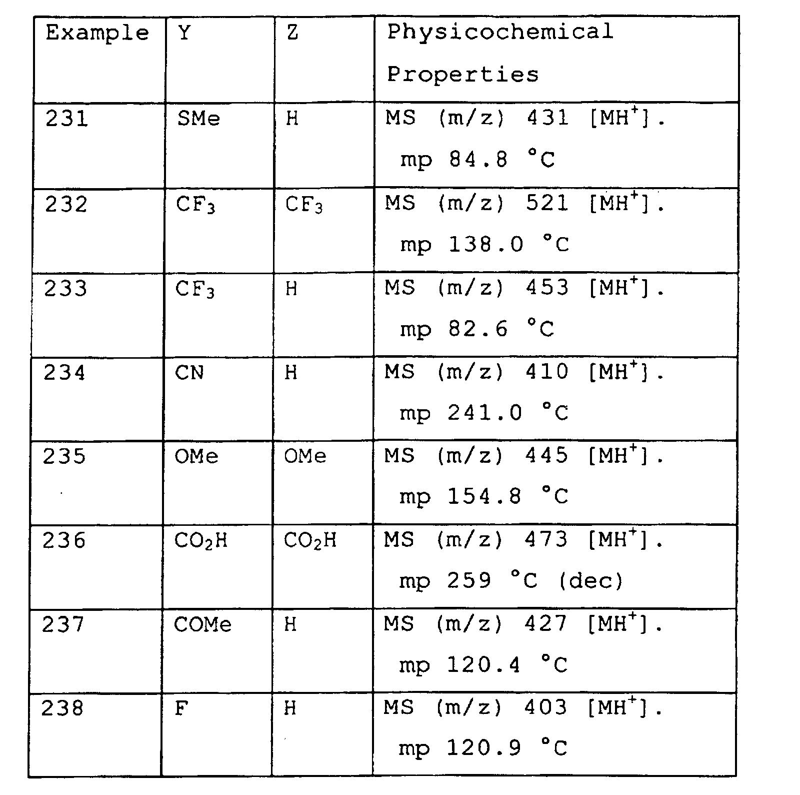

### Example 239: 5-(3-Nitrobenzyl)-3-(2,6-dichloro-4-pyridyl)-1,3-diazabicyclo[3.3.0]octane-2,4-dione.

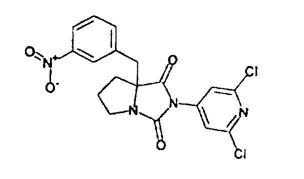

This compound was prepared in an analogous manner. MS m/z 421 (MH⁺); mp. 182.8 °C.

### Example 240: 5-(4-Cyanobenzyl)-3-(2-amino-3,5-dichlorophenyl)-1,3-diazabicyclo[3.3.0]octane-2,4-dione

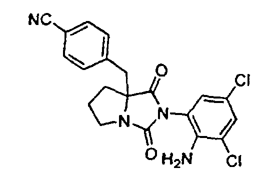

This compound was prepared in a manner similar to Examples 131 and 132 starting from 2,4-dichloro-6-nitroaniline. MS m/z 415 (MH⁺). mp. 122.2 °C.

The following compounds were prepared in a manner similar to Example 67 using the requisite acid.

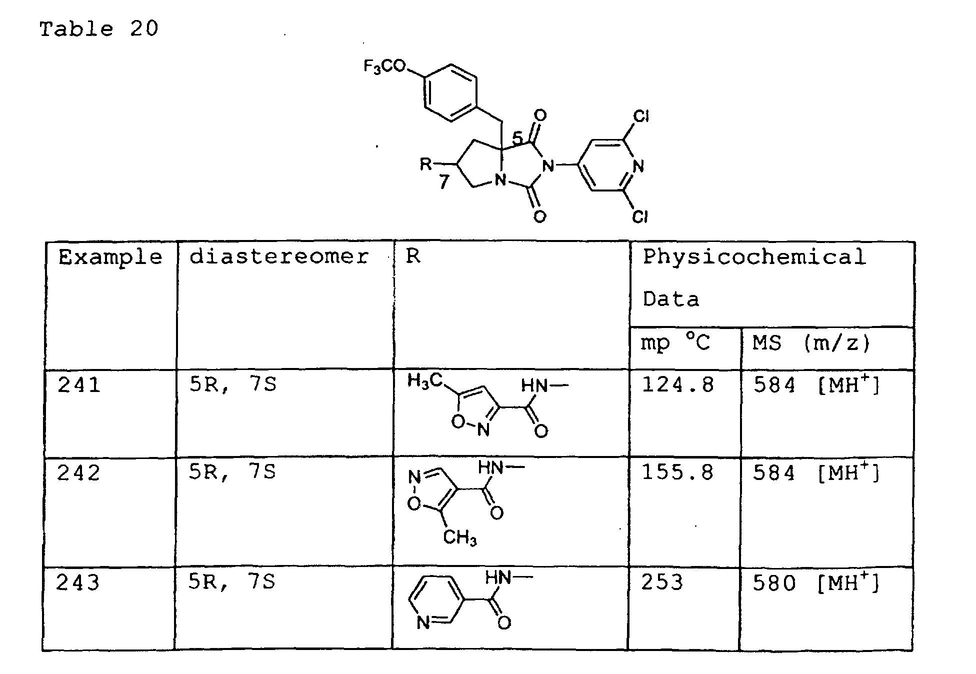

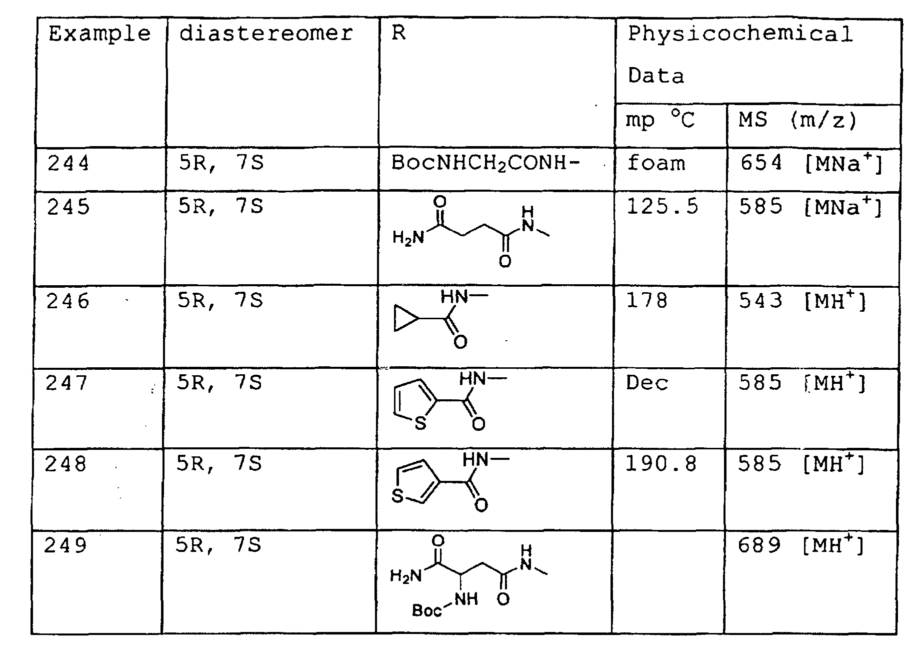

### Example 250: (5R, 7S)-5-[4-(Trifluoromethoxy)benzyl]-3-(2,6-dichloro-4-pyridyl)-7-methoxycarbonylamino-1,3-diazabicyclo[3.3.0]octane-2,4-dione

To a solution of the compound from Example 63 (0.075 g) in THF (4 mL) was added DIEA (0.10 mL) and methyl chloroformate (0.020 mL). After 3hours, the reaction was concentrated and purified by chromatography (silica gel; 98:2 CH₂Cl₂/methanol, Chromatotron) to afford the titled compound (0.0715 g): MS (m/z) 533 [MH⁺]. mp 119.8 °C.

### Example 251: (5R, 7S) -5-[4-(Trifluoromethoxy)benzyl]-3-(2,6-dichloro-4-pyridyl)-7-(1-pyrrolyl)-1,3-diazabicyclo[3.3.0]octane-2,4-dione

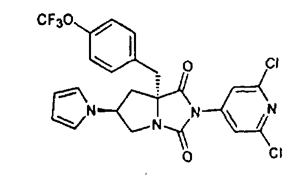

To a solution of the compound from Example 63 (0.25 g) in acetic acid (5 mL) was added sodium acetate (0.26 g) and 2,5-dimethoxyfuran (0.14 mL). After 20 minutes at reflux, the reaction was cooled, diluted with EtOAc, neutralized with solid NaHCO₃. The organic solution was collected, washed with saturated NaHCO₃ and brine, dried (Na₂SO₄), filtered, concentrated and purified by chromatography (silica gel; 95:5 CH₂Cl₂/methanol, Chromatotron) to afford the titled compound (0.145 g): MS (m/z) 525 [MH⁺]. mp 129.5 °C.

### Example 252: (5R, 7S)-5-[4-(Trifluoromethoxy)benzyl]-3-(2,6-dichloro-4-pyridyl)-7-aminoacetylamino-1,3-diazabicyclo[3.3.0]octane-2,4-dione

The titled compound was prepared from Example 244 by treatment with TFA in the usual manner. MS (m/z) 532 [MH⁺]. mp 114 °C.

### Example 253: (5R, 7S)-5-[4-(Trifluoromethoxy)benzyl]-3-(2,6-dichloro-4-pyridyl)-7-[(methansulfonylaminoacetyl)amino]-1,3-diazabicyclo[3.3.0]octane-2,4-dione

The titled compound was prepared from Example 252 in a manner similar to Example 33: MS (m/z) 632 [MNa⁺]. mp 104.9 °C.

### Example 254: (5R, 7S)-5-[4-(Trifluoromethoxy)benzyl]-3-(2,6-dichloro-4-pyridyl)-7-methanesulfonylamino-1,3-diazabicyclo[3.3.0]octane-2,4-dione

The titled compound was prepared from the compound from Example 63 in a manner similar to Example 33: MS (m/z) 575 [MNa⁺]. mp 207.2 °C.

### Example 255: (5R, 7S)-5-[4-(Trifluoromethoxy)benzyl]-3-(2,6-dichloro-4-pyridyl)-7-[(3-carbamoyl-3-aminopropanoyl)amino]-1,3-diazabicyclo[3.3.0]octane-2,4-dione

The titled compound was prepared from Example 249 by treatment with TFA in usual manner: MS (m/z) 589 [MH⁺]. mp 107.8 °C.

### Example 256: (5R, 7S)-5-[4-(Trifluoromethoxy)benzyl]-3-(2,6-dichloro-4-pyridyl)-7-[(3-carbamoyl-3-acetylaminopropanoyl)amino]-1,3-diazabicyclo[3.3.0]octane-2,4-dione

The titled compound was prepared from Example 255 in a manner similar to Example 48. MS (m/z) 653 [MNa⁺]. mp 152.8 °C.

### Example 257: (5R, 7R)-5-[4-(Trifluoromethoxy)benzyl]-3-(2,6-dichloro-4-pyridyl)-7-dimethylcarbamoyl-1,3-diazabicyclo[3.3.0]octane-2,4-dione

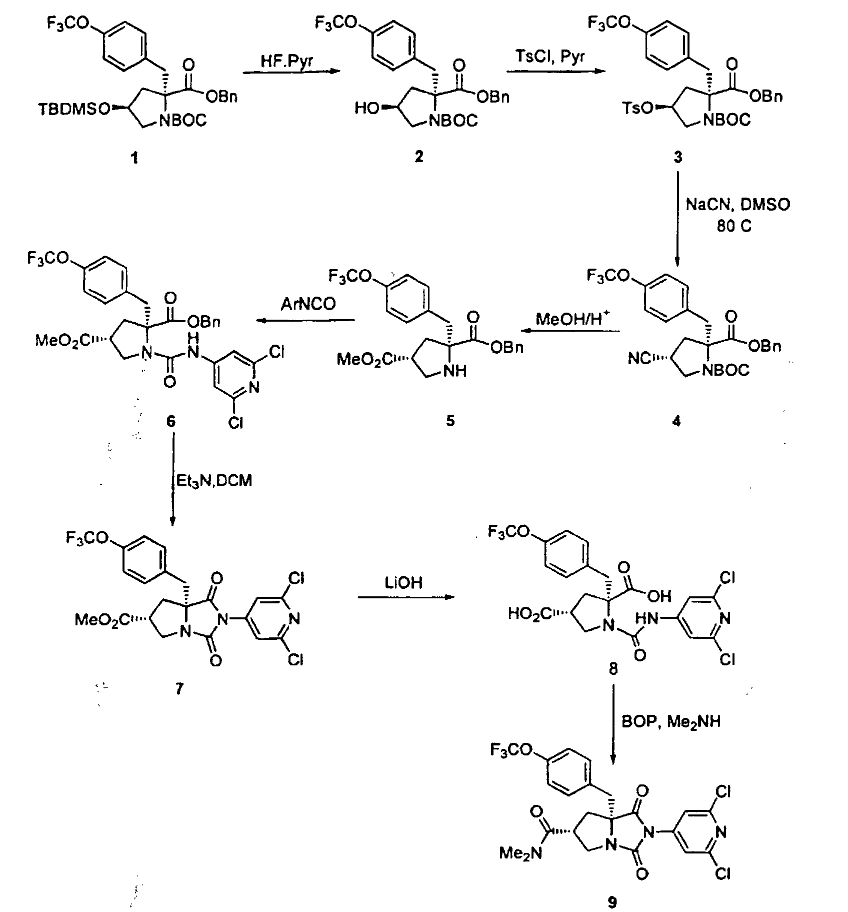

Step 1. The mixture of diastereoisomers of structure **1** (5.5g) were dissolved in THF (150 mL) and HF pyridine (10 mL) was added. The reaction mixture was stirred at room temperature for 1 hour whereupon saturated NaHCO₃ was added until no further effervescence. The aqueous phase was then separated and extracted with EtOAc. The combined organics were washed with HCl (5%), saturated NaHCO₃, then dried over Na₂SO₄, filtered and concentrated *in vacuo.* The residue was purified by liquid chromatography (SiO₂, EtOAc/Hexane = 25/75) gave **2** as a single diastereoisomer (2.9 g). MS (m/z) 496 (MH⁺).
Step 2. A mixture of the compound **2** (2.9 g), TsCl (2.23 g), pyridine (1.4 mL) and DCM (10 mL) was stirred at room temperature. After 3 days, EtOAc was added and the mixture was washed with HCl (5%). The aqueous phase was then separated and extracted with EtOAc. The combined organics were washed with NaHCO₃, then brine, dried over Na₂SO₄, filtered and concentrated *in vacuo*. The residue was purified by liquid chromatography (SiO₂, EtOAc/Hexane = 1/9) to give **3** (3.5 g). MS (m/z) 650 (MH⁺)
Step 3. The compound **3** (2.47 g) was dissolved in dry DMSO (8 mL) and powdered NaCN (340 mg) was added. The reaction mixture was heated at 80°C for 30 hours with more NaCN (2 x 100 mg) being added at 9 and 22 hours. EtOAc and brine were added and the reaction mixture was shaken. The aqueous phase was then separated and extracted with EtOAc. The combined organics were dried over Na₂SO₄, filtered and concentrated *in vacuo.* The residue was purified by liquid chromatography (SiO₂, EtOAc/Hexane = 1/9) gave **4** (0.9 g). MS (m/z) 504 (MH⁺) .
Step 4. The compound **4** (0.9g) was dissolved in dry MeOH (6 mL) and HCl (1M in Et₂O, 12 mL) was added and the reaction mixture was stirred at room temperature. After 18 hours, EtOAc and saturated NaHCO₃ were added until no further effervescence. The aqueous phase was then separated and extracted with EtOAc. The combined organics were dried over Na₂SO₄, filtered and concentrated *in vacuo.* The residue was purified by liquid chromatography (SiO₂, EtOAc/Hexane = 3/7) to give **5** (730 mg). MS (m/z) 438 (MH⁺).
Step 5. Amine **5** (720 mg) was dissolved in DCM (10 mL) and 3,5-dichloro-4-pyridyl isocyanate (343 mg) was added directly. The mixture was stirred at room temperature for 5 hours whereupon it was concentrated and purified by liquid chromatography (SiO₂, EtOAc/Hexane = 2/8) to give **6** (900 mg). MS (m/z) 626 (M⁺)
Step 6. Urea **6** (880 mg) was dissolved in DCM (16 mL) and Et₃N (0.59 mL) was added. The reaction mixture was then heated at 43°C for 18 hours, concentrated *in vacuo* and purified by liquid chromatography (SiO₂, EtOAc/Hexane = 2/8) to give **7** (508 mg). MS (m/z) 518 (M⁺).
Step 7. The compound **7** (280 mg) was dissolved in THF/MeOH and LiOH (50 mg) in H₂O (1 mL) was added. The reaction mixture was stirred at room temperature for 24 hours. HCl (5%) was added to pH=2 and the reaction mixture concentrated. The aqueous layer was extracted with EtOAc and DCM and the combined organic layers dried over Na₂SO₄ and concentrated to give the desired product **8** (305 mg). MS (m/z) 522 (M⁺).
Step 8. To a solution of the above diacid **8** (53 mg) in dry DCM (2 mL) and diisopropylethylamine (0.044 mL) was added BOP reagent (99 mg) and the solution stirred at room temperature for 2.5 hours. Dimethylamine (2M in THF, 0.066 mL) was then added. After 4 h, further BOP reagent (0.1 mmol) and dimethylamine (0.05 mmol) were added. After a total of 20 hours, EtOAc and HCl (5%) were added and the reaction mixture shaken. The aqueous phase was separated and extracted with EtOAc. The combined organics were washed with saturated NaHCO₃, brine, dried over Na₂SO₄, filtered and concentrated *in vacuo.* The residue was purified by liquid chromatography (SiO₂, EtOAc/DCM = 1/9) to give the titled compound (47 mg). MS (m/z) 531 (M⁺).

The following compounds were prepared in an analogous manner to Example 257.

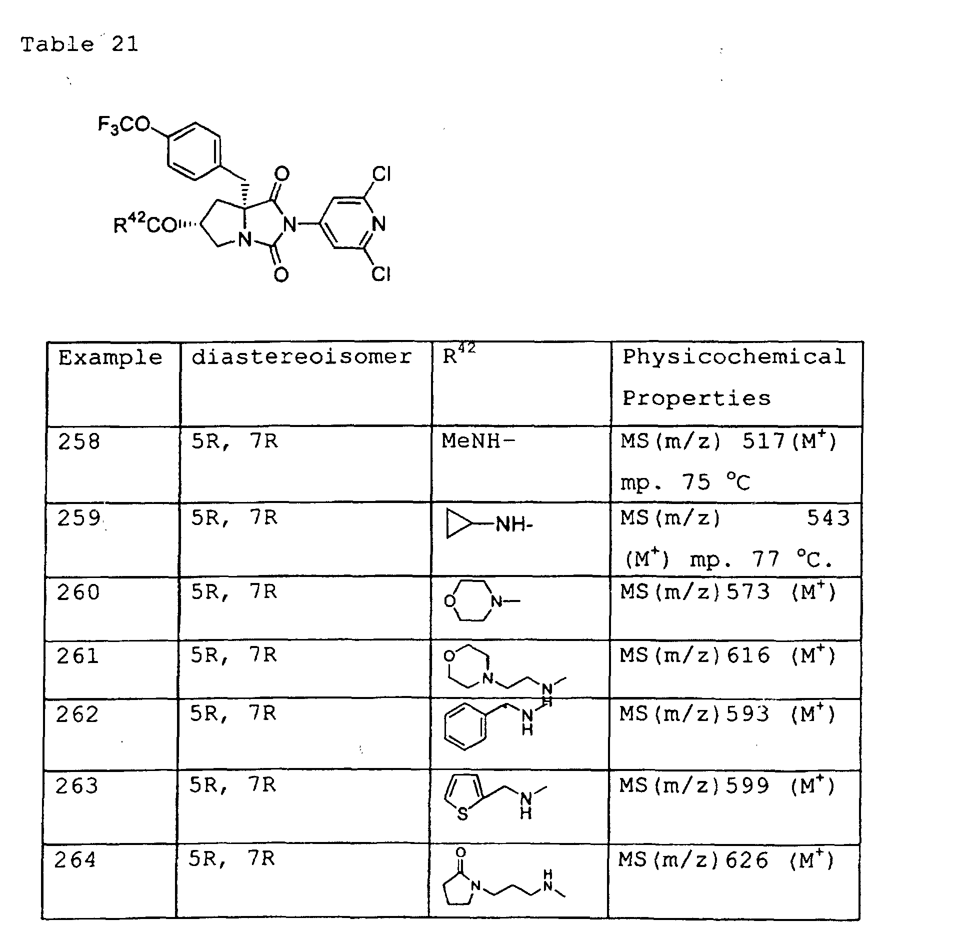

### Example 265. (5R, 7R) -5-[4-(Trifluoromethoxy)benzyl]-3-(2,6-dichloro-4-pyridyl)-7-carbamoyl-1,3-diazabicyclo[3.3.0]octane-2,4-dione

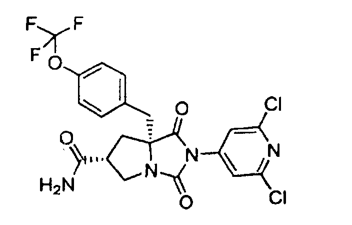

The title compound was prepared in a manner similar to Example 257 but replacing dimethyl amine with ammonium chloride in step 8. MS (m/z) 503 (M⁺)

### Example 266. (5R,7R)-5-benzyl-3-(3,5-dichlorophenyl)-7-hydroxy-1,3-diazabicyclo[3.3.0]octane-2,4-dione

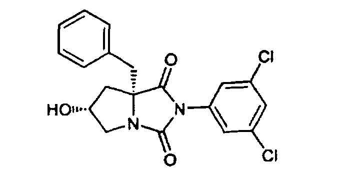

This compound was prepared in a manner similar to the compound described in Example 58. MS (m/z) 391 (MH⁺)

### Example 267. 5-Benzyl-3-(3,5-dichlorophenyl-1,3-diazabicyclo[3.3.0]octane-2,4-dione

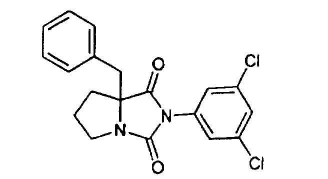

This compound was prepared in a manner similar to the compound described in Example 21. MS (m/z) 375 (MH⁺); mp. 173.8 °C

### Example 268: (5R, 7R) -5-[4-(Trifluoromethoxy)benzyl]-3-(3,5-dichlorophenyl)-7-hydroxy-1,3-diazabicyclo[3.3.0]octane-2,4-dione

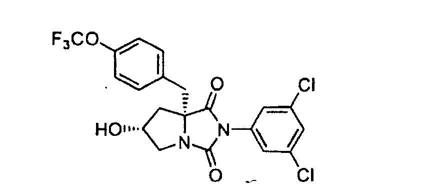

This compound was prepared in a manner similar to the compound described in Example 58. MS (m/z) 475 (MH⁺); mp. 50.4 °C

### Example 269: 6-Bromobenzyl-8-(2,6-dichloro-4-pyridyl)-1,4,8-triazabicyclo[4.3.0]nonane-3,7,9-trione.

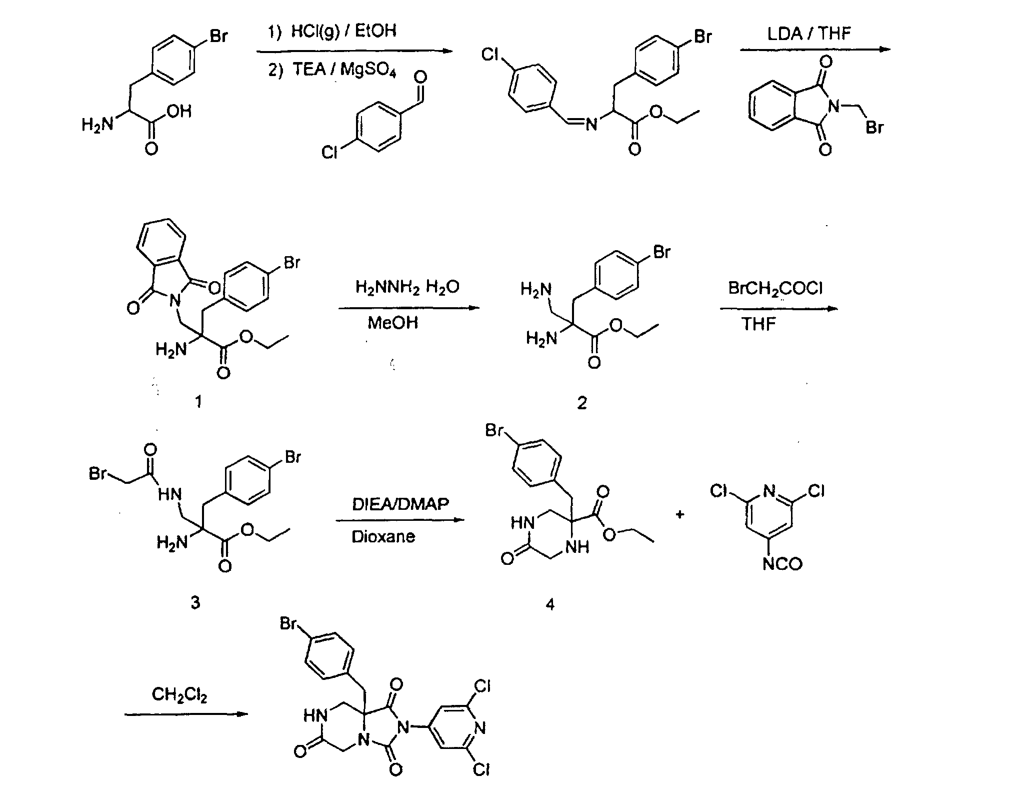

Steps 1 and 2. Compound **1** was prepared from 4-brmophenylalanine ethyl ester according to the methodology described in WO 98/39303. MS(m/z): 431(MH⁺).
Step 3. A mixture of the compound **1** and hydrazine hydrate in MeOH was heated under reflux for 24 hours. MeOH was evaporated and the compound was purified via chromatography to give the diamine. MS(m/z): 301 (MH⁺).
Steps 4 and 5. THF (15 mL) was added to the above diamine (1.1 g) and the solution was cooled in ice. Bromoacetyl chloride (0.35 mL) was added dropwise and the solution was warmed slowly to room temperature. The mixture was stirred for 5 hours and concentrated to half the volume. Et₂O was added and the mixture stirred at room temperature until precipitation was complete. The solid was filtered and dissolved in EtOAc. The solution was washed with saturated NaHCO₃ followed by brine, dried (MgSO₄), filtered and evaporated. The residue (1.09 g) was used as is for the next step.

A mixture of the above compound (0.480 mg), DIEA (0.59 mL) and DMAP (7 mg) in dioxan (20 mL) was heated at 70 °C for 24 hours. The mixture was diluted with EtOAc/water and the EtOAc layer was separated. The aqueous solution was extracted with EtOAc and the combined organic layer was dried (Na₂SO₄), filtered and concentrated. The residue was purified via HPLC (AcOH 0.1M/CH₃CN 75/25 to 0/100) to give the desired compound (0.52 mg). MS(m/z): 341 (MH⁺) .

### Step 6. To a solution of the above compound (50 mg) in dry DCM (2 mL) was added 2,6-dichloropyridyl-4-isocyanate (36 mg) and the solution stirred at room temperature for 5 hours. The solution was concentrated in vacuo and the residue purified by chromatography (silica gel; Chromatotron) to give the titled compound (0.47 mg). mp. 226 °C. MS(m/z): 484(MH⁺).

The following compounds were prepared in a manner similar to one of the above Examples.

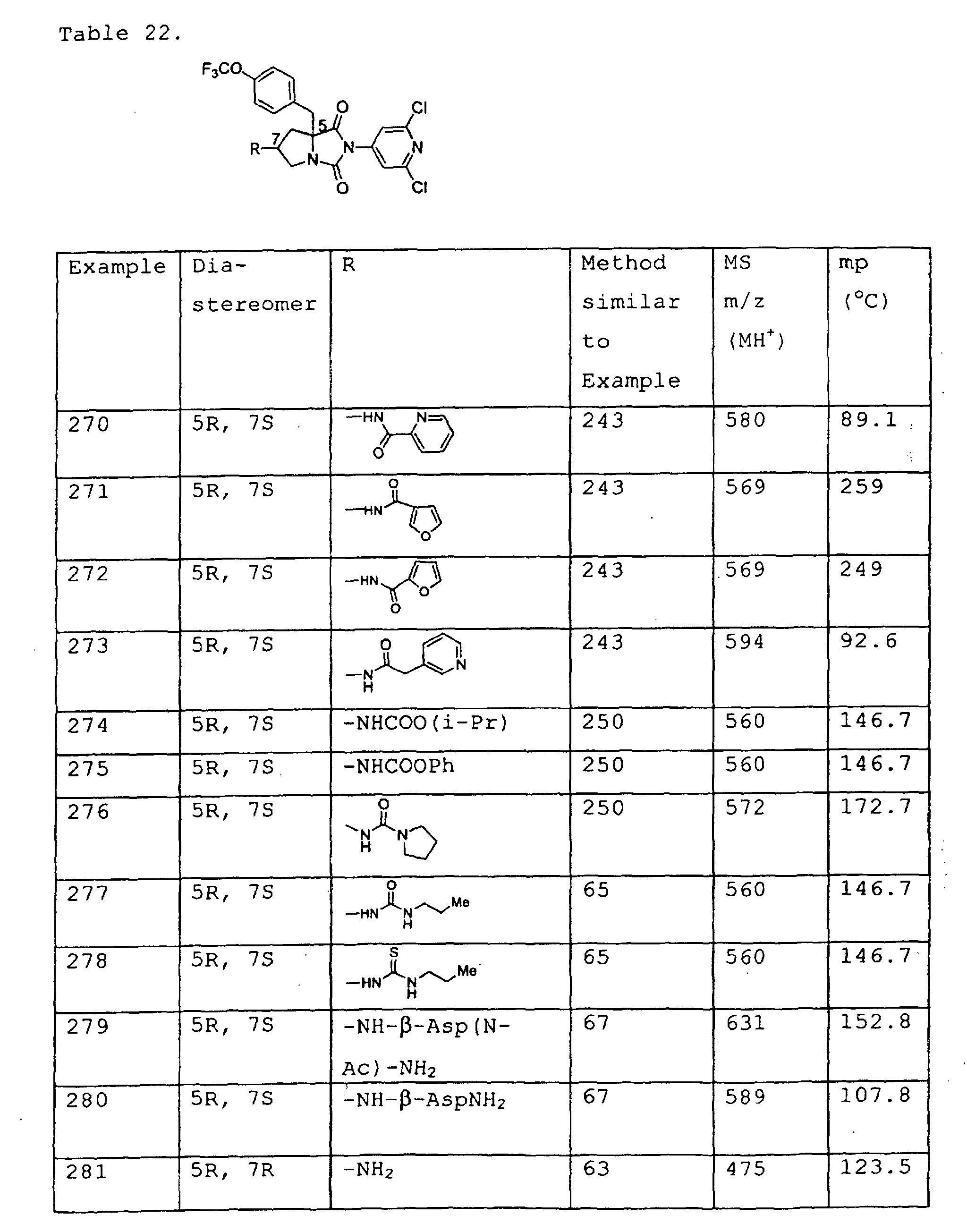

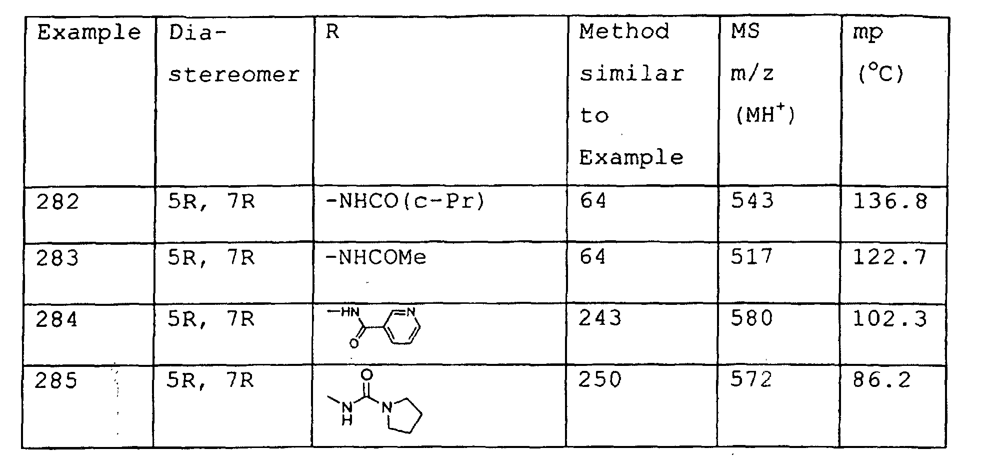

### Example 286. (5R, 7S)-5-[4-(Trifluoromethoxy)benzyl]-3-(2,6-dichloro-4-pyridyl)-7-valerylamino-1,3-diazabicyclo[3.3.0]octane-2,4-dione.

Valeryl chloride (0.031g) was added to a solution of the compound from Example 63 (0.1 g) and DIEA (0.84 g) in THF (5 mL) and the reaction mixture was stirred at room temperature for 24 hours. The mixture was concentrated and the residue was purified via HPLC (CH₃CN/0.1 M AcOH) to yield 0.075 g of the titled compound. MS (m/z) 559 (MH⁺) and 581 (MNa⁺). mp 66.8 °C.

### Example 287. (5R, 7S)-5-[4-(Trifluoromethoxy)benzyl]-3-(2,6-dichloro-4-pyridyl)-7-ureido-1,3-diazabicyclo[3.3.0]octane-2,4-dione.

A solution of the compound from Example 63 (0.114 g) in diethyl ether (5 mL) was added dropwise to a solution of N-chlorosulfonylisocyanate (30 µL) in diethyl ether (5 mL) at -15 °C. After 1 hour, the reaction mixture was concentrated. The residue was stirred with 0.5 N HCl for 30 minutes. The reaction mixture was diluted with DMSO and purified by HPLC (CH₃CN/0.1 M AcOH). The desired fraction was collected, concentrated, neutralized, extracted with EtOAc, dried (Na₂SO₄), filtered and concentrated to afford the titled compound (0.077g). MS (m/z) 518 (MH⁺). mp 141.7 °C.

### Example 288. (5R, 7S)-5-[4-(Trifluoromethoxy)benzyl]-3-(2,6-dichloro-4-pyridyl)-7-(2,5-dimethylpyrrolyl)-1,3-diazabicyclo[3.3.0]octane-2,4-dione.

A mixture of the compound from Example 63 (0.1 g), 2,5-hexanedione (0.028 g), AcOH (5 mL) in EtOH (5 mL) was refluxed for 4 hours. The mixture was concentrated and the residue was purified via HPLC (CH₃CN/0.1 M AcOH) to yield 0.065 g of the titled compound. MS (m/z) 553 (MH⁺). mp 112.4 °C.

### Example 289. (5R, 7S)-5-[4-(Trifluoromethoxy)benzyl]-3-(2,6-dichloro-4-pyridyl)-7-[2-(trifluoroacetyl)pyrrolyl]-1,3-diazabicyclo[3.3.0]octane-2,4-dione.

To a solution of the compound from Example 251 (0.078 g) in CH₂Cl₂ (10 mL) was added trifluoroacetic anhydride (0.084 g) and the mixture was stirred under N₂ overnight. The reaction mixture was concentrated and the residue was purified via HPLC (CH₃CN/0.1 M AcOH) to yield 0.060 g of the titled compound. MS (m/z) 621 (MH⁺). mp 166.6 °C.

The following compounds (5R,7S diastereomers) were prepared in a manner similar to one of the above Examples.

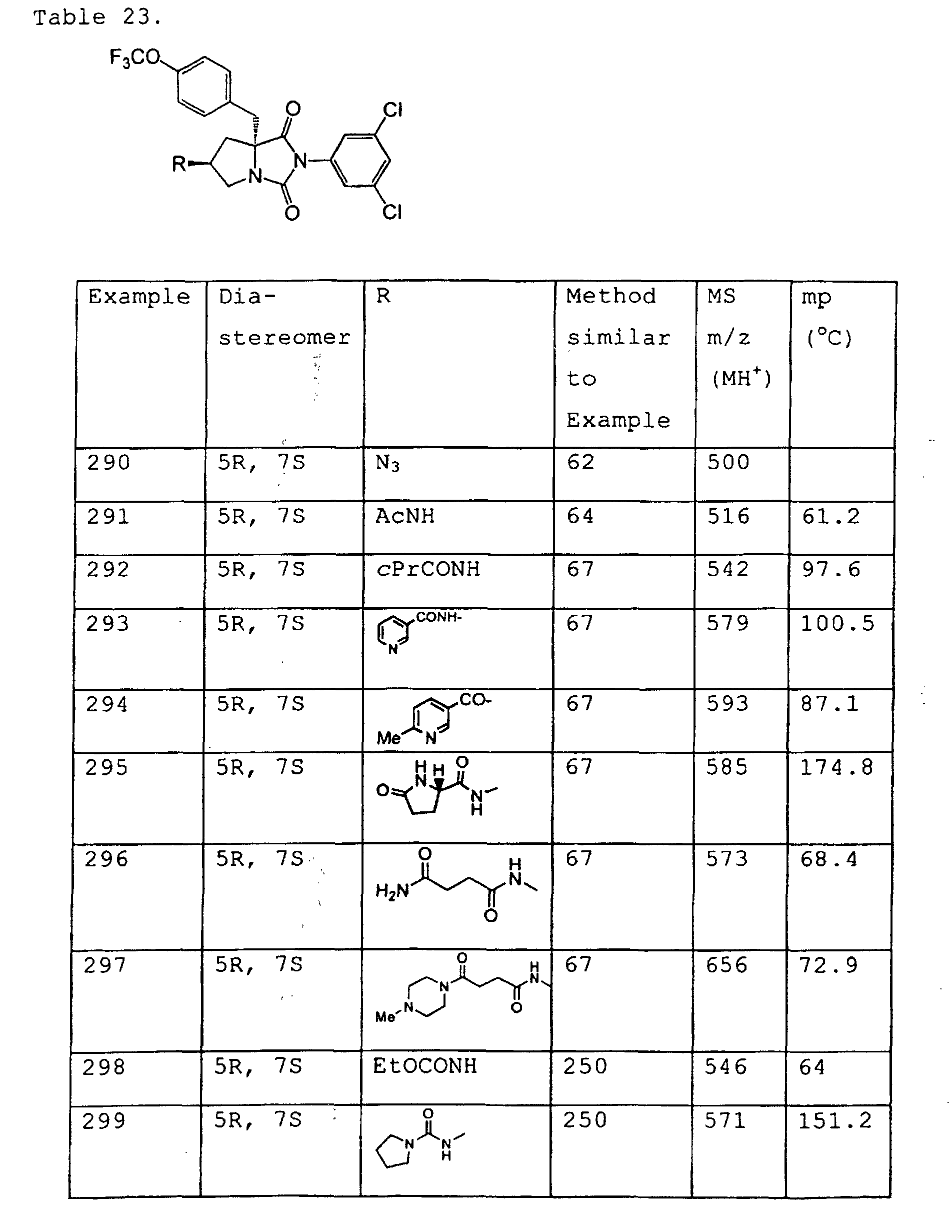

### Example 300. (5R, 7S)-5-[4-(Trifluoromethoxy)benzyl]-3-(3,5-dichlorophenyl)-7-amino-1,3-diazabicyclo[3.3.0]octane-2,4-dione.

Hydrogen (gas) was bubbled through a solution of the compound from Example 288 (0.274 g) in EtOAc (20 mL) in presence of Pd-C (5%, 20 mg) for 10 minutes and the mixture was stirred for 2 hours. The mixture was filtered through celite and the celite washed with additional EtOAc. The combined EtOAc was evaporated and the residue was purified via chromatography (hexanes-EtOAc) to yield 0.161 g of the titled compound. MS (m/z) 474 (MH⁺). mp 43.3 °C.

### Example 301. (5R, 7S)-5-[4-(Trifluoromethoxy)benzyl]-3-(3,5-dichlorophenyl)-7-(N-acety-N-methylamino)-1,3-diazabicyclo[3.3.0]octane-2,4-dione.

LDA (0.34 M in THF; 0.045 g) was added to a solution of the compound from Example 64 (0.18 g) in THF at -80 °C and the mixture was stirred for 20 minutes at that temperature. MeI (0.65 g) was added and the reaction mixture was stirred for 10 minutes. The mixture was warmed to room temperature and stirred for additional 20 minutes. It was quenched with aqueous NH₄Cl solution and the mixture was evaporated. The residue was purified via HPLC (CH₃CN/0.1 M AcOH) to yield 0.012 g of the titled compound. MS (m/z) 531 (MH⁺) and 553 (MNa+). mp 74.7 °C.

### Example 302. (5R, 7S)-5-[4-(Trifluoromethoxy)benzyl]-3-(3,5-dichlorophenyl)-7-[[4-(dimethylamino)butyryl]amino]-1,3-diazabicyclo[3.3.0]octane-2,4-dione

This compound was prepared similar to Example 67. MS (m/z) 587 (MH⁺). mp 57.9 °C.

The following compounds were prepared in a manner similar to one of the above Examples.

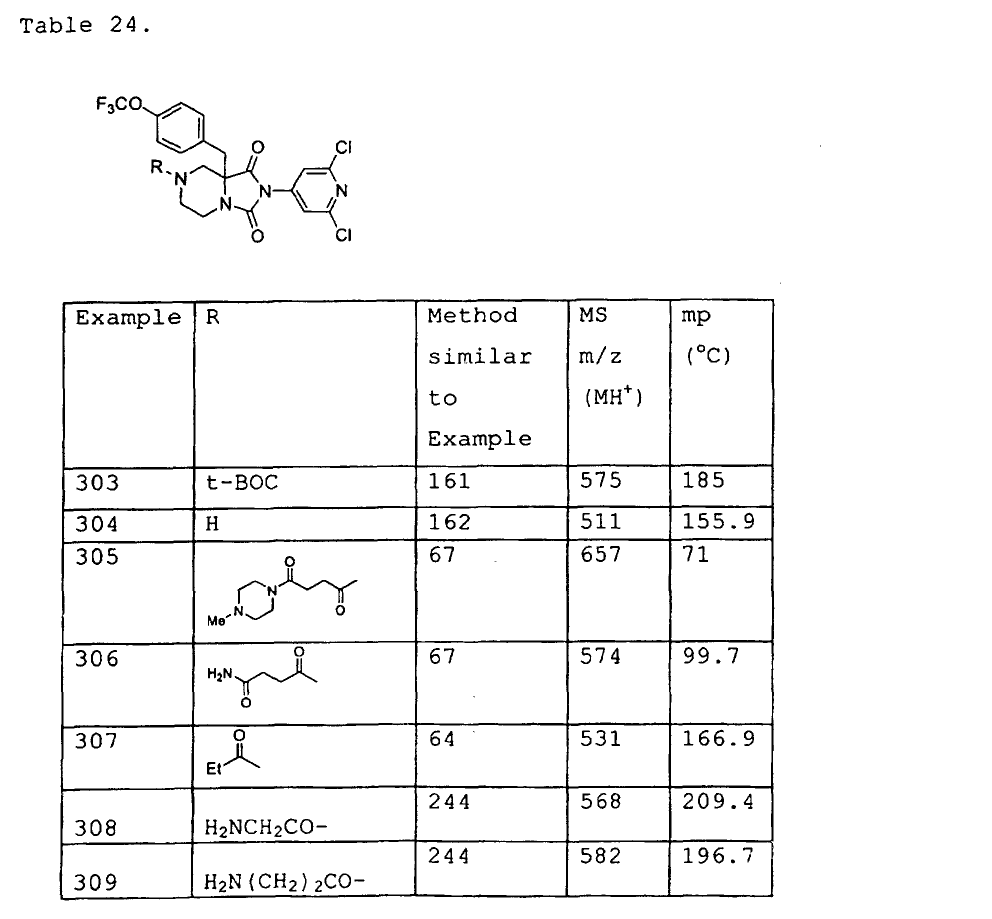

The following compounds were prepared in a manner similar to Examples 189 and 190.

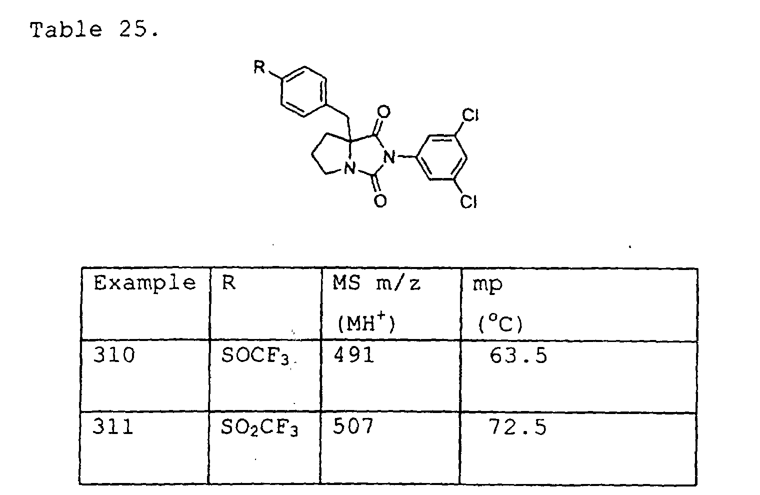

### Example 312. (5R, 7R)-5-[4-(Trifluoromethoxy)benzyl]-3-(3,5-dichlorophenyl)-7-carbamoyl-1,3-diazabicyclo[3.3.0]octane-2,4-dione.

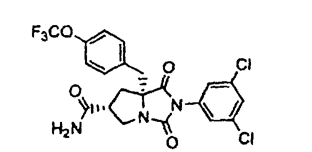

The compound from Example 257, step-7 (diacid compound **8**, 0.153 g) was treated with thionyl chloride (2 mL) and heated to reflux. After 1 hour, the reaction mixture was concentrated and evaporated with toluene. The residue was dissolved in THF (3 mL) and the mixture was added to NH₄OH (0.5 mL) in THF (5 mL) chilled with an ice bath. After 15 minutes, the mixture was added to 3N HCl (10 mL). The mixture was extracted with EtOAc. The extract was washed with brine, dried (Na₂SO₄), filtered and concentrated. Purification by HPLC afforded the titled compound (0.108 g): MS (m/z) 502 (MH+); mp 122.7 °C.

The following compounds were prepared in a manner similar to the Example 257 replacing BOP reagent with SOCl₂ in step-8 as described in Example 310.

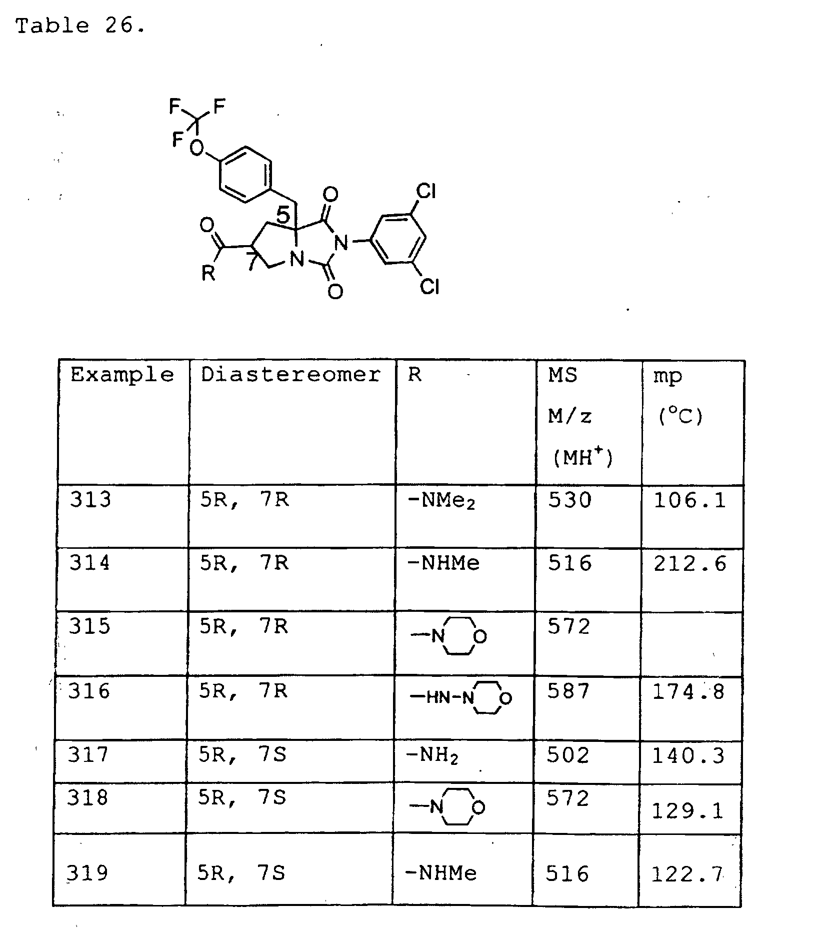

### Reference Examples

### Preparation of the substituted benzylbromide intermediate: Prototype example.

### Synthesis of 5-bromomethyl-2,2-difluorobenzodioxole.

BH₃-THF (1M, 10 mL) was added to a solution of 2,2-difluorobenzodioxole-5-carboxylic acid (0.52 g) in THF (10 mL) and the resulting mixture was stirred at room temperature for 14 hours. The reaction mixture was quenched with 2 N HCl and extracted with EtOAc. The EtOAc solution was washed with brine, dried (Na₂SO₄) and concentrated to yield the alcohol (0.42 g) that was used as is for the next step.

The above compound was dissolved in dry CH₂Cl₂ (10 mL) and Ph₃P (1.86 g) was added with stirring. After about 15 minutes a solution of CBr₄ (4.06 g) in CH₂Cl₂ was added dropwise with stirring. After 20 hours the reaction mixture was concentrated and the residue was purified via chromatography (silica gel; hexanes-20% EtOAc/hexanes) to yield the desired bromide (0.48 g).

All benzyl bromides were prepared in an analogous manner starting from the requisite acid.

### Cell Adhesion Protocol

Cell Adhesion - The recombinant protein ICAM-1•Fc was constructed from the 5 extracellular domains of human ICAM-1 and fusion with the constant region of human IgG. ICAM-1•Fc was purified by Protein A affinity chromatography and stored in aliquots at -20°C. Immobilized ICAM-1•Fc was prepared by dilution of the protein in PBS pH 7.5, transfer of 100 µl/well to Falcon Probind III plates and overnight incubation at 4°C. Wells coated with BSA served as a measure of non specific background adhesion. Washed plates were blocked with a solution of 0.25% ovalbumin in PBS for 1 h at 37°C. HBSS washed Jurkat cells were suspended to a final concentration of 2.5x10⁶/ml in TBSg adhesion buffer (24 mM Tris pH 7.4, 0.14 M NaCl, 2.7 mM KCl, 2 mM glucose, 0.1% HSA). A 100 µl volume of cells was added to the blocked and washed ICAM-1•Fc coated plates that contained 100 µl of plate buffer (TBSg, 10 mM MgCl₂, 2% DMSO) . Adhesion was for 1 h at 37°C. Non adherent cells were removed using the EL404 plate washer (BioTek Instruments; Highland Park, VT). The number of adherent cells was quantified by measuring enzymatic activity of endogenous N-acetyl-hexosaminidase using the enzyme substrate *p*-nitrophenol-N-acetyl-b-D-glucoseaminide, pNAG. The amount of liberated *p*-nitrophenol was measured by reading the optical density at 405 nm using a vertical pathway spectrophotometer to quantify cell attachment (VMAX Kinetic Microplate Reader, Molecular Devices, Menlo Park, CA). For competition studies the compounds from 100% DMSO stock solutions were diluted in plate buffer at 2-fold the required testing concentration prior to transfer to the ICAM-1·Fc coated plate and serial dilution.

The compound of the present invention has an IC₅₀ from low nM to µM in the Jurkat/ICAM -1 adhesion assay.

## Claims

1. A compound of the formula (I): 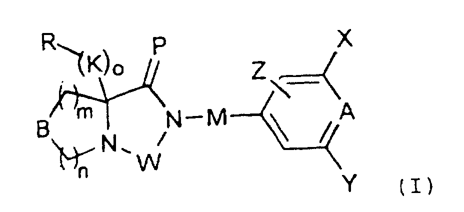 or a pharmaceutically acceptable salt thereof;
wherein
A is = C(Z¹)-, or = N-;
B is
1) -C(R¹) (R²)-,
2) -S-,
3) -SO-,
4) -SO₂-,
5) -O-,
6) -N(R³)-,
7) -N(COR⁴¹)-,
8) -N(CSR⁴¹)-,
9) -N(SO₂R⁵)-,
10) -N(R³)CO-,
11) -N(COR⁴¹)CO-,
12) -N(CSR⁴¹)CO-, or
13) -N(SO₂R⁵)CO-;
K is -CH₂-, -CH(OH)-, -C(=O), or -CF₂-;
M is a single bond, -(CH₂)ₚ-, -C(=O)- or -NH-;
W is one of the following groups: 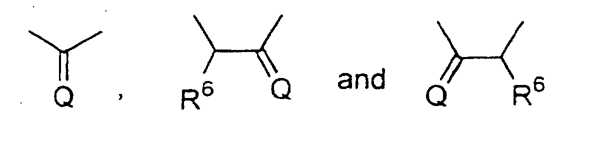 X and Y are independently
1) H,
2) halogen
3) NO₂
4) CN,
5) C₁₋₆ alkylthio,
6) NR³R⁶
7) C₁₋₆ alkyl optionally substituted with halogen,
8) C₁₋₆ alkoxy,
9) COR⁴²,
10) phenyl optionally substituted with:
a) C₁₋₆ alkyl optionally substituted with halogen,
b) C₁₋₆ alkoxy optionally substituted with halogen, or
c) CN,
11) isoxazolyl optionally substituted with C₁₋₆ alkyl,
12) pyrrolyl optionally substituted with C₁₋₆ alkoxycarbonyl or formyl, or
13) pyridyl,
Z and Z¹ are independently
1) H,
2) OH,
3) halogen,
4) NO₂,
5) CF₃,
6) NR³R⁶,
7) NHCOR⁴¹,
8) C₁₋₆ alkoxy optionally substituted with:
a) carboxyl,
b) C₁₋₆ alkoxycarbonyl, or
c) phenyl,
or
9) COR⁴²;
P and Q are independently O or S;
R is phenyl, naphthyl, pyridyl, benzofuryl or thiazolyl, and said phenyl, naphthyl, pyridyl, benzofuryl and thiazolyl may be substituted with a group selected from:
1) halogen,
2) OH,
3) CN,
4) C₁₋₆ alkyl optionally substituted with a group selected from:
a) halogen,
b) OR⁶, or
c) COR⁴¹,
5) C₁₋₆ alkoxy optionally substituted with a group selected from:
a) halogen,
b)NR³R⁶,
c) pyridyl, and
d) piperidinyl,
6) NO₂,
7) NR³R⁶,
8) NHCOR⁴¹,
9) NHSO₂R⁵,
10) COR⁴²,
11) C(=NH)NH₂,
12) CONHOH,
13) C₁₋₆ alkylthio which may be substituted with halogen,
14) C₁₋₆ alkylsulfinyl which may be substituted with halogen,
15) C₁₋₆ alkylsulfonyl which may be substituted with halogen,
16) C₁₋₃ alkylenedioxy optionally substituted with:
a) C₁₋₆ alkyl, or
b) halogen,
17) -C(=O)-(natural α-amino acid residue)
wherein said natural α-amino acid residue may be esterified with C₁₋₆ alkyl group,
18) phenyl optionally substituted with:
a) C₁₋₆ alkoxy,
b) C₁₋₆ alkyl optionally substituted with OR⁶, N(C₁₋₆ alkyl)₂ or COR⁴²,
c) CN,
d) COR⁴²,
e) C₂₋₇ alkenyl optionally substituted with COOR⁵,
f) NR⁶R⁶,
g) NO₂,
h) NHCOR⁴¹,
i) NHSO₂R⁵,
j) N(SO₂R⁵)₂,
k) NHCONHR⁵,
l) N(CONHR⁵)₂,
m) NHCSNHR⁵, or
n) pyrrolidinyl which may be substituted with C₁₋₆ alkyl,
19) furyl optionally substituted with CHO,
20) thienyl optionally substituted with CHO,
21) pyrrolyl optonally substituted with CHO and C₁₋₆ alkoxycarbonyl,
22) dihydroxazolyl optionally substituted with C₁₋₆ alkyl,
23) isoxazolyl optionally substituted with C₁₋₆ alkyl,
24) benzothienyl,
25) pyridyl,
26) tetrazolyl, and
27) thiazolyl which may be substituted with C₁₋₆ alkyl;
R¹ and R² are independently
1) H,
2) halogen,
3) OR³,
4) OCOR⁵,
5) SO₂R⁵,
6) NR³R⁶,
7) NR⁶COR⁴¹,
8) NR⁶CSR⁴¹,
9) NR⁶SO₂R⁵,
10) OCONR³R³,
11) N₃,
12) CN,
13) COR⁴², or
14) phenyl
or R¹ and R² combine with each other at the terminal thereof to form:
1) oxo,
2) methylene substituted with carboxyl, C₂₋₇ alkoxycarbonyl, or CONR^{a}R^{b}, or
3) C₂₋₃ alkylenedioxy;
R^{a} and R^{b} combine with each other at the terminals thereof to form a 3-7 membered ring together with the nitrogen atom to which they are attached, where said 3-7 membered ring may include additional heteroatoms such as oxygen, nitrogen and sulfur and may be substituted with C₁₋₆ alkyl, oxo, hydroxy, C₁₋₆ alkoxy or NR⁶R⁶;
R³ is
1) H,
2) C₁₋₆ alkyl which may be substituted with:
a) OH,
b) phenyl optionally substituted with halogen or C₁₋₆ alkoxy,
c) carboxyl,
d) carbamoyl,
e) NR⁶R⁶,,
f) C₁₋₆ alkoxycarbonyl,
g) morpholinyl,
h) pyridyl,
i) thienyl, or
j) pyrrolidinyl optionally substituted with oxo,
3) C₃₋₆ cycloalkyl,
4) phenyl which may be substituted with halogen,
5) pyridyl, which may be substituted with C₁₋₆ alkyl, or
6) morpholinyl;
R⁴¹ is
1) C₁₋₆ alkyl which may be substituted with a group selected from:
a) NR⁶R⁶,
b) carboxyl,
c) -CONR^{c}R^{d} where R^{c} and R^{d} are independently selected from hydrogen and C₁₋₆ alkyl, or R^{c} and R^{d} combine with each other at the terminals thereof to form a 3-7 membered ring together with the nitrogen atom to which they are attached, and said 1-3 heteroatoms such as oxygen, nitrogen and sulfur and may be substituted with C₁₋₆ alkyl, oxo, hydroxy, C₁₋₆ alkoxy or NR⁶R⁶,
d) C₁₋₆ alkoxycarbonylamino,
e) C₁₋₆ alkylsulfonylamino,
f) C₂₋₇ alkanoylamino, and
g) pyridyl,
2) C₁₋₆ alkoxy which may be substituted with NR⁶R⁶ or phenyl,
3) phenyl which may be substituted with:
a) carboxyl,
b) C₁₋₆ alkoxycarbonyl, or
c) NR⁶R⁶,
4) isoxazolyl which may be substituted with C₁₋₆ alkyl,
5) pyridyl,
6) thienyl,
7) furyl,
8) NR^{a}R^{b},
9) C₃₋₆ cycloalkyl, or
10) NR³R⁶;
R⁴² is
1) H,
2) OH,
3) C₁₋₆ alkyl which may be substituted with NR⁶R⁶ or phenyl,
4) C₁₋₆ alkoxy which may be substituted with NR⁶R⁶,
5) phenyl,
6) NR³R⁶, or
7) NR^{a}R^{b};
R⁵ is
1) C₁₋₆ alkyl which may be substituted with COR⁴², or
2) phenyl or naphthyl;
R⁶ is
1) H, or
2) C₁₋₆ alkyl which may be substituted with -N(C₁₋₆ alkyl)₂;
m is 0, 1, 2 or 3; n is 0, 1 or 2; o is 1 or 2; p is 1 or 2.

2. The compound according to claim 1, wherein X and Y are independently selected from:
1) halogen,
2) NO₂,
3) C₁₋₆ alkyl optionally substituted with halogen,
4) C₁₋₆ alkoxy group,
5) C₁₋₇ alkanoyl group,
6) CN,
7) carboxyl,
8) C₁₋₆ alkylthio,
9) NR³R⁶,
10) phenyl optionally substituted with:
a) C₁₋₆ alkyl optionally substituted with halogen,
b) C₁₋₆ alkoxy optionally substituted with halogen, or
c) CN,
11) isoxazolyl optionally substituted with C₁₋₆ alkyl,
12) pyrrolyl optionally substituted with C₁₋₆ alkoxycarbonyl or formyl,
13) pyridyl,
R is phenyl, naphthyl, pyridyl, benzofuryl or thiazolyl, wherein said phenyl, naphthyl, pyridyl, benzofuryl and thiazolyl may be substituted with a group selected from:
1) halogen,
2) OH,
3) CN,
4) C₁₋₆ alkyl optionally substituted with a group selected from:
a) halogen,
b) OR⁶, or
c) COR⁴¹,
5) C₁₋₆ alkoxy optionally substituted with a group selected from:
a) halogen,
b) NR³R⁶,
c) pyridyl, or
d) piperidinyl,
6) NO₂,
7) NR³R⁶,
8) NHCOR⁴¹,
9) NHSO₂R⁵,
10) COR⁴²,
11) C(=NH)NH₂,
12) CONHOH,
13) C₁₋₆ alkylthio which may be substituted with halogen,
14) C₁₋₆ alkylsulfinyl which may be substituted with halogen,
15) C₁₋₆ alkylsulfonyl which may be substituted with halogen,
16) C₁₋₃ alkylenedioxy optionally substituted with:
a) C₁₋₆ alkyl, or
b) halogen,
17) -C(=O)-(natural α-amino residue), wherein said natural α-amino acid is selected from aspartic acid, alanine, phenylalanine, and asparagine and said natural α-amino acid residue may be esterified with C₁₋₆ alkyl group,
18) phenyl optionally substituted with:
a) C₁₋₆ alkoxy,
b) C₁₋₆ alkyl optionally substituted with OR⁶, N(C₁₋₆ alkyl)₂ or COR⁴²,
c) CN,
d) COR⁴²,
e) C₂₋₇ alkenyl optionally substituted with COOR⁵,
f) NR⁶R⁶,
g) NO₂,
h) NHCOR⁴¹,
i) NHSO₂R⁵,
j) N(SO₂R⁵)₂,
k) NHCONHR⁵,
l) N(CONHR⁵)₂,
m) NHCSNHR⁵, or
n) pyrrolidinyl which may be substituted with C₁₋₆ alkyl,
19) furyl optionally substituted with CHO,
20) thienyl optionally substituted with CHO,
21) pyrrolyl optionally substituted with CHO and C₁₋₆ alkoxycarbonyl,
22) dihydroxazolyl optionally substituted with C₁₋₆ alkyl,
23) isoxazolyl optionally substituted with C₁₋₆ alkyl,
24) benzothienyl,
25) pyridyl,
26) tetrazolyl, and
27) thiazolyl which may be substituted with C₁₋₆ alkyl;
R¹ and R² are independently selected from:
1) H,
2) halogen,
3) OR³,
4) OCOR⁵,
5) NR³R⁶,
6) NR⁶COR⁴¹,
7) NHCSR⁴¹,
8) NHSO₂R⁵,
9) N₃,
10) COR⁴², or
11) phenyl;
or R¹ and R² combine with each other at the terminal thereof to form oxo;
R³ is
1) hydrogen;
2) C₁₋₆ alkyl optionally substituted with:
a) OH,
b) phenyl optionally substituted with halogen or C₁₋₆ alkoxy,
c) carboxy,
d) carbamoyl,
e) NR⁶R⁶,
f) C₁₋₆ alkoxycarbonyl,
g) morpholinyl,
h) pyridyl,
i) thienyl, or
j) pyrrolidinyl optionally substituted with oxo,
3) C₃₋₆ cycloalkyl,
4) phenyl optionally substituted with halogen,
5) pyridyl optionally substituted with C₁₋₆ alkyl, or
6) morpholinyl;
R⁴¹ is
1) C₁₋₆ alkyl optionally substituted with a group selected from:
a) NR⁶R⁶,
b) carboxyl,
c) -CONR^{c}R^{d} where R^{c} and R^{d} are independently selected from hydrogen and C₁₋₆ alkyl, or R^{c} and R^{d} combine with each other at the terminals thereof to form a 3-7 membered ring together with the nitrogen atom to which they are attached, and said 3-7 membered ring may include additional 1-3 heteroatoms such as oxygen, nitrogen and sulfur and may be substituted with C₁₋₆ alkyl, oxo, hydroxy, C₁₋₆ alkoxy or NR⁶R⁶,
d) C₁₋₆ alkoxycarbonylamino,
e) C₁₋₆ alkylsulfonylamino,
f) C₂₋₇ alkanoylamino, and
g) pyridyl;
2) C₁₋₆ alkoxy optionally substituted with NR⁶R⁶ or phenyl,
3) NR³R⁶,
4) phenyl optionally substituted with:
a) carboxyl,
b) C₁₋₆ alkoxycarbonyl, or
c) NR⁶R⁶,
5) C₃₋₆ cycloalkyl,
6) isoxazolyl optionally substituted with C₁₋₆ alkyl,
7) pyridyl,
8) thienyl,
9) furyl, or
10) NR^{a}R^{b};
R⁴² is
1) H,
2) OH,
3) C₁₋₆ alkyl optionally substituted with NR⁶R⁶ or phenyl,
4) C₁₋₆ alkoxy optionally substituted with NR⁶R⁶,
5) NR³R⁶,
6) NR^{a}R^{b}, or
7) pyridyl which may be substituted with C₁₋₆ alky;
R⁵ is
1) C₁₋₆ alkyl optionally substituted with COR⁴², or
2) phenyl or naphthyl;
R⁶ is
1) hydrogen, or
2) C₁₋₆ alkyl optionally substituted with -N(C₁₋₆ alkyl)₂.

3. The compound according to claim 1, wherein the chemical structure is the following: 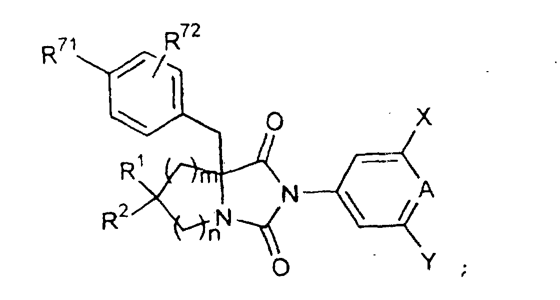 wherein
A is =C(Z¹)-, or =N-;
X and Y are independently
1) H,
2) halogen,
3) NO₂,
4) CN,
5) C₁₋₆ alkylthio,
6) NR³R⁶, or
7) C₁₋₆ alkyl optionally substituted with halogen;
Z¹ is selected from
1) H,
2) OH,
3) halogen,
4) NR³R⁶,
5) NHCOR⁴¹,
6) C₁₋₆ alkoxy optionally substituted with:
a) carboxyl,
b) C₁₋₆ alkoxycarbonyl, or
c) phenyl
and
7) COR⁴²;
R¹ and R² are independently H, halogen, OR³, NR³R⁶, NHCOR⁴¹, NHCSR⁴¹, NHSO₂R⁵, N₃, COR⁴², or phenyl;
or R¹ and R² combine with each other at the terminal thereof to form oxo;
R⁷¹ is a group selected from:
1) H,
2) OH,
3) halogen,
4) CN,
5) C₁₋₆ alkyl optionally substituted with halogen or OR⁶,
6) C₁₋₆ alkoxy optionally substituted with halogen, NR³R⁶, pyridyl, or piperidinyl
7) NO₂,
8) NR³R⁶,
9) NHCOR⁴¹,
10) NHSO₂R⁵,
11) COR⁴²,
12) C(=NH)NH₂,
13) CONHOH,
14) C₁₋₆ alkylthio,
15) C₁₋₆ alkylsulfinyl,
16) C₁₋₆ alkylsulfonyl,
17) phenyl which may be substituted with a group selected from a) C₂₋₇ alkenyl substituted with COOR⁵, b) COR⁴², c) C₁₋₆ alkyl optionally substituted with OR⁶, d) C₁₋₆ alkoxy, and e) CN,
18) thienyl which may be substituted with CHO,
19) furyl which may be substituted with CHO,
20) tetrazolyl,
21) dihydroxazolyl,
22) pyrrolyl which may be substituted with CHO,
23) isoxazolyl substituted with C₁₋₆ alkyl,
24) benzothienyl;
R⁷² is a group selected from:
1) hydrogen,
2) halogen,
3) CN,
4) C₁₋₆ alkyl optionally substituted with OR⁶, or
5) NO₂;
or, R⁷¹ and R⁷² combine with each other at the terminal thereof to form C₁₋₃ alkylenedioxy optionally substituted with halogen;
m is 0, 1, or 2, and n is 0 or 1.

4. The compound according to claim 3, wherein
A is =CH- or =N-;
X and Y are independently
1) halogen,
2) NO₂,
3) NR³R⁶, or
4) C₁₋₆ alkyl optionally substituted with halogen;
one of R¹ and R² is H, and the other is H, OH, halogen, NR³R⁶, NHCOR⁴¹, NHCSR⁴¹, NHSO₂R⁵, N₃, COR⁴², or phenyl;
or R¹ and R² combine with each other at the terminal thereof to form oxo;
R⁷¹ is a group selected from:
1) H,
2) halogen,
3) CN,
4) C₁₋₆ alkyl optionally substituted with halogen,
5) C₁₋₆ alkoxy optionally substituted with halogen,
6) COR⁴²,
7) C₁₋₆ alkylthio,
8) phenyl which may be substituted with a group selected from a) C₂₋₇ alkenyl substituted with COOR⁵, b) COR⁴², c) C₁₋₆ alkyl optionally substituted with OR⁶, d) C₁₋₆ alkoxy, and e) CN,
9) thienyl which may be substituted with CHO,
10) furyl which may be substituted with CHO,
11) pyrrolyl which may be substituted with CHO,
12) isoxazolyl substituted with C₁₋₆ alkyl;
R⁷² is hydrogen;
or, R⁷¹ and R⁷² combine with each other at the terminal thereof to form C₁₋₃ alkylenedioxy substituted with halogen;
m is l, and n is 1.

5. The compound according to claim 4, wherein
X and Y are independently halogen;
one of R¹ and R² is H, and the other is H, OH, NR³R⁶, NHCOR⁴¹, NHCSR⁴¹, or COR⁴²;
R⁴¹ is
1) C₁₋₆ alkyl optionally substituted with a group selected from:
a) NR⁶R⁶,
b) carboxyl,
c) carbamoyl,
d) piperazinylcarbonyl optionally substituted with C₁₋₆ alkyl,
e) C₂₋₇ alkanoylamino, and
f) pyridyl;
2) C₁₋₆ alkoxy,
3) NR³R⁶,
4) C₃₋₆ cycloalkyl,
5) pyridyl,
6) thienyl,
7) furyl, or
8) pyrrolidinyl;
R⁴² is NR³R⁶ or morpholinyl;
R⁷¹ is
1) halogen,
2) CN, or
3) C₁₋₆ alkoxy optionally substituted with halogen; and
R⁷² is hydrogen.

6. The compound according to claim 5, wherein
X and Y are independently halogen;
one of R¹ and R² is H, and the other is OH, NHCOR⁴¹ or COR⁴²;
R⁴¹ is
a) C₁₋₆ alkyl optionally substituted with a group selected from carboxyl, carbamoyl, and piperazinylcarbonyl substituted with C₁₋₆ alkyl,
b) NH₂,
c) NH(C₁₋₆ alkyl) ,
d) pyridyl, or
e) pyrrolidinyl,
R⁴² is NH₂, NH(C₁₋₆ alkyl) or morpholinyl;
R⁷¹ is C₁₋₆ alkoxy substituted with halogen; and
R⁷² is hydrogen.

7. The compound according to claim 1, wherein the chemical structure is the following: 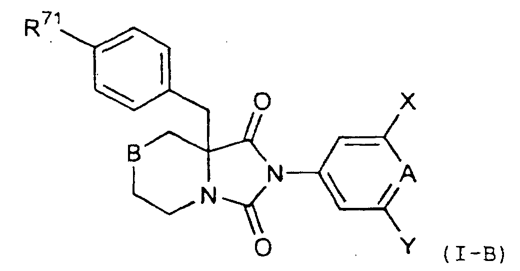 wherein A is =CH- or =N-;
B is -S-, -SO-, -SO₂-, -N(R³)-, -N(COR⁴¹)-, -N(CSR⁴¹)- or -N(SO₂R⁵)-;
X and Y are independently H, halogen, NO₂, or C₁₋₆ alkyl;
R⁷¹ is a group selected from:
1) H,
2) halogen,
3) CN,
4) C₁₋₆ alkyl optionally substituted with halogen,
5) C₁₋₆ alkoxy optionally substituted with halogen,
6) COR⁴²,
7) C₁₋₆ alkylthio,
8) phenyl,
9) thienyl,
10) furyl,
11) pyrrolyl,
12) isoxazolyl substituted with C₁₋₆ alkyl,
wherein said phenyl may be substituted with a group selected from a) C₂₋₇ alkenyl substituted with COOR⁵, b) COR⁴², c) C₁₋₆ alkyl optionally substituted with OR⁶, d) C₁₋₆ alkoxy, and e) CN, and said thienyl, furyl and pyrrolyl may be substituted with CHO.

8. The compound according to claim 7, wherein
B is -N(COR⁴¹)-;
X and Y are independently halogen;
R⁴¹ is
1) C₁₋₆ alkyl optionally substituted with a group selected from:
a) NR⁶R⁶,
b) carbamoyl, and
c) piperazinylcarbonyl optionally substituted with C₁₋₆ alkyl;
2) C₁₋₆ alkoxy, or
3) NR³R⁶;
R⁷¹ is
1) halogen, or
2) C₁₋₆ alkoxy optionally substituted with halogen.

9. The compound according to claim 1, wherein the chemical structure is the following: 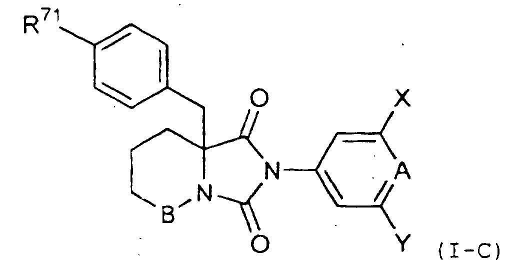 wherein A is =CH- or =N-;
B is -N(R³)-, -N(COR⁴¹)-, -N(CSR⁴¹)- or -N(SO₂R⁵)-;
X and Y are independently H, halogen, NO₂, or C₁₋₆ alkyl;
R⁷¹ is a group selected from:
1) H,
2) halogen,
3) CN,
4) C₁₋₆ alkyl optionally substituted with halogen,
5) C₁₋₆ alkoxy optionally substituted with halogen,
6) COR⁴²,
7) C₁₋₆ alkylthio,
8) phenyl,
9) thienyl,
10) furyl,
11) pyrrolyl,
12) isoxazolyl substituted with C₁₋₆ alkyl,
wherein said phenyl may be substituted with a group selected from a) C₂₋₇ alkenyl substituted with COOR⁵, b) COR⁴², c) C₁₋₆ alkyl optionally substituted with OR⁶, d) C₁₋₆ alkoxy, and e) CN, and said thienyl, furyl and pyrrolyl may be substituted with CHO.

10. The compound according to claim 1, wherein the chemical structure is the following: 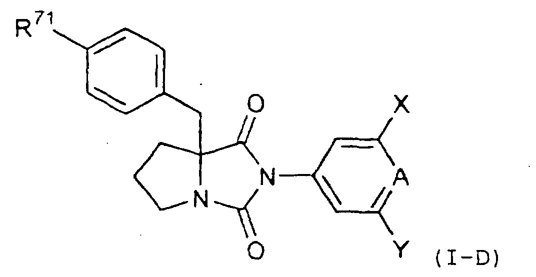 wherein
A is =CH-, or =N-
X is H or halogen;
Y is
1) pyrrolyl optionally substituted with formyl,
2) phenyl optionally substituted with a) CN, b) C₁₋₆ alkyl optionally substituted with halogen, c) C₁₋₆ alkoxy optionally substituted with halogen, or
3) isoxazolyl optionally substituted with C₁₋₆ alkyl;
R⁷¹ is a group selected from:
1) H,
2) halogen,
3) CN,
4) C₁₋₆ alkyl optionally substituted with halogen,
5) C₁₋₆ alkoxy optionally substituted with halogen,
6) COR⁴²,
7) C₁₋₆ alkylthio,
8) phenyl,
9) thienyl,
10) furyl,
11) pyrrolyl,
12) isoxazolyl substituted with C₁₋₆ alkyl,
wherein said phenyl may be substituted with a group selected from a) C₂₋₇ alkenyl substituted with COOR⁵, b) COR⁴², c) C₁₋₆ alkyl optionally substituted with OR⁶, d) C₁₋₆ alkoxy, and e) CN, and said thienyl, furyl and pyrrolyl may be substituted with CHO.

11. The compound according to claim 1, wherein the compound is selected from the group consisting of:
(5R, 7R)-5-[4-(trifluoromethoxy)benzyl]-3-(3,5-dichlorophenyl)-7-hydroxy-1,3-diazabicyclo[3.3.0]octane-2,4-dione;
(5R, 7S)-5-[4-(trifluoromethoxy)benzyl]-3-(3,5-dichlorophenyl)-7-acetylamino-1,3-diazabicyclo[3.3.0]octane-2,4-dione;
(5R, 7S)-5-[4-(trifluoromethoxy)benzyl]-3-(3,5-dichlorophenyl)-7-(3-carbamoylpropionylamino)-1,3-diazabicyclo[3.3.0]octane-2,4-dione;
(5R, 7S)-5-[4-(trifluoromethoxy)benzyl]-3-(3,5-dichlorophenyl)-7-[3-(4-methyl-1-piperazinylcarbonyl)propionylamino]-1,3-diazabicyclo[3.3.0]octane-2,4-dione;
(5R, 7S)-5-[4-(trifluoromethoxy)benzyl]-3-(3,5-dichlorophenyl) -7-nicotinoylamino-1,3-diazabicyclo[3.3.0]octane-2,4-dione;
(5R, 7S)-5-[4-(trifluoromethoxy)benzyl]-3-(3,5-dichlorophenyl)-7-(1-pyrrolidinylcarbonylamino)-1,3-diazabicyclo[3.3.0]octane-2,4-dione;
(5R, 7R)-5-[4-(trifluoromethoxy)benzyl]-3-(3,5-dichlorophenyl)-7-carbamoyl-1,3-diazabicyclo[3.3.0]octane-2,4-dione;
(5R, 7R)-5-[4-(trifluoromethoxy)benzyl]-3-(3,5-dichlorophenyl)-7-morpholinocarbonyl-1,3-diazabicyclo[3.3.0]octane-2,4-dione;
(5R, 7R)-5-[4-(trifluoromethoxy)benzyl]-3-(3,5-dichlorophenyl)-7-dimethylcarbamoyl-1,3-diazabicyclo[3.3.0]octane-2,4-dione;
(5R, 7R)-5-[4-(trifluoromethoxy)benzyl]-3-(3,5-dichlorophenyl)-7-methylcarbamoyl-1,3-diazabicyclo[3.3.0]octane-2,4-dione; and
(5R, 7R)-5-[4-(trifluoromethoxy)benzyl]-3-(3,5-dichlorophenyl)-7-morpholinocarbamoyl-1, 3-diazabicyclo[3.3.0]octane-2,4-dione.

12. A process for preparing a compound of the formula (I): 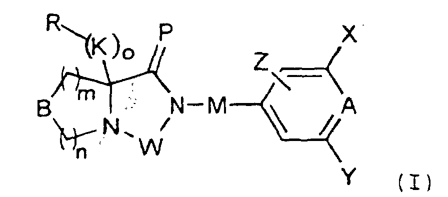 wherein the symbols are the same as defined in claim 1, or a pharmaceutically acceptable salt thereof, which comprises cyclizing the compound of the formula (II): 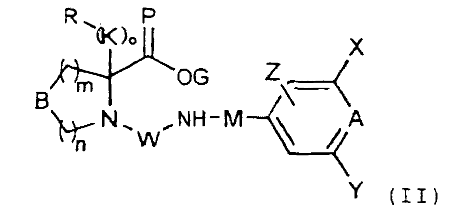 wherein OG is a hydroxyl group, a protected hydroxyl group, or a resin-bound hydroxyl group, and the other symbols are the same as defined above, and converting into the pharmaceutically acceptable salt, if necessary.

13. A process for preparing a compound of the formula (I): 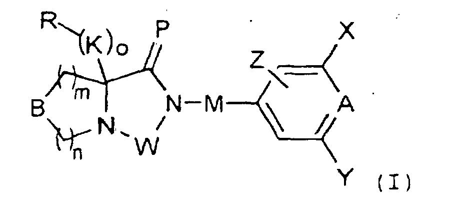 wherein the symbols are the same as defined in claim 1, or a pharmaceutically acceptable salt thereof, which comprises reacting the compound of the formula (III): 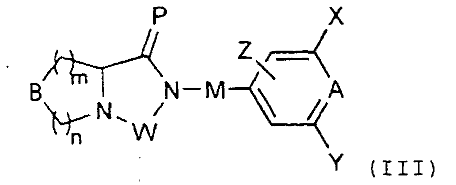 wherein the symbols are the same as defined above, with the compound of the formula (IV):
R-(K)ₒ-L (IV)
wherein L is a leaving group and the other symbols are the same as defined in claim 1, and optionally converting into the pharmaceutically acceptable salt, if necessary.

14. A process for preparing a compound of the formula (I): 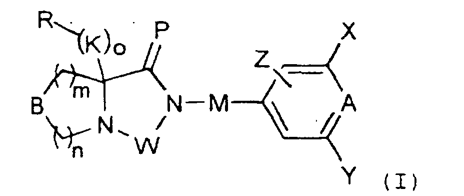 wherein the symbols are the same as defined in claim 1, or a pharmaceutically acceptable salt thereof, which comprises reacting the compound of the formula (V): 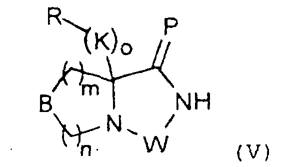 wherein the symbols are the same as defined in claim 1, with the compound of the formula (VI): 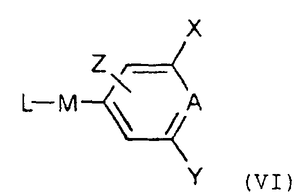 wherein L is a leaving group and the other symbols are the same as defined in claim 1 and converting the resulting compound into a pharmaceutically acceptable salt if necessary.

15. A pharmaceutical composition which comprises a therapeutically effective amount of the compound as set forth in claim 1 in admixture with a therapeutically acceptable carrier or diluent.

16. Use of the compound as set forth in claim 1 for treatment or prevention of α_{L}β₂ adhesion mediated condition in a mammal.

17. Use according to claim 16, said condition is selected from rheumatoid arthritis, asthma, allergy conditions, adult respiratory distress syndrome, AIDS, cardiovascular diseases, thrombosis or harmful platelet aggregation, reocclusion following thrombolysis, allograft rejection, reperfusion injury, stroke, psoriasis, eczema, skin inflammatory diseases such as contact dermatitis and atopic dermatitis, osteoporosis, osteoarthritis, atherosclerosis (including graft arteriosclerosis after transplantation), neoplastic diseases including metastasis of neoplastic or cancerous growth, wound healing enhancement, eye diseases such as detaching retina, Type I diabetes, multiple sclerosis, systemic lupus erythematosus (SLE), inflammatory and immunoinflammatory conditions including ophthalmic inflammatory conditions and inflammatory bowel diseases (Crohn's disease, ulcerative colitis), regional enteritis, Sjogren's Syndrome, and other autoimmune diseases, pancreatitis, delayed graft function, intimal hyperplasia; myocardial reinfarction or restenosis after surgery such as percutaneous transluminal coronary angioplasty (PTCA), rejection after transplantation (chronic rejection and acute rejection), host vs. graft or graft vs. host diseases, and cancer.

18. Use according to claim 17, wherein said diseases is selected from psoriasis, rheumatoid arthritis, inflammatory bowel diseases (Crohn's disease, ulcerative colitis), systemic lupus erythematosus, atopic dermatitis, Sjogren's Syndrome, rejection after transplantation (chronic rejection, acute rejection), and graft vs. host disease.

## Patentansprüche

1. Verbindung der Formel (I): 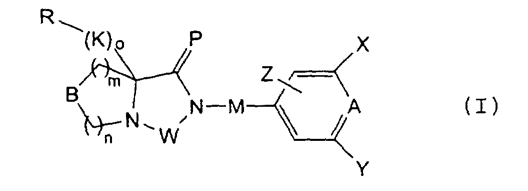 oder ein pharmazeutisch annehmbares Salz davon;
worin gilt:
A ist =C(Z¹)- oder =N-;
B ist
1) -C(R¹) (R²)-
2) -S-
3) -SO-
4) -SO₂-
5) -O-
6) -N(R³)-
7) -N(COR⁴¹)-
8) -N(CSR⁴¹)-
9) -N(SO₂R⁵)-
10) -N(R³)CO-
11) -N(COR⁴¹)CO-
12) -N(CSR⁴¹)CO- oder
13) -N(SO₂R⁵)CO-;
K ist -CH₂-, -CH(OH)-, -C(=O) oder -CF₂-;
M ist eine Einfachbindung, -(CH₂)ₚ-, -C(=O)- oder -NH-;
W ist eine der folgenden Gruppen: 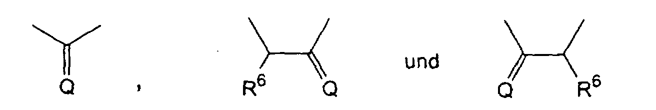 X und Y sind unabhängig voneinander
1) H
2) Halogen
3) NO₂
4) CN
5) C₁₋₆-Alkylthio
6) NR³R⁶
7) C₁₋₆-Alkyl, optional substituiert mit Halogen
8) C₁₋₆-Alkoxy
9) COR⁴²
10) Phenyl, optional substituiert mit:
a) C₁₋₆-Alkyl, optional substituiert mit Halogen
b) C₁₋₆-Alkoxy, optional substituiert mit Halogen, oder
c) CN
11) Isoxazolyl, optional substituiert mit C₁₋₆-Alkyl
12) Pyrrolyl, optional substituiert mit C₁₋₆-Alkoxycarbonyl oder Formyl, oder
13) Pyridyl;
Z and Z¹ sind jeweils unabhängig voneinander
1) H
2) OH
3) Halogen
4) NO₂
5) CF₃
6) NR³R⁶
7) NHCOR⁴¹
8) C₁₋₆-Alkoxy, optional substituiert mit:
a) Carboxyl
b) C₁₋₆-Alkoxycarbonyl oder
c) Phenyl
oder
9) COR⁴²;
P und Q sind jeweils unabhängig voneinander O oder S; R ist Phenyl, Naphthyl, Pyridyl, Benzofuryl oder Thiazolyl, wobei das Phenyl, Naphthyl, Pyridyl, Benzofuryl und Thiazolyl mit einer Gruppe substituiert sein kann, ausgewählt aus:
1) Halogen
2) OH
3) CN
4) C₁₋₆-Alkyl, optional substituiert mit einer Gruppe ausgewählt aus:
a) Halogen
b) OR⁶ oder
c) COR⁴¹
5) C₁₋₆-Alkoxy, optional substituiert mit einer Gruppe ausgewählt aus:
a) Halogen
b) NR³R⁶
c) Pyridyl und
d) Piperidinyl
6) NO₂
7) NR³R⁶
8) NHCOR⁴¹
9) NHSO₂R⁵
10) COR⁴²
11) C(=NH)NH₂
12) CONHOH
13) C₁₋₆-Alkylthio, das mit Halogen substituiert sein kann
14) C₁₋₆-Alkylsulfinyl, das mit Halogen substituiert sein kann
15) C₁₋₆-Alkylsulfonyl, das mit Halogen substituiert sein kann
16) C₁₋₃-Alkylendioxy, optional substituiert mit:
a) C₁₋₆-Alkyl oder
b) Halogen
17) -C(=O)- (natürlicher α-Aminosäurerest), worin der natürliche α-Aminosäurerest mit einer C₁₋₆-Alkylgruppe verestert sein kann
18) Phenyl, optional substituiert mit:
a) C₁₋₆-Alkoxy
b) C₁₋₆-Alkyl, optional substituiert mit OR⁶, N(C₁₋₆-Alkyl)₂ oder COR⁴²
c) CN
d) COR⁴²
e) C₂₋₇-Alkenyl, optional substituiert mit COOR⁵
f) NR⁶R⁶
g) NO₂
h) NHCOR⁴¹
i) NHSO₂R⁵
j) N(SO₂R⁵)₂
k) NHCONHR⁵
l) N (CONHR⁵)₂
m) NHCSNHR⁵ oder
n) Pyrrolidinyl, das mit C₁₋₆-Alkyl substituiert sein kann
19) Furyl, optional substituiert mit CHO
20) Thienyl, optional substituiert mit CHO
21) Pyrrolyl, optional substituiert mit CHO und C₁₋₆-Alkoxycarbonyl
22) Dihydroxazolyl, optional substituiert mit C₁₋₆-Alkyl
23) Isoxazolyl, optional substituiert mit C₁₋₆-Alkyl
24) Benzothienyl
25) Pyridyl
26) Tetrazolyl und
27) Thiazolyl, das mit C₁₋₆-Alkyl substituiert sein kann;
R¹ und R² sind unabhängig voneinander
1) H
2) Halogen
3) OR³
4) OCOR⁵
5) SO₂R⁵
6) NR³R⁶
7) NR⁶COR⁴¹
8) NR⁶CSR⁴¹
9) NR⁶SO₂R⁵
10) OCONR³R³
11) N₃
12) CN
13) COR⁴² oder
14) Phenyl
oder R¹ und R² sind miteinander an ihrem Ende verbunden, um
1) Oxo
2) Methylen, substituiert mit Carboxyl, C₂₋₇-Alkoxycarbonyl oder CONR^{a}R^{b}, oder
3) C₂₋₃-Alkylendioxy
zu bilden;
R^{a} und R^{b} sind miteinander an ihren Enden verbunden, um einen 3-7 gliedrigen Ring zusammen mit dem Stickstoffatom zu bilden, an welches sie gebunden sind,
worin der 3-7 gliedrige Ring zusätzliche Heteroatome, wie z.B. Sauerstoff, Stickstoff und Schwefel, einschließen kann und mit C₁₋₆-Alkyl, Oxo, Hydroxy, C₁₋₆-Alkoxy oder NR⁶R⁶ substituiert sein kann;
R³ ist
1) H
2) C₁₋₆-Alkyl, das substituiert sein kann mit:
a) OH
b) Phenyl, optional substituiert mit Halogen oder C₁₋₆-Alkoxy
c) Carboxyl
d) Carbamoyl
e) NR⁶R⁶
f) C₁₋₆-Alkoxycarbonyl
g) Morpholinyl
h) Pyridyl
i) Thienyl oder
j) Pyrrolidinyl, optional substituiert mit Oxo
3) C₃₋₆-Cycloalkyl
4) Phenyl, das mit Halogen substituiert sein kann
5) Pyridyl, das mit C₁₋₆-Alkyl substituiert sein kann, oder
6) Morpholinyl;
R⁴¹ ist
1) C₁₋₆-Alkyl, das mit einer Gruppe substituiert sein kann, ausgewählt aus
a) NR⁶R⁶
b) Carboxyl
c) -CONR^{c}R^{d}, worin R^{c} und R^{d} unabhängig voneinander ausgewählt sind aus Wasserstoff und C₁₋₆-Alkyl, oder R^{c} and R^{d} an ihren Enden miteinander verbunden sind, um einen 3-7 gliedrigen Ring mit dem Stickstoffatom zu bilden, an welches sie gebunden sind, und die 1-3 Heteroatome, wie z.B. Sauerstoff, Stickstoff und Schwefel, und mit C₁₋₆-Alkyl, Oxo, Hydroxy, C₁₋₆-Alkoxy oder NR⁶R⁶ substituiert sein können
d) C₁₋₆-Alkoxycarbonylamino
e) C₁₋₆-Alkylsulfonylamino
f) C₂₋₇-Alkanoylamino und
g) Pyridyl
2) C₁₋₆-Alkoxy, das mit NR⁶R⁶ oder Phenyl substituiert sein kann
3) Phenyl, das substituiert sein kann mit:
a) Carboxyl
b) C₁₋₆-Alkoxycarbonyl oder
c) NR⁶R⁶
4) Isoxazolyl, das mit C₁₋₆-Alkyl substituiert sein kann
5) Pyridyl
6) Thienyl
7) Furyl
8) NR^{a}R^{b}
9) C₃₋₆-Cycloalkyl oder
10) NR³R⁶;
R⁴² ist
1) H
2) OH
3) C₁₋₆-Alkyl, das mit NR⁶R⁶ oder Phenyl substituiert sein kann
4) C₁₋₆-Alkoxy, das mit NR⁶R⁶ substituiert sein kann
5) Phenyl
6) NR³R⁶ oder
7) NR^{a}R^{b};
R⁵ ist
1) C₁₋₆-Alkyl, das mit COR⁴² substituiert sein kann oder
2) Phenyl oder Naphthyl;
R⁶ ist
1) H oder
2) C₁₋₆-Alkyl, das mit -N(C₁₋₆ alkyl)₂ substituiert sein kann;
m ist 0, 1, 2 oder 3; n ist 0, 1 oder 2; o ist 1 oder 2; p ist 1 oder 2.

2. Verbindung gemäß Anspruch 1, worin X und Y unabhängig voneinander ausgewählt sind aus:
1) Halogen
2) NO₂
3) C₁₋₆-Alkyl, optional substituiert mit Halogen
4) C₁₋₆-Alkoxygruppe
5) C₁₋₇-Alkanoylgruppe
6) CN
7) Carboxyl
8) C₁₋₆-Alkylthio
9) NR³R⁶
10) Phenyl, optional substituiert mit:
a) C₁₋₆-Alkyl, optional substituiert mit Halogen
b) C₁₋₆-Alkoxy, optional substituiert mit Halogen oder
c) CN
11) Isoxazolyl, optional substituiert mit C₁₋₆-Alkyl
12) Pyrrolyl, optional substituiert mit C₁₋₆-Alkoxycarbonyl oder Formyl
13) Pyridyl;
R ist Phenyl, Naphthyl, Pyridyl, Benzofuryl oder Thiazolyl, worin das Phenyl, Naphthyl, Pyridyl, Benzofuryl und Thiazolyl substituiert sein kann durch eine Gruppe ausgewählt aus:
1) Halogen
2) OH
3) CN
4) C₁₋₆-Alkyl, optional substituiert mit einer Gruppe ausgewählt aus:
a) Halogen
b) OR⁶ oder
c) COR⁴¹
5) C₁₋₆-Alkoxy, optional substituiert mit einer Gruppe ausgewählt aus:
a) Halogen
b) NR³R⁶
c) Pyridyl oder
d) Piperidinyl
6) NO₂
7) NR³R⁶
8) NHCOR⁴¹
9) NHSO₂R⁵
10) COR⁴²
11) C(=NH)NH₂
12) CONHOH
13) C₁₋₆-Alkylthio, das mit Halogen substituiert sein kann
14) C₁₋₆-Alkylsulfinyl, das mit Halogen substituiert sein kann
15) C₁₋₆-Alkylsulfonyl, das mit Halogen substituiert sein kann
16) C₁₋₃-Alkylendioxy, optional substituiert mit:
a) C₁₋₆-Alkyl oder
b) Halogen
17) -C(=O)- (natürlicher α-Aminosäurerest), worin die natürliche α-Aminosäure ausgewählt ist aus Asparaginsäure, Alanin, Phenylalanin und Asparagin, und der natürliche α-Aminosäurerest mit einer C₁₋₆-Alkylgruppe verestert sein kann
18) Phenyl, optional substituiert mit:
a) C₁₋₆-Alkoxy
b) C₁₋₆-Alkyl, optional substituiert mit OR⁶, N(C₁₋₆-Alkyl)₂ oder COR⁴²
c) CN
d) COR⁴²
e) C₂₋₇-Alkenyl, optional substituiert mit COOR⁵
f) NR⁶R⁶
g) NO₂
h) NHCOR⁴¹
i) NHSO₂R⁵
j) N(SO₂R⁵)₂
k) NHCONHR⁵
l) N(CONHR⁵)₂
m) NHCSNHR⁵ oder
n) Pyrrolidinyl, das mit C₁₋₆-Alkyl substituiert sein kann,
19) Furyl, optional substituiert mit CHO
20) Thienyl, optional substituiert mit CHO
21) Pyrrolyl, optional substituiert mit CHO und C₁₋₆-Alkoxycarbonyl
22) Dihydroxazolyl, optional substituiert mit C₁₋₆-Alkyl
23) Isoxazolyl, optional substituiert mit C₁₋₆-Alkyl
24) Benzothienyl
25) Pyridyl
26) Tetrazolyl und
27) Thiazolyl, das mit C₁₋₆-Alkyl substituiert sein kann;
R¹ und R² sind unabhängig ausgewählt aus:
1) H
2) Halogen
3) OR³
4) OCOR⁵
5) NR³R⁶
6) NR⁶COR⁴¹
7) NHCSR⁴¹
8) NHSO₂R⁵
9) N₃
10) COR⁴² oder
11) Phenyl;
oder R¹ and R² sind miteinander an ihren Enden verbunden, um Oxo zu bilden;
R³ ist
1) Wasserstoff
2) C₁₋₆-Alkyl, optional substituiert mit:
a) OH
b) Phenyl, optional substituiert mit Halogen oder C₁₋₆-Alkoxy
c) Carboxy
d) Carbamoyl
e) NR⁶R⁶
f) C₁₋₆-Alkoxycarbonyl
g) Morpholinyl
h) Pyridyl
i) Thienyl oder
j) Pyrrolidinyl, optional substituiert mit Oxo
3) C₃₋₆-Cycloalkyl
4) Phenyl, optional substituiert mit Halogen
5) Pyridyl, optional substituiert mit C₁₋₆-Alkyl oder
6) Morpholinyl;
R⁴¹ ist
1) C₁₋₆-Alkyl, optional substituiert mit einer Gruppe ausgewählt aus:
a) NR⁶R⁶
b) Carboxyl
c) -CONR^{c}R^{d}, worin R^{c} und R^{d} unabhängig ausgewählt sind aus Wasserstoff und C₁₋₆-Alkyl, oder R^{c} und R^{d} miteinander an ihren Enden verbunden sind, um einen 3-7 gliedrigen Ring zusammen mit dem Stickstoffatom zu bilden, an welches sie gebunden sind, worin der 3-7 gliedrige Ring zusätzlich 1-3 Heteroatome, wie z.B. Sauerstoff, Stickstoff und Schwefel, einschließen kann und mit C₁₋₆-Alkyl, Oxo, Hydroxy, C₁₋₆-Alkoxy oder NR⁶R⁶ substituiert sein kann
d) C₁₋₆-Alkoxycarbonylamino
e) C₁₋₆-Alkylsulfonylamino
f) C₂₋₇-Alkanoylamino und
g) Pyridyl
2) C₁₋₆-Alkoxy, optional substituiert mit NR⁶R⁶ oder Phenyl
3) NR³R⁶
4) Phenyl, optional substituiert mit:
a) Carboxyl
b) C₁₋₆-Alkoxycarbonyl oder
c) NR⁶R⁶
5) C₃₋₆-Cycloalkyl
6) Isoxazolyl, optional substituiert mit C₁₋₆-Alkyl
7) Pyridyl
8) Thienyl
9) Furyl oder
10) NR^{a}R^{b};
R⁴² ist
1) H
2) OH
3) C₁₋₆-Alkyl, optional substituiert mit NR⁶R⁶ oder Phenyl
4) C₁₋₆-Alkoxy, optional substituiert mit NR⁶R⁶
5) NR³R⁶
6) NR^{a}R^{b} oder
7) Pyridyl, das mit C₁₋₆-Alkyl substituiert sein kann;
R⁵ ist
1) C₁₋₆-Alkyl, optional substituiert mit COR⁴² oder
2) Phenyl oder Naphthyl;
R⁶ ist
1) Wasserstoff oder
2) C₁₋₆-Alkyl, optional substituiert mit -N(C₁₋₆-Alkyl)₂.

3. Verbindung gemäß Anspruch 1, worin die chemische Struktur die folgende ist: 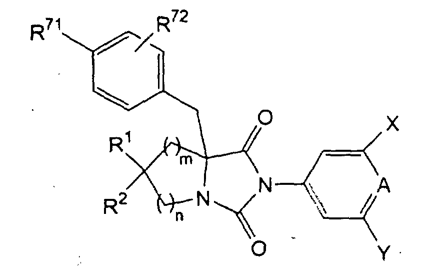 worin gilt:
A ist =C(Z¹)- oder =N-
X und Y sind jeweils unabhängig voneinander
1) H
2) Halogen
3) NO₂
4) CN
5) C₁₋₆-Alkylthio
6) NR³R⁶ oder
7) C₁₋₆-Alkyl, optional substituiert mit Halogen;
Z¹ ist ausgewählt aus:
1) H
2) OH
3) Halogen
4) NR³R⁶
5) NHCOR⁴¹
6) C₁₋₆-Alkoxy, optional substituiert mit:
a) Carboxyl
b) C₁₋₆-Alkoxycarbonyl oder
c) Phenyl
und
7) COR⁴²;
R¹ und R² sind unabhängig H, Halogen, OR³, NR³R⁶, NHCOR⁴¹, NHCSR⁴¹, NHSO₂R⁵, N₃, COR⁴² oder Phenyl;
oder R¹ and R² sind miteinander an ihren Enden verbunden, um Oxo zu bilden;
R⁷¹ ist eine Gruppe ausgewählt aus:
1) H
2) OH
3) Halogen
4) CN
5) C₁₋₆-Alkyl, optional substituiert mit Halogen oder OR⁶
6) C₁₋₆-Alkoxy, optional substituiert mit Halogen, NR³R⁶, Pyridyl oder Piperidinyl
7) NO₂
8) NR³R⁶
9) NHCOR⁴¹
10) NHSO₂R⁵
11) COR⁴²
12) C(=NH)NH₂
13) CONHOH
14) C₁₋₆-Alkylthio
15) C₁₋₆-Alkylsulfinyl
16) C₁₋₆-Alkylsulfonyl
17) Phenyl, das mit einer Gruppe substituiert sein kann, die ausgewählt ist aus a) C₂₋₇-Alkenyl substituiert mit COOR⁵, b) COR⁴², c) C₁₋₆-Alkyl, optional substituiert mit OR⁶; d) C₁₋₆-Alkoxy und e) CN
18) Thienyl, das mit CHO substituiert sein kann
19) Furyl, das mit CHO substituiert sein kann
20) Tetrazolyl
21) Dihydroxazolyl
22) Pyrrolyl, das mit CHO substituiert sein kann
23) Isoxazolyl, substituiert mit C₁₋₆-Alkyl
24) Benzothienyl;
R⁷² ist eine Gruppe ausgewählt aus:
1) Wasserstoff
2) Halogen
3) CN
4) C₁₋₆-Alkyl, optional substituiert mit OR⁶ oder
5) NO₂;
oder R⁷¹ und R⁷² sind miteinander an ihren Enden verbunden, um C₁₋₃-Alkylendioxy, optional substituiert mit Halogen, zu bilden;
m ist 0, 1 oder 2 und n ist 0 oder 1.

4. Verbindung gemäß Anspruch 3, worin
A ist =CH- oder =N-;
X und Y sind unabhängig
1) Halogen
2) NO₂
3) NR³R⁶ oder
4) C₁₋₆-Alkyl, optional substituiert mit Halogen;
ein Vertreter aus R¹ und R² ist H und das andere ist H, OH, Halogen, NR³R⁶, NHCOR⁴¹, NHCSR⁴¹, NHSO₂R⁵, N₃, COR⁴² oder Phenyl;
oder R¹ und R² sind miteinander an ihren Enden verbunden, um Oxo zu bilden;
R⁷¹ ist eine Gruppe ausgewählt aus:
1) H
2) Halogen
3) CN
4) C₁₋₆-Alkyl, optional substituiert mit Halogen
5) C₁₋₆-Alkoxy, optional substituiert mit Halogen
6) COR⁴²
7) C₁₋₆-Alkylthio
8) Phenyl, das substituiert sein kann mit einer Gruppe ausgewählt aus a) C₂₋₇-Alkenyl substituiert mit COOR⁵, b) COR⁴², c) C₁₋₆-Alkyl, optional substituiert mit OR⁶, d) C₁₋₆-Alkoxy und e) CN
9) Thienyl, das mit CHO substituiert sein kann
10) Furyl, das mit CHO substituiert sein kann
11) Pyrrolyl, das mit CHO substituiert sein kann
12) Isoxazolyl, substituiert mit C₁₋₆-Alkyl;
R⁷² ist Wasserstoff;
oder R⁷¹ und R⁷² sind miteinander an ihren Enden verbunden, um C₁₋₃-Alkylendioxy, substituiert mit Halogen, zu bilden;
m ist 1 und n ist 1.

5. Verbindung gemäß Anspruch 4, worin gilt:
X und Y unabhängig Halogen sind;
eines von R¹ und R² ist H und das andere ist H, OH, NR³R⁶, NHCOR⁴¹, NHCSR⁴¹ oder COR⁴²;
R⁴¹ ist
1) C₁₋₆-Alkyl, optional substituiert mit einer Gruppe ausgewählt aus:
a) NR⁶R⁶
b) Carboxyl
c) Carbamoyl
d) Piperazinylcarbonyl, optional substituiert mit C₁₋₆-Alkyl
e) C₂₋₇-Alkanoylamino und
f) Pyridyl
2) C₁₋₆-Alkoxy
3) NR³R⁶
4) C₃₋₆-Cycloalkyl
5) Pyridyl
6) Thienyl
7) Furyl oder
8) Pyrrolidinyl;
R⁴² ist NR³R⁶ oder Morpholinyl;
R⁷¹ ist
1) Halogen
2) CN oder
3) C₁₋₆-Alkoxy, optional substituiert mit Halogen; und
R⁷² ist Wasserstoff.

6. Verbindung gemäß Anspruch 5, worin
X und Y unabhängig Halogen sind;
eines von R¹ und R² ist H und das andere ist OH, NHCOR⁴¹ oder COR⁴²;
R⁴¹ ist
a) C₁₋₆-Alkyl, optional substituiert mit einer Gruppe ausgewählt aus Carboxyl, Carbamoyl und Piperazinylcarbonyl, das mit C₁₋₆-Alkyl substituiert ist
b) NH₂
c) NH(C₁₋₆-Alkyl)
d) Pyridyl oder
e) Pyrrolidinyl
R⁴² ist NH₂, NH(C₁₋₆-Alkyl) oder Morpholinyl;
R⁷¹ ist C₁₋₆-Alkoxy, substituiert mit Halogen; und
R⁷² ist Wasserstoff.

7. Verbindung gemäß Anspruch 1, worin die chemische Struktur die folgende ist: 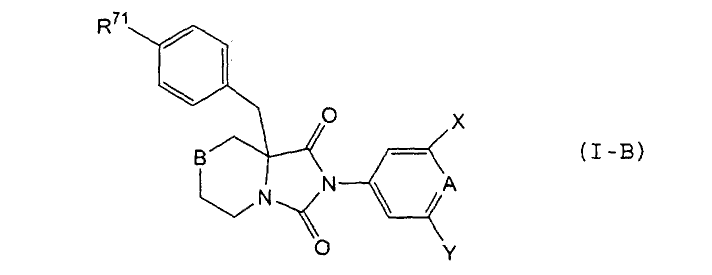 worin
A ist =CH- oder =N-;
B ist -S-, -SO-, -SO₂-, -N(R³)-, -N(COR⁴¹)- oder -N(SO₂R⁵)-;
X und Y sind unabhängig H, Halogen, NO₂ oder C₁₋₆-Aklyl; R⁷¹ ist eine Gruppe ausgewählt aus:
1) H
2) Halogen
3) CN
4) C₁₋₆-Alkyl, optional substituiert mit Halogen
5) C₁₋₆-Alkoxy, optional substituiert mit Halogen
6) COR⁴²
7) C₁₋₆-Alkylthio
8) Phenyl
9) Thienyl
10) Furyl
11) Pyrrolyl
12) Isoxazolyl, substituiert mit C₁₋₆-Alkyl,
worin das Phenyl substituiert sein kann mit einer Gruppe ausgewählt aus a) C₂₋₇-Alkenyl, substituiert mit COOR⁵, b) COR⁴², c) C₁₋₆-Alkyl, optional substituiert mit OR⁶, d) C₁₋₆-Alkoxy und e) CN, und das Thienyl, Furyl und Pyrrolyl mit CHO substituiert sein kann.

8. Verbindung gemäß Anspruch 7, worin gilt:
B ist -N(COR⁴¹)-;
X und Y sind unabhängig Halogen;
R⁴¹ ist
1) C₁₋₆-Alkyl, optional substituiert mit einer Gruppe ausgewählt aus:
a) NR⁶R⁶
b) Carbamoyl und
c) Piperazinylcarbonyl, optional substituiert mit C₁₋₆-Alkyl
2) C₁₋₆-Alkoxy oder
3) NR³R⁶;
R⁷¹ ist
1) Halogen oder
2) C₁₋₆-Alkoxy, optional substituiert mit Halogen.

9. Verbindung gemäß Anspruch 1, worin die chemische Struktur die folgende ist: 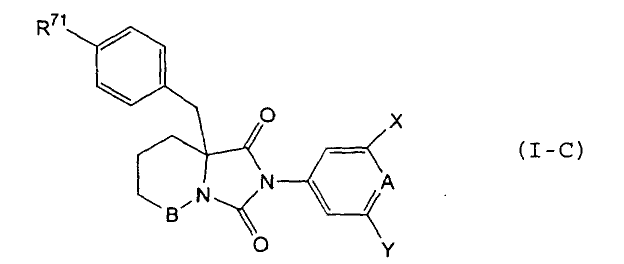 worin
A ist =CH- oder =N-;
B ist -N(R³)-, -N(COR⁴¹)-, -N(CSR⁴¹)- oder -N (SO₂R⁵)-;
X und Y sind unabhängig H, Halogen, NO₂ oder C₁₋₆-Alkyl; R⁷¹ ist eine Gruppe ausgewählt aus:
1) H
2) Halogen
3) CN
4) C₁₋₆-Alkyl, optional substituiert mit Halogen
5) C₁₋₆-Alkoxy, optional substituiert mit Halogen
6) COR⁴²
7) C₁₋₆-Alkylthio
8) Phenyl
9) Thienyl
10) Furyl
11) Pyrrolyl
12) Isoxazolyl, substituiert mit C₁₋₆-Alkyl,
worin das Phenyl substituiert sein kann mit einer Gruppe ausgewählt aus a) C₂₋₇-Alkenyl, substituiert mit COOR⁵, b) COR⁴², c) C₁₋₆-Alkyl, optional substituiert mit OR⁶, d) C₁₋₆-Alkoxy und e) CN, und das Thienyl, Furyl und Pyrrolyl mit CHO substituiert sein kann.

10. Verbindung gemäß Anspruch 1, worin die chemische Struktur die folgende ist: 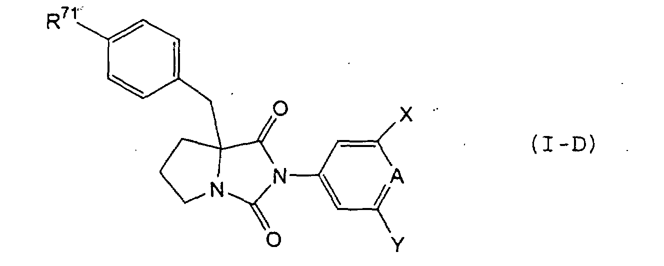 worin gilt:
A ist =CH- oder =N-;
X ist H oder Halogen;
Y ist
1) Pyrrolyl, optional substituiert mit Formyl
2) Phenyl, optional substituiert mit a) CN, b) C₁₋₆-Alkyl, optional substituiert mit Halogen, c) C₁₋₆-Alkoxy, optional substituiert mit Halogen oder
3) Isoxazolyl, optional substituiert mit C₁₋₆-Alkyl; R⁷¹ ist eine Gruppe ausgewählt aus:
1) H
2) Halogen
3) CN
4) C₁₋₆-Alkyl, optional substituiert mit Halogen
5) C₁₋₆-Alkoxy, optional substituiert mit Halogen
6) COR⁴²
7) C₁₋₆-Alkylthio
8) Phenyl
9) Thienyl
10) Furyl
11) Pyrrolyl
12) Isoxazolyl, substituiert mit C₁₋₆-Alkyl,
worin das Phenyl substituiert sein kann mit einer Gruppe ausgewählt aus a) C₂₋₇-Alkenyl, substituiert mit COOR⁵, b) COR⁴², c) C₁₋₆-Alkyl, optional substituiert mit OR⁶, d) C₁₋₆-Alkoxy und e) CN, und das Thienyl, Furyl und Pyrrolyl mit CHO substituiert sein kann.

11. Verbindung gemäß Anspruch 1, worin die Verbindung ausgewählt ist aus der Gruppe bestehend aus:
(5R,7R)-5-[4-(Trifluormethoxy)benzyl]-3-(3,5-dichlorphenyl)-7-hydroxy-1,3-diazabicyclo[3.3.0]octan-2,4-dion;
(5R,7S)-5-[4-(Trifluormethoxy)benzyl]-3-(3,5-dichlorphenyl)-7-acetylamino-1,3-diazabicyclo[3.3.0]octan-2,4-dion;
(5R,7S)-5-[4-(Trifluormethoxy)benzyl]-3-(3,5-dichlorphenyl)-7-(3-carbamoylpropionylamino)-1,3-diazabicyclo[3.3.0]octan-2,4-dion;
(5R,7S)-5-[4-(Trifluormethoxy)benzyl]-3-(3,5-dichlorphenyl)-7-[3-(4-methyl-1-piperazinylcarbonyl)propionylamino]-1,3-diazabicyclo[3.3.0]octan-2,4-dion;
(5R,7S)-5-[4-(Trifluormethoxy)benzyl]-3-(3,5-dichlorphenyl)-7-nicotinoylamino-1,3-diazabicyclo[3.3.0]octan-2,4-dion;
(5R,7S)-5-[4-(Trifluormethoxy)benzyl]-3-(3,5-dichlorphenyl)-7-(1-pyrrolidinylcarbomylamino-1,3-diazabicyclo[3.3.0]octan-2,4-dion;
(5R,7R)-5-[4-(Trifluormethoxy)benzyl]-3-(3,5-dichlorphenyl)-7-carbamoyl-1,3-diazabicyclo[3.3.0]octan-2,4-dion;
(5R,7R)-5-[4-(Trifluormethoxy)benzyl]-3-(3,5-dichlorphenyl)-7-morpholinocarbonyl-1,3-diazabicyclo[3.3.0]octan-2,4-dion;
(5R,7R)-5-[4-(Trifluormethoxy)benzyl]-3-(3,5-dichlorphenyl)-7-dimethylcarbamoyl-1,3-diazabicyclo[3.3.0]octan-2,4-dion;
(5R,7R)-5-[4-(Trifluormethoxy)benzyl]-3-(3,5-dichlorphenyl)-7-methylcarbamoyl-1,3-diazabicyclo[3.3.0]octan-2,4-dion und
(5R,7R)-5-[4-(Trifluormethoxy)benzyl]-3-(3,5-dichlorphenyl)-7-morpholinocarbamoyl-1,3-diazabicyclo[3.3.0]octan-2,4-dion.

12. Verfahren für die Herstellung einer Verbindung der Formel (I) : 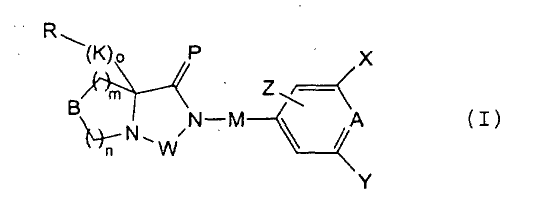 worin die Symbole dieselben wie definiert in Anspruch 1 sind, oder ein pharmazeutisch annehmbares Salz davon, welches die Zyklisierung der Verbindung der Formel (II): 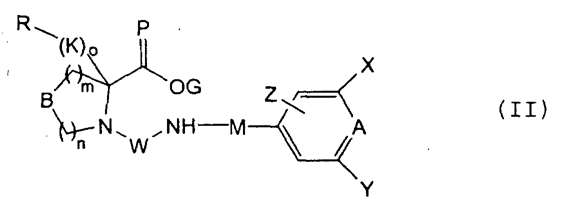 worin OG eine Hydroxylgruppe, eine geschützte Hydroxylgruppe oder eine harzgebundene Hydroxylgruppe ist, und die anderen Symbole dieselben wie oben definiert sind, und, falls notwendig, Umwandlung in das pharmazeutisch annehmbare Salz umfaßt.

13. Verfahren für die Herstellung einer Verbindung der Formel (I): 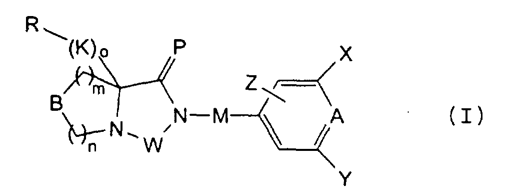 worin die Symbole dieselben wie definiert in Anspruch 1 sind, oder ein pharmazeutisch annehmbares Salz davon, welches die Umsetzung der Verbindung der Formel (III): 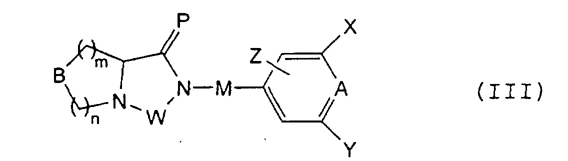 worin die Symbole dieselben wie oben definiert sind, mit der Verbindung der Formel (IV):
R―(K)ₒ―L (IV)
umfaßt, worin L eine Abgangsgruppe ist und die anderen Symbole dieselben wie definiert in Anspruch 1 sind, und, falls notwendig, optionale Umwandlung in das pharmazeutisch annehmbare Salz.

14. Verfahren für die Herstellung einer Verbindung der Formel (I): 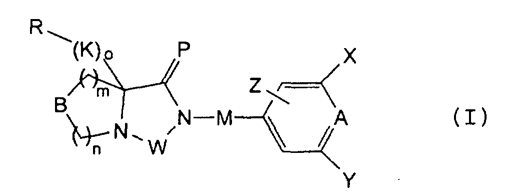 worin die Symbole dieselben wie definiert in Anspruch 1 sind, oder ein pharmazeutisch annehmbares Salz davon, das die Umsetzung der Verbindung der Formel (V): 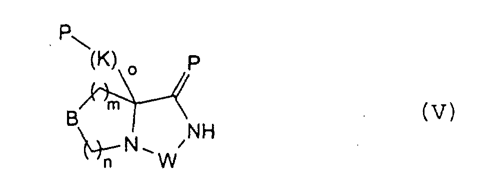 worin die Symbole dieselben wie definiert in Anspruch 1 sind, mit der Verbindung der Formel (VI): 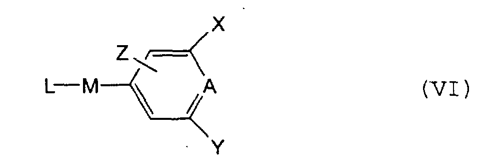 worin L eine Abgangsgruppe ist und die anderen Symbole dieselben wie definiert in Anspruch 1 sind, und, falls notwendig, Umsetzung der resultierenden Verbindung in ein pharmazeutisch annehmbares Salz umfaßt.

15. Pharmazeutische Zusammensetzung, die eine therapeutisch wirksame Menge der wie in Anspruch 1 beschriebenen Verbindung im Gemisch mit einem therapeutisch annehmbaren Träger oder Verdünnungsmittel umfaßt.

16. Verwendung der wie in Anspruch 1 beschriebenen Verbindung für die Behandlung oder Prävention von einem α_{L}β₂ adhäsionsvermittelten Zustand in einem Säugetier.

17. Verwendung gemäß Anspruch 16, wobei der Zustand ausgewählt ist aus rheumatoider Arthritis, Asthma, allergischem Zustand, respiratorischer Insuffizienz des Erwachsenen, AIDS, Herz-Kreislauf-Erkrankungen, Thrombose oder schädlicher Blutplättchenaggregation, Reokklusion nach Thrombolyse, Fremdimplantatabstoßung, Reperfusionsverletzung, Schlaganfall, Psoriasis, Ekzeme, Hautentzündungskrankungen, wie z.B. Kontaktdermatitis oder atopische Dermatitis, Osteoporose, Osteoarthritis, Atherosklerose (einschließlich Transplantat-Arteriosklerose nach Transplantation), neoplastische Erkrankungen, einschließlich Metastase von neoplastischem oder kanzerösem Wachstum, Wundheilungsverbesserung, Augenkrankheiten, wie z.B. Ablösen der Retina, Typ I-Diabetes, multiple Sklerose, systemischer Lupus erythematodes (SLE), entzündliche und immunoentzündliche Zustände, einschließlich entzündliche Augenzustände und entzündliche Darmkrankheiten (Morbus Crohn, Colitis ulcerosa), Enteritis regionalis, Sjogren-Syndrom, und andere Autoimmunkrankheiten, Pankreatitis, verzögerte Transplantatfunktion, Intimahyperplasie; Myokardreinfarkt oder Restenosierung nach Operation, wie z.B. perkutane transluminale Koronarangioplastie (PTCA), Abstoßung nach Transplantation (chronische Abstoßung und akute Abstoßung), Wirt-Transplantat- oder Transplantat-Wirt-Krankheiten und Krebs.

18. Verwendung gemäß Anspruch 17, worin die Krankheiten ausgewählt sind aus Psoriasis, rheumatoider Arthritis, entzündlichen Darmkrankheiten (Morbus Crohn, Colitis ulcerosa) systemischer Lupus erythematodes, atopischer Dermatitis, Sjogren-Syndrom, Abstoßung nach Transplantation (chronische Abstoßung, akute Abstoßung) und Transplantat-Wirt-Krankheit.

## Revendications

1. Composé de formule (I) : 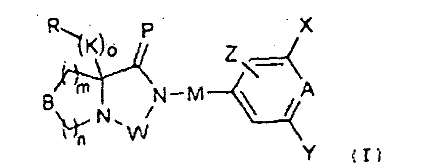 ou un sel pharmaceutiquement acceptable de celui-ci, dans lequel :
A est =C(Z¹)- ou =N- ;
B est :
1) -C(R¹)(R²)-,
2) -S-,
3) -SO-,
4) -SO₂-,
5) -O-,
6) -N(R³)-,
7) -N(COR⁴¹)-,
8) -N(CSR⁴¹)-,
9) -N(SO₂R⁵)-,
10) -N(R³)CO-,
11) -N(COR⁴¹)CO-,
12) -N(CSR⁴¹)CO-, ou
13) -N(SO₂R⁵)CO- ;
K est -CH₂-, -CH(OH)-, -C(=O)- ou -CF₂- ;
M est une simple liaison, -(CH₂)ₚ-, -C(=O)- ou -NH- ;
W est l'un des groupes suivants : 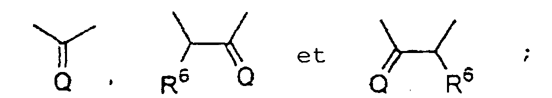
X et Y sont indépendamment :
1) H,
2) un halogène,
3) NO₂,
4) CN,
5) un groupe (alkyl en C₁₋₆)thio,
6) NR³R⁶,
7) un groupe alkyle en C₁₋₆ éventuellement substitué par un halogène,
8) un groupe alcoxy en C₁₋₆,
9) COR⁴²,
10) un groupe phényle éventuellement substitué par :
a) un groupe alkyle en C₁₋₆ éventuellement substitué par un halogène,
b) un groupe alcoxy en C₁₋₆ éventuellement substitué par un halogène, ou
c) CN,
11) un groupe isoxazolyle éventuellement substitué par un groupe alkyle en C₁₋₆,
12) un groupe pyrrolyle éventuellement substitué par un groupe (alcoxy en C₁₋₆)carbonyle ou formyle, ou
13) un groupe pyridyle ;
Z et Z¹ sont indépendamment :
1) H,
2) OH,
3) un halogène,
4) NO₂,
5) CF₃,
6) NR³R⁶,
7) NHCOR⁴¹,
8) un groupe alcoxy en C₁₋₆ éventuellement substitué par :
a) un groupe carboxyle,
b) un groupe (alcoxy en C₁₋₆)carbonyle, ou
c) un groupe phényle, ou
9) COR⁴² ;
P et Q sont indépendamment O ou S ;
R est un groupe phényle, naphtyle, pyridyle, benzofuryle ou thiazolyle, et lesdits groupes phényle, naphtyle, pyridyle, benzofuryle et thiazolyle peuvent être substitués par un groupe choisi parmi :
1) un halogène,
2) OH,
3) CN,
4) un groupe alkyle en C₁₋₆ éventuellement substitué par un groupe choisi parmi :
a) un halogène,
b) OR⁶, et
c) COR⁴¹,
5) un groupe alcoxy en C₁₋₆ éventuellement substitué par un groupe choisi parmi :
a) un halogène,
b) NR³R⁶,
c) un groupe pyridyle, et
d) un groupe pipéridinyle,
6) NO₂,
7) NR³R⁶,
8) NHCOR⁴¹,
9) NHSO₂R⁵,
10) COR⁴²,
11) C(=NH)NH₂,
12) CONHOH,
13) un groupe (alkyl en C₁₋₆)thio qui peut être substitué par un halogène,
14) un groupe (alkyl en C₁₋₆)sulfinyle qui peut être substitué par un halogène,
15) un groupe (alkyl en C₁₋₆)sulfonyle qui peut être substitué par un halogène,
16) un groupe (alkylène en C₁₋₃)dioxy éventuellement substitué par :
a) un groupe alkyle en C₁₋₆, ou
b) un halogène,
17) un groupe -C(=O)-(résidu acide α-aminé naturel) dans lequel ledit résidu acide α-aminé naturel peut être estérifié avec un groupe alkyle en C₁₋₆,
18) un groupe phényle éventuellement substitué par :
a) un groupe alcoxy en C₁₋₆,
b) un groupe alkyle en C₁₋₆ éventuellement substitué par un groupe OR⁶, N(alkyle en C₁₋₆)₂ ou COR⁴²,
c) CN,
d) COR⁴²,
e) un groupe alcényle en C₂₋₇ éventuellement substitué par COOR⁵,
f) NR⁶R⁶,
g) NO₂,
h) NHCOR⁴¹,
i) NHSO₂R⁵,
j) N(SO₂R⁵)₂,
k) NHCONHR⁵,
l) N(CONHR⁵)₂,
m) NHCSNHR⁵, ou
n) un groupe pyrrolidinyle qui peut être substitué par un groupe alkyle en C₁₋₆,
19) un groupe furyle éventuellement substitué par CHO,
20) un groupe thiényle éventuellement substitué par CHO,
21) un groupe pyrrolyle éventuellement substitué par CHO et un groupe (alcoxy en C₁₋₆)carbonyle,
22) un groupe dihydroxazolyle éventuellement substitué par un groupe alkyle en C₁₋₆,
23) un groupe isoxazolyle éventuellement substitué par un groupe alkyle en C₁₋₆,
24) un groupe benzothiényle,
25) un groupe pyridyle,
26) un groupe tétrazolyle, et
27) un groupe thiazolyle qui peut être substitué par un groupe alkyle en C₁₋₆ ;
R¹ et R² sont indépendamment :
1) H,
2) un halogène,
3) OR³,
4) OCOR⁵,
5) SO₂R⁵,
6) NR³R⁶,
7) NR⁶COR⁴¹,
8) NR⁶CSR⁴¹,
9) NR⁶SO₂R⁵,
10) OCONR³R³,
11) N₃,
12) CN,
13) COR⁴², ou
14) un groupe phényle,
ou R¹ et R² se combinent l'un avec l'autre à leur extrémité pour former :
1) un groupe oxo,
2) un groupe méthylène substitué par un groupe carboxyle, (alcoxy en C₂₋₇)carbonyle ou CONR^{a}R^{b}, ou
3) un groupe (alkylène en C₂₋₃)dioxy ;
R^{a} et R^{b} se combinent l'un avec l'autre à leurs extrémités pour former un cycle à 3-7 chaînons conjointement avec l'atome d'azote auquel ils sont attachés, ledit cycle à 3-7 chaînons pouvant comporter des hétéroatomes supplémentaires tels qu'un oxygène, un azote et un soufre et pouvant être substitué par un groupe alkyle en C₁₋₆, oxo, hydroxyle, alcoxy en C₁₋₆ ou NR⁶R⁶ ;
R³ est :
1) H,
2) un groupe alkyle en C₁₋₆ qui peut être substitué par :
a) OH,
b) un groupe phényle éventuellement substitué par un halogène ou un groupe alcoxy en C₁₋₆,
c) un groupe carboxyle,
d) un groupe carbamoyle,
e) NR⁶R⁶,
f) un groupe (alcoxy en C₁₋₆)carbonyle,
g) un groupe morpholinyle,
h) un groupe pyridyle,
i) un groupe thiényle, ou
j) un groupe pyrrolidinyle éventuellement substitué par un groupe oxo,
3) un groupe cycloalkyle en C₃₋₆,
4) un groupe phényle qui peut être substitué par un halogène,
5) un groupe pyridyle qui peut être substitué par un groupe alkyle en C₁₋₆, ou
6) un groupe morpholinyle ;
R⁴¹ est :
1) un groupe alkyle en C₁₋₆ qui peut être substitué par un groupe choisi parmi :
a) NR⁶R⁶,
b) un groupe carboxyle,
c) -CONR^{c}R^{d}, où R^{c} et R^{d} sont indépendamment choisis parmi un hydrogène et un groupe alkyle en C₁₋₆, ou R^{c} et R^{d} se combinent l'un avec l'autre à leurs extrémités pour former un cycle à 3-7 chaînons conjointement avec l'atome d'azote auquel ils sont attachés, ledit cycle à 3-7 chaînons pouvant comporter 1-3 hétéroatomes supplémentaires tels qu'un oxygène, un azote et un soufre et pouvant être substitué par un groupe alkyle en C₁₋₆, oxo, hydroxyle, alcoxy en C₁₋₆ ou NR⁶R⁶,
d) un groupe (alcoxy en C₁₋₆)carbonylamino,
e) un groupe (alkyl en C₁₋₆)sulfonylamino,
f) un groupe (alcanoyl en C₂₋₇)amino, et
g) un groupe pyridyle,
2) un groupe alcoxy en C₁₋₆ qui peut être substitué par NR⁶R⁶ ou un groupe phényle,
3) un groupe phényle qui peut être substitué par :
a) un groupe carboxyle,
b) un groupe (alcoxy en C₁₋₆)carbonyle, ou
c) NR⁶R⁶,
4) un groupe isoxazolyle qui peut être substitué par un groupe alkyle en C₁₋₆,
5) un groupe pyridyle,
6) un groupe thiényle,
7) un groupe furyle,
8) NR^{a}R^{b},
9) un groupe cycloalkyle en C₃₋₆, ou
10) NR³R⁶ ;
R⁴² est :
1) H,
2) OH,
3) un groupe alkyle en C₁₋₆ qui peut être substitué par NR⁶R⁶ ou un groupe phényle,
4) un groupe alcoxy en C₁₋₆ qui peut être substitué par NR⁶R⁶,
5) un groupe phényle,
6) NR³R⁶, ou
7) NR^{a}R^{b} ;
R⁵ est :
1) un groupe alkyle en C₁₋₆ qui peut être substitué par COR⁴², ou
2) un groupe phényle ou naphtyle ;
R⁶ est :
1) H, ou
2) un groupe alkyle en C₁₋₆ qui peut être substitué par un groupe -N(alkyle en C₁₋₆)₂ ;
m vaut 0, 1, 2 ou 3 ; n vaut 0, 1 ou 2 ; o vaut 1 ou 2 ; et p vaut 1 ou 2.

2. Composé selon la revendication 1, dans lequel X et Y sont indépendamment choisis parmi :
1) un halogène,
2) NO₂,
3) un groupe alkyle en C₁₋₆ éventuellement substitué par un halogène,
4) un groupe alcoxy en C₁₋₆,
5) un groupe alcanoyle en C₁₋₇,
6) CN,
7) un groupe carboxyle,
8) un groupe (alkyl en C₁₋₆)thio,
9) NR³R⁶,
10) un groupe phényle éventuellement substitué par :
a) un groupe alkyle en C₁₋₆ éventuellement substitué par un halogène,
b) un groupe alcoxy en C₁₋₆ éventuellement substitué par un halogène, ou
c) CN,
11) un groupe isoxazolyle éventuellement substitué par un groupe alkyle en C₁₋₆,
12) un groupe pyrrolyle éventuellement substitué par un groupe (alcoxy en C₁₋₆)carbonyle ou formyle, et
13) un groupe pyridyle ;
R est un groupe phényle, naphtyle, pyridyle, benzofuryle ou thiazolyle, lesdits groupes phényle, naphtyle, pyridyle, benzofuryle et thiazolyle pouvant être substitués par un groupe choisi parmi :
1) un halogène,
2) OH,
3) CN,
4) un groupe alkyle en C₁₋₆ éventuellement substitué par un groupe choisi parmi :
a) un halogène,
b) OR⁶, et
c) COR⁴¹,
5) un groupe alcoxy en C₁₋₆ éventuellement substitué par un groupe choisi parmi :
a) un halogène,
b) NR³R⁶,
c) un groupe pyridyle, et
d) un groupe pipéridinyle,
6) NO₂,
7) NR³R⁶,
8) NHCOR⁴¹,
9) NHSO₂R⁵,
10) COR⁴²,
11) C(=NH)NH₂,
12) CONHOH,
13) un groupe (alkyl en C₁₋₆)thio qui peut être substitué par un halogène,
14) un groupe (alkyl en C₁₋₆)sulfinyle qui peut être substitué par un halogène,
15) un groupe (alkyl en C₁₋₆)sulfonyle qui peut être substitué par un halogène,
16) un groupe (alkylène en C₁₋₃)dioxy éventuellement substitué par :
a) un groupe alkyle en C₁₋₆, ou
b) un halogène,
17) un groupe -C(=O)-(résidu acide α-aminé naturel) dans lequel ledit résidu acide α-aminé naturel est choisi parmi l'acide aspartique, l'alanine, la phénylalanine et l'asparagine, et ledit résidu acide α-aminé naturel peut être estérifié avec un groupe alkyle en C₁₋₆,
18) un groupe phényle éventuellement substitué par :
a) un groupe alcoxy en C₁₋₆,
b) un groupe alkyle en C₁₋₆ éventuellement substitué par un groupe OR⁶, N(alkyle en C₁₋₆)₂ ou COR⁴²,
c) CN,
d) COR⁴²,
e) un groupe alcényle en C₂₋₇ éventuellement substitué par COOR⁵,
f) NR⁶R⁶,
g) NO₂,
h) NHCOR⁴¹,
i) NHSO₂R⁵,
j) N(SO₂R⁵)₂,
k) NHCONHR⁵,
l) N(CONHR⁵)₂,
m) NHCSNHR⁵, ou
n) un groupe pyrrolidinyle qui peut être substitué par un groupe alkyle en C₁₋₆,
19) un groupe furyle éventuellement substitué par CHO,
20) un groupe thiényle éventuellement substitué par CHO,
21) un groupe pyrrolyle éventuellement substitué par CHO et un groupe (alcoxy en C₁₋₆)carbonyle,
22) un groupe dihydroxazolyle éventuellement substitué par un groupe alkyle en C₁₋₆,
23) un groupe isoxazolyle éventuellement substitué par un groupe alkyle en C₁₋₆,
24) un groupe benzothiényle,
25) un groupe pyridyle,
26) un groupe tétrazolyle, et
27) un groupe thiazolyle qui peut être substitué par un groupe alkyle en C₁₋₆ ;
R¹ et R² sont indépendamment choisis parmi :
1) H,
2) un halogène,
3) OR³,
4) OCOR⁵,
5) NR³R⁶,
6) NR⁶COR⁴¹,
7) NHCSR⁴¹,
8) NHSO₂R⁵,
9) N₃,
10) COR⁴², et
11) un groupe phényle,
ou R¹ et R² se combinent l'un avec l'autre à leur extrémité pour former un groupe oxo ;
R³ est :
1) un hydrogène,
2) un groupe alkyle en C₁₋₆ éventuellement substitué par :
a) OH,
b) un groupe phényle éventuellement substitué par un halogène ou un groupe alcoxy en C₁₋₆,
c) un groupe carboxyle,
d) un groupe carbamoyle,
e) NR⁶R⁶,
f) un groupe (alcoxy en C₁₋₆)carbonyle,
g) un groupe morpholinyle,
h) un groupe pyridyle,
i) un groupe thiényle, ou
j) un groupe pyrrolidinyle éventuellement substitué par un groupe oxo,
3) un groupe cycloalkyle en C₃₋₆,
4) un groupe phényle éventuellement substitué par un halogène,
5) un groupe pyridyle éventuellement substitué par un groupe alkyle en C₁₋₆, ou
6) un groupe morpholinyle ;
R⁴¹ est :
1) un groupe alkyle en C₁₋₆ éventuellement substitué par un groupe choisi parmi :
a) NR⁶R⁶,
b) un groupe carboxyle,
c) -CONR^{c}R^{d}, où R^{c} et R^{d} sont indépendamment choisis parmi un hydrogène et un groupe alkyle en C₁₋₆, ou R^{c} et R^{d} se combinent l'un avec l'autre à leurs extrémités pour former un cycle à 3-7 chaînons conjointement avec l'atome d'azote auquel ils sont attachés, ledit cycle à 3-7 chaînons pouvant comporter 1-3 hétéroatomes supplémentaires tels qu'un oxygène, un azote et un soufre et pouvant être substitué par un groupe alkyle en C₁₋₆, oxo, hydroxyle, alcoxy en C₁₋₆ ou NR⁶R⁶ ;
d) un groupe (alcoxy en C₁₋₆)carbonylamino,
e) un groupe (alkyl en C₁₋₆)sulfonylamino,
f) un groupe (alcanoyl en C₂₋₇)amino, et
g) un groupe pyridyle,
2) un groupe alcoxy en C₁₋₆ éventuellement substitué par NR⁶R⁶ ou un groupe phényle,
3) NR³R⁶,
4) un groupe phényle éventuellement substitué par :
a) un groupe carboxyle,
b) un groupe (alcoxy en C₁₋₆)carbonyle, ou
c) NR⁶R⁶,
5) un groupe cycloalkyle en C₃₋₆,
6) un groupe isoxazolyle éventuellement substitué par un groupe alkyle en C₁₋₆,
7) un groupe pyridyle,
8) un groupe thiényle,
9) un groupe furyle, ou
10) NR^{a}R^{b} ;
R⁴² est :
1) H,
2) OH,
3) un groupe alkyle en C₁₋₆ éventuellement substitué par NR⁶R⁶ ou un groupe phényle,
4) un groupe alcoxy en C₁₋₆ éventuellement substitué par NR⁶R⁶,
5) NR³R⁶,
6) NR^{a}R^{b}, ou
7) un groupe pyridyle qui peut être substitué par un groupe alkyle en C₁₋₆ ;
R⁵ est :
1) un groupe alkyle en C₁₋₆ éventuellement substitué par COR⁴², ou
2) un groupe phényle ou naphtyle ; et
R⁶ est :
1) un hydrogène, ou
2) un groupe alkyle en C₁₋₆ éventuellement substitué par un groupe -N(alkyle en C₁₋₆)₂.

3. Composé selon la revendication 1, sa structure chimique étant la suivante : 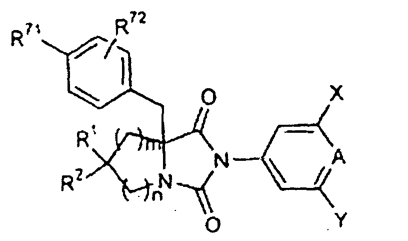 dans laquelle :
A est =C(Z¹)- ou =N- ;
X et Y sont indépendamment :
1) H,
2) un halogène,
3) NO₂,
4) CN,
5) un groupe (alkyl en C₁₋₆)thio,
6) NR³R⁶, ou
7) un groupe alkyle en C₁₋₆ éventuellement substitué par un halogène ;
Z¹ est choisi parmi :
1) H,
2) OH,
3) un halogène,
4) NR³R⁶,
5) NHCOR⁴¹,
6) un groupe alcoxy en C₁₋₆ éventuellement substitué par :
a) un groupe carboxyle,
b) un groupe (alcoxy en C₁₋₆)carbonyle, ou
c) un groupe phényle, et
7) COR⁴² ;
R¹ et R² sont indépendamment H, un halogène, OR³, NR³R⁶, NHCOR⁴¹, NHCSR⁴¹, NHSO₂R⁵, N₃, COR⁴² ou un groupe phényle, ou R¹ et R² se combinent l'un avec l'autre à leur extrémité pour former un groupe oxo ;
R⁷¹ est un groupe choisi parmi :
1) H,
2) OH,
3) un halogène
4) CN,
5) un groupe alkyle en C₁₋₆ éventuellement substitué par un halogène ou OR⁶,
6) un groupe alcoxy en C₁₋₆ éventuellement substitué par un halogène, NR³R⁶, un groupe pyridyle ou pipéridinyle,
7) NO₂,
8) NR³R⁶,
9) NHCOR⁴¹,
10) NHSO₂R⁵,
11) COR⁴²,
12) C(=NH)NH₂,
13) CONHOH,
14) un groupe (alkyl en C₁₋₆)thio,
15) un groupe (alkyl en C₁₋₆)sulfinyle,
16) un groupe (alkyl en C₁₋₆)sulfonyle,
17) un groupe phényle qui peut être substitué par un groupe choisi parmi :
a) un groupe alcényle en C₂₋₇ substitué par COOR⁵,
b) COR⁴²,
c) un groupe alkyle en C₁₋₆ éventuellement substitué par OR⁶,
d) un groupe alcoxy en C₁₋₆, et
e) CN,
18) un groupe thiényle qui peut être substitué par CHO,
19) un groupe furyle qui peut être substitué par CHO,
20) un groupe tétrazolyle,
21) un groupe dihydroxazolyle,
22) un groupe pyrrolyle qui peut être substitué par CHO,
23) un groupe isoxazolyle substitué par un groupe alkyle en C₁₋₆, et
24) un groupe benzothiényle ;
R⁷² est un groupe choisi parmi :
1) un hydrogène,
2) un halogène,
3) CN,
4) un groupe alkyle en C₁₋₆ éventuellement substitué par OR⁶, et
5) NO₂ ;
ou R⁷¹ et R⁷² se combinent l'un avec l'autre à leur extrémité pour former un groupe (alkylène en C₁₋₃)dioxy éventuellement substitué par un halogène ;
m vaut 0, 1 ou 2, et n vaut 0 ou 1.

4. Composé selon la revendication 3, dans lequel :
A est =CH- ou =N- ;
X et Y sont indépendamment :
1) un halogène,
2) NO₂,
3) NR³R⁶, ou
4) un groupe alkyle en C₁₋₆ éventuellement substitué par un halogène ;
l'un parmi R¹ et R² est H, et l'autre est H, OH, un halogène, NR³R⁶, NHCOR⁴¹, NHCSR⁴¹, NHSO₂R⁵, N₃, COR⁴² ou un groupe phényle,
ou R¹ et R² se combinent l'un avec l'autre à leur extrémité pour former un groupe oxo ;
R⁷¹ est un groupe choisi parmi :
1) H,
2) un halogène
3) CN,
4) un groupe alkyle en C₁₋₆ éventuellement substitué par un halogène,
5) un groupe alcoxy en C₁₋₆ éventuellement substitué par un halogène,
6) COR⁴²,
7) un groupe (alkyl en C₁₋₆)thio,
8) un groupe phényle qui peut être substitué par un groupe choisi parmi :
a) un groupe alcényle en C₂₋₇ substitué par COOR⁵,
b) COR⁴²,
c) un groupe alkyle en C₁₋₆ éventuellement substitué par OR⁶,
d) un groupe alcoxy en C₁₋₆, et
e) CN,
9) un groupe thiényle qui peut être substitué par CHO,
10) un groupe furyle qui peut être substitué par CHO,
11) un groupe pyrrolyle qui peut être substitué par CHO, et
12) un groupe isoxazolyle substitué par un groupe alkyle en C₁₋₆ ;
R⁷² est un hydrogène ;
ou R⁷¹ et R⁷² se combinent l'un avec l'autre à leur extrémité pour former un groupe (alkylène en C₁₋₃)dioxy substitué par un halogène ;
m vaut 1, et n vaut 1.

5. Composé selon la revendication 4, dans lequel :
X et Y sont indépendamment un halogène ;
l'un parmi R¹ et R² est H, et l'autre est H, OH, NR³R⁶, NHCOR⁴¹, NHCSR⁴¹ ou COR⁴² ;
R⁴¹ est :
1) un groupe alkyle en C₁₋₆ éventuellement substitué par un groupe choisi parmi :
a) NR⁶R⁶,
b) un groupe carboxyle,
c) un groupe carbamoyle,
d) un groupe pipérazinylcarbonyle éventuellement substitué par un groupe alkyle en C₁₋₆,
e) un groupe (alcanoyl en C₂₋₇)amino, et
f) un groupe pyridyle,
2) un groupe alcoxy en C₁₋₆,
3) NR³R⁶,
4) un groupe cycloalkyle en C₃₋₆,
5) un groupe pyridyle,
6) un groupe thiényle,
7) un groupe furyle, ou
8) un groupe pyrrolidinyle ;
R⁴² est NR³R⁶ ou un groupe morpholinyle ;
R⁷¹ est :
1) un halogène,
2) CN, ou
3) un groupe alcoxy en C₁₋₆ éventuellement substitué par un halogène ; et
R⁷² est un hydrogène.

6. Composé selon la revendication 5, dans lequel :
X et Y sont indépendamment un halogène ;
l'un parmi R¹ et R² est H, et l'autre est OH, NHCOR⁴¹ ou COR⁴² ;
R⁴¹ est :
1) un groupe alkyle en C₁₋₆ éventuellement substitué par un groupe choisi parmi les groupes carboxyle, carbamoyle, et pipérazinylcarbonyle substitué par un groupe alkyle en C₁₋₆,
2) NH₂,
3) un groupe NH (alkyle en C₁₋₆),
4) un groupe pyridyle, ou
5) un groupe pyrrolidinyle ;
R⁴² est NH₂, un groupe NH(alkyle en C₁₋₆) ou morpholinyle ;
R⁷¹ est un groupe alcoxy en C₁₋₆ substitué par un halogène ; et
R⁷² est un hydrogène.

7. Composé selon la revendication 1, sa structure chimique étant la suivante : 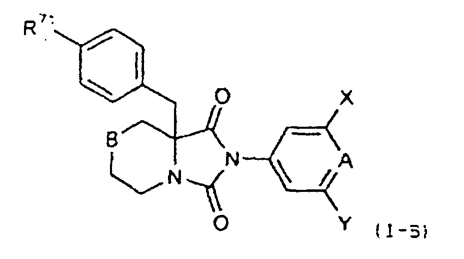 dans laquelle :
A est =CH- ou =N- ;
B est -S-, -SO-, -SO₂-, -N(R³)-, -N(COR⁴¹)-, -N(CSR⁴¹)- ou -N(SO₂R⁵)- ;
X et Y sont indépendamment H, un halogène, NO₂ ou un groupe alkyle en C₁₋₆ ;
R⁷¹ est un groupe choisi parmi :
1) H,
2) un halogène
3) CN,
4) un groupe alkyle en C₁₋₆ éventuellement substitué par un halogène,
5) un groupe alcoxy en C₁₋₆ éventuellement substitué par un halogène,
6) COR⁴²,
7) un groupe (alkyl en C₁₋₆)thio,
8) un groupe phényle,
9) un groupe thiényle,
10) un groupe furyle,
11) un groupe pyrrolyle, et
12) un groupe isoxazolyle substitué par un groupe alkyle en C₁₋₆,
ledit groupe phényle pouvant être substitué par un groupe choisi parmi :
a) un groupe alcényle en C₂₋₇ substitué par COOR⁵,
b) COR⁴²,
c) un groupe alkyle en C₁₋₆ éventuellement substitué par OR⁶,
d) un groupe alcoxy en C₁₋₆, et
e) CN,
et lesdits groupes thiényle, furyle et pyrrolyle pouvant être substitués par CHO.

8. Composé selon la revendication 7, dans lequel :
B est -N(COR⁴¹)- ;
X et Y sont indépendamment un halogène ;
R⁴¹ est :
1) un groupe alkyle en C₁₋₆ éventuellement substitué par un groupe choisi parmi :
a) NR⁶R⁶,
b) un groupe carbamoyle, et
c) un groupe pipérazinylcarbonyle éventuellement substitué par un groupe alkyle en C₁₋₆,
2) un groupe alcoxy en C₁₋₆, ou
3) NR³R⁶ ; et
R⁷¹ est :
1) un halogène, ou
2) un groupe alcoxy en C₁₋₆ éventuellement
substitué par un halogène.

9. Composé selon la revendication 1, sa structure chimique étant la suivante : 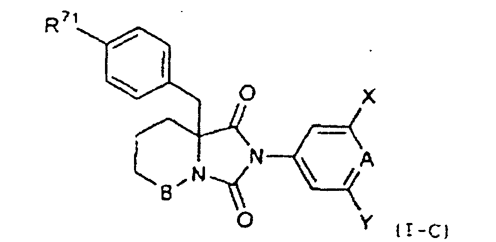 dans laquelle :
A est =CH- ou =N- ;
B est -N(R³)-, -N(COR⁴¹)-, -N(CSR⁴¹)- ou -N(SO₂R⁵)- ;
X et Y sont indépendamment H, un halogène, NO₂ ou un groupe alkyle en C₁₋₆ ;
R⁷¹ est un groupe choisi parmi :
1) H,
2) un halogène
3) CN,
4) un groupe alkyle en C₁₋₆ éventuellement substitué par un halogène,
5) un groupe alcoxy en C₁₋₆ éventuellement substitué par un halogène,
6) COR⁴²,
7) un groupe (alkyl en C₁₋₆)thio,
8) un groupe phényle,
9) un groupe thiényle,
10) un groupe furyle,
11) un groupe pyrrolyle, et
12) un groupe isoxazolyle substitué par un groupe alkyle en C₁₋₆,
ledit groupe phényle pouvant être substitué par un groupe choisi parmi :
a) un groupe alcényle en C₂₋₇ substitué par COOR⁵,
b) COR⁴²,
c) un groupe alkyle en C₁₋₆ éventuellement substitué par OR⁶,
d) un groupe alcoxy en C₁₋₆, et
e) CN,
et lesdits groupes thiényle, furyle et pyrrolyle pouvant être substitués par CHO.

10. Composé selon la revendication 1, sa structure chimique étant la suivante : 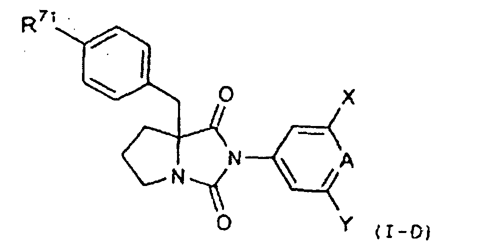 dans laquelle :
A est =CH- ou =N- ;
X est H ou un halogène ;
Y est :
1) un groupe pyrrolyle éventuellement substitué par un groupe formyle,
2) un groupe phényle éventuellement substitué par :
a) CN,
b) un groupe alkyle en C₁₋₆ éventuellement substitué par un halogène, ou
c) un groupe alcoxy en C₁₋₆ éventuellement substitué par un halogène, ou
3) un groupe isoxazolyle éventuellement substitué par un groupe alkyle en C₁₋₆ ;
R⁷¹ est un groupe choisi parmi :
1) H,
2) un halogène
3) CN,
4) un groupe alkyle en C₁₋₆ éventuellement substitué par un halogène,
5) un groupe alcoxy en C₁₋₆ éventuellement substitué par un halogène,
6) COR⁴²,
7) un groupe (alkyl en C₁₋₆)thio,
8) un groupe phényle,
9) un groupe thiényle,
10) un groupe furyle,
11) un groupe pyrrolyle, et
12) un groupe isoxazolyle substitué par un groupe alkyle en C₁₋₆,
ledit groupe phényle pouvant être substitué par un groupe choisi parmi :
a) un groupe alcényle en C₂₋₇ substitué par COOR⁵,
b) COR⁴²,
c) un groupe alkyle en C₁₋₆ éventuellement substitué par OR⁶,
d) un groupe alcoxy en C₁₋₆, et
e) CN,
et lesdits groupes thiényle, furyle et pyrrolyle pouvant être substitués par CHO.

11. Composé selon la revendication 1, le composé étant choisi dans le groupe constitué par :
la (5R,7R)-5-[4-(trifluorométhoxy)benzyl]-3-(3,5-dichlorophényl)-7-hydroxy-1,3-diazabicyclo[3.3.0]octane-2,4-dione ;
la (5R,7S)-5-[4-(trifluorométhoxy)benzyl]-3-(3,5-dichlorophényl)-7-acétylamino-1,3-diazabicyclo[3.3.0]octane-2,4-dione ;
la (5R,7S)-5-[4-(trifluorométhoxy)benzyl]-3-(3,5-dichlorophényl)-7-(3-carbamoylpropionylamino)-1,3-diazabicyclo[3.3.0]octane-2,4-dione ;
la (5R,7S)-5-[4-(trifluorométhoxy)benzyl]-3-(3,5-dichlorophényl)-7-[3-(4-méthyl-1-pipérazinylcarbonyl)propionylamino]-1,3-diazabicyclo[3.3.0]octane-2,4-dione ;
la (5R,7S)-5-[4-(trifluorométhoxy)benzyl]-3-(3,5-dichlorophényl)-7-nicotinoylamino-1,3-diazabicyclo[3.3.0]octane-2,4-dione ;
la (5R,7S)-5-[4-(trifluorométhoxy)benzyl]-3-(3,5-dichlorophényl)-7-(1-pyrrolidinylcarbonylamino)-1,3-diazabicyclo[3.3.0]octane-2,4-dione ;
la (5R,7R)-5-[4-(trifluorométhoxy)benzyl]-3-(3,5-dichlorophényl)-7-carbamoyl-1,3-diazabicyclo[3.3.0]octane-2,4-dione ;
la (5R,7R)-5-[4-(trifluorométhoxy)benzyl]-3-(3,5-dichlorophényl)-7-morpholinocarbonyl-1,3-diazabicyclo[3.3.0]octane-2,4-dione ;
la (5R,7R)-5-[4-(trifluorométhoxy)benzyl]-3-(3,5-dichlorophényl)-7-diméthylcarbamoyl-1,3-diazabicyclo[3.3.0]octane-2,4-dione ;
la (5R,7R)-5-[4-(trifluorométhoxy)benzyl]-3-(3,5-dichlorophényl)-7-méthylcarbamoyl-1,3-diazabicyclo[3.3.0]octane-2,4-dione ; et
la (5R,7R)-5-[4-(trifluorométhoxy)benzyl]-3-(3,5-dichlorophényl)-7-morpholinocarbamoyl-1,3-diazabicyclo[3.3.0]octane-2,4-dione.

12. Procédé de préparation d'un composé de formule (I) : 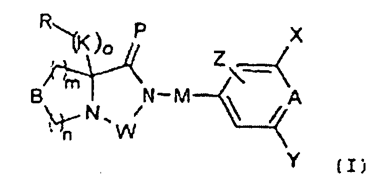 dans laquelle les symboles sont identiques à ceux définis dans la revendication 1, ou d'un sel pharmaceutiquement acceptable de celui-ci, qui comprend la cyclisation du composé de formule (II) : 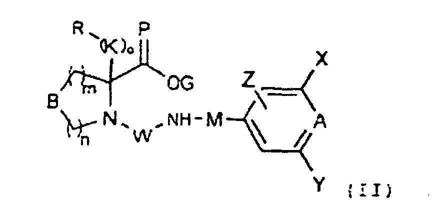 dans laquelle OG est un groupe hydroxyle, un groupe hydroxyle protégé ou un groupe hydroxyle lié à une résine, et les autres symboles sont identiques à ceux définis ci-dessus, et sa transformation en sel pharmaceutiquement acceptable, si nécessaire.

13. Procédé de préparation d'un composé de formule (I) : 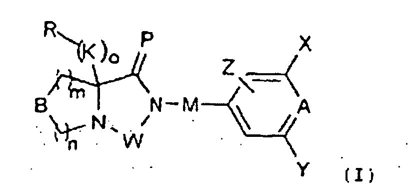 dans laquelle les symboles sont identiques à ceux définis dans la revendication 1, ou d'un sel pharmaceutiquement acceptable de celui-ci, qui comprend la réaction du composé de formule (III) : 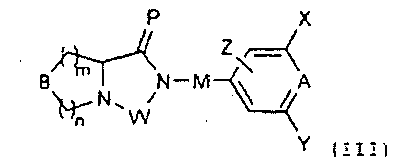 dans laquelle les symboles sont identiques à ceux définis ci-dessus, avec le composé de formule (IV) :
R-(K)ₒ-L (IV)
dans laquelle L est un groupe partant et les autres symboles sont identiques à ceux définis dans la revendication 1, et éventuellement sa transformation en sel pharmaceutiquement acceptable, si nécessaire.

14. Procédé de préparation d'un composé de formule (I) : 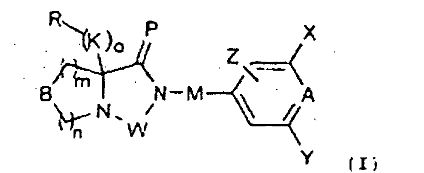 dans laquelle les symboles sont identiques à ceux définis dans la revendication 1, ou d'un sel pharmaceutiquement acceptable de celui-ci, qui comprend la réaction du composé de formule (V) : 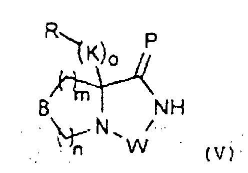 dans laquelle les symboles sont identiques à ceux définis dans la revendication 1, avec le composé de formule (VI) : 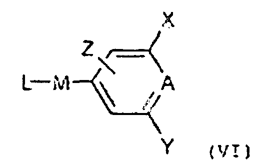 dans laquelle L est un groupe partant et les autres symboles sont identiques à ceux définis dans la revendication 1, et la transformation du composé résultant en sel pharmaceutiquement acceptable, si nécessaire.

15. Composition pharmaceutique qui comprend une quantité thérapeutiquement efficace du composé selon la revendication 1 en mélange avec un support ou diluant thérapeutiquement acceptable.

16. Utilisation du composé selon la revendication 1 pour le traitement ou la prévention d'un état médié par l'adhérence de α_{L}β₂ chez un mammifère.

17. Utilisation selon la revendication 16, ledit état étant choisi parmi la polyarthrite rhumatoïde, l'asthme, les états allergiques, le syndrome de détresse respiratoire de l'adulte, le SIDA, les maladies cardiovasculaires, la thrombose ou l'agrégation nocive des plaquettes, la réocclusion suivant une thrombolyse, le rejet d'allogreffe, les lésions de reperfusion, l'accident vasculaire cérébral, le psoriasis, l'eczéma, les maladies inflammatoires de la peau telles que la dermatite de contact et la dermatite atopique, l'ostéoporose, l'arthrose, l'athérosclérose (y compris l'artériosclérose du greffon après transplantation), les maladies néoplasiques, y compris la métastase d'une croissance néoplasique ou cancéreuse, l'amélioration de la cicatrisation, les maladies de l'oeil telles que le décollement de la rétine, le diabète de type I, la sclérose en plaques, le lupus érythémateux disséminé (LED), les états inflammatoires et immuno-inflammatoires, y compris les états inflammatoires ophtalmiques et les maladies inflammatoires de l'intestin (maladie de Crohn, colite ulcéreuse), l'entérite régionale, le syndrome de Sjogren, ainsi que les autres maladies auto-immunes, la pancréatite, le fonctionnement retardé du greffon, l'hyperplasie intimale, le réinfarctus du myocarde ou la resténose suivant une opération telle qu'une angioplastie coronaire transluminale percutanée (ACTP), le rejet après transplantation (rejet chronique et rejet aigu), les maladies de l'hôte contre le greffon ou du greffon contre l'hôte, et le cancer.

18. Utilisation selon la revendication 17, dans laquelle ladite maladie est choisie parmi le psoriasis, la polyarthrite rhumatoïde, les maladies inflammatoires de l'intestin (maladie de Crohn, colite ulcéreuse), le lupus érythémateux disséminé, la dermatite atopique, le syndrome de Sjogren, le rejet après transplantation (rejet chronique, rejet aigu), et la maladie du greffon contre l'hôte.
